# EUROPEAN PATENT SPECIFICATION

(11) **EP 3 765 458 B1**
(45) Date of publication and mention of the grant of the patent: **11.01.2023**
(21) Application number: 19713945.4
(22) Date of filing: 13.03.2019
(51) Int. Cl.: C07D 417/06, C07D 417/14, C07D 495/04, A61P 25/28, A61K 31/427, A61K 31/445

(54) **O-GLYCOPROTEIN-2-ACETAMIDO-2-DEOXY-3-D-GLYCOPYRANOSIDASE INHIBITORS**
O-GLYCOPROTEIN-2-ACETAMIDO-2-DEOXY-3-D-GLYCOPYRANOSIDASE-INHIBITOREN
INHIBITEURS D'O-GLYCOPROTÉINE-2-ACÉTAMIDO-2-DÉSOXY-3-D-GLYCOPYRANOSIDASE

(30) Priority: 14.03.2018 US 201862642932 P; 27.06.2018 US 201862690536 P; 17.07.2018 US 201862699443 P
(43) Date of publication of application: 20.01.2021
(73) Proprietor: Biogen MA Inc., Cambridge, MA 02142 (US)
(72) Inventor: GENUNG, Nathan, Cambridge, MA 02142 (US); GUCKIAN, Kevin M., Cambridge, MA 02142 (US); VESSELS, Jeffrey, Cambridge, MA 02142 (US); ZHANG, Lei, Cambridge, MA 02142 (US); GIANATASSIO, Ryan, Cambridge, MA 02142 (US); LIN, Edward Yin Shiang, Cambridge, MA 02142 (US); XIN, Zhili, Cambridge, MA 02142 (US)
(74) Representative: HGF
(86) International application number: PCT/US2019/021995
(87) International publication number: WO 2019/178191

(56) References cited:
- WO-A1-2014/159234
- WO-A1-2017/106254
- WO-A1-2018/109198
- WO-A1-2018/109202
- WO-A1-2018/154133

## Description

### CROSS REFERENCE TO RELATED APPLICATIONS

This application claims to U.S. Provisional Patent Application No. 62/699,443, filed July 17, 2018; U.S. Provisional Patent Application No. 62/690,536, filed June 27, 2018; and U.S. Provisional Patent Application No. 62/642,932, filed March 14, 2018.

### BACKGROUND

A wide range of cellular proteins, both nuclear and cytoplasmic, are post-translationally modified by the addition of the monosaccharide 2-acetamido-2-deoxy-β-D-glucopyranoside (β-N-acetyl glucosamine) which is attached via an O-glycosidic linkage. This monosaccharide is generally referred to as O-linked N-acetylglucosamine or O-GlcNAc. The enzyme responsible for post-translationally linking β-N-acetylglucosamine (GlcNAc) to specific serine and threonine residues of numerous nucleocytoplasmic proteins is O-GlcNAc transferase (OGTase). A second enzyme, known as O-glycoprotein-2-acetamido-2-deoxy-3-D-glucopyranosidase or O-GlcNAcase or OGA, removes this post-translational modification to liberate proteins, making the O-GlcNAc-modification a dynamic cycle occurring several times during the lifetime of a protein.

O-GlcNAc-modified proteins regulate a wide range of vital cellular functions including, e.g., transcription, proteasomal degradation and cellular signaling. O-GlcNAc is also found on many structural proteins, including the cytoskeletal protein "tau" which is responsible for stabilizing a key cellular network of microtubules that is essential for distributing proteins and nutrients within neurons. Importantly, tau has been clearly implicated in the etiology of several diseases including tauopathies, Alzheimer's disease, Parkinson's disease, dementia and cancer.

It is well established that Alzheimer's disease and a number of related tauopathies including Progressive Supranuclear Palsy (PSP) and amyotrophic lateral sclerosis (ALS) are characterized, in part, by the development of neurofibrillary tangles (NFTs). These NFTs are aggregates of paired helical filaments (PHFs) and are composed of an abnormal form of tau. In AD patients, tau becomes hyperphosphorylated, thereby disrupting its normal function, forming PHFs and ultimately aggregating to form NFTs.

Six isoforms of tau are found in the human brain. In AD patients, all six isoforms of tau are found in NFTs, and all are markedly hyperphosphorylated. Tau in healthy brain tissue bears only 2 or 3 phosphate groups, whereas those found in the brains of AD patients bear, on average, 8 phosphate groups.

It has recently emerged that increases in phosphorylation levels result in decreased O-GlcNAc levels and conversely, increased O-GlcNAc levels correlate with decreased phosphorylation levels. It has been shown that decreased glucose availability in brain leads to tau hyperphosphorylation. The gradual impairment of glucose transport and metabolism leads to decreased O-GlcNAc and hyperphosphorylation of tau (and other proteins). Accordingly, the inhibition of O-GlcNAcase, which prevents hyperphosphorylation of tau by preventing removal of O-GlcNac from tau, should compensate for the age-related impairment of glucose metabolism within the brains of health individuals as well as patients suffering from Alzheimer's disease or related neurodegenerative diseases.

However, a major challenge in developing inhibitors for blocking the function of mammalian glycosidases, including O-GlcNAcase, is the large number of functionally related enzymes present in tissues of higher eukaryotes. Accordingly, the use of non-selective inhibitors in studying the cellular and organismal physiological role of one particular enzyme is complicated because complex phenotypes arise from the concomitant inhibition of such functionally related enzymes. In the case of β-N-acetylglucosaminidases, existing compounds that act to block O-GlcNAcase function are non-specific and act potently to inhibit the lysosomal β-hexosaminidases.

In view of foregoing technical challenge, and given the potential for regulation of O-GlcNAcase for treatment of AD, tauopathies and other neurological diseases, there remains a need for development of potent and selective O-GlcNAcase inhibitors.

WO2014/159234, WO2017/106254, WO2018/154133, WO2018/109202 and WO2018/109198 describe compounds that inhibit GlcNAcase.

### SUMMARY

Described herein are compounds that are useful treating various diseases, disorders and medical conditions, including but not limited to those associated with proteins that are modified by O-GlcNAcase.

A first aspect of a compound of the present invention is represented by the following structural formulae:
(i) 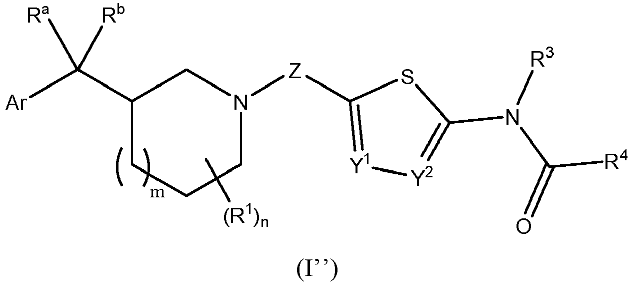 or a pharmaceutically acceptable salt thereof, wherein:
   Ar is an optionally substituted 5- to 10-membered heteroaryl, an optionally substituted phenyl or an optionally substituted phenyl fused to an optionally substituted non-aromatic 5-to 6-membered heterocycle;
   Y¹ and Y² are each CR^{c} or N, wherein at least one of Y¹ or Y² is N;
   Z is CR²R², C(=O), (CR²R²)₂, CH₂C(=O), or C(=O)CH₂;
   R^{a} and R^{b} are each independently -H, halo, C₁-C₄ alkyl, C₁-C₄ haloalkyl, or C₁-C₄ alkoxy, or R^{a} and R^{b} taken together with their intervening carbon atom form a C₃-C₆ cycloalkyl;
   R^{c} is halo, C₁-C₄ alkyl, C₁-C₄ haloalkyl, or C₁-C₄ alkoxy;
   m is 0 or 1;
   n is 0 or an integer from 1 to 7;
   when n is other than 0, R¹, for each occurrence, is independently halo, C₁-C₄ alkyl, C₁-C₄ haloalkyl, C₁-C₄ alkoxy or C₁-C₄ haloalkoxy;
   R², for each occurrence, is independently -H, halo, C₁-C₄ alkyl, C₁-C₄ haloalkyl, C₃-C₁₀ cycloalkyl, or C₃-C₁₀ halocycloalkyl;
   or alternatively two R² together with the carbon atom to which they are attached form a C₃-C₁₀ cycloalkyl;
   R³ is -H or C₁-C₄ alkyl; and
   R⁴ is -H, C₁-C₄ alkyl, C₁-C₄ haloalkyl, or C₃-C₆ cycloalkyl;
   or alternatively R³ and R⁴ taken together with their intervening atoms form an optionally substituted 5- to 7-membered heterocyclyl; or
(ii) 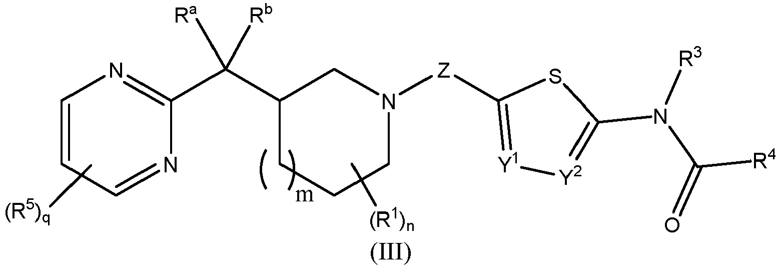 or a pharmaceutically acceptable salt thereof; wherein:
   Y¹ and Y² are each CR^{c} or N, wherein at least one of Y¹ or Y² is N;
   Z is CR²R², C(=O), (CR²R²)₂, CH₂C(=O), or C(=O)CH₂;
   R^{a}, R^{b} and R^{c} are each independently -H, halo, C₁-C₄ alkyl, C₁-C₄ haloalkyl, or C₁-C₄ alkoxy, or R^{a} and R^{b} taken together with their intervening carbon atom form a C₃-C₆ cycloalkyl;
   m is 0 or 1;
   n is 0 or an integer from 1 to 7;
   when n is other than 0, R¹, for each occurrence, is independently halo, C₁-C₄ alkyl, C₁-C₄ haloalkyl, C₁-C₄ alkoxy or C₁-C₄ haloalkoxy;
   R², for each occurrence, is independently -H, halo, C₁-C₄ alkyl, C₁-C₄ haloalkyl, C₃-C₁₀ cycloalkyl, or C₃-C₁₀ halocycloalkyl;
   or alternatively two R² together with the carbon atom to which they are attached form a C₃-C₁₀ cycloalkyl;
   R³ is -H or C₁-C₄ alkyl; and
   R⁴ is -H, C₁-C₄ alkyl, C₁-C₄ haloalkyl, or C₃-C₆ cycloalkyl;
   or alternatively R³ and R⁴ taken together with their intervening atoms form an optionally substituted 5- to 7-membered heterocyclyl;
   R⁵, for each occurrence, is selected from C₁-C₄ alkyl, C₁-C₄ haloalkyl, C₃-C₆ cycloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, -C(=O)R^{x}, phenyl and monocyclic heteroaryl; wherein
      when R⁵ is a C₁-C₄ alkyl group, the C₁-C₄ alkyl group is optionally and independently substituted with -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{x}, -C(=O)NR^{x}R^{y}, -NR^{x}C(=O)R^{y}, -C(=S)NR^{x}R^{y} -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{y}, C₃-C₆ cycloalkyl (optionally substituted with one or more groups selected from -CH₃, halomethyl, halo, methoxy and halomethoxy), monocyclic heteroaryl (optionally substituted with one or more groups selected from -CH₃, halomethyl, halo, methoxy or halomethoxy) and phenyl (optionally substituted with one or more groups selected from - CH₃, halomethyl, halo, methoxy and halomethoxy);
      when R⁵ is a C₃-C₆ cycloalkyl, phenyl or a monocyclic heteroaryl, the cycloalkyl, phenyl or a monocyclic heteroaryl is optionally and independently substituted with C₁-C₄ alkyl, C₁-C₄ haloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{y}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{x};
      each R^{x} and each R^{y} is independently -H, C₁-C₄ alkyl, or C₃-C₈ cycloalkyl; wherein the C₁-C₄ alkyl or C₃-C₈ cycloalkyl represented by R^{x} or R^{y} is optionally substituted with one or more substituents selected from halo, hydroxyl, C₃-C₆ cycloalkyl and phenyl (optionally substituted with one or more groups selected from -CH₃, halomethyl, halo, methoxy or halomethoxy);
      R^{z} is -H, C₁-C₄ alkyl, or C₃-C₈ cycloalkyl; wherein the C₁-C₄ alkyl or C₃-C₈ cycloalkyl group represented by R^{z} is optionally substituted with one or more substituents selected from halo, hydroxyl, C₃-C₆ cycloalkyl and phenyl (optionally substituted with one or more groups selected from -CH₃, halomethyl, halo, methoxy and halomethoxy); and
      i is 0, 1, or 2; and
      q 0, 1, 2, or 3.

Provided is a pharmaceutical composition comprising at least one compound described herein, or a pharmaceutically acceptable salt thereof, and at least one pharmaceutically acceptable excipient.

Also provided is a compound described herein, or a pharmaceutically acceptable salt thereof, or a pharmaceutical composition comprising at least one compound described herein, or a pharmaceutically acceptable salt thereof, and at least one pharmaceutically acceptable excipient for use in the treatment of a disease or condition selected from a neurodegenerative disease, a tauopathy, diabetes, cancer and stress.

Also provided is a compound described herein, or a pharmaceutically acceptable salt thereof, or an effective amount of a pharmaceutical composition comprising at least one compound described herein, or a pharmaceutically acceptable salt thereof, and at least one pharmaceutically acceptable excipient for use in the inhibition of O-GlcNAcase.

Also provided is a compound described herein, or a pharmaceutically acceptable salt thereof, or an effective amount of a pharmaceutical composition comprising at least one compound described herein, or a pharmaceutically acceptable salt thereof, and at least one pharmaceutically acceptable excipient for use in the treatment of a disease or condition characterized by hyperphosphorylation of tau in the brain.

### DETAILED DESCRIPTION

Described herein are compounds that are useful treating various diseases, disorders and medical conditions, including but not limited to those associated with proteins that are modified by O-GlcNAcase.

In a first embodiment, a compound of the present invention is represented by the following structural formula: 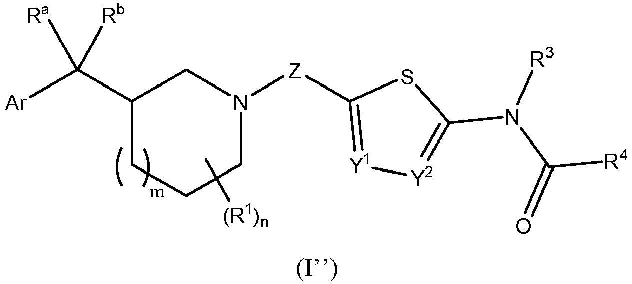 or a pharmaceutically acceptable salt thereof, wherein the variables are as defined in the summary above.

In a second embodiment, a compound of the present invention is represented by the following structural formula: 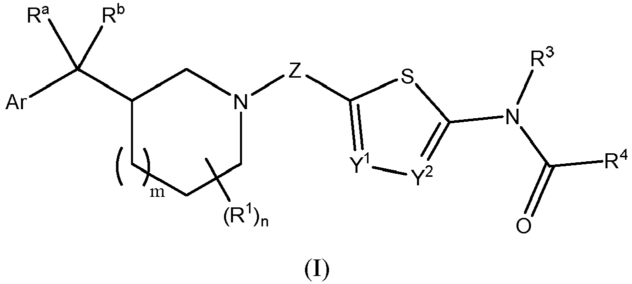 or a pharmaceutically acceptable salt thereof, wherein R^{a}, R^{b} and R^{c} are each independently - H, halo, C₁-C₄ alkyl, or C₁-C₄ haloalkyl, or R^{a} and R^{b} taken together with their intervening carbon atom form a C₃-C₆ cycloalkyl; wherein the remaining variables are as defined in the first embodiment.

In a third embodiment, a compound of the invention is represented by the following structural formulas: 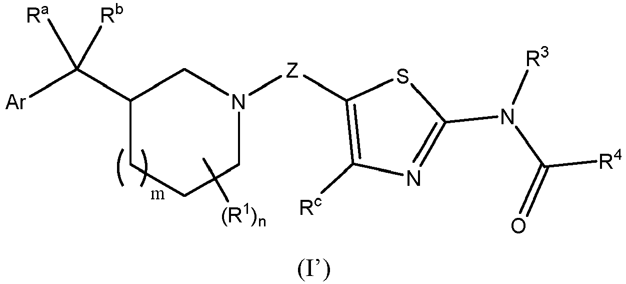 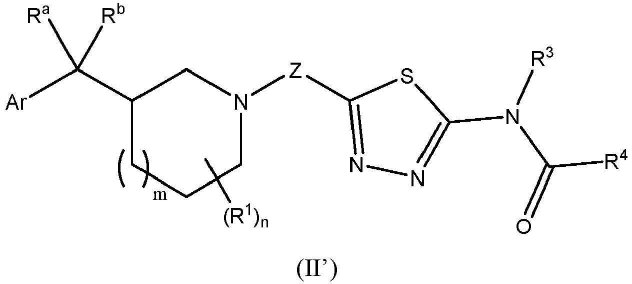 or a pharmaceutically acceptable salt thereof; wherein the variables are as defined in the first or second embodiments.

In a fourth embodiment, a compound of the invention is represented by the following structural formulas: 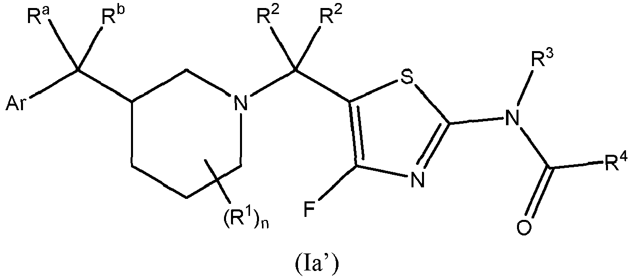 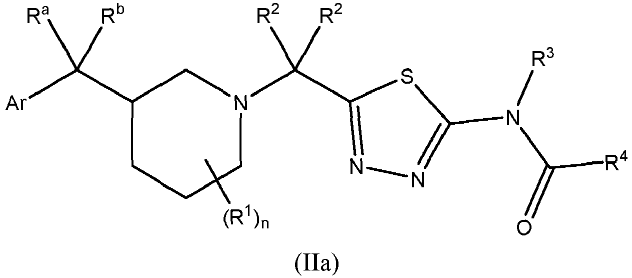 or a pharmaceutically acceptable salt thereof; wherein R^{a} and R^{b} are each independently -H or C₁-C₄ alkyl; R², for each occurrence, is independently -H, halo, C₁-C₄ alkyl; and the remaining variables are as defined in the first, second, or third embodiments.

In a fifth embodiment, a compound of the invention is represented by the following structural formulas: 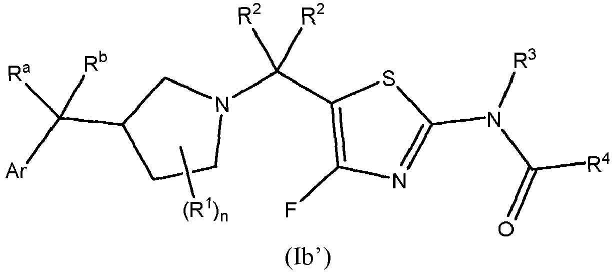 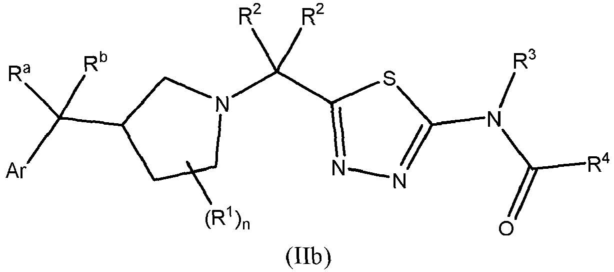 or a pharmaceutically acceptable salt thereo; wherein R², for each occurrence, is independently -H, halo, C₁-C₄ alkyl; and the remaining variables are as defined in the first, second, or third embodiments.

In a sixth embodiment, a compound of the invention is represented by the following structural formulas: 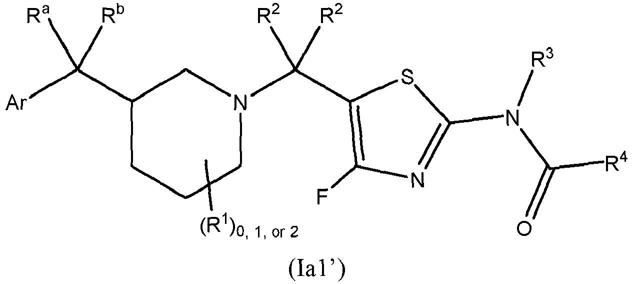 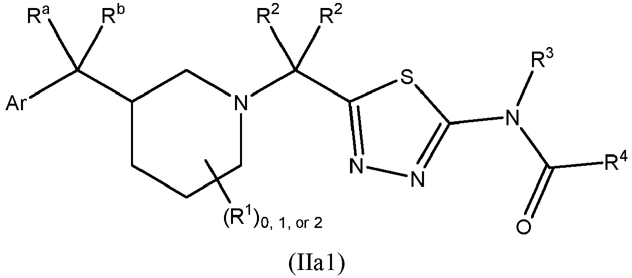 or a pharmaceutically acceptable salt thereof; wherein R^{a} and R^{b} are each independently -H or methyl; R¹ is halo, C₁-C₄ alkyl or C₁-C₄ haloalkyl; R², for each occurrence, is independently -H or C₁-C₄ alkyl; and the remaining variables are as defined in the first, second, third, or fourth embodiments.

In a seventh embodiment, a compound of the invention is represented by the following structural formulas: 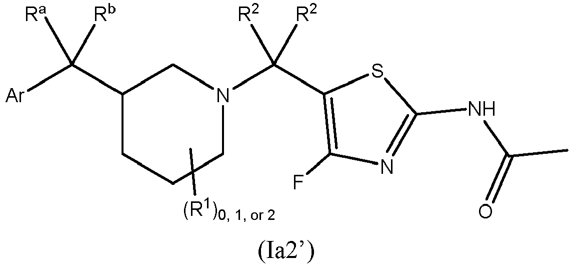 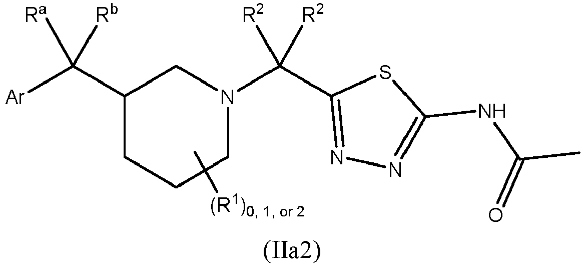 or a pharmaceutically acceptable salt thereof; wherein R^{a} and R^{b} are each independently -H or methyl; R², for each occurrence, is independently -H or methyl; R¹ is -F or methyl; and the remaining variables are as defined in the first, second, third, fourth or sixth embodiments.

In an eighth embodiment, a compound of the invention is represented by the following structural formulas: 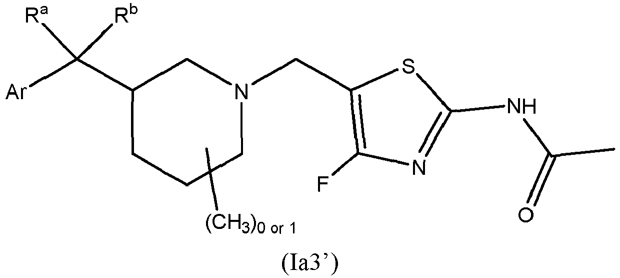 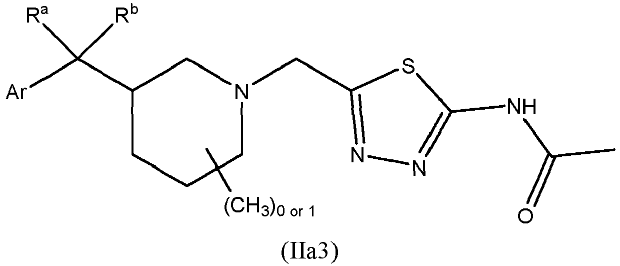 or a pharmaceutically acceptable salt thereof; wherein Ar is as defined in the first, second, third, fourth, sixth, or seventh embodiments.

In a ninth embodiment, a compound of the invention is represented by the following structural formulas: 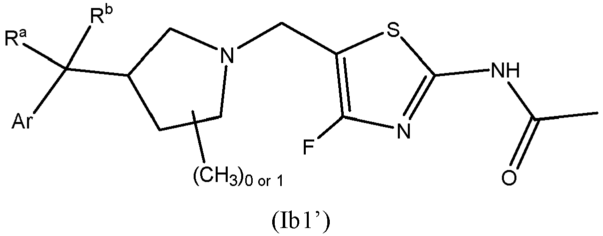 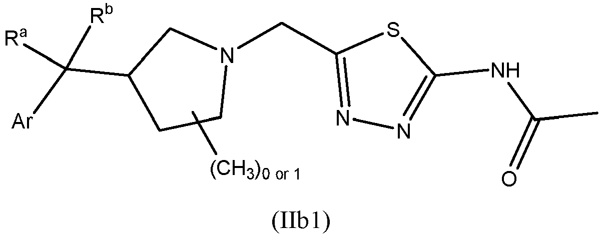 or a pharmaceutically acceptable salt thereof; wherein Ar is as defined in the first, second, third, or fifth embodiments.

In a tenth embodiment, in a compound of the invention in accordance to the first, second, third, fourth, fifth, sixth, seventh, eighth, or ninth embodiments, or a pharmaceutically acceptable salt thereof, Ar is optionally substituted pyrazolyl, optionally substituted imidazolyl, optionally substituted thiazolyl, optionally substituted phenyl, optionally substituted pyridinyl, optionally substituted pyrimidinyl, optionally substituted pyrazinyl, optionally substituted imidazo[1,2-a]pyridinyl, optionally substituted thieno[2,3-d]pyrimidinyl, or optionally substituted thieno[3,2-d]pyrimidinyl. In a compound of the invention in accordance to the first, second, third, fourth, fifth, sixth, seventh, eighth, or ninth embodiments, or a pharmaceutically acceptable salt thereof, Ar is optionally substituted oxadiazolyl, optionally substituted 1,2,3-triazol-1-yl, optionally substituted triazolo[4,3-a]pyridin-3-yl, or optionally substituted 1H-benzo[d]imidazol-1-yl.

In an eleventh embodiment, in a compound of the invention in accordance to the first, second, third, fourth, fifth, sixth, seventh, eighth, ninth, or tenth embodiments, or a pharmaceutically acceptable salt thereof, Ar is optionally substituted 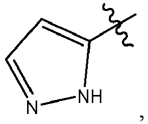 optionally substituted 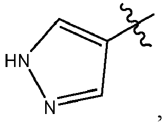 optionally substituted 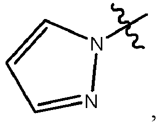 optionally substituted 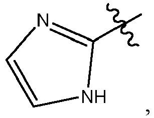 optionally substituted 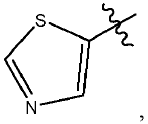 optionally substituted 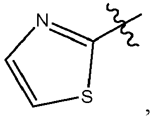 optionally substituted 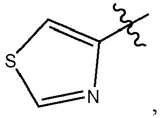 optionally substituted 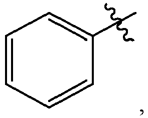 optionally substituted 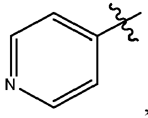 , optionally substituted 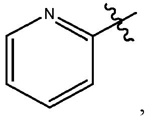 optionally substituted 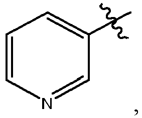 optionally substituted 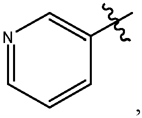 optionally substituted 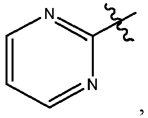 optionally substituted 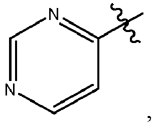 optionally substituted 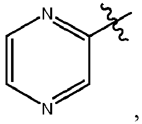 optionally substituted 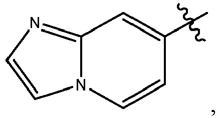 or optionally substituted 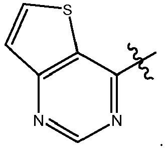 In a compound of the invention in accordance to the first, second, third, fourth, fifth, sixth, seventh, eighth, ninth, or tenth embodiments, or a pharmaceutically acceptable salt thereof, Ar is optionally substituted 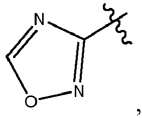 optionally substituted 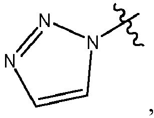 optionally substituted 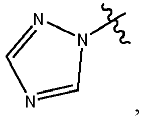 optionally substituted 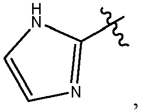 optionally substituted 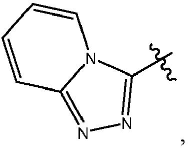 or optionally substituted

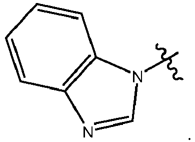

In a twelfth embodiment, in a compound of the invention in accordance to the first, second, third, fourth, fifth, sixth, seventh, eighth, ninth, tenth, or eleventh embodiments, or a pharmaceutically acceptable salt thereof, Ar is optionally substituted 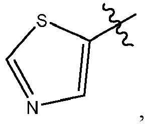 optionally substituted 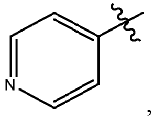 optionally substituted 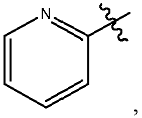 optionally substituted 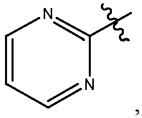 optionally substituted 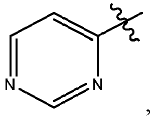 optionally substituted 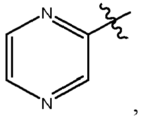 optionally substituted 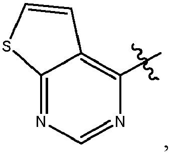 or optionally substituted 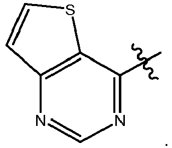 In a compound of the invention in accordance to the first, second, third, fourth, fifth, sixth, seventh, eighth, ninth, tenth, or eleventh embodiments, or a pharmaceutically acceptable salt thereof, Ar is optionally substituted

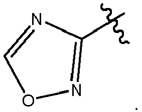

In a thirteenth embodiment, in a compound of the invention in accordance to the first, second, third, fourth, fifth, sixth, seventh, eighth, ninth, tenth, eleventh, or twelfth embodiments, or a pharmaceutically acceptable salt thereof, Ar is optionally substituted with one or more groups selected from C₁-C₄ alkyl, C₁-C₄ haloalkyl, C₃-C₆ cycloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, -C(=O)R^{x}, phenyl and monocyclic heteroaryl; wherein
the C₁-C₄ alkyl group substituent on Ar is optionally substituted with -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{x}, -C(=O)NR^{x}R^{y}, -NR^{x}C(=O)R^{y}, -C(=S)NR^{x}R^{y} -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{y}, C₃-C₆ cycloalkyl (optionally substituted with one or more groups selected from -CH₃, halomethyl, halo, methoxy and halomethoxy), monocyclic heteroaryl (optionally substituted with one or more groups selected from -CH₃, halomethyl, halo, methoxy or halomethoxy) and phenyl (optionally substituted with one or more groups selected from - CH₃, halomethyl, halo, methoxy and halomethoxy);
the C₃-C₆ cycloalkyl, phenyl and monocyclic heteroaryl group substituent on Ar are optionally and independently substituted with C₁-C₄ alkyl, C₁-C₄ haloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{y}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{x};
each R^{x} and each R^{y} is independently -H, C₁-C₄ alkyl, or C₃-C₈ cycloalkyl; wherein the C₁-C₄ alkyl or C₃-C₈ cycloalkyl represented by R^{x} or R^{y} is optionally substituted with one or more substituents selected from halo, hydroxyl, C₃-C₆ cycloalkyl and phenyl (optionally substituted with one or more groups selected from -CH₃, halomethyl, halo, methoxy or halomethoxy);
R^{z} is -H, C₁-C₄ alkyl, or C₃-C₈ cycloalkyl; wherein the C₁-C₄ alkyl or C₃-C₈ cycloalkyl group represented by R^{z} is optionally substituted with one or more substituents selected from halo, hydroxyl, C₃-C₆ cycloalkyl and phenyl (optionally substituted with one or more groups selected from -CH₃, halomethyl, halo, methoxy and halomethoxy); and
i is 0, 1, or 2.

In a fourteenth embodiment, in a compound of the invention in accordance to the first, second, third, fourth, fifth, sixth, seventh, eighth, ninth, tenth, eleventh, twelfth, or thirteenth embodiments, or a pharmaceutically acceptable salt thereof, Ar is optionally substituted with one with one or more groups selected from C₁-C₄ alkyl, C₁-C₄ haloalkyl, C₃-C₆cycloalkyl, halo, -CN, -OR^{z}, -NR^{x}R^{y}, -C(=O)NR^{x}R^{y}, -C(=S)NR^{x}R^{y}, -O(C=O)NR^{x}R^{y}, -O(C=S)NR^{x}R^{y}, -C(=O)OR^{x}, -NR^{x}C(=O)R^{y} phenyl, -C(=O)R^{x}, and optionally substituted monocyclic heteroaryl.

In a fifteenth embodiment, in a compound of the invention in accordance to the first, second, third, fourth, fifth, sixth, seventh, eighth, ninth, tenth, eleventh, twelfth, thirteenth, or fourteenth embodiments, or a pharmaceutically acceptable salt thereof, Ar is optionally substituted with one with one or more groups selected from C₁-C₄ alkyl, C₁-C₄ haloalkyl, C₃-C₆ cycloalkyl, halo, -OR^{z}, -C(=O)R^{x}, and monocyclic heteroaryl optionally substituted with C₁-C₄ alkyl, C₁-C₄ haloalkyl, halo.

In a sixteenth embodiment, in a compound of the invention in accordance to the first, second, third, fourth, fifth, sixth, seventh, eighth, ninth, tenth, eleventh, twelfth, thirteenth, fourteenth, or fifteenth embodiments, or a pharmaceutically acceptable salt thereof, Ar is optionally substituted with one with one or more groups selected from -CH₃, - CH₂CH₃, halomethyl, cyclopentyl, cyclobutyl, halo, -OR^{z}, -C(=O)R^{x}, and a 5- or 6-membered monocyclic heteroaryl containing one or two heteroatoms selected from S and N and optionally substituted with C₁-C₄ alkyl; wherein R^{x} is -H or C₁-C₄ alkyl; and wherein R^{z} is optionally substituted C₁-C₄ alkyl.

In a seventeenth embodiment, in a compound of the invention in accordance to the first, second, third, fourth, fifth, sixth, seventh, eighth, ninth, tenth, eleventh, twelfth, thirteenth, fourteenth, fifteenth, or sixteenth embodiment, or a pharmaceutically acceptable salt thereof, Ar is optionally substituted with one with one or more groups selected from-CH₃, -CH₂CH₃, -CHF₂, -CF₃, cyclopentyl, cyclobutyl, -F, -Cl, -Br, -OCH₃, -C(=O)CH₃, and a thiazolyl. In another embodiment, n a compound of the invention in accordance to the first, second, third, fourth, fifth, sixth, seventh, eighth, ninth, tenth, eleventh, twelfth, thirteenth, fourteenth, fifteenth, or sixteenth embodiment, or a pharmaceutically acceptable salt thereof, Ar is optionally substituted with one with one or more groups selected from-CH₃, -CH₂CH₃, -CHF₂, -CF₃, cyclopentyl, cyclobutyl, -F, -Br, -OCH₃, -C(=O)CH₃, and a thiazolyl.

In an eighteenth embodiment, a compound of the invention is represented by the following structural formula: 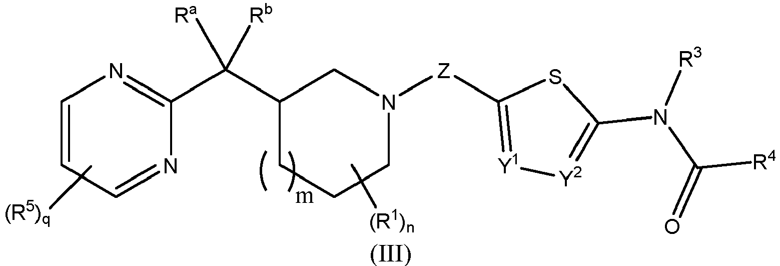 or a pharmaceutically acceptable salt thereof, wherein R⁵, for each occurrence, is selected from C₁-C₄ alkyl, C₁-C₄ haloalkyl, C₃-C₆ cycloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, -C(=O)R^{x}, phenyl and monocyclic heteroaryl;
wherein
when R⁵ is a C₁-C₄ alkyl group, the C₁-C₄ alkyl group is optionally and independently substituted with -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{x}, -C(=O)NR^{x}R^{y}, -NR^{x}C(=O)R^{y}, -C(=S)NR^{x}R^{y} -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{y}, C₃-C₆ cycloalkyl (optionally substituted with one or more groups selected from -CH₃, halomethyl, halo, methoxy and halomethoxy), monocyclic heteroaryl (optionally substituted with one or more groups selected from -CH₃, halomethyl, halo, methoxy or halomethoxy) and phenyl (optionally substituted with one or more groups selected from - CH₃, halomethyl, halo, methoxy and halomethoxy);
when R⁵ is a C₃-C₆ cycloalkyl, phenyl or a monocyclic heteroaryl, the cycloalkyl, phenyl or a monocyclic heteroaryl is optionally and independently substituted with C₁-C₄ alkyl, C₁-C₄ haloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{y}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{x};
each R^{x} and each R^{y} is independently -H, C₁-C₄ alkyl, or C₃-C₈ cycloalkyl; wherein the C₁-C₄ alkyl or C₃-C₈ cycloalkyl represented by R^{x} or R^{y} is optionally substituted with one or more substituents selected from halo, hydroxyl, C₃-C₆ cycloalkyl and phenyl (optionally substituted with one or more groups selected from -CH₃, halomethyl, halo, methoxy or halomethoxy);
R^{z} is -H, C₁-C₄ alkyl, or C₃-C₈ cycloalkyl; wherein the C₁-C₄ alkyl or C₃-C₈ cycloalkyl group represented by R^{z} is optionally substituted with one or more substituents selected from halo, hydroxyl, C₃-C₆ cycloalkyl and phenyl (optionally substituted with one or more groups selected from -CH₃, halomethyl, halo, methoxy and halomethoxy); and
i is 0, 1, or 2; and
q 0, 1, 2, or 3;
wherein the remaining variables are as defined in the first embodiment.

In a nineteenth embodiment, a compound of the invention is represented by the one of the following structural formulas: 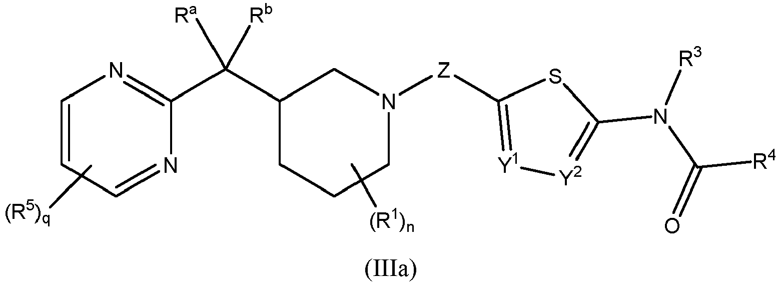 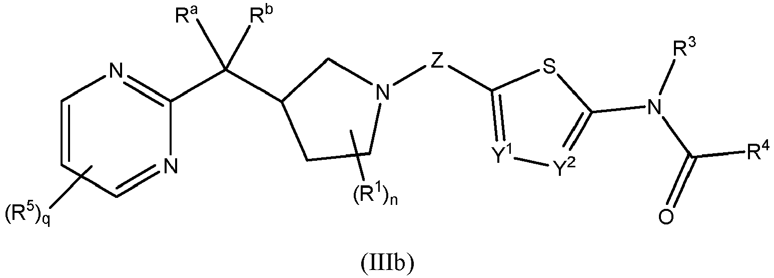 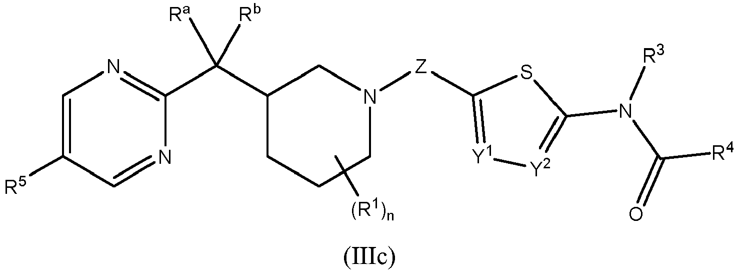 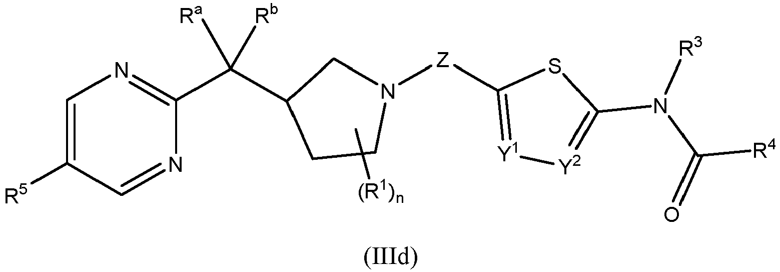 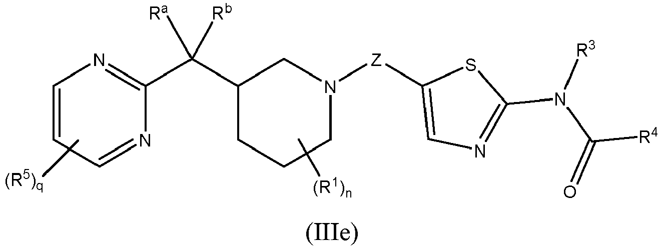 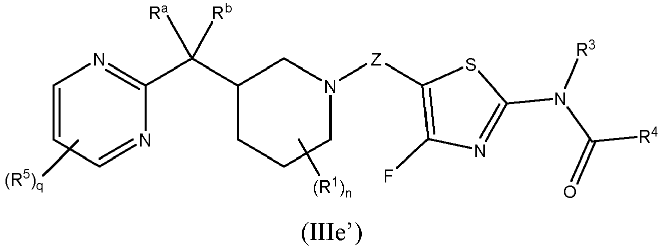 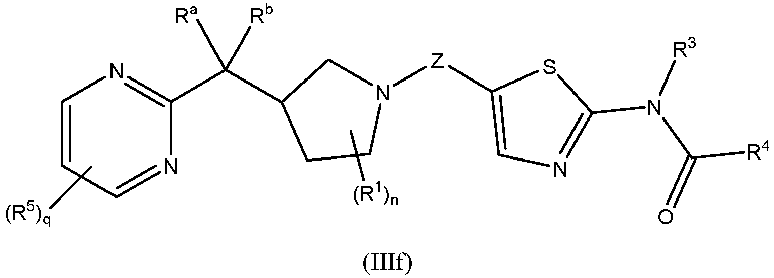 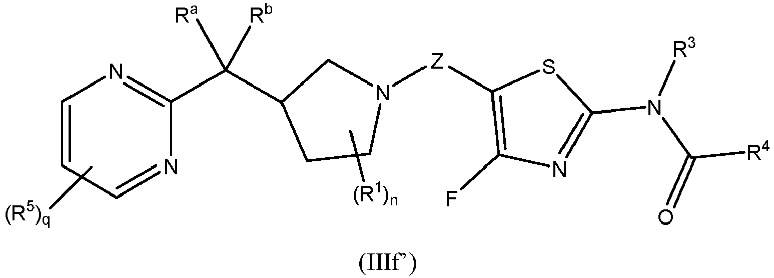 or a pharmaceutically acceptable salt thereof, wherein q is 0, 1, 2, or 3; and wherein the remaining variables are as defined in the eighteenth embodiment.

In a twentieth embodiment, a compound of the invention is represented by the one of the following structural formulas: 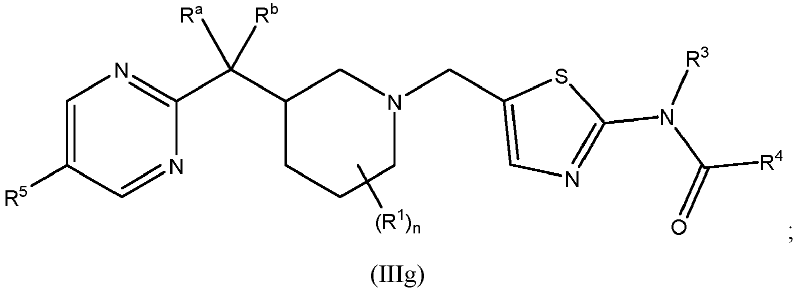 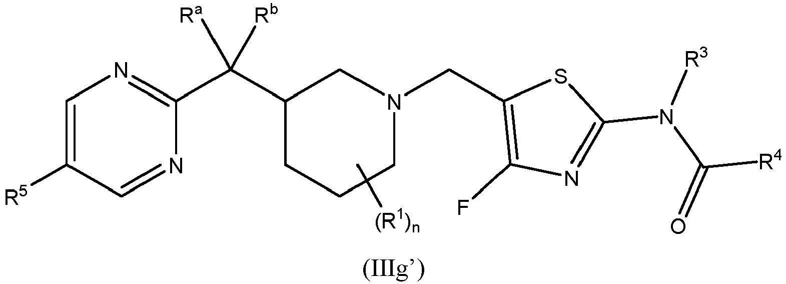 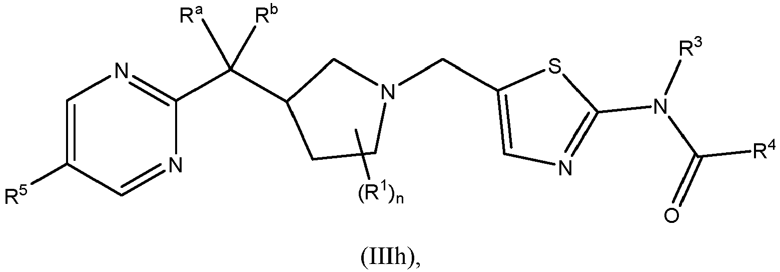 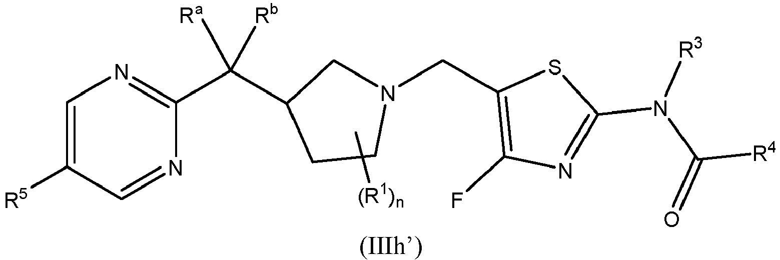 or a pharmaceutically acceptable salt thereof, wherein the remaining variables are as defined in the eighteenth embodiment.

In a twenty-first embodiment, a compound of the invention is represented by the following structural formula: 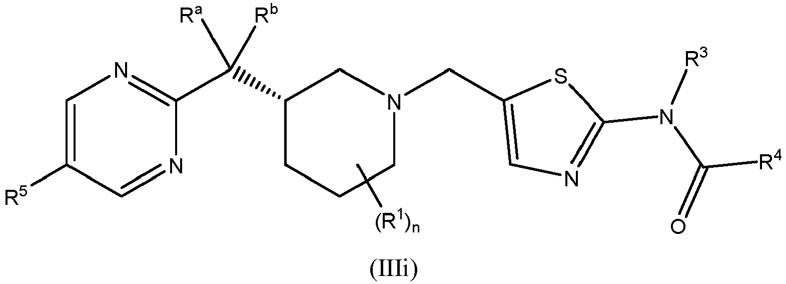 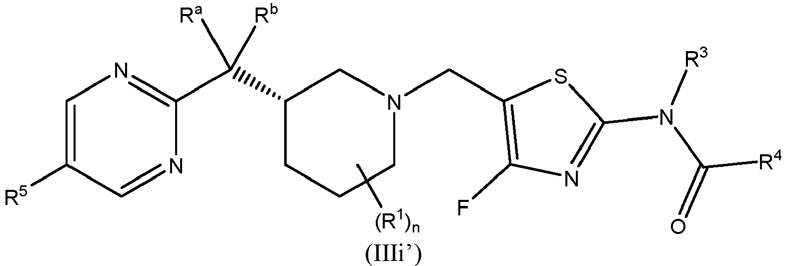 or a pharmaceutically acceptable salt thereof, wherein the remaining variables are as defined in the eighteenth embodiment.

In a twenty-second embodiment, a compound of the invention is represented by the following structural formula: 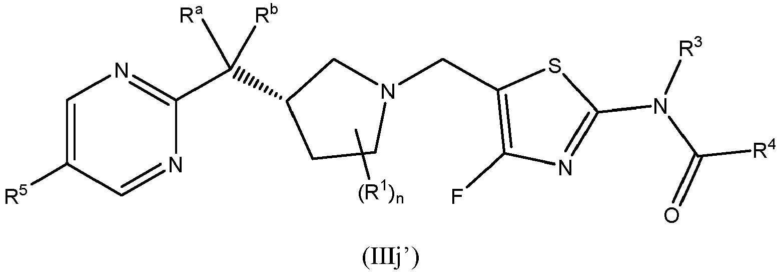 or a pharmaceutically acceptable salt thereof, wherein the remaining variables are as defined in the eighteenth embodiment.

In a twenty-third embodiment, a compound of the invention is represented by the following structural formula: 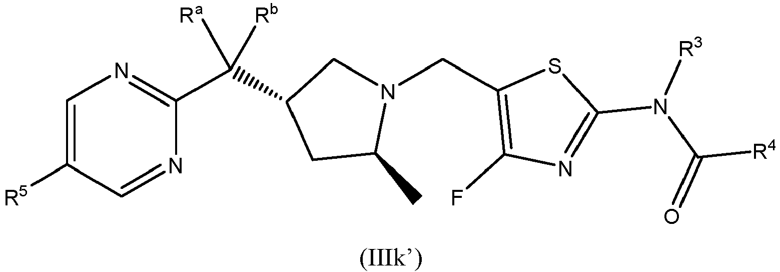 or a pharmaceutically acceptable salt thereof, wherein the remaining variables are as defined in the eighteenth embodiment.

In a twenty-fourth embodiment, in a compound of the invention in accordance to the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, twenty-first, twenty-second, or twenty-third embodiment, or a pharmaceutically acceptable salt thereof, R³ is -H,

In a twenty-fifth embodiment, in a compound of the invention in accordance to the irst, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, twenty-first, twenty-second, twenty-third, or twenty-fourth embodiment, or a pharmaceutically acceptable salt thereof, R⁴ is -CH₃,

In a twenty-fourth embodiment, in a compound of the invention in accordance to the irst, second, third, fourth, fifth, sixth, seventh, eighth, ninth, tenth, eleventh, twelfth, thirteenth, fourteenth, fifteenth, sixteenth, seventeenth, eighteenth nineteenth, twentieth, twenty-first, twenty-second, or twenty-third embodiments, or a pharmaceutically acceptable salt thereof, R⁵ is selected from C₁-C₄ alkyl, C₁-C₄ haloalkyl, C₃-C₆ cycloalkyl, halo, -CN, -OR^{z}, -NR^{x}R^{y}, -C(=O)NR^{x}R^{y}, -C(=S)NR^{x}R^{y}, -O(C=O)NR^{x}R^{y}, -O(C=S)NR^{x}R^{y}, -C(=O)OR^{x}, -NR^{x}C(=O)R^{y} phenyl, -C(=O)R^{x}, and optionally substituted monocyclic heteroaryl.

In a twenty-fifth embodiment, in a compound of the invention in accordance to the irst, second, third, fourth, fifth, sixth, seventh, eighth, ninth, tenth, eleventh, twelfth, thirteenth, fourteenth, fifteenth, sixteenth, seventeenth, eighteenth nineteenth, twentieth, twenty-first, twenty-second, twenty-third, or twenty-fourth embodiments, or a pharmaceutically acceptable salt thereof, R⁵ is selected from C₁-C₄ alkyl, C₁-C₄ haloalkyl, C₃-C₆ cycloalkyl, halo, -OR^{z}, -C(=O)R^{x}, and monocyclic heteroaryl optionally substituted with C₁-C₄ alkyl, C₁-C₄ haloalkyl, halo.

In a twenty-sixth embodiment, in a compound of the invention in accordance to the irst, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, twenty-first, twenty-second, twenty-third, twenty-fourth, or twenty-fifth embodiments, or a pharmaceutically acceptable salt thereof, R⁵ is selected from -CH₃, -CH₂CH₃, halomethyl, cyclopentyl, cyclobutyl, halo, -OR^{z}, -C(=O)R^{x}, and a 5- or 6-membered monocyclic heteroaryl containing one or two heteroatoms selected from S and N and optionally substituted with C₁-C₄ alkyl; wherein R^{x} is -H or C₁-C₄ alkyl; and wherein R^{z} is optionally substituted C₁-C₄ alkyl.

In a twenty-seventh embodiment, in a compound of the invention in accordance to the irst, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, twenty-first, twenty-second, twenty-third, twenty-fourth, twenty-fifth, twenty-sixth embodiment, or a pharmaceutically acceptable salt thereof, R⁵ is selected from -CH₃, - CH₂CH₃, -CHF₂, -CF₃, cyclopentyl, cyclobutyl, -F, -Br, -Cl, -OCH₃, -C(=O)CH₃, and a thiazolyl.

In a twenty-eighth embodiment, in a compound of the invention in accordance to the irst, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, twenty-first, twenty-second, twenty-third, twenty-fourth, twenty-fifth, twenty-sixth, or twenty-seventh embodiment, or a pharmaceutically acceptable salt thereof, R⁵ is selected from -F, -Br, and Cl.

In a twenty-ninth embodiment, in a compound of the invention in accordance to the irst, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, twenty-first, twenty-second, twenty-third, twenty-fourth, twenty-fifth, twenty-sixth, twenty-seventh, or twenty-eighth embodiment, or a pharmaceutically acceptable salt thereof, one of R^{a} and R^{b} is -H and the other is selected from -CH₃, -CF₃, and -OCH₃.

In a thirtieth embodiment, in a compound of the invention in accordance to the irst, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, twenty-first, twenty-second, twenty-third, twenty-fourth, twenty-fifth, twenty-sixth, twenty-seventh, twenty-eighth, or twenty-ninth embodiment, or a pharmaceutically acceptable salt thereof, n is 0 or 1.

In one embodiment, a compound of the invention may be any one of the compounds described herein in the working examples, including salts and neutral forms thereof.

As used herein, the term "alkyl" refers to a fully saturated branched or straightchained hydrocarbon moiety. Unless otherwise specified, the alkyl comprises 1 to 12 carbon atoms, preferably 1 to 8 carbon atoms, more preferably 1 to 6 carbon atoms or most preferably 1 to 4 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl and n-hexyl.

As used herein, the term "alkoxy" refers to the group -OR, in which R is an alkyl or a cycloalkyl, as that term is defined above. Non-limiting examples of alkoxy groups include: -OCH₃, -OCH₂CH₃, -OCH₂CH₂CH₃, -OCH(CH₃)₂, -OCH(CH₂)₂, -O-cyclopropyl, - O-cyclobutyl, -O-cyclopentyl and -O-cyclohexyl.

The number of carbon atoms in a group is specified herein by the prefix "Cₓ₋ₓₓ", wherein x and xx are integers. For example, "C₁₋₄ alkyl" is an alkyl group which has from 1 to 4 carbon atoms.

As used herein, the term "halogen" or "halo" may be fluoro, chloro, bromo or iodo.

As used herein, the term "haloalkyl" refers to an alkyl, as defined herein, that is substituted by one or more halo groups as defined herein.

As used herein, the terms "heterocyclyl", "heterocyclyl group", "heterocyclic" and "heterocyclic ring" are used interchangeably to refer to a saturated, unsaturated non-aromatic, monocyclic or bicyclic (e.g., fused) ring system which has from 3- to 12-ring members, or in particular 3- to 6- ring members or 5- to 7- ring members, at least one of which is a heteroatom, and up to 4 (e.g., 1, 2, 3 or 4) of which may be heteroatoms, wherein the heteroatoms are independently selected from O, S and N, and wherein C can be oxidized (e.g., C(=O)), N can be oxidized (e.g., N(O)) or quaternized (e.g. N⁺), and S can be optionally oxidized to sulfoxide and sulfone. Examples of non-aromatic heterocyclyls include aziridinyl, azetidinyl, oxetanyl, thietanyl, pyrrolidinyl, tetrahydrofuranyl, thiolanyl, imidazolidinyl, pyrazolidinyl, isoxazolidinyl, isothiazolidinyl, piperidinyl, tetrahydropyranyl, thianyl, piperazinyl, morpholinyl, thiomorpholinyl, dioxanyl, dithianyl, azepanyl, oxepanyl, thiepanyl, dihydrofuranyl, imidazolinyl, dihydropyranyl, hydantoinyl, pyrrolidinonyl, tetrahydrothiopyranyl, tetrahydropyridinyl, and thiopyranyl, and the like. Examples of bicyclic nonaromatic heterocyclic ring systems include benzo[1,3]dioxolyl, tetrahydroindolyl, and 2-azaspiro[3.3]heptanyl.

As used herein, the terms "heteroaryl", "heteroaryl group", "heteroaromatic" and "heteroaromatic ring" are used interchangeably to refer to an aromatic 5- to 12-membered monocyclic or bicyclic ring system, having 1 to 4 heteroatoms independently selected from O, S and N, and wherein N can be oxidized (e.g., N(O)) or quaternized, and S can be optionally oxidized to sulfoxide and sulfone. "Heteroaryl" includes a heteroaromatic group that is fused to a phenyl group or non-aromatic heterocycle such as tetrahydrofuran, pyran, pyrrolidine, piperidine, and the like. As used herein, the heteroaryl group Ar can be attached to the rest of a compound of the invention at any ring that has an open valency. Examples of heteroaryls include pyrrolyl, furanyl, imidazolyl, pyrazolyl, oxazolyl, isoxazolyl, thiazolyl, isothiazolyl, oxadiazolyl, thiadiazolyl, triazolyl, tetrazolyl, pyridyl, pyrazinyl, pyrimidyl, pyridazinyl, oxazinyl, thiazinyl, dioxinyl, triazinyl, tetrazinyl, azepinyl, oxepinyl, thiepinyl, thiazepinyl, 1-oxo-pyridyl, thienyl, valerolactamyl, azaindolyl, benzimidazolyl, benzo[1,4]dioxinyl, benzofuryl, benzoisoxazolyl, benzoisothiazolyl, benzothiadiazolyl, benzothiazolyl, benzothienyl, benzotriazolyl, benzoxadiazolyl, benzoxazolyl, cyclopentaimidazolyl, cyclopentatriazolyl, imidazo[1,2-a]pyridyl, indazolyl, indolizinyl, indolyl, isoquinolinyl, oxazolopyridinyl, purinyl, pyrazolo[3,4]pyrimidinyl, pyridopyazinyl, pyridopyrimidinyl, pyrrolo[2,3]pyrimidinyl, pyrrolopyrazolyl, pyrroloimidazolyl, pyrrolotriazolyl, quinazolinyl, quinolinyl, thiazolopyridinyl, naphthyridyl.

As used herein the term "an optionally substituted phenyl fused to an optionally substituted non-aromatic 5- to 6-membered heterocycle" refers to the fused ring system where it is the phenyl ring that is that is directly linked to the rest of the compound.

As used herein, the term "cycloalkyl" refers to completely saturated monocyclic or bicyclic (e.g., fused) hydrocarbon groups of 3-12 carbon atoms, 3-6 carbon atoms or 5-7 carbon atoms.

As used herein, the term "halocycloalkyl" refers to a cycloalkyl, as defined herein, that is substituted by one or more halo groups as defined herein.

"The term "fused ring system," as used herein, is a ring system having at least two rings and in which two rings share two adjacent ring atoms.

A substituted alkyl, phenyl, heteroaryl, non-aromatic heterocyclyl or heterocyclyl group is an alkyl, phenyl, heteroaryl, non-aromatic heterocyclyl or heterocyclyl group that has one or more substituents. Suitable substituents are those that do not significantly decrease the O-GlcNAcase inhibitory activity of a compound of formula (I"), (I), (I'), (Ia), (Ia') (Ib), (Ib') (Ia1), (Ia1'), (Ia2), (Ia2'), (Ia3), (Ia3'), (Ib1), (Ib1'), (II'), (IIa), (IIb), (IIa1), (IIa2), (IIa3), (IIb1), (III), (IIIa), (IIIb), (IIIc), (IIId), (IIIe), (IIIe'), (IIIf), (IIIf'), (IIIg), (IIIg'), (IIIh), (IIIh'), (IIIi), (IIIi'), (IIIj'), (IIIk') (hereinafter referred thereto collectively as "formulas (I") through (IIIk")") or a pharmaceutically acceptable salt thereof. Examples of suitable substituents for an alkyl, phenyl, heteroaryl, non-aromatic heterocyclyl or heterocyclyl group include but are not limited to C₁-C₄ alkyl, C₁-C₄ haloalkyl, C₃-C₆ cycloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, -C(=O)R^{x}, phenyl and monocyclic heteroaryl. The C₁-C₄ alkyl group substituent is optionally substituted with -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{x}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{y}, C₃-C₆ cycloalkyl (optionally substituted with one or more groups selected from -CH₃, halomethyl, halo, methoxy and halomethoxy), monocyclic heteroaryl (optionally substituted with one or more groups selected from -CH₃, halomethyl, halo, methoxy or halomethoxy) and phenyl (optionally substituted with one or more groups selected from -CH₃, halomethyl, halo, methoxy and halomethoxy). The C₃-C₆ cycloalkyl, phenyl and monocyclic heteroaryl group substituents are optionally and independently substituted with C₁-C₄ alkyl, C₁-C₄ haloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{y}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{x}. In these substituents, each R^{x} and each R^{y} is independently -H, C₁-C₄ alkyl, or C₃-C₈ cycloalkyl, where the C₁-C₄ alkyl or C₃-C₈ cycloalkyl represented by R^{x} or R^{y} is optionally substituted with one or more substituents selected from halo, hydroxyl, C₃-C₆ cycloalkyl and phenyl (optionally substituted with one or more groups selected from -CH₃, halomethyl, halo, methoxy or halomethoxy). In these substituents, R^{z} is -H, C₁-C₄ alkyl, or C₃-C₈ cycloalkyl, where the C₁-C₄ alkyl or C₃-C₈ cycloalkyl group represented by R^{z} is optionally substituted with one or more substituents selected from halo, hydroxyl, C₃-C₆ cycloalkyl and phenyl (optionally substituted with one or more groups selected from -CH₃, halomethyl, halo, methoxy and halomethoxy). In these substituents, i is 0, 1, or 2.

Pharmaceutically acceptable salts of the compounds disclosed herein are also included in the invention. In cases where a compound provided herein is sufficiently basic or acidic to form stable nontoxic acid or base salts, preparation and administration of the compounds as pharmaceutically acceptable salts may be appropriate. Examples of pharmaceutically acceptable salts are organic acid addition salts formed with acids which form a physiologically acceptable anion, for example, tosylate, methanesulfonate, acetate, citrate, malonate, tartarate, succinate, benzoate, ascorbate, α-ketoglutarate or α-glycerophosphate. Inorganic salts may also be formed, including hydrochloride, sulfate, nitrate, bicarbonate and carbonate salts.

Pharmaceutically acceptable salts may be obtained using standard procedures well known in the art, for example by reacting a sufficiently basic compound such as an amine with a suitable acid; affording a physiologically acceptable anion. Alkali metal (for example, sodium, potassium or lithium) or alkaline earth metal (for example calcium) salts of carboxylic acids can also be made.

Pharmaceutically acceptable base addition salts can be prepared from inorganic and organic bases. Suitable bases include but are not limited to alkali metal hydroxides, alkaline earth metal hydroxides, carbonates, bicarbonates, and the like.

The disclosed compounds, or pharmaceutically acceptable salts thereof, can contain one or more asymmetric centers in the molecule. In accordance with the present disclosure any structure that does not designate the stereochemistry is to be understood as embracing all the various stereoisomers (e.g., diastereomers and enantiomers) in pure or substantially pure form, as well as mixtures thereof (such as a racemic mixture, or an enantiomerically enriched mixture). It is well known in the art how to prepare such optically active forms (for example, resolution of the racemic form by recrystallization techniques, synthesis from optically-active starting materials, by chiral synthesis or chromatographic separation using a chiral stationary phase). The disclosed compounds may exist in tautomeric forms and mixtures and separate individual tautomers are contemplated. In addition, some compounds may exhibit polymorphism.

When a particular steroisomer (e.g., enantiomer, diasteromer, etc.) of a compound used in the disclosed methods is depicted by name or structure, the stereochemical purity of the compounds is at least 60%, 70%, 80%, 90%, 95%, 97%, 99%, 99.5% or 99.9%. "Stererochemical purity" means the weight percent of the desired stereoisomer relative to the combined weight of all stereoisomers.

When the stereochemistry of a disclosed compound is named or depicted by structure, and the named or depicted structure encompasses more than one stereoisomer (e.g., as in a diastereomeric pair), it is to be understood that one of the encompassed stereoisomers or any mixture of the encompassed stereoisomers are included. It is to be further understood that the stereoisomeric purity of the named or depicted stereoisomers at least 60%, 70%, 80%, 90%, 99% or 99.9% by weight. The stereoisomeric purity in this case is determined by dividing the total weight in the mixture of the stereoisomers encompassed by the name or structure by the total weight in the mixture of all of the stereoisomers.

The term "Peak 1" as used herein refers to the first eluding peak during the separation of enantiomers and/or diastereomers, which is followed by the subsequently eluding "Peak 2", and optionally, "Peak 3", and "Peak 4".

In one embodiment, any position occupied by hydrogen is meant to include enrichment by deuterium above the natural abundance of deuterium as well. For example, one or more hydrogen atoms are replaced with deuterium at an abundance that is at least 3340 times greater than the natural abundance of deuterium, which is 0.015% (i.e., at least 50.1% incorporation of deuterium), at least 3500 (52.5% deuterium incorporation at each designated deuterium atom), at least 4000 (60% deuterium incorporation), at least 4500 (67.5% deuterium incorporation), at least 5000 (75% deuterium), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation). In one embodiment, hydrogen is present at all positions at its natural abundance. The compounds or pharmaceutically acceptable salts thereof as described herein, may exist in tautomeric forms and mixtures and separate individual tautomers are contemplated.

One aspect of the invention includes a compound of any one of formulas (I") through (IIIk') or a pharmaceutically acceptable salt thereof, for use in inhibiting a glycosidase and/or a glycosidase signaling pathway in a cell. The glycosidase is preferably a glycoside hydrolase, more preferably a family 84 glycoside hydrolase, even more preferably O-glycoprotein-2-acetamido-2-deoxy-3-D-glucopyranosidase (O-GlcNAcase or OGA), most preferably a mammalian O-GlcNAcase. In one embodiment, the cell is contacted in vitro or in vivo. In one embodiment, contacting the cell includes administering the compound to a subj ect.

One aspect of the invention includes a a compound of any one of formulas (I") through (IIIk') or a pharmaceutically acceptable salt thereof, for use in inhibiting a glycosidase and/or a glycosidase signaling pathway in a subject in need thereof, thereby activating the glycosidase in the subject. The glycosidase is preferably a glycoside hydrolase, more preferably a family 84 glycoside hydrolase, even more preferably O-glycoprotein-2-acetamido-2-deoxy-3-D-glucopyranosidase (O-GlcNAcase or OGA), most preferably a mammalian O-GlcNAcase.

One aspect of the invention includes a compound of any one of formulas (I") through (IIIk') or a pharmaceutically acceptable salt thereof, for promoting survival of a eukaryotic cell (e.g., a mammalian cell) or increasing the lifespan of the cell

One aspect of the invention includes a compound of any one of formulas (I") through (IIIk') or a pharmaceutically acceptable salt thereof, for use in treating a disease or a condition that is caused, mediated and/or propagated by O-GlcNAcase activity in a subject. Preferably, the disease or condition is a neurological disorder, diabetes, cancer or stress. More preferably, the disease or condition is a neurological disorder. In one embodiment, the neurological disorder is one or more tauopathies selected from Acute ischemic stroke (AIS), Alzheimer's disease, Dementia, Amyotrophic lateral sclerosis (ALS), Amyotrophic lateral sclerosis with cognitive impairment (ALSci), Argyrophilic grain dementia, Bluit disease, Corticobasal degeneration (CBP), Dementia pugilistica, Diffuse neurofibrillary tangles with calcification, Down's syndrome, epilepsy, Familial British dementia, Familial Danish dementia, Frontotemporal dementia with parkinsonism linked to chromosome 17 (FTDP-17), Gerstmann-Straussler-Scheinker disease, Guadeloupean parkinsonism, Hallevorden-Spatz disease (neurodegeneration with brain iron accumulation type 1), ischemic stroke, mild cognitive impairment (MCI), Multiple system atrophy, Myotonic dystrophy, Niemann-Pick disease (type C), Pallido-ponto-nigral degeneration, Parkinsonism-dementia complex of Guam, Pick's disease (PiD), Postencephalitic parkinsonism (PEP), Prion diseases (including Creutzfeldt- Jakob Disease (GJD), Variant Creutzfeldt-Jakob Disease (vCJD), Fatal Familial Insomnia, Kuru, Progressive supercortical gliosis, Progressive supranuclear palsy (PSP), Steele- Richardson-Olszewski syndrome, Subacute sclerosing panencephalitis, Tangle-only dementia, Huntington's disease, and Parkinson's disease. In another embodiment, the neurological disorder is one or more tauopathies selected from Acute ischemic stroke (AIS), Alzheimer's disease, Dementia, Amyotrophic lateral sclerosis (ALS), Amyotrophic lateral sclerosis with cognitive impairment (ALSci), Argyrophilic grain dementia, epilepsy, mild cognitive impairment (MCI), Huntington's disease, and Parkinson's disease. In yet another embodiment, the neurological disorder is Alzheimer's disease.

One aspect of the invention includes a compound of any one of formulas (I") through (IIIk') or a pharmaceutically acceptable salt thereof for use in treating a disease or a condition that is characterized by hyperphosphorylation of tau (e.g., hyperphosphorylation of tau in the brain) in a subject. In one embodiment, the disease or condition is selected from Acute ischemic stroke (AIS), Alzheimer's disease, Dementia, Amyotrophic lateral sclerosis (ALS), Amyotrophic lateral sclerosis with cognitive impairment (ALSci), Argyrophilic grain dementia, Bluit disease, Corticobasal degeneration (CBP), Dementia pugilistica, Diffuse neurofibrillary tangles with calcification, Down's syndrome, epilepsy, Familial British dementia, Familial Danish dementia, Frontotemporal dementia with parkinsonism linked to chromosome 17 (FTDP-17), Gerstmann-Straussler-Scheinker disease, Guadeloupean parkinsonism, Hallevorden-Spatz disease (neurodegeneration with brain iron accumulation type 1), ischemic stroke, mild cognitive impairment (MCI), Multiple system atrophy, Myotonic dystrophy, Niemann-Pick disease (type C), Pallido-ponto-nigral degeneration, Parkinsonism-dementia complex of Guam, Pick's disease (PiD), Postencephalitic parkinsonism (PEP), Prion diseases (including Creutzfeldt- Jakob Disease (GJD), Variant Creutzfeldt-Jakob Disease (vCJD), Fatal Familial Insomnia, Kuru, Progressive supercortical gliosis, Progressive supranuclear palsy (PSP), Steele- Richardson-Olszewski syndrome, Subacute sclerosing panencephalitis, Tangle-only dementia, Huntington's disease, and Parkinson's disease. In another embodiment, the disease or condition is selected from Acute ischemic stroke (AIS), Alzheimer's disease, Dementia, Amyotrophic lateral sclerosis (ALS), Amyotrophic lateral sclerosis with cognitive impairment (ALSci), Argyrophilic grain dementia, epilepsy, ischemic stroke, mild cognitive impairment (MCI), Huntington's disease, and Parkinson's disease. In yet another embodiment, the disease or condition is Alzheimer's disease.

As used herein, the term "subject" and "patient" may be used interchangeably, and means a mammal in need of treatment, e.g., companion animals (e.g., dogs, cats and the like), farm animals (e.g., cows, pigs, horses, sheep, goats and the like) and laboratory animals (e.g., rats, mice, guinea pigs and the like). Typically, the subject is a human in need of treatment.

As used herein, the term "treating" or 'treatment" refers to obtaining desired pharmacological and/or physiological effect. The effect can be therapeutic, which includes achieving, partially or substantially, one or more of the following results: partially or totally reducing the extent of the disease, disorder or syndrome; ameliorating or improving a clinical symptom or indicator associated with the disorder; and delaying, inhibiting or decreasing the likelihood of the progression of the disease, disorder or syndrome.

The term "an effective amount" means an amount of the compound of any one of formulas (I") through (IIIk') or a pharmaceutically acceptable salt thereof, e.g., 0.1 mg to 1000 mg/kg body weight, when administered to a subject, which results in beneficial or desired results, including clinical results, i.e., reversing, alleviating, inhibiting, reducing or slowing the progression of a disease or condition treatable by a compound of any one of formulas (I") through (IIIk') or a pharmaceutically acceptable salt thereof, reducing the likelihood of recurrence of a disease or condition treatable by a compound of any one of formulas (I") through (IIIk') or a pharmaceutically acceptable salt thereof or one or more symptoms thereof, e.g., as determined by clinical symptoms, compared to a control. The expression "an effective amount" also encompasses the amounts which are effective for increasing normal physiological function.

Another embodiment of the present invention is a pharmaceutical composition comprising at least one compound described herein, or a pharmaceutically acceptable salt thereof, and at least one pharmaceutically acceptable carrier.

Also included is a compound of any one of formulas (I") through (IIIk') or a pharmaceutically acceptable salt thereof for use in the treatment of a subject with one or more diseases or conditions described herein. Further included are pharmaceutical compositions comprising a compound of any one of formulas (I") through (IIIk') or a pharmaceutically acceptable salt thereof, optionally together with a pharmaceutically acceptable carrier, for use in the treatment of one or more diseases or conditions described herein.

The term "pharmaceutically acceptable carrier" refers to a non-toxic carrier, diluent, adjuvant, vehicle or excipient that does not adversely affect the pharmacological activity of the compound with which it is formulated, and which is also safe for human use. Pharmaceutically acceptable carriers that may be used in the compositions of this disclosure include, but are not limited to, ion exchangers, alumina, aluminum stearate, magnesium stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (e.g., microcrystalline cellulose, hydroxypropyl methylcellulose, lactose monohydrate, sodium lauryl sulfate, and crosscarmellose sodium), polyethylene glycol, sodium carboxymethylcellulose, polyacrylates, waxes, polyethylene-polyoxypropylene-block polymers, polyethylene glycol and wool fat.

Other excipients, such as flavoring agents; sweeteners; and preservatives, such as methyl, ethyl, propyl and butyl parabens, can also be included. More complete listings of suitable excipients can be found in the Handbook of Pharmaceutical Excipients (5th Ed., a Pharmaceutical Press (2005)). A person skilled in the art would know how to prepare formulations suitable for various types of administration routes. Conventional procedures and ingredients for the selection and preparation of suitable formulations are described, for example, in Remington's Pharmaceutical Sciences (2003, 20th edition) and in The United States Pharmacopeia: The National Formulary (USP 24 NF19) published in 1999.

A compound of any one of formulas (I") through (IIIk') or a pharmaceutically acceptable salt thereof, or the compositions of the present teachings may be administered, for example, by oral, parenteral, sublingual, topical, rectal, nasal, buccal, vaginal, transdermal, patch, pump administration or via an implanted reservoir, and the pharmaceutical compositions would be formulated accordingly. Parenteral administration includes intravenous, intraperitoneal, subcutaneous, intramuscular, transepithelial, nasal, intrapulmonary, intrathecal, rectal and topical modes of administration. Parenteral administration can be by continuous infusion over a selected period of time.

Other forms of administration included in this disclosure are as described in WO 2013/075083, WO 2013/075084, WO 2013/078320, WO 2013/120104, WO 2014/124418, WO 2014/151142, and WO 2015/023915.

Useful dosages of a compound or pharmaceutically acceptable salt thereof as described herein can be determined by comparing their in vitro activity and in vivo activity in animal models. Methods for the extrapolation of effective dosages in mice and other animals, to humans are known to the art; for example, see U.S. Pat. No. 4,938,949.

### EXEMPLIFICATIONS

The following general reaction schemes, Schemes 1 to 4, provide useful details for preparing the instant compounds. The requisite intermediates are in some cases commercially available or can be prepared according to literature procedures. The illustrative reaction schemes are not limited by the compounds listed or by any particular substituents employed for illustrative purposes substituent labeling (i.e. R groups) as shown in the reaction schemes do not necessarily correlate to that used in the claims and often, for clarity, a single substituent is shown attached to the compound where multiple substituents are allowed under the definitions of any one of formulas (I") through (IIIk') hereinabove. 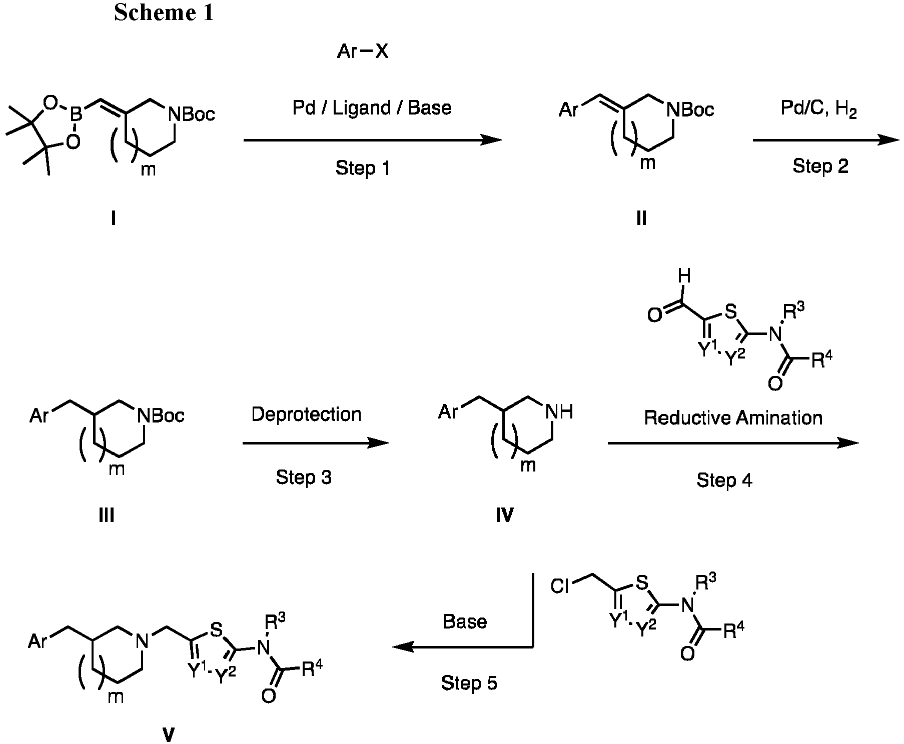 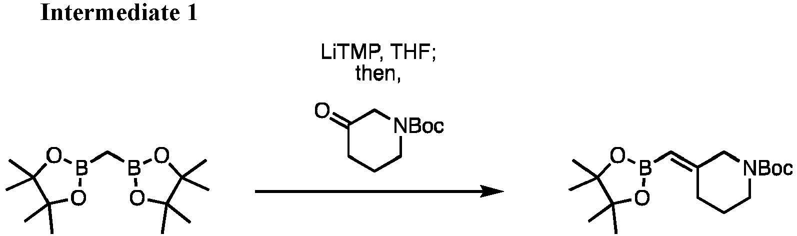 ***tert*-butyl 3-((4,4,5,5-tetramethyl-1,3,2-dioxaborolan-2-yl)methylene)piperidine-1-carboxylate:** To a solution of 2,2,6,6-tetramethylpiperidine (85.0 g, 602 mmol) in anhydrous THF (1000 mL) at 0 °C was added a 2.5M solution of n-BuLi in hexanes (288 mL, 720 mmol) under argon. The resulting mixture was stirred for 10 min. A solution of 4,4,5,5-tetramethyl-2-[(tetramethyl 1,3,2-dioxaborolan-2-yl)methyl]-1,3,2-dioxaborolane (161 g, 601 mmol) in THF (200 mL) was added at 0°C. After stirring for 15 minutes, the solution was cooled to -78°C before the dropwise addition of a solution of *tert-butyl* 3-oxopiperidine-1-carboxylate (100 g, 502 mmol) in THF (500 mL). The reaction was stirred for 1 h at -78 °C and then overnight at 0 °C. A 30% K₂CO₃ (aq) solution (500 mL) was added and the organic layer was partitioned, dried over Na₂SO₄, filtered, and concentrated *in vacuo.* The residue was purified over SiO₂ (ethyl acetate/pentane) to afford the title compound (121 g, 75%). 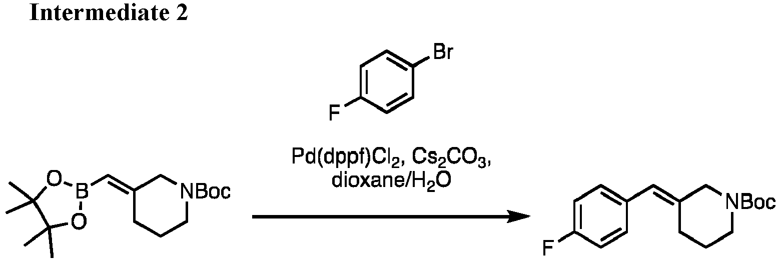 ***tert*-butyl 3-(4-fluorobenzylidene)piperidine-1-carboxylate**: To a solution of 1-bromo-4-fluorobenzene (0.2 g, 1.14 mmol) in dioxane (8.0 mL) and H₂O (2.0 mL) were added 1 *tert-*butyl 3-((4,4,5,5-tetramethyl-1,3,2-dioxaborolan-2-yl)methylene)piperidine-1-carboxylate (0.405 g, 1.26 mmol), Pd(dppf)Cl₂ (0.084 g, 0.114 mmol), Cs₂CO₃ (0.745 g, 2.29 mmol). The mixture was stirred at 90 °C for 2.0 hours under N₂. The reaction mixture was filtered and concentrated *in vacuo.* The residue was purified over SiO₂ (petroleum ether/EtOAc: 15/1) to afford the title compound (0.120 g, 36%). LCMS (ESI): [M+H] 292. 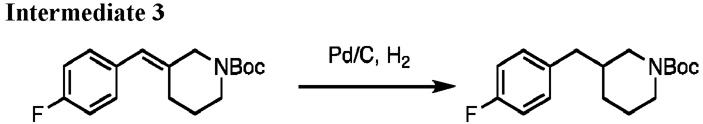 ***tert-butyl* 3-(4-fluorobenzyl)piperidine-1-carboxylate:** To a solution of *tert-butyl* 3-(4-fluorobenzylidene)piperidine-1-carboxylate (0.120 g, 0.412 mmol) in MeOH (15.0 mL) was added Pd/C (0.022 g, 10% on carbom) under N₂. The mixture was stirred at 20 °C for 1 hour under H₂ (15 psi). The mixture was filtered and concentrated *in vacuo* to afford the title compound (0.082 g, 68%). LCMS (ESI): [M+H] 294. 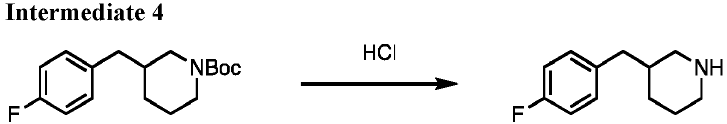 **3-(4-fluorobenzyl)piperidine:** *tert-Butyl* 3-(4-fluorobenzyl)piperidine-1-carboxylate (0.082 g, 0.280 mmol) was added to a HCl/EtOAc solution (0.280 mmol, 8.0 mL). The mixture was stirred at 18 °C for 17 h. The mixture was adjusted to pH 8 with NH₄OH (aq) and the mixture was concentrated *in vacuo* to afford the title compound (0.054 g, 99%). LCMS (ESI): [M+H] 194.

### Example 1-1 (Reference)

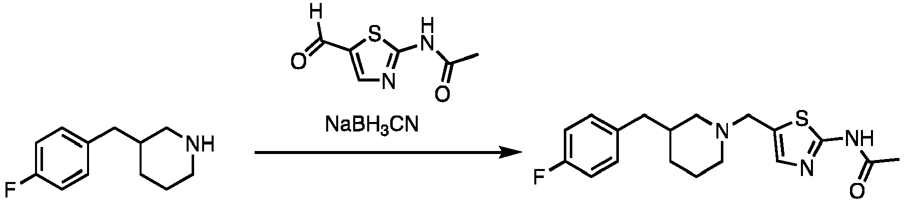

**N-(5-((3-(4-fluorobenzyl)piperidin-1-yl)methyl)thiazol-2-yl)acetamide:** To a solution of 3-(4-fluorobenzyl)piperidine (0.054 g, 0.279 mmol) in MeOH (15.0 mL) were added N-(5-formylthiazol-2-yl)acetamide (0.095 g, 0.559 mmol) and a drop of AcOH. The mixture was stirred at 50 °C for 2 h. NaBH₃CN (0.053 g, 0.838 mmol) was added and the mixture was stirred at 50 °C for 17 h. The mixture was purified by Prep-HPLC {(Column: Waters Xbridge Prep OBD C18 150×30 5u; Condition: water (0.04%NH₃H₂O+10mM NH₄HCO₃)-MeCN; Begin B: 35; End B: 65; Gradient Time (min):10; 100%B Hold Time (min): 2; FlowRate (ml/min): 25} to afford the title compound (0.038 g, 39%). LCMS: [M+H] 348. ¹HNMR (400 MHz, Methanol-*d*4) δ 7.19 (s, 1H), 7.14-7.10 (m, 2H), 6.94 (t, *J* = 9.2 Hz, 2H), 3.65 (s, 2H), 2.83-2.75 (m, 2H), 2.48 (d, *J* = 6.8 Hz, 2H), 2.19 (s, 3H), 2.02-1.99 (m, 1H), 1.82-1.66 (m, 4H), 1.57-1.47 (m, 1H), 0.99-0.91 (m, 1H).

### Example 1-2 (Reference)

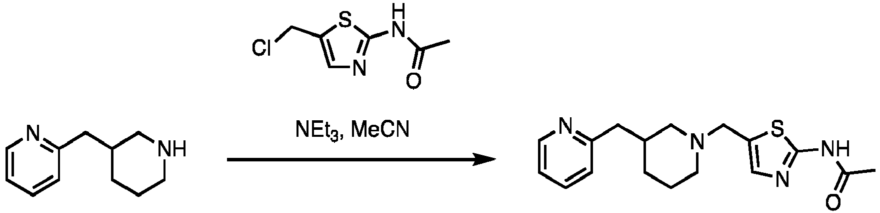

**N-(5-((3-(pyridin-2-ylmethyl)piperidin-1-yl)methyl)thiazol-2-yl)acetamide:** To a suspension of 2-(3-piperidylmethyl)pyridine HCl (0.035 g, 0.165 mol, hydrochloride) and N-[5-(chloromethyl)thiazol-2-yl]acetamide (0.032 g, 0.165 mmol) in MeCN (0.64 mL) was added triethylamine (0.05 g, 0.494 mol) and the mixture warmed to 70 °C overnight. The reaction was cooled to room temperture, and the mixture was diluted with EtOAc and washed with saturated NH₄Cl (*aq*). The organics were dried over MgSO₄, filtered and concentrated *in vacuo.* The residue was purified by Prep-HPLC {(Column: Waters XSelect CSH Prep C18 5um OBD 19×100mm; Condition: water:MeCN gradient 5-55% ACN over 7 min gradient, 0.1 Vol% ammonium hydroxide modifier} to afford the title compound (0.015 g, 27%). LCMS (ESI): [M+H] 331. ¹HNMR: (500 MHz, CDCl₃) δ 11.76 (br s, 1H), 8.45 (dd, *J*=1.8, 4.9 Hz, 1H), 8.40 (d, *J*=1.8 Hz, 1H), 7.47 (td, *J*=1.8, 7.9 Hz, 1H), 7.21 (dd, *J*=4.6, 7.0 Hz, 1H), 7.18 (s, 1H), 3.70 - 3.59 (m, 2H), 2.78 (br d, *J*=9.2 Hz, 2H), 2.60 - 2.53 (m, 1H), 2.51 - 2.43 (m, 1H), 2.31 (s, 3H), 2.07 - 1.98 (m, 1H), 1.93 - 1.81 (m, 2H), 1.66 (m, 2H), 1.56 - 1.46 (m, 1H), 1.02 - 0.91 (m, 1H).

### Example 1-3 (Reference)

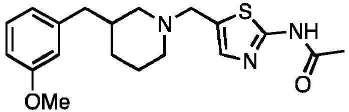

**N-(5-((3-(3-methoxybenzyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 1-bromo-3-methoxybenzene, and N-(5-formylthiazol-2-yl)acetamide. LCMS (ESI): [M+H] 360. ¹HNMR: (400 MHz, Methanol-d4) δ 7.20 (s, 1H), 7.13 (t, *J=* 8.0 Hz, 1H), 6.71-6.69 (m, 3H), 3.75 (s, 3H), 3.71-3.65 (m, 2H), 2.84-2.78 (m, 2H), 2.48-2.46 (m, 2H), 2.19 (s, 3H), 2.04-1.99 (m, 1H), 1.88-1.66 (m, 4H), 1.58-1.48 (m, 1H), 1.00-0.92 (m, 1H).

### Example 1-4 (Reference)

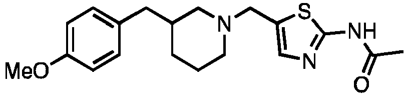

***N*-(5-((3-(4-methoxybenzyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 1-chloro-4-methoxybenzene, and *N*-(5-formylthiazol-2-yl)acetamide. LCMS (ESI): [M+H] 360. ¹HNMR: (400 MHz, CDCl₃) δ 12.43 (s, 1H), 7.17 (s, 1H), 7.04 (d, *J* = 8.4 Hz, 1H), 6.80 (d, *J* = 8.4 Hz, 1H), 3.77 (s, 3H), 3.69-3.58 (m, 2H), 2.79-2.67 (m, 2H), 2.51-2.37 (m, 2H), 2.31 (s, 3H), 2.00-1.85 (m, 1H), 1.90-1.76 (m, 2H), 1.71-1.57 (m, 2H), 1.54-1.48 (m, 1H), 0.94-0.86 (m, 1H).

### Example 1-5 (Reference)

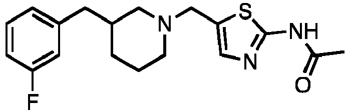

***N*-(5-((3-(3-fluorobenzyl)piperidin-1-yl)methyl)thiazol-2-yl)acetamide:** The title compound was prepared in an analogous manner of that in scheme 1 from *tert*-butyl 3-((4,4,5,5-tetramethyl-1,3,2-dioxaborolan-2-yl)methylene)piperidine-1-carboxylate, 1-bromo-3-fluorobenzene, and *N*-(5-formylthiazol-2-yl)acetamide. LCMS (ESI): [M+H] 348. ¹HNMR (400 MHz, Methanol-*d*4) δ 7.26-7.20 (m, 2H), 6.94 (d, *J* = 7.6 Hz, 1H), 6.89-6.85 (m, 2H), 3.69-3.61 (m, 2H), 2.83-2.76 (m, 2H), 2.58-2.47 (m, 2H), 2.19 (s, 3H), 2.05-1.94 (m, 1H), 1.87-1.76 (m, 2H), 1.69-1.66 (m, 2H), 1.57-1.48 (m, 1H), 1.01-0.93 (m, 1H).

### Example 1-6 (Reference)

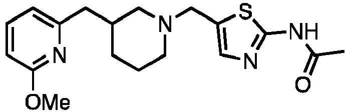

**N-(5-((3-((6-methoxypyridin-2-yl)methyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate and 2-chloro-6-methoxypyridine. LCMS (ESI): [M+H] 361. ¹HNMR: (400 MHz, CDCl₂) δ 12.41 (br s, 1H), 7.44-7.40 (m, 1H), 7.17 (s, 1H), 6.64 (d, *J* = 7.2 Hz, 1H), 6.51 (d, *J* = 8.0 Hz, 1H), 3.88 (s, 3H), 3.69-3.60 (m, 2H), 2.83-2.77 (m, 2H), 2.62-2.50 (m, 2H), 2.30 (s, 3H), 2.16-2.12 (m, 1H), 2.04-1.99 (m, 1H), 1.87-1.82 (m, 1H), 1.68-1.62 (m, 2H), 1.55-1.52 (m, 1H), 1.01-0.92 (m, 1H).

### Example 1-7 (Reference)

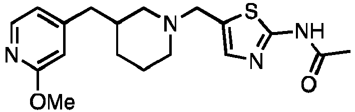

**N-(5-((3-((2-methoxypyridin-4-yl)methyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 4-bromo-2-methoxypyridine, and N-(5-formylthiazol-2-yl)acetamide. LCMS (ESI): [M+H] 361. ¹HNMR: (400 MHz, Methanol-d4) δ 7.95 (d, *J* = 5.2 Hz, 1H), 7.19 (s, 1H), 6.77 (d, *J* = 5.2 Hz, 1H), 6.59 (s, 1H), 3.86 (s, 3H), 3.67 (s, 2H), 2.82-2.74 (m, 2H), 2.51-2.49 (m, 2H), 2.19 (s, 3H), 2.07-2.00 (m, 1H), 1.93-1.84 (m, 1H), 1.82-1.77 (m, 1H), 1.69-1.67 (m, 2H), 1.59-1.55 (m, 1H), 1.03-0.96 (m, 1H).

### Example 1-8 (Reference)

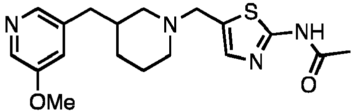

***N*-(5-((3-((5-methoxypyridin-3-yl)methyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 3-bromo-5-methoxypyridine, and N-(5-formylthiazol-2-yl)acetamide. LCMS (ESI): [M+H] 361. ¹HNMR: (400 MHz, CDCl₃) δ 11.18 (br s, 1H), 8.14 (d, *J* = 2.4 Hz, 1H), 8.00 (s, 1H), 7.17 (s, 1H), 6.98 (s, 1H), 3.84 (s, 3H), 3.64-3.63 (m, 2H), 2.78-2.76 (m, 2H), 2.53-2.46 (m, 2H), 2.29 (s, 3H), 2.02-1.98 (m, 1H), 1.88-1.82 (m, 2H), 1.67-1.63 (m, 2H), 1.55-1.50 (m, 1H), 0.96-0.94 (m, 1H).

### Example 1-9 (Reference)

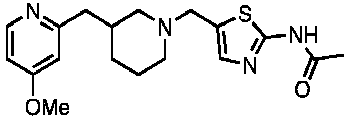

***N*-(5-((3-((4-methoxypyridin-2-yl)methyl)piperidin-1-yl)methyl)thiazol-2-yl)acetamide:** The title compound was prepared in an analogous manner of that in scheme 1 from *tert*-butyl 3-((4,4,5,5-tetramethyl-1,3,2-dioxaborolan-2-yl)methylene)piperidine-1-carboxylate, 2-bromo-5-methoxypyridine, and *N*-(5-formylthiazol-2-yl)acetamide. LCMS (ESI): [M+H] 361. ¹HNMR: (400 MHz, Methanol-*d*4) δ 8.21 (d, *J* = 6.0 Hz, 1H), 7.21 (s, 1H), 6.81-6.78 (m, 2H), 3.85 (s, 3H), 3.71-3.67 (m, 2H), 2.86-2.77 (m, 2H), 2.63 (d, *J* = 7.2 Hz, 2H), 2.20 (s, 3H), 2.08-2.02 (m, 2H), 1.89-1.82 (m, 1H), 1.75-1.61 (m, 2H), 1.58-1.52 (m, 1H), 1.06-1.03 (m, 1H).

### Example 1-10 (Reference)

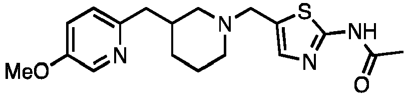

***N*-(5-((3-((5-methoxypyridin-2-yl)methyl)piperidin-1-yl)methyl)thiazol-2-yl)acetamide:** The title compound was prepared in an analogous manner of that in scheme 1 from *tert*-butyl 3-((4,4,5,5-tetramethyl-1,3,2-dioxaborolan-2-yl)methylene)piperidine-1-carboxylate, 2-bromo-5-methoxypyridine, and N-(5-formylthiazol-2-yl)acetamide. LCMS (ESI): [M+H] 361. ¹HNMR: (400 MHz, CDCl₃) δ 12.31 (br s, 1H), 8.20 (d, *J* = 2.8 Hz, 1H), 7.16 (s, 1H), 7.11-7.08 (m, 1H), 7.03-7.01 (m, 1H), 3.82 (s, 3H), 3.67-3.60 (m, 2H), 2.76 (d, *J* = 10.2 Hz, 2H), 2.74-2.60 (m, 2H), 2.30 (s, 3H), 2.03-2.00 (m, 2H), 1.87-1.85 (m, 1H), 1.65 (d, *J* = 9.6 Hz, 2H), 1.62-1.02 (m, 1H), 0.99-0.97 (m, 1H).

### Example 1-11 (Reference)

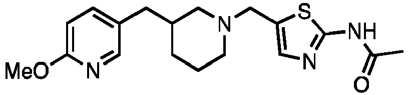

***N*-(5-((3-((6-methoxypyridin-3-yl)methyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 3-bromo-5-methoxypyridine, and *N*-(5-formylthiazol-2-yl)acetamide. LCMS (ESI): [M+H] 360. ¹HNMR: (400 MHz, Methanol-d4) δ 7.87 (d, *J* = 2.0 Hz, 1H), 7.49 (dd, *J* = 8.8, 2.0 Hz, 1H), 7.20 (s, 1H), 6.71 (d, *J* = 8.4 Hz, 1H), 3.86 (s, 3H), 3.69-3.62 (m, 2H), 2.88-2.71 (m, 2H), 2.46-2.44 (m, 2H), 2.20 (s, 3H), 2.07-1.97 (m, 1H), 1.86-1.73 (m, 2H), 1.72-1.62 (m, 2H), 1.60-1.44 (m, 1H), 1.05-0.88 (m, 1H).

### Example 1-12 (Reference)

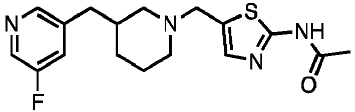

***N*-(5-((3-((5-fluoropyridin-3-yl)methyl)piperidin-1-yl)methyl)thiazol-2-yl)acetamide:** The title compound was prepared in an analogous manner of that in scheme 1 from *tert*-butyl 3-((4,4,5,5-tetramethyl-1,3,2-dioxaborolan-2-yl)methylene)piperidine-1-carboxylate, 3-bromo-5-methoxypyridine, and *N*-(5-formylthiazol-2-yl)acetamide. LCMS (ESI): [M+H] 349. ¹HNMR: (400 MHz, CDCl₃) δ 11.88 (br s, 1H), 8.30 (s, 1H), 8.22 (s, 1H), 7.21-7.17 (m, 2H), 3.68-3.59 (m, 2H), 2.77-2.74 (m, 2H), 2.63-2.47 (m, 2H), 2.31 (s, 3H), 2.08-2.03 (m, 1H), 1.93-1.83 (m, 2H), 1.73-1.68 (m, 2H), 1.56-1.50 (m, 1H), 1.03-0.93 (m, 1H).

### Example 1-13 (Reference)

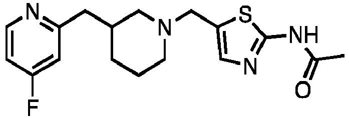

***N*-(5-((3-((4-fluoropyridin-2-yl)methyl)piperidin-1-yl)methyl)thiazol-2-yl)acetamide:** The title compound was prepared in an analogous manner of that in scheme 1 from *tert*-butyl 3-((4,4,5,5-tetramethyl-1,3,2-dioxaborolan-2-yl)methylene)piperidine-1-carboxylate, 2-bromo-4-fluoropyridine, and *N*-(5-formylthiazol-2-yl)acetamide. LCMS (ESI): [M+H] 349. ¹HNMR: (400 MHz, Methanol-d4) δ 8.46 (dd, *J* = 8.8, 5.6 Hz, 1H), 7.23 (s, 1H), 7.14-7.11 (m, 1H), 7.09-7.06 (m, 1H), 3.71 (s, 2H), 2.88-2.81 (m, 2H), 2.75-2.72 (m, 1H), 2.22 (s, 3H), 2.12-2.09 (m, 2H), 1.93-1.90 (m, 1H), 1.71-1.54 (m, 3H), 1.12-1.07 (m, 1H).

### Example 1-14 (Reference)

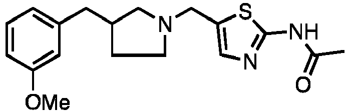

***N*-(5-((3-(3-methoxybenzyl)pyrrolidin-1-yl)methyl)thiazol-2-yl)acetamide:** The title compound was prepared in an analogous manner of that in scheme 1 from *tert*-butyl 3-((4,4,5,5-tetramethyl-1,3,2-dioxaborolan-2-yl)methylene)pyrrolidine-1-carboxylate, 1-bromo-3-methoxybenzene, and *N*-(5-formylthiazol-2-yl)acetamide. LCMS (ESI): [M+H] 346. ¹HNMR: (400 MHz, Methanol-d4) δ 7.23 (s, 1H), 7.15-7.11 (m, 1H), 6.77-6.66 (m, 3H), 3.83-3.66 (m, 5H), 2.73-2.55 (m, 5H), 2.52-2.40 (m, 1H), 2.29-2.23 (m, 1H), 2.19 (s, 3H), 1.99-1.87 (m, 1H), 1.56-1.46 (m, 1H).

### Example 1-15 (Reference)

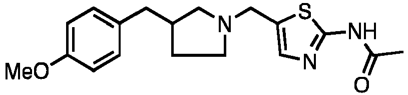

***N*-(5-((3-(4-methoxybenzyl)pyrrolidin-1-yl)methyl)thiazol-2-yl)acetamide:** The title compound was prepared in an analogous manner of that in scheme 1 from *tert*-butyl 3-((4,4,5,5-tetramethyl-1,3,2-dioxaborolan-2-yl)methylene)pyrrolidine-1-carboxylate, 1-chloro-4-methoxybenzene, and N-(5-formylthiazol-2-yl)acetamide. LCMS (ESI): [M+H] 346. ¹HNMR: (400 MHz, Methanol-d4) δ 7.24 (s, 1H), 7.07 (dd, *J* = 6.4, 2.0 Hz, 2H), 6.81 (t, *J* = 2.8 Hz, 1H), 6.79 (d, *J* = 2.0 Hz, 1H), 3.79-3.73 (m, 5H), 2.74-2.69 (m, 2H), 2.65-2.58 (m, 3H), 2.50-2.42 (m, 1H), 2.29-2.24 (m, 1H), 2.19 (s, 3H), 1.96-1.89 (m, 1H), 1.55-1.51 (m, 1H).

### Example 1-16 (Reference)

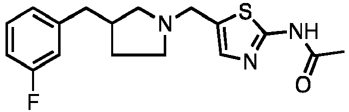

***N*-(5-((3-(3-fluorobenzyl)pyrrolidin-1-yl)methyl)thiazol-2-yl)acetamide:** The title compound was prepared in an analogous manner of that in scheme 1 from *tert*-butyl 3-((4,4,5,5-tetramethyl-1,3,2-dioxaborolan-2-yl)methylene)pyrrolidine-1-carboxylate, 1-bromo-3-fluorobenzene, and *N*-(5-formylthiazol-2-yl)acetamide. LCMS (ESI): [M+H] 334. ¹HNMR (400 MHz, DMSO-*d*6) δ 11.50 (br s, 1H), 7.32-7.26 (m, 1H), 7.22 (s, 1H), 7.04-6.97 (m, 3H), 3.72-3.63 (m, 2H), 3.32 (s, 2H), 2.69-2.58 (m, 2H), 2.58-2.52 (m, 3H), 2.45-2.35 (m, 1H), 2.17-2.14 (m, 1H), 2.10 (s, 3H), 1.88-1.79 (m, 1H), 1.45-1.37 (m, 1H).

### Example 1-17 (Reference)

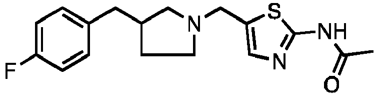

***N*-(5-((3-(4-fluorobenzyl)pyrrolidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)pyrrolidine-1-carboxylate, 1-bromo-4-fluorobenzene, and N-(5-formylthiazol-2-yl)acetamide. LCMS (ESI): [M+H] 334. ¹HNMR (400 MHz, Methanol-*d*4) δ 7.23 (s, 1H), 7.18-7.15 (m, 2H), 6.97-6.93 (m, 2H), 3.82-3.72 (m, 2H), 2.71-2.67 (m, 5H), 2.67-2.64 (m, 1H), 2.28-2.26 (m, 1H), 2.19 (s, 3H), 1.98-1.94 (m, 1H), 1.54-1.50 (m, 1H).

### Example 1-18 (Reference)

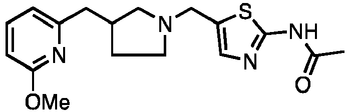

***N*-(5-((3-((6-methoxypyridin-2-yl)methyl)pyrrolidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)pyrrolidine-1-carboxylate, 2-chloro-6-methoxypyridine, and N-(5-formylthiazol-2-yl)acetamide. LCMS (ESI): [M+H] 347. ¹HNMR: (400 MHz, Methanol-*d*4) δ 7.55-7.50 (m, 1H), 7.25 (s, 1H), 6.76 (d, *J=* 6.8 Hz, 1H), 6.57 (d, *J=* 8.4 Hz, 1H), 3.85 (s, 3H), 3.84-3.74 (m, 2H), 2.80-2.68 (m, 6H), 2.36-2.37 (m, 1H), 2.19 (s, 3H), 2.03-1.96 (m, 1H), 1.62-1.54 (m, 1H).

### Example 1-19 (Reference)

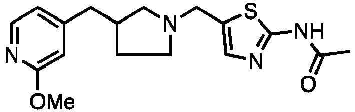

**N-(5-((3-((2-methoxypyridin-4-yl)methyl)pyrrolidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)pyrrolidine-1-carboxylate, 4-bromo-2-methoxypyridine, and N-(5-formylthiazol-2-yl)acetamide. LCMS (ESI): [M+H] 347. ¹HNMR: (400 MHz, Methanol-*d*4) δ 7.97 (d, *J=* 5.6 Hz, 1H), 7.23 (s, 1H), 6.81 (d, *J* = 5.6 Hz, 1H), 6.63 (s, 1H), 3.86 (s, 3H), 3.83-3.73 (m, 2H), 2.73-2.69 (m, 5H), 2.67-2.64 (m, 1H), 2.29-2.27 (m, 1H), 2.17 (s, 3H), 2.19-2.00 (m, 1H), 1.52-1.50 (m, 1H).

### Example 1-20 (Reference)

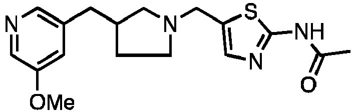

***N*-(5-((3-((5-methoxypyridin-3-yl)methyl)pyrrolidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)pyrrolidine-1-carboxylate, 3-bromo-5-methoxypyridine, and N-(5-formylthiazol-2-yl)acetamide. LCMS (ESI): [M+H] 347. ¹HNMR: (400 MHz, CDCl₃) δ 12.36 (s, 1H), 8.13 (d, *J=* 2.4 Hz, 1H), 8.04 (d, *J=* 1.6 Hz, 1H), 7.18 (s, 1H), 6.99-6.98 (m, 1H), 3.82 (s, 3H), 3.80-3.71 (m, 2H), 2.70-2.62 (m, 5H), 2.53-2.44 (m, 1H), 2.30 (s, 3H), 2.29-2.25 (m, 1H), 2.02-1.93 (m, 1H), 1.54-1.46 (m, 1H).

### Example 1-21 (Reference)

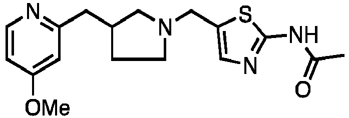

***N*-(5-((3-((4-methoxypyridin-2-yl)methyl)pyrrolidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)pyrrolidine-1-carboxylate, 2-bromo-4-methoxypyridine, and N-(5-formylthiazol-2-yl)acetamide. LCMS (ESI): [M+H] 347. ¹HNMR: (400 MHz, Methanol-*d*4) δ 8.22 (d, *J=* 6.0 Hz, 1H), 7.24 (s, 1H), 6.84 (d, *J* = 2.4 Hz, 1H), 6.82-6.80 (m, 1H), 3.86 (s, 3H), 3.84-3.75 (m, 2H), 2.79 (d, *J=* 7.6 Hz, 2H), 2.74-2.59 (m, 4H), 2.34-2.29 (m, 1H), 2.19 (s, 3H), 2.03-1.92 (m, 1H), 1.62-1.53 (m, 1H).

### Example 1-22 (Reference)

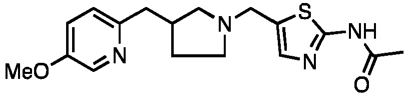

**N-(5-((3-((5-methoxypyridin-2-yl)methyl)pyrrolidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)pyrrolidine-1-carboxylate, 2-bromo-5-methoxypyridine, and N-(5-formylthiazol-2-yl)acetamide. LCMS (ESI): [M+H] 347. ¹HNMR: (400 MHz, CDCl₃) δ 11.55 (br s, 1H), 8.21 (d, *J* = 2.8 Hz, 1H), 7.19 (s, 1H), 7.12-7.09 (m, 1H), 7.05-7.03 (m, 1H), 3.83 (s, 3H), 3.80-3.71 (m, 2H), 2.79 (d, *J=* 7.2 Hz, 2H), 2.78-2.60 (m, 4H), 2.31-2.29 (m, 4H), 1.97-1.55 (m, 1H), 1.54-1.52 (m, 1H).

### Example 1-23 (Reference)

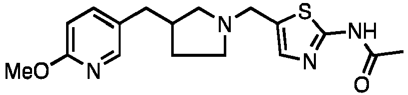

***N*-(5-((3-((6-methoxypyridin-3-yl)methyl)pyrrolidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)pyrrolidine-1-carboxylate, 5-bromo-2-methoxypyridine, and N-(5-formylthiazol-2-yl)acetamide. LCMS (ESI): [M+H] 347. ¹HNMR: (400 MHz, Methanol-*d*4) δ 7.92 (d, *J* = 1.6 Hz, 1H), 7.53 (dd, *J* = 8.8, 2.4 Hz, 1H), 7.24 (s, 1H), 6.72 (d, *J=* 8.8 Hz, 1H), 3.86 (s, 3H), 3.83-3.74 (m, 2H), 2.75-2.66 (m, 2H), 2.66-2.57 (m, 3H), 2.51-2.39 (m, 1H), 2.30-2.25 (m, 1H), 2.19 (s, 3H), 2.00-1.91 (m, 1H), 1.57-1.48 (m, 1H).

### Example 1-24 (Reference)

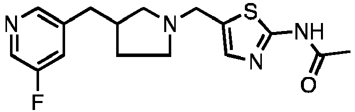

***N*-(5-((3-((5-fluoropyridin-3-yl)methyl)pyrrolidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)pyrrolidine-1-carboxylate, 3-bromo-5-fluoropyridine, and N-(5-formylthiazol-2-yl)acetamide. LCMS (ESI): [M+H] 335. ¹HNMR: (400 MHz, CDCl₃) δ 12.44 (br s, 1H), 8.29 (d, *J=* 2.8 Hz, 1H), 8.25 (s, 1H), 7.22-7.18 (m, 2H), 3.80-3.71 (m, 2H), 2.72-2.62 (m, 5H), 2.52-2.43 (m, 1H), 2.30 (s, 3H), 2.28-2.26 (m, 1H), 2.02-1.94 (m, 1H), 1.54-1.45 (m, 1H).

### Example 1-25 (Reference)

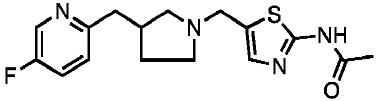

**N-(5-((3-((5-fluoropyridin-2-yl)methyl)pyrrolidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)pyrrolidine-1-carboxylate, 2-bromo-5-fluoropyridine, and N-(5-formylthiazol-2-yl)acetamide. LCMS (ESI): [M+H] 335. ¹HNMR: (400 MHz, CDCl₃) δ 11.55 (s, 1H), 8.36 (d, *J=* 3.2 Hz, 1H), 7.32-7.27 (m, 1H), 7.19 (s, 1H), 7.13-7.10 (m, 1H), 3.80-3.72 (m, 2H), 2.85-2.82 (m, 2H), 2.74-2.61 (m, 4H), 2.32-2.30 (m, 1H), 2.29 (s, 3H), 2.00-1.95 (m, 1H), 1.57-1.51 (m, 1H).

### Example 1-26 (Reference)

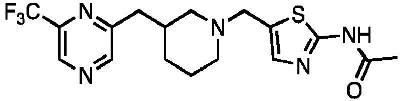

**N-(5-((3-((6-(trifluoromethyl)pyrazin-2-yl)methyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 2-chloro-6-(trifluoromethyl)pyrazine, and N-[5-(chloromethyl)thiazol-2-yl]acetamide. LCMS (ESI): [M+H] 400. ¹HNMR: (500 MHz, CDCl₃) δ 11.56 (br s, 1H), 8.78 (s, 1H), 8.64 (s, 1H), 7.18 (s, 1H), 3.75 - 3.58 (m, 2H), 2.95 - 2.68 (m, 3H), 2.31 (s, 3H), 2.23 - 2.09 (m, 2H), 2.04 - 1.91 (m, 1H), 1.74 - 1.52 (m, 4H), 1.09 (m, 1H).

### Example 1-27 (Reference)

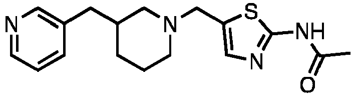

### N-(5-((3-(pyridin-3-ylmethyl)piperidin-1-yl)methyl)thiazol-2-yl)acetamide

The title compound was prepared in an analogous manner of that in scheme 1 from tert-butyl 3-((4,4,5,5-tetramethyl-1,3,2-dioxaborolan-2-yl)methylene)piperidine-1-carboxylate 3-bromopyridine, and N-[5-(chloromethyl)thiazol-2-yl]acetamide. LCMS (ESI): [M+H] 331. ¹HNMR: (500 MHz, CDCl₃) δ 11.79 (br s, 1H), 8.54 (dd, *J*=1.8, 4.9 Hz, 1H), 7.59 (dt, *J*=1.8, 7.6 Hz, 1H), 7.19 (s, 1H), 7.14 (d, *J*=7.9 Hz, 1H), 7.13 - 7.09 (m, 1H), 3.75 - 3.56 (m, 2H), 2.89 - 2.76 (m, 2H), 2.76 - 2.63 (m, 2H), 2.32 (s, 3H), 2.12 (m, 1H), 2.08 - 2.01 (m, 1H), 1.90 (br t, *J*=10.4 Hz, 1H), 1.78 - 1.62 (m, 2H), 1.60 - 1.47 (m, 1H), 1.12 - 0.96 (m, 1H).

### Example 1-28 (Reference)

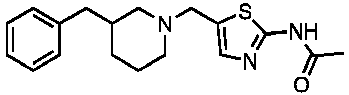

**N-(5-((3-benzylpiperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, bromobenzene, and N-[5-(chloromethyl)thiazol-2-yl]acetamide. LCMS (ESI): [M+H] 330. ¹HNMR: (500 MHz, CDCl₃) δ 11.61 (br s, 1H), 7.32 - 7.24 (m, 2H), 7.22 - 7.08 (m, 4H), 3.73 - 3.58 (m, 2H), 2.80 (br d, *J*=7.9 Hz, 2H), 2.60 - 2.51 (m, 1H), 2.50 - 2.42 (m, 1H), 2.31 (s, 3H), 1.99 (br t, *J*=10.4 Hz, 1H), 1.93 - 1.86 (m, 1H), 1.85 - 1.79 (m, 1H), 1.71 - 1.62 (m, 2H), 1.56 - 1.45 (m, 1H), 1.00 - 0.87 (m, 1H).

### Example 1-29 (Reference)

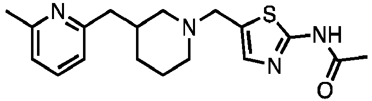

### N-(5-((3-((6-methylpyridin-2-yl)methyl)piperidin-1-yl)methyl)thiazol-2-yl)acetamide

The title compound was prepared in an analogous manner of that in scheme 1 from *tert-butyl* 3-((4,4,5,5-tetramethyl-1,3,2-dioxaborolan-2-yl)methylene)piperidine-1-carboxylate, 2-bromo-6-methylpyridine, and N-[5-(chloromethyl)thiazol-2-yl]acetamide. LCMS (ESI): [M+H] 345. ¹HNMR: (500 MHz, CDCl₃) δ 12.11 (br s, 1H), 7.46 (t, *J*=7.6 Hz, 1H), 7.17 (s, 1H), 6.95 (d, *J*=7.3 Hz, 1H), 6.91 (d, *J*=7.3 Hz, 1H), 3.64 (q, *J*=14.0 Hz, 2H), 2.79 (br d, *J*=8.5 Hz, 2H), 2.71 - 2.61 (m, 2H), 2.52 (s, 3H), 2.31 (s, 3H), 2.16 - 2.06 (m, 1H), 2.02 (br t, *J*=10.1 Hz, 1H), 1.86 (br t, *J*=10.4 Hz, 1H), 1.71 - 1.62 (m, 2H), 1.59 - 1.46 (m, 1H), 1.07 - 0.95 (m, 1H).

### Example 1-30 (Reference)

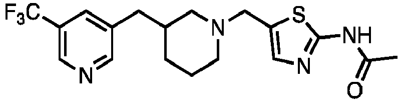

**N-(5-((3-((5-(trifluoromethyl)pyridin-3-yl)methyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 3-bromo-5-(trifluoromethyl)pyridine, and N-[5-(chloromethyl)thiazol-2-yl]acetamide. LCMS (ESI): [M+H] 399. ¹HNMR: (500 MHz, CDCl₃) δ 11.74 (br s, 1H), 8.73 (s, 1H), 8.60 (s, 1H), 7.70 (s, 1H), 7.18 (s, 1H), 3.74 - 3.58 (m, 2H), 2.76 (m, 2H), 2.71 - 2.63 (m, 1H), 2.56 (m, 1H), 2.31 (s, 3H), 2.08 (m, 1H), 1.90 (m, 2H), 1.75 - 1.60 (m, 2H), 1.59 - 1.48 (m, 1H), 1.06 - 0.95 (m, 1H).

### Example 1-31 (Reference)

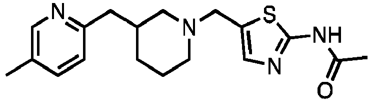

### N-(5-((3-((5-methylpyridin-2-yl)methyl)piperidin-1-yl)methyl)thiazol-2-yl)acetamide:

The title compound was prepared in an analogous manner of that in scheme 1 from tert-butyl 3-((4,4,5,5-tetramethyl-1,3,2-dioxaborolan-2-yl)methylene)piperidine-1-carboxylate, 2-chloro-5-methylpyridine, and N-[5-(chloromethyl)thiazol-2-yl]acetamide. LCMS (ESI): [M+H] 345. ¹HNMR: (500 MHz, CDCl₃) δ 11.94 (br s, 1H), 8.35 (s, 1H), 7.38 (dd, *J*=1.8, 7.9 Hz, 1H), 7.17 (s, 1H), 7.02 (d, *J*=7.9 Hz, 1H), 3.72 - 3.56 (m, 2H), 2.84 - 2.73 (m, 2H), 2.65 (dq, *J*=7.3, 13.6 Hz, 2H), 2.31 (s, 3H), 2.29 (s, 3H), 2.14 - 1.97 (m, 2H), 1.87 (br t, *J*=10.4 Hz, 1H), 1.70 - 1.61 (m, 2H), 1.58 - 1.46 (m, 1H), 1.06 - 0.95 (m, 1H).

### Example 1-32 (Reference)

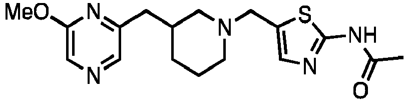

### N-(5-((3-((6-methoxypyrazin-2-yl)methyl)piperidin-1-yl)methyl)thiazol-2-yl)acetamide:

The title compound was prepared in an analogous manner of that in scheme 1 from tert-butyl 3-((4,4,5,5-tetramethyl-1,3,2-dioxaborolan-2-yl)methylene)piperidine-1-carboxylate, 2-bromo-6-methoxypyrazine, and N-[5-(chloromethyl)thiazol-2-yl]acetamide. LCMS (ESI): [M+H] 361.

### Example 1-33 (Reference)

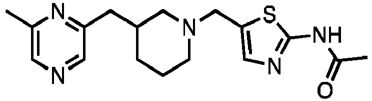

### N-(5-((3-((6-methylpyrazin-2-yl)methyl)piperidin-1-yl)methyl)thiazol-2-yl)acetamide:

The title compound was prepared in an analogous manner of that in scheme 1 from tert-butyl 3-((4,4,5,5-tetramethyl-1,3,2-dioxaborolan-2-yl)methylene)piperidine-1-carboxylate, 2-bromo-6-methylpyrazine, and N-[5-(chloromethyl)thiazol-2-yl]acetamide. LCMS (ESI): [M+H] 346. ¹HNMR: (500 MHz, CDCl₃) δ 11.80 (br s, 1H), 8.27 (s, 1H), 8.21 (s, 1H), 7.18 (s, 1H), 3.70 - 3.59 (m, 2H), 2.78 (br s, 2H), 2.67 (m, 2H), 2.54 (s, 3H), 2.31 (s, 3H), 2.18 - 2.00 (m, 2H), 1.94 - 1.85 (m, 1H), 1.65 (m, 2H), 1.54 (m, 1H), 1.13 - 0.96 (m, 1H).

### Example 1-34 (Reference)

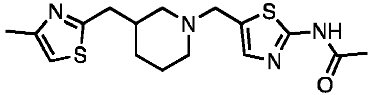

### N-(5-((3-((4-methylthiazol-2-yl)methyl)piperidin-1-yl)methyl)thiazol-2-yl)acetamide:

The title compound was prepared in an analogous manner of that in scheme 1 from tert-butyl 3-((4,4,5,5-tetramethyl-1,3,2-dioxaborolan-2-yl)methylene)piperidine-1-carboxylate, 2-bromo-4-methylthiazole, and N-[5-(chloromethyl)thiazol-2-yl]acetamide. LCMS (ESI): [M+H] 351. ¹HNMR: (500 MHz, Methanol-*d*4) δ 7.54 (s, 1H), 7.04 (s, 1H), 4.88-4.99 (m, 1H), 4.46-4.56 (m, 2H), 4.41 (s, 1H), 3.51 (br d, *J*=11.0 Hz, 2H), 2.84-3.05 (m, 3H), 2.74 (br t, *J*=12.2 Hz, 1H), 2.30-2.42 (m, 3H), 2.18-2.25 (m, 4H), 1.92-2.06 (m, 1H), 1.87 (br d, *J*=14.0 Hz, 1H), 1.73 (br d, *J*=13.4 Hz, 1H), 1.22-1.39 (m, 1H).

### Example 1-35 (Reference)

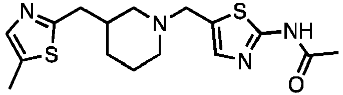

### N-(5-((3-((5-methylthiazol-2-yl)methyl)piperidin-1-yl)methyl)thiazol-2-yl)acetamide:

The title compound was prepared in an analogous manner of that in scheme 1 from tert-butyl 3-((4,4,5,5-tetramethyl-1,3,2-dioxaborolan-2-yl)methylene)piperidine-1-carboxylate, 2-bromo-5-methylthiazole, and N-[5-(chloromethyl)thiazol-2-yl]acetamide. LCMS (ESI): [M+H] 351. ¹HNMR: (500 MHz, Methanol-*d*4) δ 7.57 (s, 1H), 7.40-7.46 (m, 1H), 4.94-5.04 (m, 1H), 4.53 (s, 2H), 3.53 (br d, *J*=10.4 Hz, 2H), 2.95-3.06 (m, 2H), 2.90 (br s, 1H), 2.77 (br t, *J*=11.9 Hz, 1H), 2.45 (d, *J*=1.2 Hz, 3H), 2.18-2.29 (m, 4H), 1.95-2.10 (m, 1H), 1.83-1.94 (m, 1H), 1.75 (br d, *J*=13.4 Hz, 1H), 1.42 (s, 1H), 1.21-1.37 (m, 1H).

### Example 1-36 (Reference)

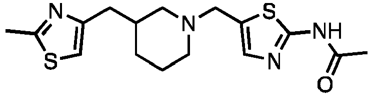

### N-(5-((3-((2-methylthiazol-4-yl)methyl)piperidin-1-yl)methyl)thiazol-2-yl)acetamide:

The title compound was prepared in an analogous manner of that in scheme 1 from *tert-butyl* 3-((4,4,5,5-tetramethyl-1,3,2-dioxaborolan-2-yl)methylene)piperidine-1-carboxylate, 4-bromo-2-methylthiazole, and N-[5-(chloromethyl)thiazol-2-yl]acetamide. LCMS (ESI): [M+H] 351. ¹HNMR: (500 MHz, Methanol-*d*4) δ 7.57 (s, 1H), 7.30 (s, 1H), 4.52 (br s, 2H),3.44-3.56 (m, 2H), 2.86-2.93 (m, 1H), 2.70-2.82 (m, 6H), 2.18-2.23 (m, 4H), 1.94-2.03 (m, 1H), 1.86 (br d, *J*=13.4 Hz, 1H), 1.76 (br d, *J*=12.8 Hz, 1H), 1.20-1.32 (m, 1H).

### Example 1-37 (Reference)

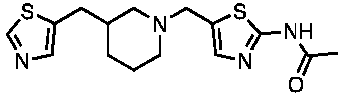

**N-(5-((3-(thiazol-5-ylmethyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 5-bromothiazole, and N-[5-(chloromethyl)thiazol-2-yl]acetamide. LCMS (ESI): [M+H] 337. ¹HNMR: (500 MHz, DMSO-*d*6) δ 11.93 (s, 1H), 8.91 (s, 1H), 7.61 (s, 1H), 7.22 (s, 1H), 4.04 (s, 6H), 3.50-3.64 (m, 3H), 2.64-2.82 (m, 5H), 2.52-2.56 (m, 1H), 2.11 (s, 4H), 1.91-2.00 (m, 1H), 1.69-1.80 (m, 2H), 1.57-1.65 (m, 2H), 1.36-1.44 (m, 1H), 0.94 (br d, J=9.8 Hz, 1H).

### Example 1-38 (Reference)

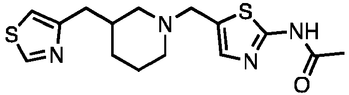

**N-(5-((3-(thiazol-4-ylmethyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 4-bromothiazole, and N-[5-(chloromethyl)thiazol-2-yl]acetamide. LCMS (ESI): [M+H] 337. ¹HNMR: (500 MHz, DMSO-*d*6) δ 11.92 (s, 1H), 8.99 (d, *J*=1.8 Hz, 1H), 7.30 (d, *J*=1.8 Hz, 1H), 7.21 (s, 1H), 4.04 (s, 5H), 3.49-3.63 (m, 2H), 2.59-2.74 (m, 5H), 2.11 (s, 4H), 1.87-1.99 (m, 2H), 1.75 (br t, *J*=10.4 Hz, 1H), 1.58 (br d, *J*=10.4 Hz, 2H), 1.34-1.44 (m, 1H), 0.92 (br d, *J*=9.8 Hz, 1H).

### Example 1-39 (Reference)

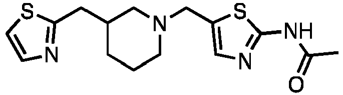

**N-(5-((3-(thiazol-2-ylmethyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 2-bromothiazole, and N-[5-(chloromethyl)thiazol-2-yl]acetamide. LCMS (ESI): [M+H] 337. ¹HNMR: (500 MHz, Methanol-*d*4) δ 7.66 (d, *J*=3.7 Hz, 1H), 7.44 (d, *J*=3.7 Hz, 1H), 7.21 (s, 1H), 3.67 (s, 2H), 2.96 (d, *J*=7.3 Hz, 2H), 2.83 (br t, *J*=11.6 Hz, 2H), 2.15-2.24 (m, 4H), 2.01-2.12 (m, 2H), 1.88 (br t, *J*=10.4 Hz, 1H), 1.68-1.81 (m, 2H), 1.52-1.63 (m, 1H), 1.07 (br dd, *J*=11.6, 2.4 Hz, 1H).

### Example 1-40 (Reference)

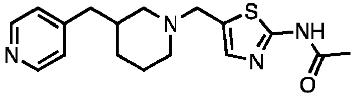

### N-(5-((3-(pyridin-4-ylmethyl)piperidin-1-yl)methyl)thiazol-2-yl)acetamide

The title compound was prepared in an analogous manner of that in scheme 1 from tert-butyl 3-((4,4,5,5-tetramethyl-1,3,2-dioxaborolan-2-yl)methylene)piperidine-1-carboxylate, 4-bromopyridine, and N-[5-(chloromethyl)thiazol-2-yl]acetamide. LCMS (ESI): [M+H] 331. ¹HNMR: (500 MHz, Methanol-*d*4) δ 8.38 (br d, *J*=5.5 Hz, 2H), 7.23-7.29 (m, 2H), 7.20 (s, 1H), 3.66 (s, 2H), 2.69-2.87 (m, 2H), 2.49-2.68 (m, 2H), 2.21 (s, 3H), 2.00-2.12 (m, 2H), 1.93 (td, J=7.0, 3.1 Hz, 1H), 1.76-1.87 (m, 1H), 1.64-1.76 (m, 2H), 1.46-1.62 (m, 1H), 1.03 (br d, J=9.8 Hz, 1H).

### Example 1-41 (Reference)

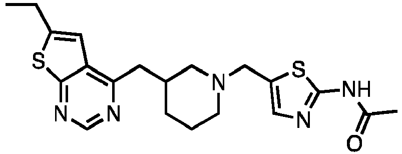

***N*-(5-((3-((6-ethylthieno[2,3-*d*]pyrimidin-4-yl)methyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 4-chloro-6-ethylthieno[2,3-d]pyrimidine, and N-[5-(chloromethyl)thiazol-2-yl]acetamide. LCMS (ESI): [M+H] 416. ¹HNMR: (500 MHz, Methanol-*d*4) δ 8.81-8.90 (m, 1H), 7.55 (s, 1H), 7.32 (dd, *J*=2.4, 1.2 Hz, 1H), 4.51 (br d, *J*=6.1 Hz, 2H), 3.53 (br d, *J*=11.6 Hz, 2H), 3.02-3.20 (m, 4H), 2.92 (br s, 1H), 2.79-2.88 (m, 1H), 2.45 (br s, 1H), 2.23 (s, 3H), 1.96-2.10 (m, 1H), 1.71-1.94 (m, 2H), 1.34-1.44 (m, 5H), 1.20-1.33 (m, 1H).

### Example 1-42 (Reference)

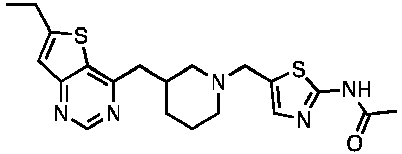

***N*-(5-((3-((6-ethylthieno[3,2-*d*]pyrimidin-4-yl)methyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 4-chloro-6-ethylthieno[3,2-d]pyrimidine, and N-[5-(chloromethyl)thiazol-2-yl]acetamide. LCMS (ESI): [M+H] 416. ¹HNMR: (500 MHz, Methanol-*d*4) δ 8.96-9.00 (m, 1H), 7.51-7.61 (m, 1H), 7.31-7.36 (m, 1H), 4.90-4.94 (m, 2H), 4.48-4.55 (m, 2H), 3.01-3.18 (m, 4H), 2.83-2.97 (m, 2H), 2.75-2.83 (m, 1H), 2.21-2.24 (m, 4H), 1.95-2.07 (m, 1H), 1.90 (br d, *J*=13.4 Hz, 1H), 1.68-1.84 (m, 2H), 1.35-1.50 (m, 5H), 0.96 (t, *J*=7.3 Hz, 1H).

### Example 1-43 (Reference)

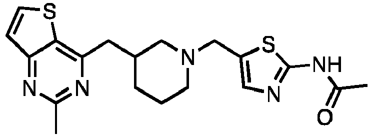

***N*-(5-((3-((2-methylthieno[3,2-dlpyrimidin-4-yl)methyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 4-chloro-2-methylthieno[3,2-*d*]pyrimidine, and N-[5-(chloromethyl)thiazol-2-yl]acetamide. LCMS (ESI): [M+H] 402. ¹HNMR: (500 MHz, Methanol-*d*4) δ 8.33 (d, *J=5.5* Hz, 1H), 7.56 (s, 1H), 7.51 (d, *J*=5.5 Hz, 1H), 4.56 (br d, *J*=14.0 Hz, 1H), 4.49 (d, *J*=14.0 Hz, 1H), 3.60-3.74 (m, 1H), 3.52-3.59 (m, 1H), 3.11-3.18 (m, 1H), 3.03-3.11 (m, 1H), 2.91-3.01 (m, 1H), 2.79-2.90 (m, 2H), 2.74 (s, 3H), 2.66 (s, 3H), 2.52-2.62 (m, 1H), 2.23 (s, 4H).

### Example 1-44 (Reference)

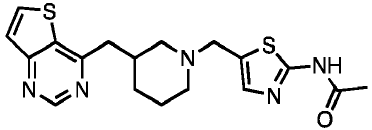

***N*-(5-((3-(thieno[3,2-*d*]pyrimidin-4-ylmethyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 4-chlorothieno[3,2-d]pyrimidine, and N-[5-(chloromethyl)thiazol-2-yl]acetamide. LCMS (ESI): [M+H] 388. ¹HNMR: (500 MHz, Methanol-*d*4) δ 9.06 (s, 1H), 8.35 (d, *J*=5.5 Hz, 1H), 7.51-7.63 (m, 3H), 2.63-2.67 (m, 2H), 2.53 (s, 1H), 2.23-2.24 (m, 3H), 1.97-2.11 (m, 2H), 1.91 (br d, *J*=14.0 Hz, 2H), 1.78 (br d, *J*=14.0 Hz, 1H), 1.38-1.45 (m, 5H).

### Example 1-45 (Reference)

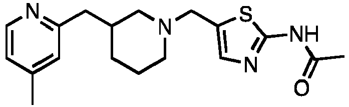

### N-(5-((3-((4-methylpyridin-2-yl)methyl)piperidin-1-yl)methyl)thiazol-2-yl)acetamide:

The title compound was prepared in an analogous manner of that in scheme 1 from tert-butyl 3-((4,4,5,5-tetramethyl-1,3,2-dioxaborolan-2-yl)methylene)piperidine-1-carboxylate, 2-bromo-4-methylpyridine, and N-[5-(chloromethyl)thiazol-2-yl]acetamide. LCMS (ESI): [M+H] 345. ¹HNMR: (500 MHz, Methanol-*d*4) δ 8.60 (d, *J*=6.1 Hz, 1H), 7.77-7.83 (m, 2H), 7.59 (s, 1H), 3.42-3.59 (m, 2H), 2.90-3.09 (m, 4H), 2.83-2.88 (m, 1H), 2.66 (d, *J*=1.8 Hz, 4H), 2.20-2.25 (m, 3H), 2.00 (br d, *J*=12.8 Hz, 1H), 1.81 (s, 1H), 1.79 (br s, 1H), 1.24-1.44 (m, 1H).

### Example 1-46 (Reference)

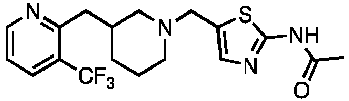

**N-(5-((3-((3-(trifluoromethyl)pyridin-2-yl)methyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 2-bromo-3-(trifluoromethyl)pyridine, and N-[5-(chloromethyl)thiazol-2-yl]acetamide. LCMS (ESI): [M+H] 399. ¹HNMR: (500 MHz, Methanol-*d*4) δ 8.74 (br dd, *J*=7.9, 4.9 Hz, 1H), 8.11 (t, *J*=6.4 Hz, 1H), 7.40-7.51 (m, 2H), 3.48-3.61 (m, 3H), 2.79-3.05 (m, 6H), 2.18-2.28 (m, 2H), 1.94-2.10 (m, 1H), 1.87 (s, 1H), 1.84 (br s, 1H), 1.72-1.82 (m, 1H), 1.28-1.43 (m, 1H).

### Example 1-47 (Reference)

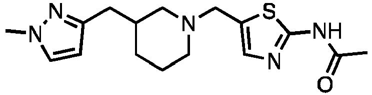

***N*-(5-((3-((1-methyl-1*H*-pyrazol-3-yl)methyl)piperidin-1-yl)methyl)thiazol-2-yl)acetamide:** The title compound was prepared in an analogous manner of that in scheme 1 from tert-butyl 3-((4,4,5,5-tetramethyl-1,3,2-dioxaborolan-2-yl)methylene)piperidine-1-carboxylate, 3-bromo-1-methyl-1H-pyrazole, and N-[5-(chloromethyl)thiazol-2-yl]acetamide. LCMS (ESI): [M+H] 334. ¹HNMR: (500 MHz, Methanol-*d*4) δ 7.42 (d, *J*=1.8 Hz, 1H), 7.22 (s, 1H), 6.04 (d, *J*=1.8 Hz, 1H), 3.80 (s, 3H), 3.67 (s, 2H), 2.86 (br d, *J*=7.9 Hz, 2H), 2.43-2.53 (m, 2H), 2.18-2.22 (m, 3H), 1.97-2.07 (m, 1H), 1.82-1.92 (m, 1H), 1.66-1.79 (m, 3H), 1.51-1.60 (m, 1H), 0.97 (br dd, *J*=11.9, 3.4 Hz, 1H). 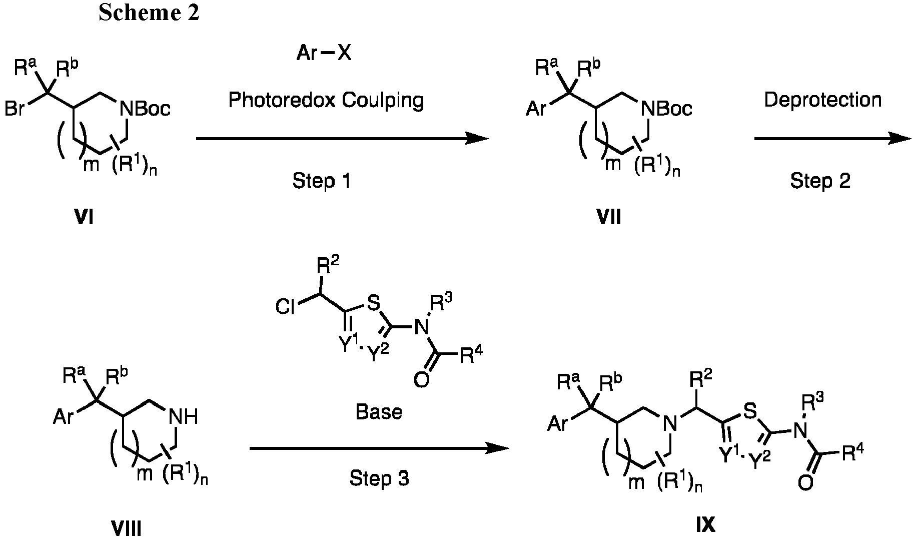 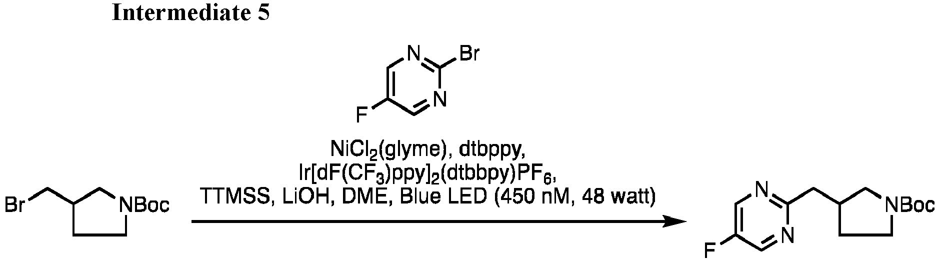 ***tert-butyl* 3-((5-fluoropyrimidin-2-yl)methyl)pyrrolidine-1-carboxylate:** A solution of NiCl₂(glyme) (0.084 g, 0.384 mmol), 4-*tert*-butyl-2-(4-*tert*-butyl-2-pyridyl)pyridine, and Ir{dF(CF₃)ppy}₂(dtbpy)PF₆ (0.086 g, 0.077 mmol) in DME (80 mL) was sparged with N₂ for 15 min. The nickel solution was added to a mixture of tert-butyl 3-(bromomethyl)pyrrolidine-1-carboxylate (2.03 g, 7.68 mmol), 2-bromo-5-fluoro-pyrimidine (1.70 g, 9.61 mmol), tris(trimethylsilyl)silane (2.87 g, 11.5 mmol, 3.54 mL), and lithium hydroxide (0.736 mg, 30.7 mmol). After the mixture was sparged with N₂ (15 min), the reaction was irradiated with blue LEDs (48 watts 450 hv) overnight at 40 °C. Celite was added to the reaction, and the mixture was filtered and concentrated *in vacuo.* The residue was purified over SiO₂ (0-100% EtOAc:heptane) to afford the title compound (0.760 g). LCMS (ESI): [M - t-Bu] 226. ¹HNMR: (500 MHz, CDCl₃) δ 8.39 (br d, *J*=9.46 Hz, 2 H), 3.30 - 3.46 (m, 2 H), 3.10 - 3.21 (m, 1 H), 2.85 - 2.96 (m, 3 H), 2.54 - 2.66 (m, 1 H), 1.80 - 1.91 (m, 1 H), 1.42 - 1.57 (m, 1 H), 1.27 - 1.35 (m, 9 H). 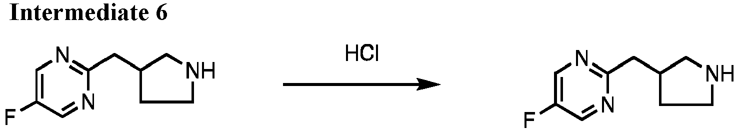 **5-fluoro-2-(pyrrolidin-3-ylmethyl)pyrimidine hydrochloride:** A solution of HCl in dioxane (4.0 M, 6.75 mL, 27.0 mmol) was added to a solution of tert-butyl 3-[(5-fluoropyrimidin-2-yl)methyl]pyrrolidine-1-carboxylate (0.760 g, 2.70 mmol) in CH₂Cl₂ (40.0 mL). After 2 h, the solution was decanted off. The residue was dissolved in MeOH and concentrated *in vacuo* to afford the title compound. LCMS (ESI): [M+H] 182.

### Examples 2-1 and 2-2

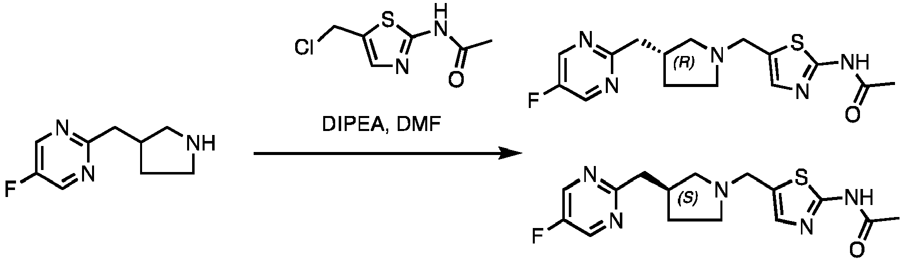

**(*R*)*-N-*(5-((3-((5-fluoropyrimidin-2-yl)methyl)pyrrolidin-1-yl)methyl)thiazol-2-yl)acetamide** and **(S)-N-(5-((3-((5-fluoropyrimidin-2-yl)methyl)pyrrolidin-1-yl)methyl)thiazol-2-yl)acetamide:** Absolute configuration assigned arbitrarily. N-[5-(chloromethyl)thiazol-2-yl]acetamide (0.428 g, 2.25 mmol) was added to a solution of 5-fluoro-2-(pyrrolidin-3-ylmethyl)pyrimidine hydrochloride (0.489 g, 2.25 mmol) and diisopropylethylamine (1.45 g, 11.2 mmol, 1.96 mL) in DMF (5.00 mL). After 2h, the mixture was concentrated *in vacuo.* The residue was dissolved in CH₂Cl₂, washed with saturated NaHCO₃ (aq) and concentrated *in vacuo.* The residue was purified over SiO₂ (40g, 0-15% CH₂Cl₂:MeOH) to afford the title compound as a racemic mixture (0.411 g, 54%). The enantiomers were separated using chiral SFC. Column: CHIRALPAK AD-H 30x250mm, 5um. Method: 35% Isopropanol w/ 0.1% diethyl amine in CO2 at 100mL/min. Peak 1: 0.137 g. LCMS: [M+H] 336. ¹HNMR: (500 MHz, CDCl₃) δ 12.07 (br s, 1 H), 8.48 - 8.59 (m, 2 H), 7.22 (s, 1 H), 3.79 (s, 2 H), 3.05 (d, *J*=7.32 Hz, 2 H), 2.74 - 2.88 (m, 2 H), 2.67 - 2.74 (m, 1 H), 2.58 - 2.64 (m, 1 H), 2.35 (dd, *J*=8.39, 6.56 Hz, 1 H), 2.32 (s, 3 H), 1.98 - 2.07 (m, 1 H), 1.61 (ddt, *J*=12.63, 8.16, 6.16, 6.16 Hz, 1 H).8

Peak 2. 0.124 g. LCMS: [M+H] 336. ¹HNMR: (500 MHz, CDCl₃) δ 11.56 (br s, 1 H), 8.53 (s, 2 H), 7.22 (s, 1 H), 3.79 (s, 2 H), 3.05 (d, *J*=7.17 Hz, 2 H), 2.75 - 2.87 (m, 2 H), 2.68 - 2.74 (m, 1 H), 2.60 (td, *J*=8.58, 6.03 Hz, 1 H), 2.32 - 2.37 (m, 1 H), 2.32 (s, 3 H), 1.99 - 2.08 (m, 1 H), 1.57 - 1.65 (m, 1 H).

### Example 2-3 (Reference)

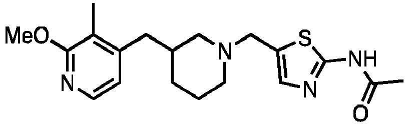

***N*-(5-((3-((2-methoxy-3-methylpyridin-4-yl)methyl)piperidin-1-yl)methyl)thiazol-2-yl)acetamide:** The title compound was prepared in an analogous manner of that in scheme 2 from tert-butyl 3-(bromomethyl)piperidine-1-carboxylate, 4-bromo-2-methoxy-3-methylpyridine, and N-[5-(chloromethyl)thiazol-2-yl]acetamide. LCMS (ESI): [M+H] 376. ¹HNMR: (500 MHz, CDCl₃) δ 7.79 (d, *J*=5.49 Hz, 1H), 7.21 (s, 1H), 6.70 (d, *J*=4.88 Hz, 1H), 3.89 (s, 3H), 3.67 (s, 1H), 3.60-3.70 (m, 1H), 2.71-2.89 (m, 2H), 2.47-2.60 (m, 2H), 2.20 (s, 3H), 2.12 (s, 3H), 2.06 (br t, *J*=11.29 Hz, 1H), 1.79-1.93 (m, 2H), 1.62-1.75 (m, 2H), 1.46-1.60 (m, 1H), 1.46-1.60 (m, 1H), 0.94-1.12 (m, 1H).

### Example 2-4

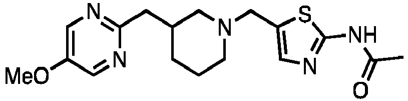

***N*-(5-((3-((5-methoxypyrimidin-2-yl)methyl)piperidin-1-yl)methyl)thiazol-2-yl)acetamide:** The title compound was prepared in an analogous manner of that in scheme 2 from tert-butyl 3-(bromomethyl)piperidine-1-carboxylate, 2-bromo-5-methoxypyrimidine, and N-[5-(chloromethyl)thiazol-2-yl]acetamide. LCMS (ESI): [M+H] 362. ¹HNMR: (500 MHz, CDCl₃) δ 11.71 (br s, 1H), 8.33 (s, 2H), 7.18 (s, 1H), 3.90 (s, 3H), 3.73 - 3.56 (m, 2H), 2.95 - 2.70 (m, 4H), 2.30 (s, 3H), 2.27 - 2.16 (m, 1H), 2.06 - 1.93 (m, 1H), 1.93 - 1.83 (m, 1H), 1.73 - 1.62 (m, 2H), 1.56 (m, 1H), 1.11 - 0.94 (m, 1H).

### Example 2-5 (Reference)

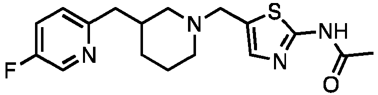

### N-(5-((3-((5-fluoropyridin-2-yl)methyl)piperidin-1-yl)methyl)thiazol-2-yl)acetamide:

The title compound was prepared in an analogous manner of that in scheme 2 from *tert-butyl* 3-(bromomethyl)piperidine-1-carboxylate, 2-bromo-5-fluoropyridine, and N-[5-(chloromethyl)thiazol-2-yl]acetamide. LCMS (ESI): [M+H] 349. ¹HNMR: (500 MHz, CDCl₃) δ 12.58 (br s, 1H), 8.44 (d, *J*=3.1 Hz, 1H), 7.58 (br d, *J*=11.0 Hz, 1H), 7.51 (dt, *J*=3.1, 8.2 Hz, 1H), 7.29 (dd, *J*=4.3, 9.2 Hz, 1H), 4.52 - 4.25 (m, 2H), 3.70 - 3.43 (m, 2H), 2.92 - 2.75 (m, 1H), 2.63 - 2.41 (m, 2H), 2.36 (s, 3H), 2.24 (m, 1H), 2.18 - 2.05 (m, 1H), 2.05 - 1.83 (m, 3H), 1.34 - 1.13 (m, 1H).

### Example 2-6

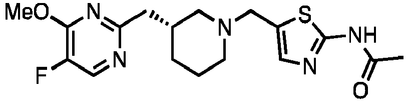

**(*R*)*-N-*(5-((3-((5-fluoro-4-methoxypyrimidin-2-yl)methyl)piperidin-1-yl)methyl)thiazol-2-yl)acetamide:** The title compound was prepared in an analogous manner of that in scheme 2 from *tert-butyl* (S)-3-(bromomethyl)piperidine-1-carboxylate, 2-chloro-5-fluoro-4-methoxypyrimidine and N-[5-(chloromethyl)thiazol-2-yl]acetamide. LCMS (ESI): [M+H] 380.

### Example 2-7

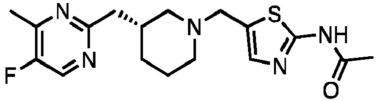

**(*R*)*-N-*(5-((3-((5-fluoro-4-methylpyrimidin-2-yl)methyl)piperidin-1-yl)methyl)thiazol-2-yl)acetamide:** The title compound was prepared in an analogous manner of that in scheme 2 from tert-butyl (S)-3-(bromomethyl)piperidine-1-carboxylate, 2-chloro-5-fluoro-4-methylpyrimidine and N-[5-(chloromethyl)thiazol-2-yl]acetamide. LCMS (ESI): [M+H] 381.

### Example 2-8 (Reference)

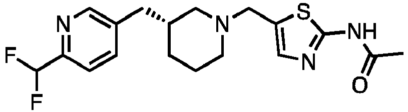

***(R)-N-(5-((3-((6-(difluoromethyl)pyridin-3-yl)methyl)piperidin-1-yl)methyl)thiazol-2-*yl)acetamide:** The title compound was prepared in an analogous manner of that in scheme 2 from tert-butyl (S)-3-(bromomethyl)piperidine-1-carboxylate, 5-bromo-2-(difluoromethyl)pyridine and *N*-[5-(chloromethyl)thiazol-2-yl]acetamide. LCMS (ESI): [M+H] 381. ¹HNMR: (500 MHz, CDCl₃) δ 11.58 (br s, 1H), 8.43 (d, *J*=1.8 Hz, 1H), 7.62 (dd, *J*=2.4, 7.9 Hz, 1H), 7.58 - 7.53 (m, 1H), 7.18 (s, 1H), 6.63 (t, *J*=55.5 Hz, 1H), 3.71 - 3.57 (m, 2H), 2.75 (br d, *J*=7.9 Hz, 2H), 2.64 (dd, *J*=7.6, 13.7 Hz, 1H), 2.54 (dd, *J*=7.6, 13.7 Hz, 1H), 2.31 (s, 3H), 2.06 (br t, *J*=10.1 Hz, 1H), 1.98 - 1.79 (m, 2H), 1.73 - 1.58 (m, 2H), 1.56 - 1.46 (m, 1H), 1.09 - 0.89 (m, 1H).

### Example 2-9

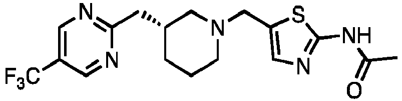

***(R)-N-*(5-((3-((5-(trifluoromethyl)pyrimidin-2-yl)methyl)piperidin-1-yl)methyl)thiazol-2-yl)acetamide:** The title compound was prepared in an analogous manner of that in scheme 2 from tert-butyl (S)-3-(bromomethyl)piperidine-1-carboxylate, 2-bromo-5-(trifluoromethyl)pyrimidine, and N-[5-(chloromethyl)thiazol-2-yl]acetamide. LCMS (ESI): [M+H] 400. ¹HNMR: (500 MHz, CDCl₃) δ 11.58 (br s, 1H), 8.90 (s, 2H), 7.18 (s, 1H), 3.72 - 3.59 (m, 2H), 3.02 - 2.91 (m, 2H), 2.80 (br t, *J*=11.3 Hz, 2H), 2.30 (s, 3H), 2.06 (br t, *J*=10.1 Hz, 1H), 1.94 (br t, *J*=10.1 Hz, 1H), 1.74 - 1.65 (m, 2H), 1.64 - 1.52 (m, 2H), 1.15 - 1.01 (m, 1H).

### Example 2-10

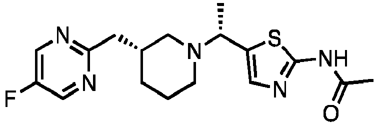

***N*-(5-((R)-1-((*R*)-3-((5-fluoropyrimidin-2-yl)methyl)piperidin-1-yl)ethyl)thiazol-2-yl)acetamide:** Relative configuration assigned arbitrarily. The title compound was prepared in an analogous manner of that in scheme 2 from *tert-butyl* (S)-3-(bromomethyl)piperidine-1-carboxylate, 2-bromo-5-(trifluoromethyl)pyrimidine, and *N*-(5-(1-chloroethyl)thiazol-2-yl)acetamide. The chiral separation method: Column: CHIRALPAK IA 30x250mm, 5um; Method: 40% Ethanol with 0.1% diethyl amine in CO₂ (flow rate: 100mL/min), ABPR 120bar, MBPR 40psi. The product was peak 1. LCMS (ESI): [M+H] 364. ¹HNMR: (500 MHz, CDCl₃) δ 11.34 (br s, 1H), 8.53 (s, 2H), 7.10 (s, 1H), 3.91 (q, *J*=6.7 Hz, 1H), 2.91 - 2.79 (m, 3H), 2.70 (m, 1H), 2.30 (s, 3H), 2.27 - 2.18 (m, 1H), 2.14 - 2.01 (m, 2H), 1.71 - 1.50 (m, 4H), 1.39 (d, *J*=6.7 Hz, 3H), 1.08 - 0.96 (m, 1H).

### Example 2-11

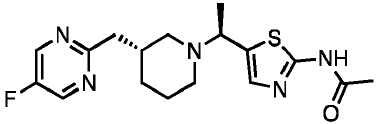

***N*-(5-((S)-1-((*R*)-3-((5-fluoropyrimidin-2-yl)methyl)piperidin-1-yl)ethyl)thiazol-2-yl)acetamide:** Relative configuration assigned arbitrarily. The chiral separation method: Column: CHIRALPAK IA 30x250mm, 5um; Method: 40% Ethanol with 0.1% diethyl amine in CO₂ (flow rate: 100mL/min), ABPR 120bar, MBPR 40psi. The product was peak 2. LCMS (ESI): [M+H] 364.

### Example 2-12

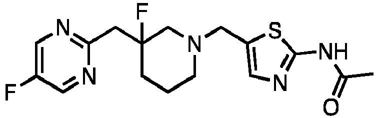

***N*-(5-((3-fluoro-3-((5-fluoropyrimidin-2-yl)methyl)piperidin-1-yl)methyl)thiazol-2-yl)acetamide:** The title compound was prepared in an analogous manner of that in scheme 2 from tert-butyl 3-(bromomethyl)-3-fluoropiperidine-1-carboxylate, 2-bromo-5-fluoropyrimidine, and N-[5-(chloromethyl)thiazol-2-yl]acetamide. LCMS (ESI): [M+H] 368. ¹HNMR: (500 MHz, CDCl₃) δ 12.24 (s, 1 H), 8.56 (s, 2 H), 7.23 (s, 1 H), 3.70 - 3.86 (m, 2 H), 3.30 - 3.52 (m, 2 H), 2.61 - 2.75 (m, 2 H), 2.51 (br s, 2 H), 1.80 - 1.89 (m, 1 H), 1.67 - 1.79 (m, 3 H).

### Example 2-13

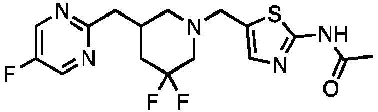

**N-(5-((3,3-difluoro-5-((5-fluoropyrimidin-2-yl)methyl)piperidin-1-yl)methyl)thiazol-2-yl)acetamide:** The title compound was prepared in an analogous manner of that in scheme 2 from tert-butyl 5-(bromomethyl)-3,3-difluoropiperidine-1-carboxylate, 2-bromo-5-fluoropyrimidine, and *N*-[5-(chloromethyl)thiazol-2-yl]acetamide. LCMS (ESI): [M+H] 386. ¹HNMR: (500 MHz, CDCl₃) δ 12.23 (br s, 1 H), 8.54 (s, 2 H), 7.22 (s, 1 H), 3.72 - 3.88 (m, 2 H), 2.98 - 3.08 (m, 2 H), 2.91 - 2.95 (m, 2 H), 2.52 - 2.65 (m, 1 H), 2.34 - 2.43 (m, 1 H), 2.11 - 2.21 (m, 1 H), 2.07 (t, *J*=10.83 Hz, 1 H), 1.45 - 1.61 (m, 1 H).

### Example 2-14

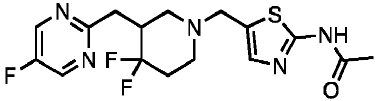

***N*-(5-((4,4-difluoro-3-((5-fluoropyrimidin-2-yl)methyl)piperidin-1-yl)methyl)thiazol-2-yl)acetamide:** The title compound was prepared in an analogous manner of that in scheme 2 from tert-butyl 3-(bromomethyl)-4,4-difluoropiperidine-1-carboxylate, 2-bromo-5-fluoropyrimidine, and N-[5-(chloromethyl)thiazol-2-yl]acetamide. LCMS (ESI): [M+H] 386. ¹HNMR: (500 MHz, CDCl₃) δ 12.25 (br s, 1 H), 8.32 - 8.35 (m, 1 H), 8.33 (s, 1 H), 6.99 (s, 1 H), 3.51 - 3.57 (m, 1 H), 3.43 - 3.48 (m, 1 H), 3.19 (dd, *J*=14.96, 3.36 Hz, 1 H), 2.71 - 2.77 (m, 1 H), 2.55 - 2.68 (m, 3 H), 2.16 - 2.26 (m, 1 H), 2.13 (s, 3 H), 1.76 - 1.97 (m, 2 H).

### Example 2-15 (Reference)

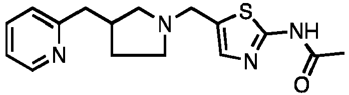

**N-(5-((3-(pyridin-2-ylmethyl)pyrrolidin-1-yl)methyl)thiazol-2-yl)acetamide:** The title compound was prepared in an analogous manner of that in scheme 2 from tert-butyl 3-(bromomethyl)pyrrolidine-1-carboxylate, 2-bromopyridine, and N-[5-(chloromethyl)thiazol-2-yl]acetamide. LCMS (ESI): [M+H] 317. ¹HNMR: (500 MHz, CDCl₃) δ 12.33 (br s, 1 H), 8.48 - 8.57 (m, 1 H), 7.59 (td, *J*=7.63, 1.83 Hz, 1 H), 7.21 (s, 1 H), 7.14 (d, *J*=7.78 Hz, 1 H), 7.11 (ddd, *J*=7.48, 4.88, 1.07 Hz, 1 H), 3.73 - 3.84 (m, 2 H), 2.87 (d, *J*=7.48 Hz, 2 H), 2.60 - 2.80 (m, 4 H), 2.33 - 2.37 (m, 1 H), 2.32 (s, 3 H), 1.96 - 2.05 (m, 1 H), 1.57 (ddt, *J*=12.66, 8.20, 6.28, 6.28 Hz, 1 H).

### Example 2-16

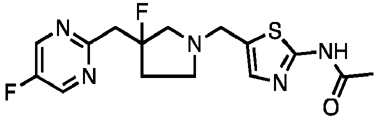

***N*-(5-((3-fluoro-3-((5-fluoropyrimidin-2-yl)methyl)pyrrolidin-1-yl)methyl)thiazol-2-yl)acetamide:** The title compound was prepared in an analogous manner of that in scheme 2 from tert-butyl 3-(bromomethyl)-3-fluoropyrrolidine-1-carboxylate, 2-bromo-5-fluoropyrimidine, and N-[5-(chloromethyl)thiazol-2-yl]acetamide. LCMS (ESI): [M+H] 354. ¹HNMR: (500 MHz, CDCl₃) δ 11.49 (br s, 1 H), 8.58 (s, 2 H), 7.23 (s, 1 H), 3.83 (d, *J*=1.83 Hz, 2 H), 3.38 - 3.53 (m, 2 H), 3.08 - 3.22 (m, 1 H), 2.79 - 2.94 (m, 2 H), 2.71 (td, *J*=8.28, 4.35 Hz, 1 H), 2.31 -234 (m, 3 H), 2.11 - 2.31 (m, 2 H).

### Example 2-17

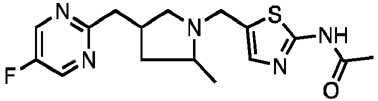

***N*-(5-((4-((5-fluoropyrimidin-2-yl)methyl)-2-methylpyrrolidin-1-yl)methyl)thiazol-2-yl)acetamide:** The title compound was prepared in an analogous manner of that in scheme 2 from tert-butyl 4-(bromomethyl)-2-methylpyrrolidine-1-carboxylate, 2-bromo-5-fluoropyrimidine, and N-[5-(chloromethyl)thiazol-2-yl]acetamide. The resulting enantiomers were separated using chiral SFC. LCMS (ESI): [M+H] 350. ¹HNMR: (400 MHz, Methanol-*d4)* δ 8.59-8.65 (m, 2H), 7.23 (d, *J*=5.52 Hz, 1H), 4.03-4.09 (m, 1H), 3.55 (t, *J*=14.56 Hz, 1H), 2.81-3.13 (m, 3H), 2.45-2.80 (m, 3H), 2.20 (d, *J*=1.00 Hz, 3H), 2.07-2.16 (m, 1H), 1.60-1.86 (m, 1H), 1.16 (dd, *J*=6.02, 8.28 Hz, 3H).

### Example 2-18

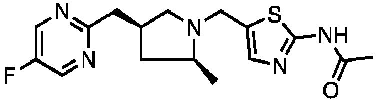

***N*-(5-(((2S,4S)-4-((5-fluoropyrimidin-2-yl)methyl)-2-methylpyrrolidin-1-yl)methyl)thiazol-2-yl)acetamide:** Relative and absolute configuration assigned arbitrarily. The chiral separation method: Column: CHIRALPAK AD-H; Method: 40% Ethanol with 0.1% diethyl amine in CO₂ (flow rate: 100mL/min), ABPR 120bar, MBPR 40psi. The product was peak 4. LCMS (ESI): [M+H] 350. ¹HNMR: (500 MHz, CDCl₃) δ 12.28 (br s, 1 H), 8.52 (s, 2 H), 7.20 (s, 1 H), 4.05 (dd, *J*=14.27, 0.99 Hz, 1 H), 3.54 (d, *J*=14.50 Hz, 1 H), 3.00 - 3.10 (m, 2 H), 2.87 (dd, *J*=9.54, 3.89 Hz, 1 H), 2.59 - 2.69 (m, 1 H), 2.49 - 2.59 (m, 2 H), 2.33 (s, 3 H), 2.10 (ddd, *J*=12.51, 8.24, 6.41 Hz, 1 H), 1.27 - 1.35 (m, 1 H), 1.17 (d, *J*=5.95 Hz, 3 H).

### Example 2-19

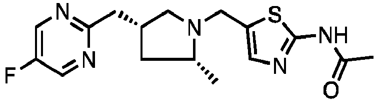

***N*-(5-(((2R,4R)-4-((5-fluoropyrimidin-2-yl)methyl)-2-methylpyrrolidin-1-yl)methyl)thiazol-2-yl)acetamide:** Relative and absolute configuration assigned arbitrarily. The chiral separation method used: Column: CHIRALPAK AD-H ; Method: 40% Ethanol with 0.1% diethyl amine in CO₂ (flow rate: 100mL/min), ABPR 120bar, MBPR 40psi. The product was peak 3. LCMS (ESI): [M+H] 350. ¹HNMR: (500 MHz, CDCl₃) δ 12.32 (br s, 1 H), 8.51 (s, 2 H), 7.20 (s, 1 H), 4.06 (dd, *J*=14.19, 0.92 Hz, 1 H), 3.53 (d, *J*=14.19 Hz, 1 H), 3.14 (dd, *J*=9.00, 7.17 Hz, 1 H), 2.91 - 3.03 (m, 2 H), 2.69 - 2.80 (m, 1 H), 2.57 - 2.66 (m, 1 H), 2.32 (s, 3 H), 2.06 (t, *J*=9.00 Hz, 1 H), 1.72 - 1.79 (m, 2 H), 1.64 - 1.72 (m, 1 H), 1.15 (d, *J*=6.10 Hz, 3 H).

### Example 2-20

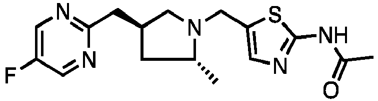

***N*-(5-(((2R,4S)-4-((5-fluoropyrimidin-2-yl)methyl)-2-methylpyrrolidin-1-yl)methyl)thiazol-2-yl)acetamide:** Relative and absolute configuration assigned arbitrarily. The chiral separation method: Column: CHIRALPAK AD-H ; Method: 40% Ethanol with 0.1% diethyl amine in CO₂ (flow rate: 100mL/min), ABPR 120bar, MBPR 40psi. The product was peak 2. LCMS (ESI): [M+H] 350. ¹HNMR: (500 MHz, CDCl₃) δ 12.50 (br s, 1 H), 8.50 (s, 2 H), 7.19 (s, 1 H), 4.05 (dd, *J*=14.11, 1.14 Hz, 1 H), 3.53 (d, *J*=14.34 Hz, 1 H), 3.14 (dd, *J*=9.08, 7.10 Hz, 1 H), 2.96 (d, J=7.02 Hz, 2 H), 2.74 (ddqd, *J*=9.92, 9.00, 7.10, 7.02, 7.02, 6.26 Hz, 1 H), 2.62 (ddq, *J*=7.78, 7.78, 6.10, 6.10, 6.10 Hz, 1 H), 2.30 - 2.34 (m, 3 H), 2.06 (t, *J*=9.00 Hz, 1 H), 1.76 (ddd, *J*=12.97, 7.78, 6.26 Hz, 1 H), 1.68 (ddd, *J*=12.82, 9.92, 7.78 Hz, 1 H), 1.15 (d, *J*=5.95 Hz, 3 H).

### Example 2-21

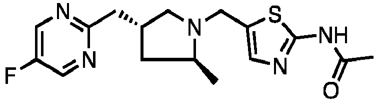

***N*-(5-(((2*S*,4*R*)-4-((5-fluoropyrimidin-2-yl)methyl)-2-methylpyrrolidin-1-yl)methyl)thiazol-2-yl)acetamide:** Relative and absolute configuration assigned arbitrarily. The chiral separation method: Column: CHIRALPAK AD-H ; Method: 40% Ethanol with 0.1% diethyl amine in CO₂ (flow rate: 100mL/min), ABPR 120bar, MBPR 40psi. The product was peak 1. LCMS (ESI): [M+H] 350. ¹HNMR: (500 MHz, CDCl₃) δ 12.46 (br s, 1 H), 8.50 (d, *J*=0.61 Hz, 2 H), 7.18 (t, *J=* 0.99 Hz, 1 H), 4.03 (dd, *J*=14.27, 1.14 Hz, 1 H), 3.53 (d, *J*=14.50 Hz, 1 H), 2.99 - 3.08 (m, 2 H), 2.57 - 2.67 (m, 1 H), 2.55 (ddq, *J*=9.46, 6.41, 6.10, 6.10, 6.10 Hz, 1 H), 2.47 - 2.52 (m, 1 H), 2.31 (s, 3 H), 2.08 (ddd, *J*=12.47, 8.28, 6.41 Hz, 1 H), 1.29 (ddd, *J*=12.66, 9.46, 7.32 Hz, 1 H), 1.15 (d, *J*=6.10 Hz, 3 H).

### Example 2-22

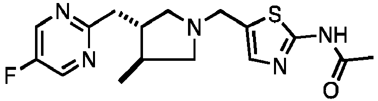

***N*-(5-(((*trans*)-3-((5-fluoropyrimidin-2-yl)methyl)-4-methylpyrrolidin-1-yl)methyl)thiazol-2-yl)acetamide:** The title compound was prepared in an analogous manner of that in scheme 2 from *tert-butyl* (trans)-3-(bromomethyl)-4-methylpyrrolidine-1-carboxylate, 2-bromo-5-fluoropyrimidine, and *N*-[5-(chloromethyl)thiazol-2-yl]acetamide. LCMS (ESI): [M+H] 350. ¹HNMR: (500 MHz, CDCl₃) δ 12.36 (br s, 1 H), 8.44 (s, 2 H), 7.12 (s, 1 H), 3.67 (q, *J*=13.89 Hz, 2 H), 3.04 (dd, *J*=13.89, 5.95 Hz, 1 H), 2.89 (dd, *J*=13.66, 9.08 Hz, 1 H), 2.79 (t, *J*=8.24 Hz, 1 H), 2.68 (t, *J*=8.62 Hz, 1 H), 2.44 (dd, *J*=9.08, 6.79 Hz, 1 H), 2.24 (s, 3 H), 2.16 - 2.22 (m, 2 H), 1.93 (spt, *J*=6.99 Hz, 1 H), 0.90 (d, *J*=6.71 Hz, 3 H).

### Example 2-23

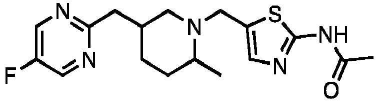

***N*-(5-((5-((5-fluoropyrimidin-2-yl)methyl)-2-methylpiperidin-1-yl)methyl)thiazol-2-yl)acetamide:** The title compound was prepared in an analogous manner of that in scheme 2 from tert-butyl 5-(bromomethyl)-2-methylpiperidine-1-carboxylate, 2-bromo-5-fluoropyrimidine, and N-[5-(chloromethyl)thiazol-2-yl]acetamide. The *cis* and *trans* diastereomers were separated over silica gel. The resulting enantiomers were separated using chiral SFC.

### Example 2-24

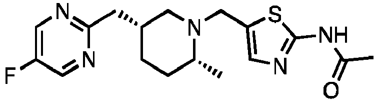

***N*-(5-(((2*R*,5*R*)-5-((5-fluoropyrimidin-2-yl)methyl)-2-methylpiperidin-1-yl)methyl)thiazol-2-yl)acetamide:** Absolute configuration assigned arbitrarily. The chiral separation method: Column: CHIRALPAK IA 30x250mm, 5um; Method: 40% Ethanol with 0.1% diethyl amine in CO₂ (flow rate: 100mL/min), ABPR 120bar, MBPR 40psi. The product was peak 1. LCMS (ESI): [M+H] 364. ¹HNMR: (400 MHz, Methanol-*d*4) δ 8.59 (d, *J*=0.75 Hz, 2H), 7.17 (s, 1H), 3.84 (dd, *J*=0.75, 14.05 Hz, 1H), 3.63 (d, *J*=14.31 Hz, 1H), 2.89-3.04 (m, 2H), 2.68-2.81 (m, 1H), 2.46-2.55 (m, 1H), 2.33-2.42 (m, 1H), 2.22-2.31 (m, 1H), 2.19 (s, 3H), 1.63-1.74 (m, 1H), 1.48-1.63 (m, 2H), 1.36-1.47 (m, 1H), 1.11 (d, *J*=6.53 Hz, 3H).

### Example 2-25

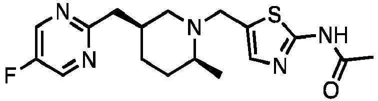

***N*-(5-(((2S,5S)-5-((5-fluoropyrimidin-2-yl)methyl)-2-methylpiperidin-1-yl)methyl)thiazol-2-yl)acetamide:** Absolute configuration assigned arbitrarily. The chiral separation method: Column: CHIRALPAK IA 30x250mm, 5um; Method: 40% Ethanol with 0.1% diethyl amine in CO₂ (flow rate: 100mL/min), ABPR 120bar, MBPR 40psi. The product was peak 2. LCMS (ESI): [M+H] 364. ¹HNMR: (400 MHz, Methanol-*d*4) δ 8.59 (d, *J*=0.75 Hz, 2H), 7.17 (s, 1H), 3.79-3.89 (m, 1H), 3.62 (d, *J*=14.31 Hz, 1H), 2.89-3.04 (m, 2H), 2.68-2.80 (m, 1H), 2.46-2.54 (m, 1H), 2.34-2.41 (m, 1H), 2.22-2.32 (m, 1H), 2.19 (s, 3H), 1.63-1.74 (m, 1H), 1.48-1.63 (m, 2H), 1.36-1.48 (m, 1H), 1.11 (d, J=6.53 Hz, 3H).

### Example 2-26

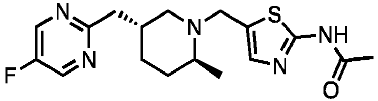

***N*-(5-(((2*S*,5*R*)-5-((5-fluoropyrimidin-2-yl)methyl)-2-methylpiperidin-1-yl)methyl)thiazol-2-yl)acetamide:** Absolute configuration assigned arbitrarily. The chiral separation method: Column: CHIRALPAK IA 30x250mm, 5um; Method: 40% Ethanol with 0.1% diethyl amine in CO₂ (flow rate: 100mL/min), ABPR 120bar, MBPR 40psi. The product was peak 1. LCMS (ESI): [M+H] 364. ¹HNMR: (400 MHz, Methanol-*d*4) δ 8.62 (d, *J*=0.75 Hz, 2H), 7.21 (s, 1H), 3.99 (d, *J*=14.81 Hz, 1H), 3.79 (d, *J*=14.81 Hz, 1H), 2.72-2.86 (m, 3H), 2.11-2.33 (m, 5H), 1.87-1.97 (m, 1H), 1.63-1.74 (m, 2H), 1.28-1.44 (m, 1H), 1.21 (d, *J*=6.02 Hz, 3H), 0.99-1.15 (m, 1H).

### Example 2-27

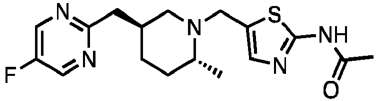

***N*-(5-(((2R,5S)-5-((5-fluoropyrimidin-2-yl)methyl)-2-methylpiperidin-1-yl)methyl)thiazol-2-yl)acetamide:** Absolute configuration assigned arbitrarily. The chiral separation method: Column: CHIRALPAK IA 30x250mm, 5um; Method: 40% Ethanol with 0.1% diethyl amine in CO₂ (flow rate: 100mL/min), ABPR 120bar, MBPR 40psi. The product was peak 2. LCMS (ESI): [M+H] 364. ¹HNMR: (400 MHz, Methanol-*d*4) δ 8.62 (s, 2H), 7.21 (s, 1H), 3.99 (d, *J*=14.81 Hz, 1H), 3.79 (d, *J*=14.81 Hz, 1H), 2.73-2.85 (m, 3H), 2.12-2.33 (m, 5H), 1.87-1.96 (m, 1H), 1.63-1.73 (m, 2H), 1.28-1.42 (m, 1H), 1.21 (d, *J*=6.27 Hz, 3H), 0.99-1.15 (m, 1H).

### Example 2-28

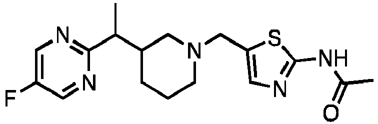

***N*-(5-((5-((5-fluoropyrimidin-2-yl)methyl)-2-methylpiperidin-1-yl)methyl)thiazol-2-yl)acetamide:** The title compound was prepared in an analogous manner of that in scheme 2 from tert-butyl 3-(1-bromoethyl)piperidine-1-carboxylate, 2-bromo-5-fluoropyrimidine, and N-[5-(chloromethyl)thiazol-2-yl]acetamide. The resulting enantiomers were separated using chiral SFC.

### Example 2-29

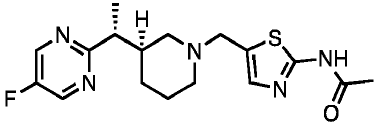

***N*-(5-(((*S*)-3-((*R*)-1-(5-fluoropyrimidin-2-yl)ethyl)piperidin-1-yl)methyl)thiazol-2-yl)acetamide:** Relative and absolute configuration assigned arbitrarily. The chiral separation method: Column: CHIRALPAK IG 30x250mm, 5um; 30% Ethanol w/ 0.1% DEA in CO₂ (flow rate: 100mL/min), ABPR 120bar, MBPR 40psi, column temperature 40 deg °C. The product was peak 1. LCMS (ESI): [M+H] 364.

### Example 2-30

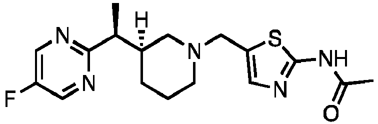

***N*-(5-(((*S*)-3-((*S*)-1-(5-fluoropyrimidin-2-yl)ethyl)piperidin-1-yl)methyl)thiazol-2-yl)acetamide:** Relative and absolute configuration assigned arbitrarily. The chiral separation method used: Column: CHIRALPAK IG 30×250mm, 5um; 30% Ethanol w/ 0.1% DEA in CO₂ (flow rate: 100mL/min), ABPR 120bar, MBPR 40psi, column temperature 40 deg °C. The product was peak 2. LCMS (ESI): [M+H] 364.

### Example 2-31

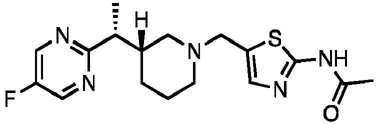

***N*-(5-(((*R*)-3-((*R*)-1-(5-fluoropyrimidin-2-yl)ethyl)piperidin-1-yl)methyl)thiazol-2-**yl)acetamide: Relative and absolute configuration assigned arbitrarily. The chiral separation method: CHIRALPAK IG 30x250mm, 5um; 30% Ethanol w/ 0.1% DEA in CO₂ (flow rate: 100mL/min), ABPR 120bar, MBPR 40psi, column temperature 40 deg °C. The product was peak 3. LCMS (ESI): [M+H] 364.

### Example 2-32

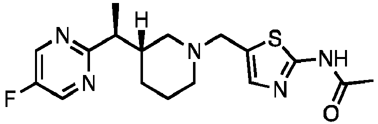

***N*-(5-(((*R*)-3-((*S*)-1-(5-fluoropyrimidin-2-yl)ethyl)piperidin-1-yl)methyl)thiazol-2-yl)acetamide:** Relative and absolute configuration assigned arbitrarily. The chiral separation method: Column: CHIRALPAK IG 30x250mm, 5um; 30% Ethanol w/ 0.1% DEA in CO₂ (flow rate: 100mL/min), ABPR 120bar, MBPR 40psi, column temperature 40 deg °C. The product was peak 4. LCMS (ESI): [M+H] 364.

### Example 2-33

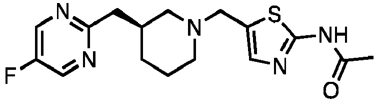

***N*-[5-[[(3*S*)-3-[(5-fluoropyrimidin-2-yl)methyl]-1-piperidyl]methyl]thiazol-2-yl]acetamide:** The title compound was prepared in an analogous manner of that in scheme 2 from *tert-butyl* (R)-3-(bromomethyl)piperidine-1-carboxylate, 2-bromo-5-fluoro-pyrimidine and *N*-[5-(chloromethyl)thiazol-2-yl]acetamide. LCMS (ESI): [M+H] 350. ¹HNMR: (500 MHz, CDCl₃) δ 12.09 (br s, 1H), 8.52 (s, 2H), 7.18 (s, 1H), 3.59-3.71 (m, 2H), 2.86 (d, *J*=6.71 Hz, 2H), 2.81 (br t, J=10.68 Hz, 2H), 2.31 (s, 3H), 2.18-2.28 (m, 1H), 1.99-2.07 (m, 1H), 1.90 (br t,*J*=10.38 Hz, 1H), 1.64-1.73 (m, 2H), 1.51-1.61 (m, 1H), 1.00-1.09 (m, 1H).

### Example 2-34

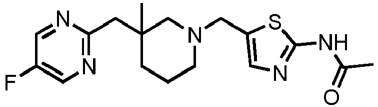

***N*-(5-((3-((5-fluoropyrimidin-2-yl)methyl)-3-methylpiperidin-1-yl)methyl)thiazol-2-yl)acetamide:** The title compound was prepared in an analogous manner of that in scheme 2 from *tert-butyl* 3-(bromomethyl)-3-methylpiperidine-1-carboxylate, 2-bromo-5-fluoropyrimidine, and *N*-(5-(chloromethyl)thiazol-2-yl)acetamide. LCMS: [M+H] 364. ¹H NMR (400 MHz, METHANOL-d₄) δ 8.57-8.63 (m, 2H), 7.17 (s, 1H), 3.85 (d, *J*=14.31 Hz, 1H), 3.59-3.70 (m, 1H), 2.89-3.03 (m, 2H), 2.74 (br s, 1H), 2.45-2.56 (m, 1H), 2.33-2.43 (m, 1H), 2.26 (ddd, *J*=3.76, 7.53, 11.29 Hz, 1H), 2.19 (s, 3H), 1.64-1.74 (m, 1H), 1.49-1.63 (m, 2H), 1.39-1.48 (m, 1H), 1.12 (d, *J*=6.53 Hz, 3H).

### Example 2-35

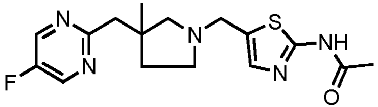

***N*-(5-((3-((5-fluoropyrimidin-2-yl)methyl)-3-methylpyrrolidin-1-yl)methyl)thiazol-2-yl)acetamide:** The title compound was prepared in an analogous manner of that in scheme 2 from *tert*-butyl 3-(bromomethyl)-3-methylpyrrolidine-1-carboxylate, 2-bromo-5-fluoropyrimidine, and *N*-(5-(chloromethyl)thiazol-2-yl)acetamide. LCMS (ESI): [M+H] 350. ¹H NMR (400 MHz, METHANOL-d4) δ 8.64 (d, *J*=0.75 Hz, 2H), 7.21 (s, 1H), 3.75 (s, 2H), 3.01 (s, 2H), 2.91 (d, *J*=9.54 Hz, 1H), 2.63 (dt, *J*=2.26, 7.03 Hz, 2H), 2.34 (d, *J*=9.54 Hz, 1H), 2.20 (s, 3H), 2.13 (td, *J*=7.28, 12.80 Hz, 1H), 1.58 (ddd, *J*=6.15, 7.22, 12.99 Hz, 1H), 1.10 (s, 3H).

### Example 2-36

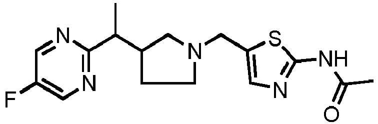

***N*-(5-((3-(1-(5-fluoropyrimidin-2-yl)ethyl)pyrrolidin-1-yl)methyl)thiazol-2-yl)acetamide:** The title compound was prepared in an analogous manner of that in scheme 2 from *tert*-butyl 3-(1-bromoethyl)pyrrolidine-1-carboxylate, 2-bromo-5-fluoropyrimidine, and *N-*(5-(chloromethyl)thiazol-2-yl)acetamide. LCMS (ESI): [M+H] 350. ¹H NMR (400 MHz, METHANOL-d4) δ 8.69 (s, 1H), 8.63-8.77 (m, 1H), 7.51-7.64 (m, 1H), 4.50-4.70 (m, 2H), 3.41-3.91 (m, 1H), 2.69-3.26 (m, 4H), 2.28-2.55 (m, 1H), 2.22 (d, *J*=4.02 Hz, 3H), 1.91-2.12 (m, 1H), 1.54-1.87 (m, 1H), 1.26-1.42 (m, 3H).

### Example 2-37

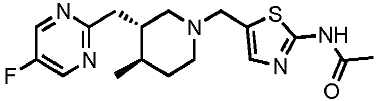

***N*-(5-(((3S,4R)-3-((5-fluoropyrimidin-2-yl)methyl)-4-methylpiperidin-1-yl)methyl)thiazol-2-yl)acetamide:** The title compound was prepared in an analogous manner of that in scheme 2 from *trans*-tert-butyl 3-(bromomethyl)-4-methylpiperidine-1-carboxylate, 2-bromo-5-fluoropyrimidine, and *N*-(5-(chloromethyl)thiazol-2-yl)acetamide, followed by chiral separation; absolute configuration assigned arbitrarily. The chiral separation method used: Column: CHIRALPAK AD-H 30x250mm, 5um; Method: 40% Ethanol in 0.1% diethyl amine in CO₂ (flow rate: 100mL/min), ABPR 120bar, MBPR 40psi. The product was peak 1. LCMS (ESI): [M+H] 364. ¹H NMR (400 MHz, METHANOL-d4) δ 8.56 (s, 2H), 7.15 (s, 1H), 3.66 (dd, *J*=0.75, 14.05 Hz, 1H), 3.47-3.57 (m, 1H), 2.96-3.08 (m, 1H), 2.86-2.95 (m, 1H), 2.65 (br s, 1H), 2.28-2.52 (m, 3H), 2.20 (s, 4H), 1.79 (dt, *J*=3.64, 7.34 Hz, 1H), 1.52-1.72 (m, 2H), 0.96 (d, *J*=7.03 Hz, 3H).

### Example 2-38

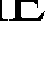

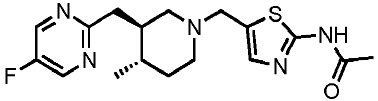

***N*-(5-(((3R,4S)-3-((5-fluoropyrimidin-2-yl)methyl)-4-methylpiperidin-1-yl)methyl)thiazol-2-yl)acetamide:** The title compound was prepared in an analogous manner of that in scheme 2 from *trans*-tert-butyl 3-(bromomethyl)-4-methylpiperidine-1-carboxylate, 2-bromo-5-fluoropyrimidine, and *N*-(5-(chloromethyl)thiazol-2-yl)acetamide, followed by chiral separation; absolute configuration assigned arbitrarily. The chiral separation method used was: Column: CHIRALPAK AD-H 30x250mm, 5um; Method: 40% Ethanol in 0.1% diethyl amine in CO₂ (flow rate: 100mL/min), ABPR 120bar, MBPR 40psi. The product was peak 2. LCMS (ESI): [M+H] 364. ¹H NMR (400 MHz, METHANOL-d4) δ 8.56 (s, 2H), 7.15 (s, 1H), 3.62-3.70 (m, 1H), 3.48-3.57 (m, 1H), 2.97-3.09 (m, 1H), 2.86-2.95 (m, 1H), 2.65 (br s, 1H), 2.27-2.50 (m, 3H), 2.20 (s, 4H), 1.80 (tt, *J*=3.89, 7.40 Hz, 1H), 1.52-1.72 (m, 2H), 0.96 (d, *J*=7.03 Hz, 3H).

### Example 2-39

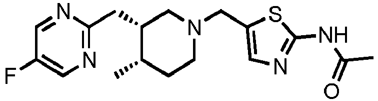

***N*-(5-(((3S,4S)-3-((5-fluoropyrimidin-2-yl)methyl)-4-methylpiperidin-1-yl)methyl)thiazol-2-yl)acetamide:** The title compound was prepared in an analogous manner of that in scheme 2 from *cis-tert-*butyl 3-(bromomethyl)-4-methylpiperidine-1-carboxylate, 2-bromo-5-fluoropyrimidine, and *N*-(5-(chloromethyl)thiazol-2-yl)acetamide, followed by chiral separation; absolute configuration assigned arbitrarily. The chiral separation method used: Column: CHIRALPAK AD-H 30x250mm, 5um. Method: 40% Ethanol w/ 0.1% DEA in CO2 (flow rate: 100mL/min), ABPR 120bar, MBPR 40psi. The product was peak 1. LCMS (ESI): [M+H] 364. ¹H NMR (400 MHz, METHANOL-d4) δ 8.56 (s, 2H), 7.15 (s, 1H), 3.62-3.73 (m, 1H), 3.53 (br d, *J*=14.05 Hz, 1H), 2.96-3.09 (m, 1H), 2.86-2.95 (m, 1H), 2.66 (br s, 1H), 2.28-2.54 (m, 3H), 2.20 (s, 4H), 1.74-1.87 (m, 1H), 1.50-1.73 (m, 2H), 0.96 (d, *J*=7.03 Hz, 3H).

### Example 2-40

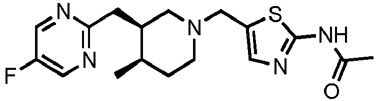

***N*-(5-(((3R,4R)-3-((5-fluoropyrimidin-2-yl)methyl)-4-methylpiperidin-1-yl)methyl)thiazol-2-yl)acetamide:** The title compound was prepared in an analogous manner of that in scheme 2 from *cis-tert-butyl* 3-(bromomethyl)-4-methylpiperidine-1-carboxylate, 2-bromo-5-fluoropyrimidine, and *N*-(5-(chloromethyl)thiazol-2-yl)acetamide, followed by chiral separation; absolute configuration assigned arbitrarily. The chiral separation method used was: Column: CHIRALPAK AD-H 30×250mm, 5um. Method: 40% Ethanol w/ 0.1% DEA in CO₂ (flow rate: 100mL/min), ABPR 120bar, MBPR 40psi. The product was peak 2. LCMS (ESI): [M+H] 364. ¹H NMR (400 MHz, METHANOL-d4) δ 8.56 (s, 2H), 7.15 (s, 1H), 3.62-3.72 (m, 1H), 3.49-3.57 (m, 1H), 2.97-3.10 (m, 1H), 2.85-2.95 (m, 1H), 2.66 (br s, 1H), 2.28-2.52 (m, 3H), 2.20 (s, 4H), 1.75-1.89 (m, 1H), 1.51-1.73 (m, 2H), 0.96 (d, *J*=7.03 Hz, 3H).

### Example 2-41

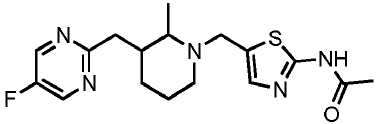

***N*-(5-((3-((5-fluoropyrimidin-2-yl)methyl)-2-methylpiperidin-1-yl)methyl)thiazol-2-yl)acetamide:** The title compound was prepared in an analogous manner of that in scheme 2 from *tert*-butyl 3-(bromomethyl)-2-methylpiperidine-1-carboxylate, 2-bromo-5-fluoropyrimidine, and *N*-(5-(chloromethyl)thiazol-2-yl)acetamide. LCMS (ESI): [M+H] 364. ¹H NMR (400 MHz, CDCl₃) δ 11.42 (br s, 1H), 8.44-8.59 (m, 2H), 7.19 (br d, *J*=8.03 Hz, 1H), 3.58-3.92 (m, 2H), 2.87 (br d, *J*=7.28 Hz, 3H), 2.47 (br d, *J*=9.03 Hz, 3H), 2.30 (s, 3H), 1.15-1.46 (m, 3H), 1.15-1.46 (m, 1H), 1.03 (br s, 3H).

### Example 2-42

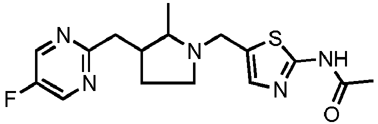

***N*-(5-((3-((5-fluoropyrimidin-2-yl)methyl)-2-methylpyrrolidin-1-yl)methyl)thiazol-2-yl)acetamide:** The title compound was prepared in an analogous manner of that in scheme 2 from *tert*-butyl 3-(bromomethyl)-2-methylpyrrolidine-1-carboxylate, 2-bromo-5-fluoropyrimidine, and *N*-(5-(chloromethyl)thiazol-2-yl)acetamide. LCMS (ESI): [M+H] 350. ¹H NMR (400 MHz, CDCl₃) δ 11.52 (br s, 1H), 8.52 (s, 2H), 7.22 (br s, 1H), 4.05 (br d, *J*=13.80 Hz, 1H), 3.72 (br s, 1H), 2.69-3.20 (m, 5H), 2.36-2.59 (m, 1H), 2.31 (s, 3H), 1.79 (br s, 1H), 1.59 (br s, 1H), 1.11 (br d, *J*=5.02 Hz, 3H).

### Example 2-43

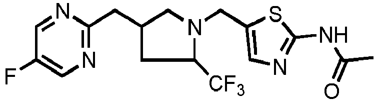

***N*-(5-((4-((5-fluoropyrimidin-2-yl)methyl)-2-(trifluoromethyl)pyrrolidin-1-yl)methyl)thiazol-2-yl)acetamide:** The title compound was prepared in an analogous manner of that in scheme 2 from, *tert-butyl* 4-(bromomethyl)-2-(trifluoromethyl)pyrrolidine-1-carboxylate, 2-bromo-5-fluoropyrimidine, and *N*-(5-(chloromethyl)thiazol-2-yl)acetamide. The resulting enantiomers were separated using chiral SFC.

### Example 2-44

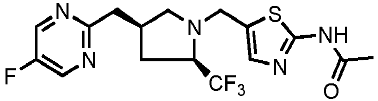

***N*-(5-(((2*R*,4*S*)-4-((5-fluoropyrimidin-2-yl)methyl)-2-(trifluoromethyl)pyrrolidin-1-yl)methyl)thiazol-2-yl)acetamide:** Relative and absolute configuration assigned arbitrarily. Column: CHIRALPAK AD-H 30x250mm, 5um; Method: 40% Ethanol in 0.1% diethyl amine in CO₂ (flow rate: 100mL/min), ABPR 120bar, MBPR 40psi. The product was peak 1. LCMS (ESI): [M+H] 404. ¹H NMR (500 MHz, CDCl₃) δ 11.89 (br s, 1H), 8.51 (s, 2H), 7.21 (s, 1H), 3.92 - 4.23 (m, 2H), 3.30 - 3.40 (m, 1H), 3.13 - 3.20 (m, 1H), 2.93 - 3.07 (m, 2H), 2.77 - 2.90 (m, 1H), 2.39 (dd, *J*=10.07, 8.85 Hz, 1H), 2.32 (s, 3H), 2.12 (br dd, *J*=13.50, 6.94 Hz, 1H), 1.77 (dt, *J*=13.58, 10.83 Hz, 1H).

### Example 2-45

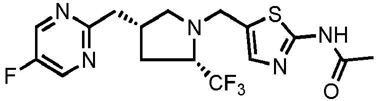

***N*-(5-(((2*S*,4*R*)-4-((5-fluoropyrimidin-2-yl)methyl)-2-(trifluoromethyl)pyrrolidin-1-yl)methyl)thiazol-2-yl)acetamide:** Relative and absolute configuration assigned arbitrarily. Column: CHIRALPAK AD-H 30x250mm, 5um; Method: 40% Ethanol in 0.1% diethyl amine in CO₂ (flow rate: 100mL/min), ABPR 120bar, MBPR 40psi. The product was peak 2. LCMS (ESI): [M+H] 404. ¹H NMR (500 MHz, CDCl₃) δ 11.97 (br s, 1H), 8.52 (s, 2H), 7.22 (s, 1H), 3.96 - 4.23 (m, 2H), 3.30 - 3.43 (m, 1H), 3.18 (dd, *J*=8.01, 6.48 Hz, 1H), 2.93 - 3.09 (m, 2H), 2.78 - 2.91 (m, 1H), 2.40 (dd, *J*=10.07, 8.85 Hz, 1H), 2.30 - 2.36 (m, 3H), 2.14 (ddd, J=13.47, 7.06, 1.30 Hz, 1H), 1.78 (dt, *J*=13.54, 10.78 Hz, 1H).

### Example 2-46

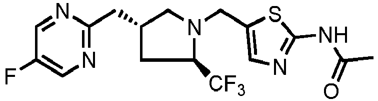

***N*-(5-(((2*R*,4*R*)-4-((5-fluoropyrimidin-2-yl)methyl)-2-(trifluoromethyl)pyrrolidin-1-yl)methyl)thiazol-2-yl)acetamide:** Relative and absolute configuration assigned arbitrarily. Column: CHIRALPAK AD-H 30x250mm, 5um; Method: 40% Ethanol in 0.1% diethyl amine in CO₂ (flow rate: 100mL/min), ABPR 120bar, MBPR 40psi. The product was peak 3. LCMS (ESI): [M+H] 404. ¹H NMR (500 MHz, CDCl₃) δ 10.69 (br s, 1H), 8.45 (s, 2H), 7.19 (s, 5H), 3.90 - 4.15 (m, 2H), 3.14 (qd, *J*=7.43, 2.59 Hz, 1H), 2.95 - 3.05 (m, 1H), 2.78 - 2.94 (m, 3H), 2.67 (td, *J*=9.77, 6.10 Hz, 1H), 2.23 (s, 3H), 1.99 (ddt, *J*=12.57, 10.59, 7.34, 7.34 Hz, 1H), 1.48 - 1.61 (m, 1H).

### Example 2-47

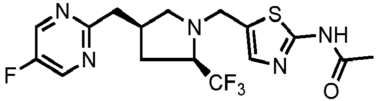

***N*-(5-(((2*R*,4*S*)-4-((5-fluoropyrimidin-2-yl)methyl)-2-(trifluoromethyl)pyrrolidin-1-yl)methyl)thiazol-2-yl)acetamide:** Relative and absolute configuration assigned arbitrarily. Column: CHIRALPAK AD-H 30x250mm, 5um; Method: 40% Ethanol in 0.1% diethyl amine in CO₂ (flow rate: 100mL/min), ABPR 120bar, MBPR 40psi. The product was peak 4. LCMS (ESI): [M+H] 404. ¹H NMR (500 MHz, CDCl₃) δ 10.73 (br s, 1H), 8.45 (s, 2H), 7.17 - 7.22 (m, 5H), 3.90 - 4.15 (m, 2H), 3.14 (qd, *J*=7.43, 2.75 Hz, 1H), 2.97 - 3.04 (m, 1H), 2.78 - 2.95 (m, 3H), 2.67 (td, *J*=9.77, 6.10 Hz, 1H), 2.23 (s, 3H), 1.99 (ddt, *J*=12.59, 10.49, 7.38, 7.38 Hz, 1H), 1.57 (m, 1H).

### Example 2-48

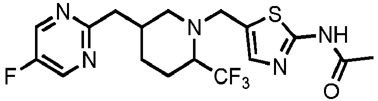

***N*-(5-((5-((5-fluoropyrimidin-2-yl)methyl)-2-(trifluoromethyl)piperidin-1-yl)methyl)thiazol-2-yl)acetamide:** The title compound was prepared in an analogous manner of that in scheme 2 from, *tert-butyl* 5-(bromomethyl)-2-(trifluoromethyl)piperidine-1-carboxylate, 2-bromo-5-fluoropyrimidine, and *N*-(5-(chloromethyl)thiazol-2-yl)acetamide. The resulting isomers were separated using: Column: CHIRALPAK AD-H 30x250mm, 5um; Method: 30% Isopropanol in 0.1% diethyl amine in CO₂ (flow rate: 100mL/min), ABPR 120bar, MBPR 40psi.

### Example 2-49

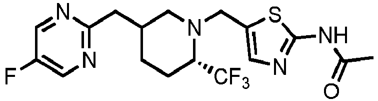

Relative and absolute configuration assigned arbitrarily; the product was peak 1. LCMS (ESI): [M+H] 487. ¹H NMR (500 MHz, CDCl₃) δ 12.15 (br s, 1H), 8.52 (s, 2H), 7.22 (s, 1H), 3.93 - 4.18 (m, 2H), 3.27 - 3.40 (m, 1H), 2.80 - 2.91 (m, 2H), 2.74 - 2.80 (m, 1H), 2.64 - 2.74 (m, 1H), 2.31 - 2.36 (m, 3H), 2.20 - 2.31 (m, 1H), 1.94 - 2.03 (m, 1H), 1.77 - 1.89 (m, 1H), 1.62 (br d, *J*=12.21 Hz, 1H), 1.36 - 1.49 (m, 1H).

### Example 2-50

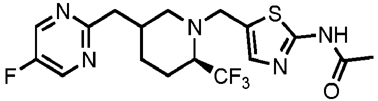

Relative and absolute configuration assigned arbitrarily; the product was peak 2. LCMS (ESI): [M+H] 487. ¹H NMR (500 MHz, CDCl₃) δ 12.15 (br s, 1H), 8.52 (s, 2H), 7.22 (s, 1H), 3.96 - 4.15 (m, 2H), 3.25 - 3.39 (m, 1H), 2.80 - 2.90 (m, 2H), 2.74 - 2.80 (m, 1H), 2.65 - 2.73 (m, 1H), 2.30 - 2.35 (m, 3H), 2.21 - 2.29 (m, 1H), 1.94 - 2.02 (m, 1H), 1.78 - 1.89 (m, 1H), 1.62 (br d, *J*=12.21 Hz, 1H), 1.37 - 1.49 (m, 1H).

### Example 2-51

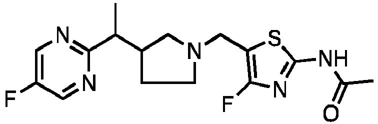

***N*-(4-fluoro-5-((3-(1-(5-fluoropyrimidin-2-yl)ethyl)pyrrolidin-1-yl)methyl)thiazol-2-yl)acetamide:** The title compound was prepared in an analogous manner of that in example 1-1 from 5-fluoro-2-(1-(pyrrolidin-3-yl)ethyl)pyrimidine and *N*-(4-fluoro-5-formylthiazol-2-yl)acetamide. LCMS (ESI): [M+H] 368. ¹H NMR (400 MHz, Methanol-d₄) δ 8.66 (dd, *J*=0.88, 1.63 Hz, 2H), 3.82-3.96 (m, 2H), 3.00-3.21 (m, 2H), 2.81-2.96 (m, 1H), 2.67-2.80 (m, 2H), 2.40-2.66 (m, 1H), 2.19 (d, *J*=4.77 Hz, 3H), 1.72-1.83 (m, 1H), 1.34-1.57 (m, 1H), 1.30 (dd, *J*=7.03, 11.29 Hz, 3H).

### Example 2-52

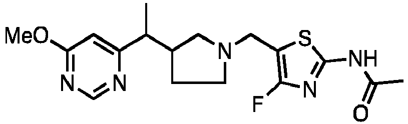

***N*-(4-fluoro-5-((3-(1-(6-methoxypyrimidin-4-yl)ethyl)pyrrolidin-1-yl)methyl)thiazol-2-yl)acetamide:** The title compound was prepared in an analogous manner of that in scheme 2 from 4-methoxy-6-(1-(pyrrolidin-3-yl)ethyl)pyrimidine and *N*-(4-fluoro-5-formylthiazol-2-yl)acetamide. LCMS (ESI): [M+H] 380. ¹H NMR (400 MHz, Methanol-d₄) δ 8.69 (s, 1H), 6.75-6.85 (m, 1H), 4.38-4.66 (m, 2H), 3.99 (d, *J*=0.75 Hz, 1H), 3.96-4.06 (m, 1H), 3.96-4.06 (m, 1H), 3.39-3.92 (m, 2H), 3.13 (td, *J*=1.63, 3.26 Hz, 1H), 2.84 (br d, *J*=7.53 Hz, 2H), 2.28-2.55 (m, 1H), 2.18-2.25 (m, 3H), 1.43-2.09 (m, 2H), 1.30 (dd, *J*=6.65, 9.91 Hz, 3H).

### Example 2-53

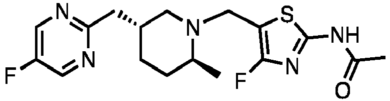

***N*-(4-fluoro-5-(((2*S*,5*R*)-5-((5-fluoropyrimidin-2-yl)methyl)-2-methylpiperidin-1-yl)methyl)thiazol-2-yl)acetamide:** The title compound was prepared according to the general procedure described in scheme 2 and using 5-fluoro-2-(((3*R*,6*S*)-6-methylpiperidin-3-yl)methyl)pyrimidine and *N*-(4-fluoro-5-formylthiazol-2-yl)acetamide. LCMS (ESI): [M+H] 382. ¹HNMR: (400 MHz, Methanol-d₄) δ 8.66 (d, *J*=1.00 Hz, 2H), 3.72-3.96 (m, 2H), 2.71-2.92 (m, 3H), 2.13-2.38 (m, 2H), 2.22 (s, 3H), 1.92-2.07 (m, 1H), 1.63-1.77 (m, 2H), 1.28-1.47 (m, 1H), 1.22 (d, *J*=6.27 Hz, 3H), 1.00-1.16 (m, 1H). 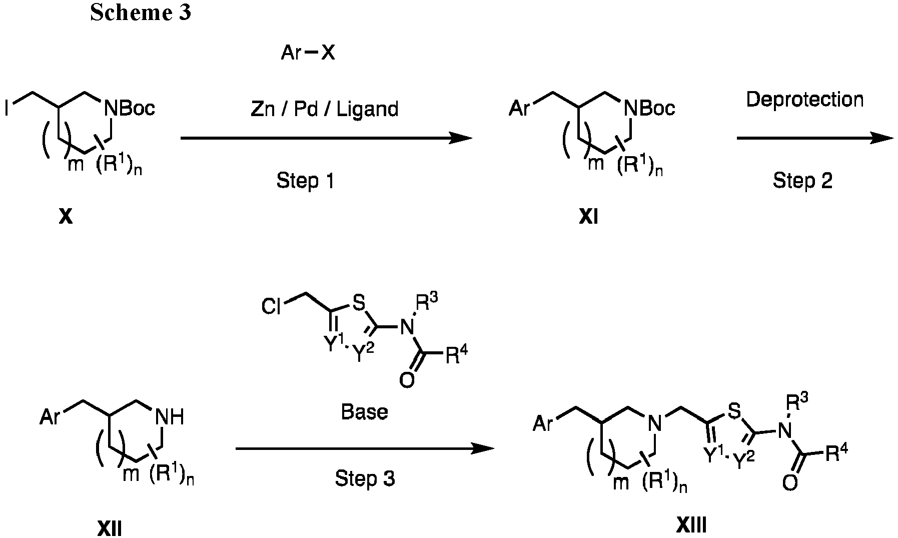 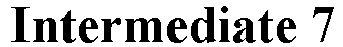 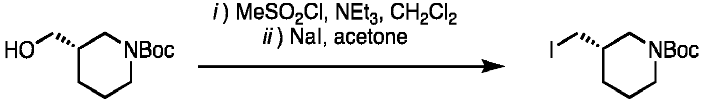 ***tert-butyl* (3*S*)-3-(iodomethyl)piperidine-1-carboxylate:** *tert-Butyl* (3*S*)-3-(hydroxymethyl)piperidine-1-carboxylate (20.0 g, 92.9 mmol) was dissolved in CH₂Cl₂ (25.0 mL) and triethylamine (11.3 g, 111.5 mmol, 15.5 mL) was added. The mixture was cooled to 0 °C and methanesulfonyl chloride (12.8 g, 111.5 mmol, 8.63 mL) was slowly added. After the addition was complete, the reaction was warmed to room temperature and stirred for 3 h. The reaction mixture was filtered over celite, washed with water (25 mL), saturated Na₂CO₃ (aq) (25 mL), dried over Na₂SO₄, filtered, and concentrated *in vacuo.* To the resulting oil was added heptane (25 mL). The precipitate was filtered and washed with another portion of heptane (70 mL). The crude mesylate was dissolved in acetone (150 mL), and sodium iodide (27.85 g, 185.8 mmol) was added to the mixture; which was subsequently stirred at reflux overnight. The reaction mixture was cooled to room temperature, filtered, and concentrated *in vacuo.* The residue was dissolved in diethyl ether (300 mL), and the solution was washed with water (50 mL), saturated Na₂CO₃ (aq) (40 mL), 5% Na₂S₂O₃ (aq) (40 mL), and brine (40 mL). The solution was dried over Na₂SO₄, filtered, and concentrated *in vacuo* to afford the title compound (23.90 g, 79%). ¹HNMR: (500 MHz, CDCl₃) δ 3.93-4.27 (m, 1H), 3.85 (br d, *J*=13.43 Hz, 1H), 3.09 (br d, *J*=6.71 Hz, 2H), 2.43-2.93 (m, 2H), 1.93 (br d, *J*=10.38 Hz, 1H), 1.58-1.71 (m, 2H), 1.40-1.54 (m, 10H), 1.25 (m, 1H). 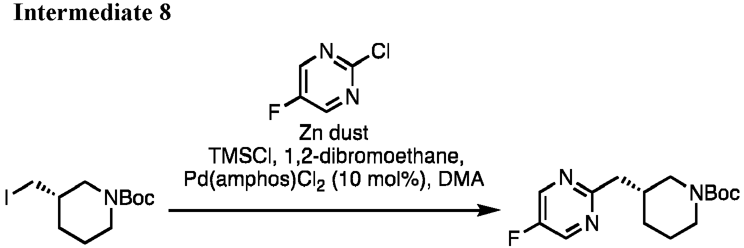 ***tert-butyl* (3*R*)-3-[(5-fluoropyrimidin-2-yl)methyl]piperidine-1-carboxylate:** To a slurry of zinc dust (0.817 g, 12.5 mmol) in DMA (2 mL) was added a mixture of TMSCl/1,2-dibromoethane (7/5 v/v, 0.24 mL) over a 10 minute period. The mixture was stirred for 15 minutes and a solution of *tert-butyl* (*S*)-3-(iodomethyl)piperidine-1-carboxylate (3.25 g, 10.0 mmol) in DMA (5 mL) was added slowly. Stirring was continued for 2 hours. To the mixture was added 2-chloro-5-fluoro-pyrimidine (0.884 g, 6.67 mmol), and Pd(amphos)Cl₂ (0.473 g, 0.667 mmol). The mixture was heated at 80 °C overnight. After cooling to room temperature, the mixture was diluted with EtOAc and filtered over a pad of Celite. The filtrate was washed with water, dried over MgSO₄, filtered, and concentrated *in vacuo.* The residue was purified over SiO₂ (0-100% EtOAc/heptanes) to afford the title compound (1.78 g, 90 %). LCMS (ESI): [(-*t*-Bu) M+H] 240, [(-Boc) M+H] 196. ¹HNMR: (500 MHz, CDCl₃) δ 8.53 (s, 2H), 3.74-4.08 (m, 2H), 2.84-2.93 (m, 2H), 2.76-2.83 (m, 1H), 2.43-2.72 (m, 1H), 2.11 (m, 1H), 1.77 (br d, *J*=10.99 Hz, 1H), 1.62-1.70 (m, 1H), 1.42 (s, 10H), 1.17-1.31 (m, 1H). 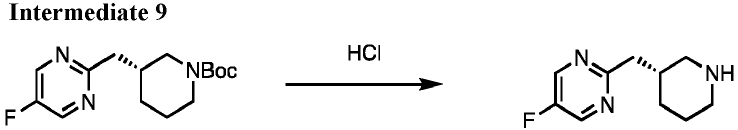 **5-fluoro-2-[[(3*R*)-3-piperidyl]methyl]pyrimidine hydrochloride:** Acetyl chloride (15.8 g, 201.5 mmol, 14.4 mL) was added dropwise over 10 minutes to vigorously stirred MeOH (110 mL) at 0 °C and the mixture was stirred for a further 20 min. To the mentholic HCl solution was added *tert-butyl* (3*R*)-3-[(5-fluoropyrimidin-2-yl)methyl]piperidine-1-carboxylate (5.95 g, 20.15 mmol) as a solution in MeOH (30 mL) dropwise at 0 °C. The resulting mixture was warmed to room temperature and stirred for a further 2 hours. The mixture was concentrated *in vacuo* to afford the title compound (4.60 g, 98%). LCMS (ESI): [M+H] 232. ¹HNMR: (400 MHz, Methanol-d₄) δ 8.67-8.76 (m, 2H), 3.27-3.38 (m, 2H), 2.82-2.97 (m, 3H), 2.70-2.81 (m, 1H), 2.34-2.46 (m, 1H), 1.78-1.93 (m, 2H), 1.72 (s, 1H), 1.26-1.39 (m, 1H).

### Example 3-1

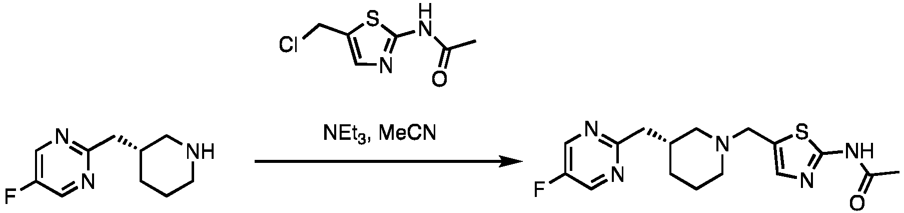

***N*-[5-[[(3*R*)-3-[(5-fluoropyrimidin-2-yl)methyl]-1-piperidyl]methyl]thiazol-2-yl]acetamide:** To a suspension of 5-fluoro-2-[[(3*R*)-3-piperidyl]methyl]pyrimidine hydrochloride (7.90 g, 34.1 mmol,) and *N*-[5-(chloromethyl)thiazol-2-yl]acetamide (6.83 g, 35.8 mmol) in MeCN (170 mL) was added triethylamine (10.35 g, 102 mmol, 14.2 mL); which was subsequently warmed to 70 °C overnight. The reaction was cooled to room temperature, and the mixture was diluted with EtOAc and washed with saturated NH₄Cl (*aq*)*.* The organics were dried over MgSO₄, filtered and concentrated *in vacuo.* The residue was purified over SiO₂ (24g, 0-100% EtOAc:iPrOH (3:1 v/v)-heptane) to afford the title compound (8.24 g, 69%). LCMS (ESI): [M+H] 350. ¹HNMR: (500 MHz, CDCl₃) δ 11.89 (br s, 1H), 8.52 (s, 2H), 7.18 (s, 1H), 3.65 (q, *J*=13.63 Hz, 2H), 2.86 (d, *J*=7.33 Hz, 2H), 2.77-2.84 (m, 2H), 2.31 (s, 3H), 2.18-2.28 (m, 1H), 1.98-2.07 (m, 1H), 1.90 (br t, *J*=10.38 Hz, 1H), 1.66-1.71 (m, 2H), 1.51-1.61 (m, 1H), 1.00-1.09 (m, 1H).

### Example 3-2

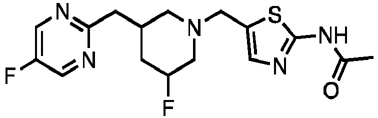

***N*-(5-((3-fluoro-5-((5-fluoropyrimidin-2-yl)methyl)piperidin-1-yl)methyl)thiazol-2-yl)acetamide:** The title compound was prepared in an analogous manner of that in scheme 3 from *tert*-butyl 3-fluoro-5-(iodomethyl)piperidine-1-carboxylate, 2-chloro-5-fluoro-pyrimidine, and *N*-[5-(chloromethyl)thiazol-2-yl]acetamide. LCMS (ESI): [M+H] 368. ¹HNMR: (500 MHz, CDCl₃) δ 11.96 (br s, 1 H), 8.54 (s, 2 H), 7.21 (s, 1 H), 4.66 (tt, *J*=9.99, 4.73 Hz, 0.5 H), 4.56 (tt, *J*=9.96, 4.77 Hz, 0.5 H), 3.68 - 3.82 (m, 2 H), 3.18 (dt, *J*=9.99, 4.92 Hz, 1 H), 2.88 - 3.00 (m, 3 H), 2.85 (br d, *J*=10.53 Hz, 1 H), 2.34 - 2.40 (m, 1 H), 2.28 - 2.34 (m, 4 H), 2.12 - 2.20 (m, 1 H), 2.05 (td, *J*=9.84, 5.04 Hz, 1 H), 1.90 (t, *J*=10.68 Hz, 1 H), 1.25 (quin, *J*=11.48 Hz, 1 H).

### Example 3-3

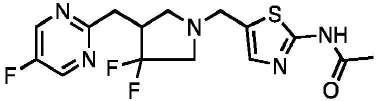

***N*-(5-((3,3-difluoro-4-((5-fluoropyrimidin-2-yl)methyl)pyrrolidin-1-yl)methyl)thiazol-2-yl)acetamide:** The title compound was prepared in an analogous manner of that in scheme 3 from *tert*-butyl 3,3-difluoro-4-(iodomethyl)pyrrolidine-1-carboxylate, 2-chloro-5-fluoro-pyrimidine, and *N*-[5-(chloromethyl)thiazol-2-yl]acetamide. LCMS: [M+H] 372. ¹HNMR (500 MHz, CDCl₃) δ 12.17 (br s, 1 H), 8.54 (s, 2 H), 7.23 (t, *J*=0.84 Hz, 1 H), 3.82 (t, *J*=0.92 Hz, 2 H), 3.34 - 3.40 (m, 1 H), 3.23 - 3.33 (m, 1 H), 3.14 (td, *J*=6.41, 3.36 Hz, 2 H), 3.04 (dd, *J*=15.03, 9.54 Hz, 1 H), 2.81 - 2.91 (m, 1 H), 2.44 - 2.50 (m, 1 H), 2.30 - 2.36 (m, 3 H).

### Example 3-4 (Reference)

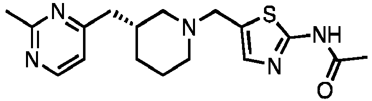

**(*R*)*-N-*(5-((3-((2-methylpyrimidin-4-yl)methyl)piperidin-1-yl)methyl)thiazol-2-yl)acetamide:** The title compound was prepared in an analogous manner of that in scheme 3 from *tert*-butyl (*S*)-3-(iodomethyl)piperidine-1-carboxylate, 4-chloro-2-methyl-pyrimidine, and *N*-[5-(chloromethyl)thiazol-2-yl]acetamide. LCMS: [M+H] 346. ¹HNMR: (400 MHz, CDCl₃) δ 11.56 (br s, 1H), 8.48 (d, *J*=5.02 Hz, 1H), 7.17 (s, 1H), 6.94 (d, *J*=5.27 Hz, 1H), 3.57-3.71 (m, 2H), 2.76 (m, 2H), 2.70 (s, 3H), 2.57-2.68 (m, 2H), 2.31 (s, 3H), 2.06-2.18 (m, 2H), 1.89 (m, 1H), 1.67 (m, 2H), 1.50-1.63 (m, 1H), 0.99-1.11 (m, 1H).

### Example 3-5 (Reference)

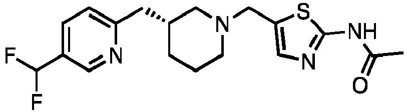

**(*R*)-*N*-(5-((3-((5-(difluoromethyl)pyridin-2-yl)methyl)piperidin-1-yl)methyl)thiazol-2-yl)acetamide:** The title compound was prepared in an analogous manner of that in scheme 3 from *tert*-butyl (*S*)-3-(iodomethyl)piperidine-1-carboxylate, 2-chloro-5-(difluoromethyl)pyridine, and *N*-[5-(chloromethyl)thiazol-2-yl]acetamide. LCMS (ESI): [M+H] 381. ¹HNMR: (400 MHz, CDCl₃) δ 11.97 (br s, 1H), 8.64 (d, *J*=1.25 Hz, 1H), 7.74 (d, *J*=7.76 Hz, 1H), 7.23 (d, *J*=8.03 Hz, 1H), 7.17 (s, 1H), 6.68 (t, *J*=56.47 Hz, 1H), 3.57-3.72 (m, 2H), 2.70-2.85 (m, 4H), 2.31 (s, 3H), 2.03-2.19 (m, 2H), 1.90 (br t, *J*=10.29 Hz, 1H), 1.67 (m, 2H), 1.48-1.60 (m, 1H), 0.99-1.13 (m, 1H).

### Example 3-6

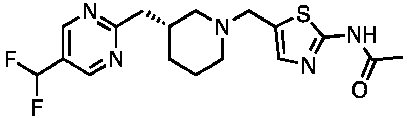

**(*R*)-*N*-(5-((3-((5-(difluoromethyl)pyrimidin-2-yl)methyl)piperidin-1-yl)methyl)thiazol-2-yl)acetamide:** The title compound was prepared in an analogous manner of that in scheme 3 from *tert*-butyl (*S*)-3-(iodomethyl)piperidine-1-carboxylate, 2-chloro-5-(difluoromethyl)pyrimidine, and *N*-[5-(chloromethyl)thiazol-2-yl]acetamide. LCMS (ESI): [M+H] 382. ¹HNMR: (400 MHz, CDCl₃) δ 11.69 (br s, 1H), 8.80 (s, 2H), 7.18 (s, 1H), 6.73 (t, *J*=55.22 Hz, 1H), 3.59-3.71 (m, 2H), 2.93 (d, *J*=7.28 Hz, 2H), 2.81 (br t, *J*=9.91 Hz, 2H), 2.30 (s, 3H), 2.01-2.11 (m, 1H), 1.93 (t, *J*=10.29 Hz, 1H), 1.52-1.74 (m, 4H), 1.02-1.14 (m, 1H).

### Example 3-7

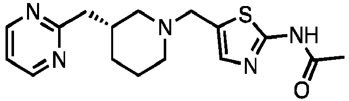

**(*R*)-*N*-(5-((3-(pyrimidin-2-ylmethyl)piperidin-1-yl)methyl)thiazol-2-yl)acetamide:** The title compound was prepared in an analogous manner of that in scheme 3 from *tert-butyl* (S)-3-(iodomethyl)piperidine-1-carboxylate, 2-chloropyrimidine, and *N*-[5-(chloromethyl)thiazol-2-yl]acetamide. LCMS (ESI): [M+H] 331. ¹HNMR: (400 MHz, CDCl₃) δ 11.39 (br s, 1H), 8.67 (d, *J*=5.02 Hz, 2H), 7.18 (s, 1H), 7.12 (t, *J*=4.89 Hz, 1H), 3.59-3.70 (m, 2H), 2.79-2.91 (m, 4H), 2.30 (s, 3H), 1.97-2.04 (m, 1H), 1.91 (t, *J*=10.42 Hz, 1H), 1.63-1.74 (m, 3H), 1.52-1.62 (m, 1H), 1.00-1.11 (m, 1H).

### Example 3-8

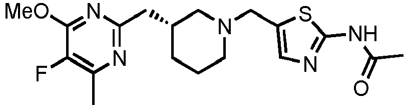

**(*R*)*-N-*(5-((3-((5-fluoro-4-methoxy-6-methylpyrimidin-2-yl)methyl)piperidin-1-yl)methyl)thiazol-2-yl)acetamide:** The title compound was prepared in an analogous manner of that in scheme 3 from *tert*-butyl (*S*)-3-(iodomethyl)piperidine-1-carboxylate, 2-bromo-5-fluoro-4-methoxy-6-methylpyrimidine, and *N*-(5-(chloromethyl)thiazol-2-yl)acetamide. LCMS (ESI): [M+H] 394. ¹H NMR (500 MHz, CDCl₃) δ 11.74 (br s, 1H), 7.18 (s, 1H), 4.00 (s, 3H), 3.65 (s, 2H), 2.80-2.91 (m, 2H), 2.66 (d, *J*=7.33 Hz, 2H), 2.40 (d, *J*=2.44 Hz, 3H), 2.31 (s, 3H), 2.17-2.27 (m, 1H), 2.01 (br t, *J*=10.38 Hz, 1H), 1.85 (br t, *J*=10.38 Hz, 1H), 1.53-1.73 (m, 3H), 0.97-1.07 (m, 1H).

### Example 3-9

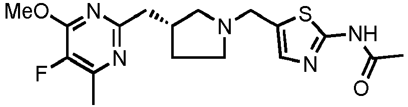

**(*R*)*-N-*(5-((3-((5-fluoro-4-methoxy-6-methylpyrimidin-2-yl)methyl)pyrrolidin-1-yl)methyl)thiazol-2-yl)acetamide:** The title compound was prepared in an analogous manner of that in scheme 3 from *tert-butyl* (*S*)-3-(iodomethyl)pyrrolidine-1-carboxylate, 2-bromo-5-fluoro-4-methoxy-6-methylpyrimidine, and *N*-(5-(chloromethyl)thiazol-2-yl)acetamide. LCMS (ESI): [M+H] 380. ¹HNMR (500 MHz, CDCl₃) δ 12.28 (br s, 1H), 7.22 (s, 1H), 4.00 (s, 3H), 3.73 - 3.85 (m, 2H), 2.82 - 2.92 (m, 3H), 2.68 - 2.81 (m, 2H), 2.52 - 2.62 (m, 1H), 2.40 (d, *J*=2.90 Hz, 3H), 2.31 (m, 4H), 1.99 - 2.09 (m, 1H), 1.58 (ddt, *J*=12.61, 8.14, 6.18, 6.18 Hz, 1H).

### Example 3-10

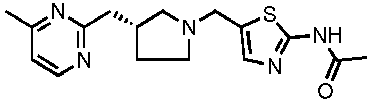

**(*R*)*-N-*(5-((3-((4-methylpyrimidin-2-yl)methyl)pyrrolidin-1-yl)methyl)thiazol-2-yl)acetamide:** The title compound was prepared in an analogous manner of that in scheme 3 from *tert-butyl* (*S*)-3-(iodomethyl)pyrrolidine-1-carboxylate, 2-bromo-4-methylpyrimidine, and *N*-(5-(chloromethyl)thiazol-2-yl)acetamide. LCMS (ESI): [M+H] 332. ¹HNMR (600 MHz, CDCl₃) δ 12.18 (br s, 1H), 8.48 (d, *J*=5.13 Hz, 1H), 7.27 - 7.32 (m, 1H), 6.98 (d, *J*=5.14 Hz, 1H), 3.84 (br s, 2H), 2.93 - 3.04 (m, 3H), 2.83 (dt, *J*=14.95, 7.38 Hz, 2H), 2.66 (br s, 1H), 2.49 (s, 3H), 2.36 - 2.47 (m, 1H), 2.31 (s, 3H), 2.05 (br dd, *J*=12.29, 5.87 Hz, 1H), 1.64 (br dd, *J*=12.29, 6.24 Hz, 1H).

### Example 3-11

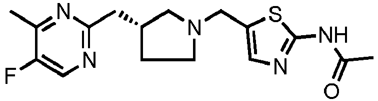

**(*R*)*-N-*(5-((3-((5-fluoro-4-methylpyrimidin-2-yl)methyl)pyrrolidin-1-yl)methyl)thiazol-2-yl)acetamide:** The title compound was prepared in an analogous manner of that in scheme 3 from *tert*-butyl (*S*)-3-(iodomethyl)pyrrolidine-1-carboxylate, 2-bromo-5-fluoro-4-methylpyrimidine, and *N*-(5-(chloromethyl)thiazol-2-yl)acetamide. LCMS (ESI): [M+H] 350. ¹H NMR (500 MHz, CDCl₃) δ 11.91 (br s, 1H), 8.35 (d, *J*=1.68 Hz, 1H), 7.22 (t, *J*=1.07 Hz, 1H), 3.78 (d, *J*=0.61 Hz, 2H), 2.98 (dt, *J*=7.25, 1.26 Hz, 2H), 2.83 - 2.87 (m, 1H), 2.70 - 2.82 (m, 2H), 2.58 (td, *J*=8.66, 6.03 Hz, 1H), 2.51 (d, *J*=2.44 Hz, 3H), 2.29 - 2.35 (m, 4H), 1.98 - 2.07 (m, 1H), 1.60 (ddt, *J*=12.51, 8.32, 6.14, 6.14 Hz, 1H).

### Example 3-12

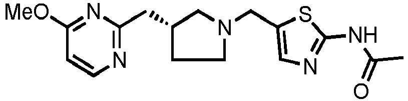

**(*R*)*-N-*(5-((3-((4-methoxypyrimidin-2-yl)methyl)pyrrolidin-1-yl)methyl)thiazol-2-yl)acetamide:** The title compound was prepared in an analogous manner of that in scheme 3 from *tert*-butyl (*S*)-3-(iodomethyl)pyrrolidine-1-carboxylate, 2-bromo-4-methoxypyrimidine, and *N*-(5-(chloromethyl)thiazol-2-yl)acetamide. LCMS (ESI): [M+H] 348. ¹H NMR (500 MHz, CDCl₃) δ 12.17 (br s, 1H), 8.24 (d, *J*=5.80 Hz, 1H), 7.13 (t, *J*=1.07 Hz, 1H), 6.41 - 6.48 (m, 1H), 3.83 - 3.92 (m, 3H), 3.65 - 3.76 (m, 2H), 2.78 - 2.88 (m, 3H), 2.70 - 2.78 (m, 1H), 2.65 (td, *J*=8.39, 5.80 Hz, 1H), 2.50 (td, *J*=8.66, 6.18 Hz, 1H), 2.24 - 2.29 (m, 1H), 2.23 (s, 3H), 1.91 - 2.03 (m, 1H), 1.53 (ddt, *J*=12.59, 8.28, 6.05, 6.05 Hz, 1H).

### Example 3-13

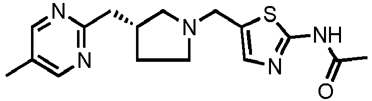

**(*R*)*-N-*(5-((3-((5-methylpyrimidin-2-yl)methyl)pyrrolidin-1-yl)methyl)thiazol-2-yl)acetamide:** The title compound was prepared in an analogous manner of that in scheme 3 from *tert-butyl* (*S*)-3-(iodomethyl)pyrrolidine-1-carboxylate, 2-bromo-5-methylpyrimidine, and *N*-(5-(chloromethyl)thiazol-2-yl)acetamide. LCMS (ESI): [M+H] 332. ¹H NMR (500 MHz, CDCl₃) δ 11.95 (br s, 1H), 8.48 (s, 2H), 7.20 (s, 1H), 2.94 - 3.03 (m, 2H), 2.75 - 2.87 (m, 2H), 2.72 (td, *J*=8.32, 5.80 Hz, 1H), 2.57 (td, *J*=8.54, 6.26 Hz, 1H), 2.30 (s, 4H), 2.28 (s, 3H), 1.95 - 2.07 (m, 1H), 1.94 - 2.05 (m, 1H), 1.54 - 1.65 (m, 1H).

### Example 3-14

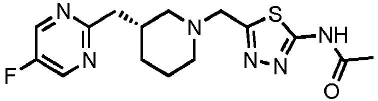

***N*-(5-((3-((5-fluoropyrimidin-2-yl)methyl)piperidin-1-yl)methyl)-1,3,4-thiadiazol-2-yl)acetamide:** The title compound was prepared according to the general procedure described in scheme 3 using 5-fluoro-2-(piperidin-3-ylmethyl)pyrimidine HCl and *N*-(5-formyl-1,3,4-thiadiazol-2-yl)acetamide. LCMS: [M+H] 351. ¹H NMR (400 MHz, METHANOL-d4) δ 8.63 (d, *J*=0.75 Hz, 2H), 3.75-3.90 (m, 2H), 2.87 (d, *J*=7.28 Hz, 2H), 2.74-2.83 (m, 2H), 2.13-2.35 (m, 5H), 2.01 (t, *J*=10.42 Hz, 1H), 1.50-1.80 (m, 3H), 0.99-1.19 (m, 1H). 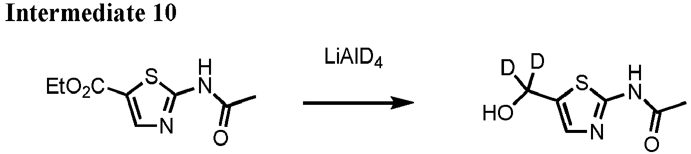 ***N*-(5-(hydroxymethyl-*d*2)thiazol-2-yl)acetamide:** To a suspension of ethyl 2-acetamidothiazole-5-carboxylate (214 mg, 1.00 mmol) in THF (5 mL) was added LiAlD₄ (250 mg, 5.96 mmol) in THF (3 mL) at 0 °C. The reaction mixture was stirred and slowly warmed to rt over. Ice was added to the mixture, followed by IN aqueous HCl. The mixture was extracted with EtOAc (x5, add solid NaCl to saturate the aqueous layer). The combined organic phases were dried over MgSO₄, filtered and concentrated. The residue was purified by normal phase column eluted with EtOAc to get the title compound (6.5 mg, 3.7%) as a white solid. LCMS: [M+H] 175. ¹H NMR (400 MHz, METHANOL-d₄) δ 7.30 (s, 1H), 2.22 (s, 3H). 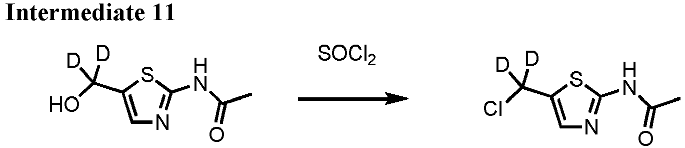 ***N*-(5-(chloromethyl-*d*2)thiazol-2-yl)acetamide:** To a solution of N-(5-(hydroxymethyl-d2)thiazol-2-yl)acetamide (6.50 mg, 37.31 umol) in DCM (1.00 mL) was added thionyl chloride (0.15 mL, 2.06 mmol). The mixture was stirred at 50 °C for 10 min. The reaction mixture was concentrated to provide the title compound (7.2 mg, 100%).

### Example 3-15

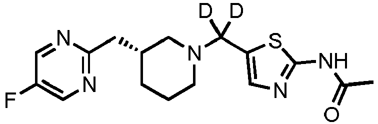

***N*-(5-((3-((5-fluoropyrimidin-2-yl)methyl)piperidin-1-yl)methyl)-1,3,4-thiadiazol-2-yl)acetamide:** The title compound was prepared in an analogous manner of that in scheme 3 from 5-fluoro-2-(piperidin-3-ylmethyl)pyrimidine HCl and N-(5-(chloromethyl-*d*2)thiazol-2-yl)acetamide. LCMS: [M+H] 352. ¹H NMR (500 MHz, METHANOL-d4) δ 8.63 (s, 2H), 7.21 (s, 1H), 2.76-2.89 (m, 4H), 2.15-2.29 (m, 4H), 2.03-2.13 (m, 1H), 1.83-1.92 (m, 1H), 1.65-1.76 (m, 2H), 1.51-1.64 (m, 1H), 1.01-1.15 (m, 1H). 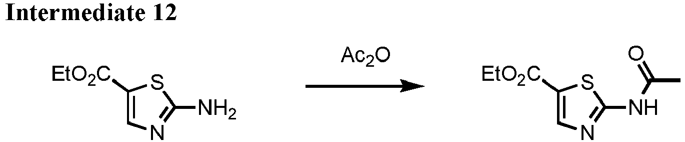 **Ethyl 2-acetamidothiazole-5-carboxylate:** To a stirred solution of ethyl 2-aminothiazole-5-carboxylate (10.00 g, 58.1 mmol), pyridine (9.47 mL, 117.3 mmol) and 4-dimethylaminopyridine (200.0 mg, 1.64 mmol) in dichloromethane (100. mL), was added acetic anhydride (8.23 mL, 87.1 mmol) at 0 °C. The reaction was heated to reflux for 2 hours. The reaction mixture was concentrated under reduced pressure and then hydrochloric acid solution (1.5 N in water, 50 mL) was added. The mixture was stirred for 10 min. The resulting precipitate was filtered and washed with water (250 mL) and heptanes (50 mL) and then dried under high vacuum to give the title compound (11.82 g, 95.0% yield). LCMS: [M+H] 215.0. ¹H NMR: (400 MHz, DMSO-d6) δ 12.55 (s, 1H), 8.12 (s, 1H), 4.27 (q, *J*=7.11 Hz, 2H), 2.19 (s, 3H), 1.28 (t, *J*=7.15 Hz, 3H). 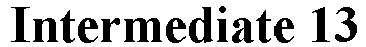 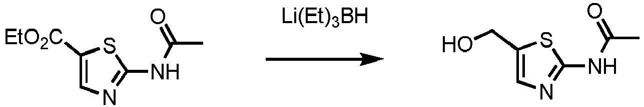 ***N*-[5-(hydroxymethyl)thiazol-2-yl]acetamide:** To a stirred solution of ethyl 2-acetamidothiazole-5-carboxylate (1.00 g, 4.67 mmol) in toluene (24 mL) was added lithium triethylborohydride (1 M in tetrahydrofuran, 9.39 mL, 9.39 mmol) slowly at 0 °C. The reaction mixture was stirred at room temperature for 2.5 hours. The reaction was cooled to 0 °C and additional lithium triethylborohydride (1 M in tetrahydrofuran, 9.39 mL, 9.39 mmol) was added slowly. The reaction was stirred 2 hours at room temperature, then cooled to 0 °C. Methanol (2 mL) was added very slowly added producing vigorous gas evolution. 5% citric acid solution was added and the mixture was stirred for 10 minutes at room temperature. The mixture was extracted with ethyl acetate, washed with saturated aqueous sodium chloride, dried over magnesium sulfate, filtered and evaporated. Purification was by silica gel chromatography using 0-10% methanol in dichloromethane as eluent to give the title compound (319.7 mg, 39.8 % yield). LCMS: [M+H] 172.9. ¹H NMR: (400 MHz, DMSO-d6) δ 11.93 (s, 1H), 7.25 (s, 1H), 5.33 (t, *J*=5.65 Hz, 1H), 4.57 (dd, *J*=0.88, 5.65 Hz, 2H), 2.12 (s, 3H). 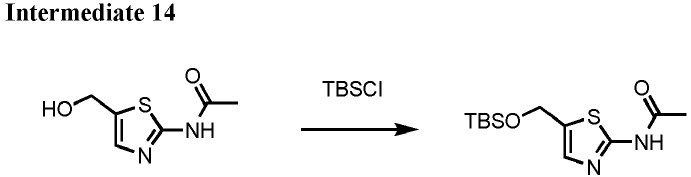 ***N*-[5-[[tert-butyl(dimethyl)silyl]oxymethyl]thiazol-2-yl]acetamide:** To a stirred mixture of *N*-[5-(hydroxymethyl)thiazol-2-yl]acetamide (319.7 mg, 1.86 mmol), 4-dimethylaminopyridine (22.7 mg, 186 umol), and triethylamine (515 uL, 3.71 mmol) in dichloromethane (9.45 mL) at 0 °C was added tert-butyl-chloro-dimethyl-silane (307.8 mg, 2.04 mmol). The reaction was stirred at room temperature overnight. The reaction was diluted with ethyl acetate, washed with 5% aqueous citric acid, washed with saturated aqueous sodium chloride, dried with magnesium sulfate, filtered, evaporated. Residue was purified by silica gel chromatography using 0-100% ethyl acetate in heptanes as eluent to give the title compound (451.6 mg, 84.8% yield). LCMS: [M+H] 286.9. ¹H NMR: (400 MHz, DMSO-d6) δ 11.99 (s, 1H), 7.30 (s, 1H), 4.80 (d, *J*=0.75 Hz, 2H), 2.12 (s, 3H), 0.86 (s, 9H), 0.07 (s, 6H). 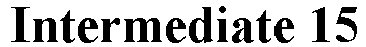 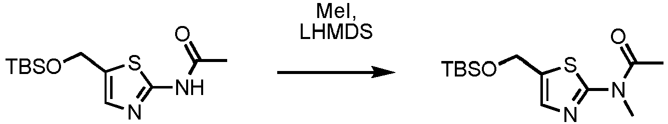 ***N*-[5-[[tert-butyl(dimethyl)silyl]oxymethyl]thiazol-2-yl]-N-methyl-acetamide:** To a solution of *N*-[5-[[tert-butyl(dimethyl)silyl]oxymethyl]thiazol-2-yl]acetamide (584.7 mg, 2.04 mmol) in tetrahydrofuran (7.5 mL) at 0 °C was added lithium bis(trimethylsilyl)amide (1.0 M in tetrahydrofuran, 2.43 mL, 2.43 mmol). The resulting suspension was stirred for 1 hour at 0 °C. To the mixture was added iodomethane (421 uL, 3.06 mmol) and the mixture was allowed to warm to room temperature. After stirring for 4 hours, the mixture was diluted with ethyl acetate, then washed with saturated aqueous ammonium chloride. The organics were dried over magnesium sulfate, filtered and concentrated *in vacuo.* The material was purified over silica gel using 0-100% ethyl acetate in heptanes as eluent to provide the title compound (335.2, 54.7% yield). LCMS: [M+H] 300.9. 1H NMR: (400 MHz, METHANOL-d4) δ 7.31 (s, 1H), 4.86 (d, *J*=1.00 Hz, 2H), 3.68 (s, 3H), 2.41 (s, 3H), 0.92 (s, 9H), 0.11 (s, 6H). 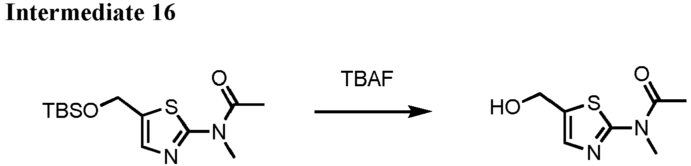 ***N*-[5-(hydroxymethyl)thiazol-2-yl]-*N*-methyl-acetamide:** *N*-[5-[[tert-butyl(dimethyl)silyl] oxymethyl]thiazol-2-yl]-N-methyl-acetamide (335.2 mg, 1.12 mmol) was dissolved in tetrahydrofuran (6.4 mL). To this was added tetrabutylammonium fluoride (1 M in tetrahydrofuran, 2.23 mL, 2.23 mmol) dropwise at 0 °C. After 30 min, the mixture was diluted with ethyl acetate, washed with saturated aqueous ammonium chloride, washed with brine, dried over magnesium sulfate, filtered, and concentrated *in vacuo.* The residue was purified by silica gel chromatography using 0-10% methanol in methylene chloride as eluent to give the title compound (159.8 mg, 76.6% yield). LCMS: [M+H] 186.9. ¹H NMR: (400 MHz, METHANOL-d4) δ 7.35 (s, 1H), 4.71 (d, *J*=0.75 Hz, 2H), 3.68 (s, 3H), 2.41 (s, 3H). 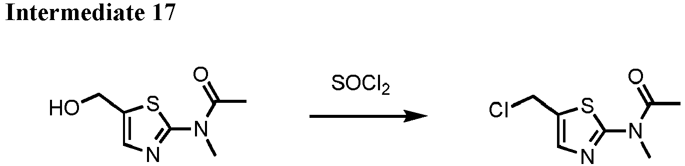 ***N*-[5-(chloromethyl)thiazol-2-yl]-*N*-methyl-acetamide:** To a stirred solution of *N*-[5-(hydroxymethyl)thiazol-2-yl]-N-methyl-acetamide (50.0 mg, 268 umol) in dichloromethane (5.0 mL), thionyl chloride (60.0 uL, 822 umol) was added slowly at 0 °C. The reaction was heated to reflux for 1.5 hours. The reaction was concentrated under reduced pressure. The residue was dissolved in dichloromethane then evaporated (repeat) to provide the title compound. LCMS: [M+H] 201.1 for methyl ether (MeOH as solvent for analytical sample).

### Example 3-16

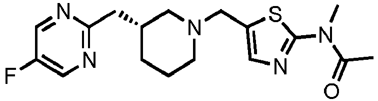

***N*-[5-[[(3R)-3-[(5-fluoropyrimidin-2-yl)methyl]-1-piperidyl]methyl]thiazol-2-yl]-N-methyl-acetamide:** The title compound was prepared in an analogous manner of that in scheme 3 from 5-fluoro-2-(piperidin-3-ylmethyl)pyrimidine HCl and *N*-[5-(chloromethyl)thiazol-2-yl]-*N*-methyl-acetamide. LCMS: [M+H] 364.2. ¹H NMR (400 MHz, METHANOL-d4) δ 8.63 (d, *J*=0.75 Hz, 2H), 7.28 (s, 1H), 3.67 (s, 2H), 3.66 (s, 3H), 2.78-2.87 (m, 4H), 2.41 (s, 3H), 2.23 (m, 1H), 2.02-2.10 (m, 1H), 1.86 (t, *J*=10.67 Hz, 1H), 1.65-1.74 (m, 2H), 1.50-1.63 (m, 1H), 0.99-1.11 (m, 1H).

### Example 3-17

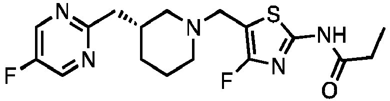

**(*R*)*-N-*(4-fluoro-5-((3-((5-fluoropyrimidin-2-yl)methyl)piperidin-1-yl)methyl)thiazol-2-yl)propionamide:** The title compound was prepared according to the general procedure described in scheme 3 and using (*R*)-5-fluoro-2-(piperidin-3-ylmethyl)pyrimidine and *N*-(4-fluoro-5-formylthiazol-2-yl)propionamide. LCMS (ESI): [M+H] 382. ¹HNMR: (400 MHz, Methanol-d₄) δ 8.64 (d, *J*=0.75 Hz, 2H), 3.64 (s, 2H), 2.73-3.00 (m, 4H), 2.46 (q, *J*=7.53 Hz, 2H), 2.08-2.31 (m, 2H), 1.91-2.04 (m, 1H), 1.49-1.79 (m, 3H), 1.19 (t, *J*=7.53 Hz, 3H), 0.98-1.13 (m, 1H).

### Example 3-18

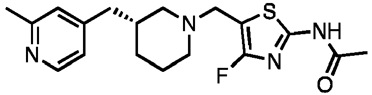

**(*R*)*-N-*(4-fluoro-5-((3-((2-methylpyridin-4-yl)methyl)piperidin-1-yl)methyl)thiazol-2-yl)acetamide:** The title compound was prepared according to the general procedure described in scheme 3 and using (*R*)-2-methyl-4-(piperidin-3-ylmethyl)pyridine and *N*-(4-fluoro-5-formylthiazol-2-yl)acetamide. LCMS (ESI): [M+H] 363. ¹HNMR: (400 MHz, Methanol-d₄) δ 8.24 (d, *J*=5.27 Hz, 1H), 7.11 (s, 1H), 7.04 (dd, *J*=1.25, 5.27 Hz, 1H), 3.59 (s, 2H), 2.69-2.92 (m, 2H), 2.54 (d, *J*=7.03 Hz, 2H), 2.47 (s, 3H), 2.18 (s, 3H), 2.07-2.22 (m, 1H), 1.78-1.92 (m, 2H), 1.62-1.78 (m, 2H), 1.45-1.62 (m, 1H), 0.93-1.15 (m, 1H).

### Example 3-19

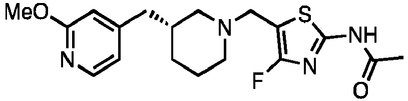

**(*R*)*-N-*(4-fluoro-5-((3-((2-methoxypyridin-4-yl)methyl)piperidin-1-yl)methyl)thiazol-2-yl)acetamide:** The title compound was prepared according to the general procedure described in scheme 3 and using (*R*)-2-methoxy-4-(piperidin-3-ylmethyl)pyridine and *N*-(4-fluoro-5-formylthiazol-2-yl)acetamide. LCMS (ESI): [M+H] 379. ¹HNMR: (400 MHz, Methanol-d₄) δ 7.97 (d, *J*=5.27 Hz, 1H), 6.78 (dd, *J*=1.38, 5.40 Hz, 1H), 6.60 (s, 1H), 3.87 (s, 3H), 3.56 (s, 2H), 2.68-2.88 (m, 2H), 2.43-2.57 (m, 2H), 2.18 (s, 3H), 2.01-2.12 (m, 1H), 1.76-1.96 (m, 2H), 1.63-1.73 (m, 2H), 1.46-1.61 (m, 1H), 0.89-1.11 (m, 1H).

### Example 3-20

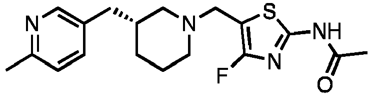

**(*R*)*-N-*(4-fluoro-5-((3-((6-methylpyridin-3-yl)methyl)piperidin-1-yl)methyl)thiazol-2-yl)acetamide:** The title compound was prepared according to the general procedure described in scheme 3 and using (*R*)-2-methyl-5-(piperidin-3-ylmethyl)pyridine and *N*-(4-fluoro-5-formylthiazol-2-yl)acetamide. LCMS (ESI): [M+H] 363. ¹HNMR: (400 MHz, Methanol-d₄) δ 8.19 (d, *J*=1.76 Hz, 1H), 7.54 (dd, *J*=2.26, 7.78 Hz, 1H), 7.21 (d, *J*=7.78 Hz, 1H), 3.60 (s, 2H), 2.71-2.92 (m, 2H), 2.53 (br d, *J*=5.52 Hz, 2H), 2.48 (s, 3H), 2.18 (s, 3H), 2.04-2.13 (m, 1H), 1.77-1.91 (m, 2H), 1.60-1.74 (m, 2H), 1.43-1.57 (m, 1H), 0.85-1.10 (m, 1H).

### Example 3-21

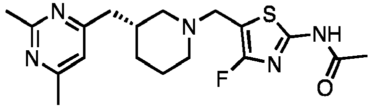

**(*R*)-*N*-(5-((3-((2,6-dimethylpyrimidin-4-yl)methyl)piperidin-1-yl)methyl)-4-fluorothiazol-2-yl)acetamide:** The title compound was prepared according to the general procedure described in scheme 3 and using (R)-2,4-dimethyl-6-(piperidin-3-ylmethyl)pyrimidine and *N*-(4-fluoro-5-formylthiazol-2-yl)acetamide. LCMS (ESI): [M+H] 378. ¹HNMR: (400 MHz, Methanol-d₄) δ 7.05 (s, 1H), 3.48-3.67 (m, 2H), 2.68-2.88 (m, 2H), 2.52-2.65 (m, 2H), 2.58 (s, 3H), 2.43 (s, 3H), 2.18 (s, 3H), 1.97-2.16 (m, 2H), 1.78-1.90 (m, 1H), 1.63-1.74 (m, 2H), 1.47-1.61 (m, 1H), 0.96-1.16 (m, 1H).

### Example 3-22

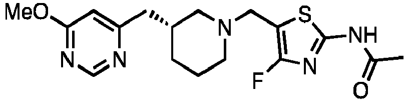

**(*R*)*-N-*(4-fluoro-5-((3-((6-methoxypyrimidin-4-yl)methyl)piperidin-1-yl)methyl)thiazol-2-yl)acetamide:** The title compound was prepared according to the general procedure described in scheme 3 and using (*R*)-4-methoxy-6-(piperidin-3-ylmethyl)pyrimidine and *N-*(4-fluoro-5-formylthiazol-2-yl)acetamide. LCMS (ESI): [M+H] 380. ¹HNMR: (400 MHz, Methanol-d₄) δ 8.61 (d, *J*=1.00 Hz, 1H), 6.71 (d, *J*=1.00 Hz, 1H), 3.97 (s, 3H), 3.52-3.66 (m, 2H), 2.75-2.88 (m, 2H), 2.48-2.66 (m, 2H), 2.18 (s, 3H), 1.99-2.12 (m, 2H), 1.81-1.95 (m, 1H), 1.47-1.78 (m, 3H), 0.95-1.17 (m, 1H).

### Example 3-23 (Reference)

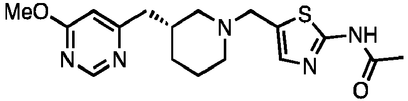

**(*R*)*-N-*(5-((3-((6-methoxypyrimidin-4-yl)methyl)piperidin-1-yl)methyl)thiazol-2-yl)acetamide:** The title compound was prepared according to the general procedure described in scheme 3 and using (*R*)-4-methoxy-6-(piperidin-3-ylmethyl)pyrimidine. LCMS (ESI): [M+H] 362. ¹HNMR: (400 MHz, Methanol-d₄) δ 8.60 (d, *J*=1.00 Hz, 1H), 7.21 (s, 1H), 6.71 (d, *J*=1.00 Hz, 1H), 3.96(s, 3H), 3.60-3.75 (m, 2H), 2.67-2.90 (m, 2H), 2.44-2.67 (m, 2H), 2..20(s, 3H), 1.96-2.16 (m, 2H), 1.76-1.95 (m, 1H), 1.46-1.76 (m, 3H), 0.96-1.15 (m, 1H).

### Example 3-24

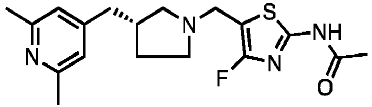

**(*S*)-*N*-(5-((3-((2,6-dimethylpyridin-4-yl)methyl)pyrrolidin-1-yl)methyl)-4-fluorothiazol-2-yl)acetamide:** The title compound was prepared in an analogous manner of that in scheme 3 from (*S*)-2,6-dimethyl-4-(pyrrolidin-3-ylmethyl)pyridine and *N*-(4-fluoro-5-formylthiazol-2-yl)acetamide. LCMS (ESI): [M+H] 363. ¹H NMR (400 MHz, Methanol-d₄) δ 6.93 (s, 2H), 3.62-3.77 (m, 2H), 2.60-2.77 (m, 5H), 2.48-2.58 (m, 1H), 2.44 (s, 6H), 2.27-2.36 (m, 1H), 2.18 (s, 3H), 1.94-2.07 (m, 1H), 1.47-1.58 (m, 1H).

### Example 3-25

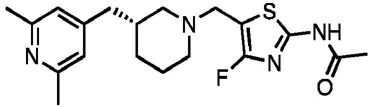

**(*R*)-*N*-(5-((3-((2,6-dimethylpyridin-4-yl)methyl)piperidin-l-yl)methyl)-4-fluorothiazol-2-yl)acetamide:** The title compound was prepared in an analogous manner of that in scheme 3 from (*R*)-2,6-dimethyl-4-(piperidin-3-ylmethyl)pyridine and *N*-(4-fluoro-5-formylthiazol-2-yl)acetamide. LCMS (ESI): [M+H] 377. ¹H NMR (400 MHz, Methanol-d₄) δ 6.89 (s, 2H), 3.51-3.63 (m, 2H), 2.82 (br d, *J*=11.04 Hz, 1H), 2.73 (br d, *J*=9.79 Hz, 1H), 2.49 (d, *J*=7.03 Hz, 2H), 2.43 (s, 6H), 2.18 (s, 3H), 2.05-2.15 (m, 1H), 1.76-1.94 (m, 2H), 1.63-1.75 (m, 2H), 1.49-1.62 (m, 1H), 0.93-1.09 (m, 1H). LCMS (ESI): [M+H] 377.

### Example 3-26

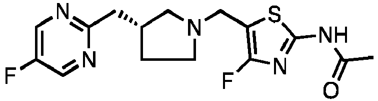

**(*R*)*-N-*(4-fluoro-5-((3-((5-fluoropyrimidin-2-yl)methyl)pyrrolidin-1-yl)methyl)thiazol-2-yl)acetamide:** The title compound was prepared in an analogous manner of that in scheme 3 from (*R*)-5-fluoro-2-(pyrrolidin-3-ylmethyl)pyrimidine and *N*-(4-fluoro-5-formylthiazol-2-yl)acetamide. LCMS (ESI): [M+H] 354. ¹H NMR (400 MHz, Methanol-d₄) δ 8.64 (d, *J*=0.75 Hz, 2H), 3.65-3.79 (m, 2H), 3.02 (d, *J*=7.53 Hz, 2H), 2.56-2.92 (m, 4H), 2.40 (dd, *J*=6.78, 9.03 Hz, 1H), 2.18 (s, 3H), 1.96-2.10 (m, 1H), 1.61 (tdd, *J*=6.40, 8.28, 12.80 Hz, 1H).

### Example 3-27

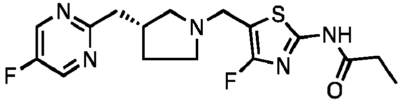

**(*R*)*-N-*(4-fluoro-5-((3-((5-fluoropyrimidin-2-yl)methyl)pyrrolidin-1-yl)methyl)thiazol-2-yl)propionamide:** The title compound was prepared in an analogous manner of that in scheme 3 from (*R*)-5-fluoro-2-(pyrrolidin-3-ylmethyl)pyrimidine and *N*-(4-fluoro-5-formylthiazol-2-yl)propionamide. LCMS (ESI): [M+H] 368. ¹H NMR (400 MHz, Methanol-d₄) δ 8.69 (s, 2H), 4.52 (s, 2H), 3.50-3.92 (m, 2H), 3.35-3.49 (m, 1H), 2.89-3.28 (m, 4H), 2.50 (q, *J*=7.53 Hz, 2H), 2.22-2.45 (m, 1H), 1.70-2.01 (m, 1H), 1.20 (t, *J*=7.53 Hz, 3H).

### Example 3-28

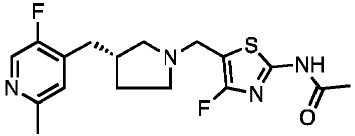

**(*S*)-*N*-(4-fluoro-5-((3-((5-fluoro-2-methylpyridin-4-yl)methyl)pyrrolidin-1-yl)methyl)thiazol-2-yl)acetamide:** The title compound was prepared in an analogous manner of that in scheme 3 from (*S*)-5-fluoro-2-methyl-4-(pyrrolidin-3-ylmethyl)pyridine and *N-*(4-fluoro-5-formylthiazol-2-yl)acetamide. LCMS (ESI): [M+H] 367. ¹H NMR (400 MHz, Methanol-d₄) δ 8.57 (d, *J*=2.76 Hz, 1H), 7.62 (d, *J*=6.27 Hz, 1H), 4.49-4.57 (m, 2H), 3.44-3.78 (m, 3H), 2.97-3.08 (m, 2H), 2.75-2.93 (m, 1H), 2.64 (s, 3H), 2.15-2.36 (m, 5H), 1.80-2.00 (m, 1H).

### Example 3-29

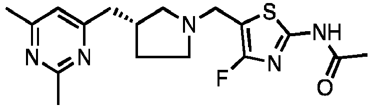

**(*R*)*-N-*(5-((3-((2,6-dimethylpyrimidin-4-yl)methyl)pyrrolidin-1-yl)methyl)-4-fluorothiazol-2-yl)acetamide:** The title compound was prepared in an analogous manner of that in scheme 3 from (*R*)-2,4-dimethyl-6-(pyrrolidin-3-ylmethyl)pyrimidine and *N*-(4-fluoro-5-formylthiazol-2-yl)acetamide. LCMS (ESI): [M+H] 364. ¹H NMR (400 MHz, Methanol-d₄) δ 7.42 (s, 1H), 4.52 (s, 2H), 3.36-3.85 (m, 4H), 2.94-3.08 (m, 3H), 2.74 (s, 3H), 2.60 (s, 3H), 2.24-2.39 (m, 1H), 2.21 (s, 3H), 1.80-1.99 (m, 1H).

### Example 3-30

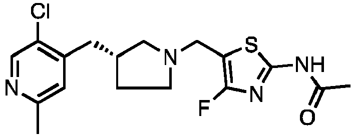

**(*S*)-*N*-(5-((3-((5-chloro-2-methylpyridin-4-yl)methyl)pyrrolidin-1-yl)methyl)-4-fluorothiazol-2-yl)acetamide:** The title compound was prepared in an analogous manner of that in scheme 3 from (*S*)-5-chloro-2-methyl-4-(pyrrolidin-3-ylmethyl)pyridine and *N*-(4-fluoro-5-formylthiazol-2-yl)acetamide. LCMS (ESI): [M+H] 383. ¹H NMR (400 MHz, Methanol-d₄) δ 8.61 (s, 1H), 7.55 (s, 1H), 4.52 (d, *J*=1.51 Hz, 2H), 3.38-3.84 (m, 3H), 3.22 (br d, *J*=8.03 Hz, 1H), 2.98-3.13 (m, 2H), 2.72-2.96 (m, 1H), 2.61 (s, 3H), 2.14-2.34 (m, 4H), 1.92 (br d, *J*=16.06 Hz, 1H).

### Example 3-31

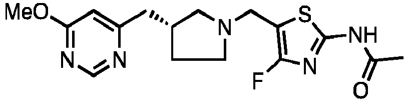

**(*R*)*-N-*(4-fluoro-5-((3-((6-methoxypyrimidin-4-yl)methyl)pyrrolidin-1-yl)methyl)thiazol-2-yl)acetamide:** The title compound was prepared in an analogous manner of that in scheme 3 from (*R*)-4-methoxy-6-(pyrrolidin-3-ylmethyl)pyrimidine and *N*-(4-fluoro-5-formylthiazol-2-yl)acetamide. LCMS (ESI): [M+H] 366. ¹H NMR (400 MHz, Methanol-d₄) δ 8.63 (d, *J*=0.75 Hz, 1H), 6.75 (d, *J*=0.75 Hz, 1H), 3.97 (s, 3H), 3.82 (d, *J*=0.75 Hz, 2H), 2.91 (dd, *J*=7.53, 9.54 Hz, 1H), 2.75-2.85 (m, 4H), 2.65-2.73 (m, 1H), 2.47 (dd, *J*=7.15, 9.66 Hz, 1H), 2.19 (s, 3H), 2.00-2.07 (m, 1H), 1.55-1.66 (m, 1H).

### Example 3-32

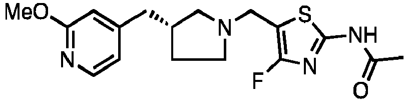

**(*S*)-*N*-(4-fluoro-5-((3-((2-methoxypyridin-4-yl)methyl)pyrrolidin-1-yl)methyl)thiazol-2-yl)acetamide:** The title compound was prepared in an analogous manner of that in scheme 3 from (*S*)-2-methoxy-4-(pyrrolidin-3-ylmethyl)pyridine and *N*-(4-fluoro-5-formylthiazol-2-yl)acetamide. LCMS (ESI): [M+H] 365. ¹H NMR (400 MHz, Methanol-d₄) δ 7.99 (d, *J*=5.27 Hz, 1H), 6.81 (dd, *J*=1.25, 5.27 Hz, 1H), 6.64 (s, 1H), 3.87 (s, 3H), 3.83 (d, *J*=2.51 Hz, 2H), 2.89 (dd, *J*=7.40, 9.66 Hz, 1H), 2.74-2.85 (m, 2H), 2.68 (dd, *J*=2.38, 7.65 Hz, 2H), 2.56 (td, *J*=7.87, 15.12 Hz, 1H), 2.44 (dd, *J*=7.28, 9.54 Hz, 1H), 2.18 (s, 3H), 1.96-2.03 (m, 1H), 1.51-1.66 (m, 1H).

### Example 3-33

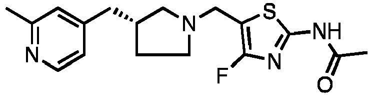

**(*S*)-*N*-(4-fluoro-5-((3-((2-methylpyridin-4-yl)methyl)pyrrolidin-1-yl)methyl)thiazol-2-yl)acetamide:** The title compound was prepared in an analogous manner of that in scheme 3 from (*S*)-2-methoxy-4-(pyrrolidin-3-ylmethyl)pyridine and *N*-(4-fluoro-5-formylthiazol-2-yl)acetamide. LCMS (ESI): [M+H] 349. ¹H NMR (400 MHz, Methanol-d₄) δ 8.26 (d, *J*=5.27 Hz, 1H), 7.15 (s, 1H), 7.08 (dd, *J*=1.00, 5.27 Hz, 1H), 3.62-3.78 (m, 2H), 2.62-2.79 (m, 5H), 2.50-2.60 (m, 1H), 2.48 (s, 3H), 2.31 (dd, *J*=6.90, 9.41 Hz, 1H), 2.16-2.20 (m, 3H), 1.94-2.05 (m, 1H), 1.46-1.61 (m, 1H).

### Example 3-34

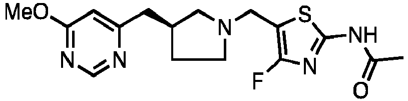

**(*S*)-*N*-(4-fluoro-5-((3-((6-methoxypyrimidin-4-yl)methyl)pyrrolidin-1-yl)methyl)thiazol-2-yl)acetamide:** The title compound was prepared in an analogous manner of that in scheme 3 from (*S*)-4-methoxy-6-(pyrrolidin-3-ylmethyl)pyrimidine and *N*-(4-fluoro-5-formylthiazol-2-yl)acetamide. LCMS (ESI): [M+H] 366. ¹H NMR (400 MHz, Methanol-d₄) δ 8.62 (d, *J*=1.00 Hz, 1H), 6.75 (d, *J*=1.00 Hz, 1H), 3.97 (s, 3H), 3.76 (d, *J*=1.51 Hz, 2H), 2.85 (dd, *J*=7.53, 9.54 Hz, 1H), 2.65-2.80 (m, 5H), 2.40 (dd, *J*=6.90, 9.41 Hz, 1H), 2.18 (s, 3H), 1.96-2.08 (m, 1H), 1.58 (tdd, *J*=6.49, 8.16, 12.99 Hz, 1H).

### Example 3-35

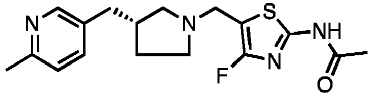

**(*S*)-*N*-(4-fluoro-5-((3-((6-methylpyridin-3-yl)methyl)pyrrolidin-1-yl)methyl)thiazol-2-yl)acetamide:** The title compound was prepared in an analogous manner of that in scheme 3 from (*S*)-4-methoxy-6-(pyrrolidin-3-ylmethyl)pyrimidine and *N*-(4-fluoro-5-formylthiazol-2-yl)acetamide. LCMS (ESI): [M+H] 349. ¹H NMR (400 MHz, Methanol-d₄) δ 8.23 (d, *J*=2.01 Hz, 1H), 7.58 (dd, *J*=2.38, 7.91 Hz, 1H), 7.21 (d, *J*=7.78 Hz, 1H), 3.64-3.76 (m, 2H), 2.61-2.80 (m, 5H), 2.43-2.55 (m, 4H), 2.31 (dd, *J*=6.78, 9.29 Hz, 1H), 2.18 (s, 3H), 1.94-2.00 (m, 1H), 1.54 (tdd, *J*=6.53, 8.22, 12.86 Hz, 1H).

### Example 3-36

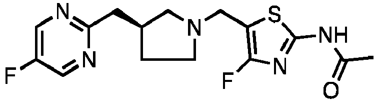

**(*S*)-*N*-(4-fluoro-5-((3-((5-fluoropyrimidin-2-yl)methyl)pyrrolidin-1-yl)methyl)thiazol-2-yl)acetamide:** The title compound was prepared in an analogous manner of that in scheme 3 from (*S*)-5-fluoro-2-(pyrrolidin-3-ylmethyl)pyrimidine and *N*-(4-fluoro-5-formylthiazol-2-yl)acetamide. LCMS (ESI): [M+H] 354. ¹H NMR (400 MHz, Methanol-d₄) δ 8.65 (d, *J*=0.75 Hz, 2H), 3.89 (s, 2H), 3.01-3.10 (m, 3H), 2.78-2.95 (m, 3H), 2.58 (dd, *J*=7.53, 10.04 Hz, 1H), 2.19 (s, 3H), 2.04-2.16 (m, 1H), 1.60-1.74 (m, 1H).

### Example 3-37

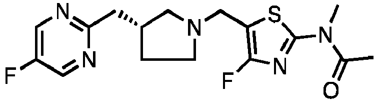

**(*R*)-*N*-(4-fluoro-5-((3-((5-fluoropyrimidin-2-yl)methyl)pyrrolidin-1-yl)methyl)thiazol-2-yl)-N-methylacetamide:** To a mixture of *N*-[4-fluoro-5-[[(3R)-3-[(5-fluoropyrimidin-2-yl)methyl]pyrrolidin-1-yl]methyl]thiazol-2-yl]acetamide (25 mg, 71 umol) and methanol (34 mg, 1.06 mmol), triphenylphosphine (37 mg, 142 umol) in THF (0.5 mL) was added isopropyl (NE)-N-isopropoxycarbonyliminocarbamate (43 mg, 212 umol). The reaction mixture was then stirred at RT overnight. Remove all the solvent. The crude was purified by chromatography on silica gel (solvent A: EtOAc, solvent B: 0-60%EtOAc-EtOH 3:1 with 2%NH4OH) to give the title compound as a white powder. LCMS (ESI): [M+H] 368. ¹H NMR (400 MHz, METHANOL-d₄) δ 8.64 (d, *J*=0.75 Hz, 2H), 3.66-3.76 (m, 2H), 3.59-3.64 (m, 3H), 3.02 (d, *J*=7.53 Hz, 2H), 2.68-2.89 (m, 3H), 2.64 (dt, *J*=6.02, 8.78 Hz, 1H), 2.35-2.44 (m, 4H), 1.96-2.08 (m, 1H), 1.61 (tdd, *J*=6.31, 8.28, 12.74 Hz, 1H).

### Example 3-38

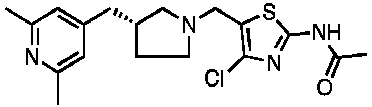

**(*S*)-*N*-(4-chloro-5-((3-((2,6-dimethylpyridin-4-yl)methyl)pyrrolidin-1-yl)methyl)thiazol-2-yl)acetamide:** The title compound was prepared in an analogous manner of that in scheme 3 from 4-methoxy-6-(1-(pyrrolidin-3-yl)ethyl)pyrimidine and *N*-(4-chloro-5-formylthiazol-2-yl)acetamide. LCMS (ESI): [M+H] 379. ¹H NMR (400 MHz, Methanol-d₄) δ 6.94 (s, 2H), 3.73-3.87 (m, 2H), 2.70-2.78 (m, 3H), 2.62-2.67 (m, 2H), 2.49-2.57 (m, 1H), 2.44 (s, 6H), 2.36 (dd, *J*=6.65, 9.41 Hz, 1H), 2.19 (s, 3H), 1.95-2.00 (m, 1H), 1.47-1.60 (m, 1H).

### Example 3-39

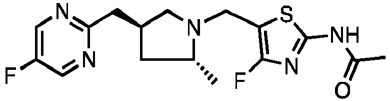

***N*-(4-fluoro-5-(((2*R*,4*S*)-4-((5-fluoropyrimidin-2-yl)methyl)-2-methylpyrrolidin-1-yl)methyl)thiazol-2-yl)acetamide:** The title compound was isolated through chiral separation (CHIRALPAK IG 30x250mm, 5um; Method: 30%MeOH w/ 0.1% DEA in CO₂ (flow rate: 100mL/min, ABPR 120bar, MBPR 40psi, column temp 40 C) of a mixture which was prepared in an analogous manner of that in scheme 3 from 5-fluoro-2-((5-methylpyrrolidin-3-yl)methyl)pyrimidine and *N*-(4-fluoro-5-formylthiazol-2-yl)acetamide. LCMS (ESI): [M+H] 368. ¹H NMR (400 MHz, Methanol-d₄) δ 8.63 (d, *J*=0.75 Hz, 2H), 3.86-3.96 (m, 1H), 3.55 (d, *J*=14.31 Hz, 1H), 3.12 (dd, *J*=7.28, 9.29 Hz, 1H), 2.95 (d, *J*=7.28 Hz, 2H), 2.53-2.83 (m, 2H), 2.11-2.24 (m, 4H), 1.73-1.85 (m, 1H), 1.64 (ddd, *J*=8.28, 9.79, 12.80 Hz, 1H), 1.15 (d, *J*=6.02 Hz, 3H).

### Example 3-40

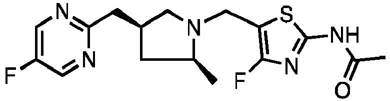

***N*-(4-fluoro-5-(((2*S*,4*S*)-4-((5-fluoropyrimidin-2-yl)methyl)-2-methylpyrrolidin-1-yl)methyl)thiazol-2-yl)acetamide:** The title compound was isolated through chiral separation (CHIRALPAK IG 30x250mm, 5um; Method: 30%MeOH w/ 0.1% DEA in CO₂ (flow rate: 100mL/min, ABPR 120bar, MBPR 40psi, column temp 40 C) of a mixture which was prepared in an analogous manner of that in scheme 3 from 5-fluoro-2-((5-methylpyrrolidin-3-yl)methyl)pyrimidine and *N*-(4-fluoro-5-formylthiazol-2-yl)acetamide. LCMS (ESI): [M+H] 368. ¹H NMR (400 MHz, Methanol-d₄) δ 8.63 (d, *J*=0.75 Hz, 2H), 3.90 (dd, *J*=1.00, 14.56 Hz, 1H), 3.52 (d, *J*=14.56 Hz, 1H), 3.01 (dd, *J*=2.26, 7.28 Hz, 2H), 2.87 (dd, *J*=3.64, 9.41 Hz, 1H), 2.52-2.70 (m, 3H), 2.18 (s, 3H), 2.11 (ddd, *J*=6.27, 8.28, 12.55 Hz, 1H), 1.21-1.29 (m, 1H), 1.16 (d, *J*=6.02 Hz, 3H).

### Example 3-41

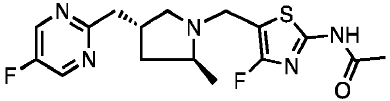

***N*-(4-fluoro-5-(((2*S*,4*R*)-4-((5-fluoropyrimidin-2-yl)methyl)-2-methylpyrrolidin-1-yl)methyl)thiazol-2-yl)acetamide:** The title compound was isolated through chiral separation (CHIRALPAK IG 30x250mm, 5um; Method: 30% MeOH w/ 0.1% DEA in CO₂ (flow rate: 100mL/min, ABPR 120bar, MBPR 40psi, column temp 40 C) of a mixture which was prepared in an analogous manner of that in scheme 3 from 5-fluoro-2-((5-methylpyrrolidin-3-yl)methyl)pyrimidine and *N*-(4-fluoro-5-formylthiazol-2-yl)acetamide. LCMS (ESI): [M+H] 368. ¹H NMR (400 MHz, Methanol-d₄) δ 8.63 (d, *J*=0.75 Hz, 2H), 3.91 (dd, *J*=0.75, 14.56 Hz, 1H), 3.55 (d, *J*=14.56 Hz, 1H), 3.12 (dd, *J*=7.03, 9.29 Hz, 1H), 2.95 (d, *J*=7.53 Hz, 2H), 2.67-2.80 (m, 1H), 2.57-2.67 (m, 1H), 2.12-2.22 (m, 4H), 1.79 (ddd, *J*=5.77, 7.65, 13.18 Hz, 1H),

### Example 3-42

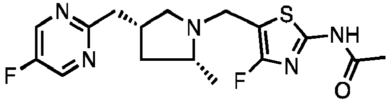

***N*-(4-fluoro-5-(((2*R*,4*R*)-4-((5-fluoropyrimidin-2-yl)methyl)-2-methylpyrrolidin-1-yl)methyl)thiazol-2-yl)acetamide:** The title compound was isolated through chiral separation (CHIRALPAK IG 30x250mm, 5um; Method: 30% MeOH w/ 0.1% DEA in CO₂ (flow rate: 100mL/min, ABPR 120bar, MBPR 40psi, column temp 40 C) of a mixture which was prepared in an analogous manner of that in scheme 3 from 5-fluoro-2-((5-methylpyrrolidin-3-yl)methyl)pyrimidine and *N*-(4-fluoro-5-formylthiazol-2-yl)acetamide. LCMS (ESI): [M+H] 368. ¹H NMR (400 MHz, Methanol-d₄) δ 8.63 (d, *J*=0.75 Hz, 1H), 8.55-8.71 (m, 1H), 3.90 (dd, *J*=1.00, 14.56 Hz, 1H), 3.52 (d, *J*=14.31 Hz, 1H), 3.01 (dd, *J*=2.26, 7.28 Hz, 2H), 2.87 (dd, *J*=3.76, 9.54 Hz, 1H), 2.59-2.71 (m, 1H), 2.51-2.59 (m, 2H), 2.18 (s, 3H), 2.11 (ddd, *J*=6.40, 8.22, 12.49 Hz, 1H), 1.22-1.29 (m, 1H), 1.16 (d, *J*=6.02 Hz, 3H).

### Example 3-43

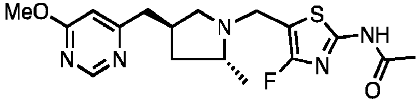

***N*-(4-fluoro-5-(((2*R*,4*S*)-4-((6-methoxypyrimidin-4-yl)methyl)-2-methylpyrrolidin-1-yl)methyl)thiazol-2-yl)acetamide:** The title compound was isolated through chiral separation (CHIRALPAK AD-H 30x250mm, 5um; Method: 45%MeOH w/ 0.1% DEA in CO₂ (flow rate: 100mL/min, ABPR 120bar, MBPR 40psi, column temp 40 C) of a mixture which was prepared in an analogous manner of that in scheme 3 from 4-methoxy-6-((5-methylpyrrolidin-3-yl)methyl)pyrimidine and *N*-(4-fluoro-5-formylthiazol-2-yl)acetamide. LCMS (ESI): [M+H] 380. ¹H NMR (400 MHz, Methanol-d₄) δ 8.61 (d, *J*=1.00 Hz, 1H), 6.73 (d, *J*=1.00 Hz, 1H), 3.97 (s, 3H), 3.88-3.94 (m, 1H), 3.55 (d, *J*=14.56 Hz, 1H), 3.07 (dd, *J*=6.90, 9.16 Hz, 1H), 2.53-2.73 (m, 4H), 2.18 (s, 3H), 2.12 (t, *J*=9.03 Hz, 1H), 1.68-1.79 (m, 1H), 1.55-1.66 (m, 1H), 1.14 (d, *J*=6.02 Hz, 3H).

### Example 3-44

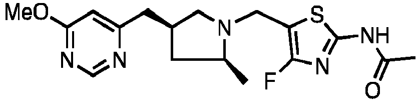

***N*-(4-fluoro-5-(((2*S*,4*S*)-4-((6-methoxypyrimidin-4-yl)methyl)-2-methylpyrrolidin-1-yl)methyl)thiazol-2-yl)acetamide:** The title compound was isolated through chiral separation (CHIRALPAK AD-H 30x250mm, 5um; Method: 45% MeOH w/ 0.1% DEA in CO₂ (flow rate: 100mL/min, ABPR 120bar, MBPR 40psi, column temp 40 C) of a mixture which prepared in an analogous manner of that in scheme 3 from 4-methoxy-6-((5-methylpyrrolidin-3-yl)methyl)pyrimidine and *N*-(4-fluoro-5-formylthiazol-2-yl)acetamide. LCMS (ESI): [M+H] 380. ¹H NMR (400 MHz, Methanol-d₄) δ 8.61 (d, *J*=1.00 Hz, 1H), 6.72 (d, *J*=1.00 Hz, 1H), 3.96 (s, 3H), 3.91 (dd, *J*=1.00, 14.56 Hz, 1H), 3.51 (d, *J*=14.31 Hz, 1H), 2.67-2.82 (m, 3H), 2.46-2.63 (m, 3H), 2.19 (s, 3H), 2.04-2.16 (m, 1H), 1.20-1.25 (m, 1H), 1.18 (d, *J*=6.02 Hz, 3H).

### Example 3-45

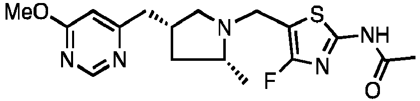

***N*-(4-fluoro-5-(((2*R*,4*R*)-4-((6-methoxypyrimidin-4-yl)methyl)-2-methylpyrrolidin-1-yl)methyl)thiazol-2-yl)acetamide:** The title compound was isolated through chiral separation (CHIRALPAK AD-H 30x250mm, 5um; Method: 45% MeOH w/ 0.1% DEA in CO₂ (flow rate: 100mL/min, ABPR 120bar, MBPR 40psi, column temp 40 C) of a mixture which prepared in an analogous manner of that in scheme 3 from 4-methoxy-6-((5-methylpyrrolidin-3-yl)methyl)pyrimidine and *N*-(4-fluoro-5-formylthiazol-2-yl)acetamide. LCMS (ESI): [M+H] 380. ¹H NMR (400 MHz, Methanol-d₄) δ 8.61 (d, *J*=1.00 Hz, 1H), 6.72 (d, *J*=1.00 Hz, 1H), 3.96 (s, 3H), 3.91 (dd, *J*=1.13, 14.43 Hz, 1H), 3.51 (d, *J*=14.31 Hz, 1H), 2.68-2.81 (m, 3H), 2.46-2.61 (m, 3H), 2.19 (s, 3H), 2.05-2.15 (m, 1H), 1.19-1.24 (m, 1H), 1.17 (d, *J*=6.02 Hz, 3H).

### Example 3-46

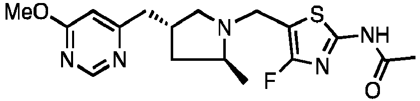

***N*-(4-fluoro-5-(((2*S*,4*R*)-4-((6-methoxypyrimidin-4-yl)methyl)-2-methylpyrrolidin-1-yl)methyl)thiazol-2-yl)acetamide:** The title compound was isolated through chiral separation (CHIRALPAK AD-H 30x250mm, 5um; Method: 45% MeOH w/ 0.1% DEA in CO₂ (flow rate: 100mL/min, ABPR 120bar, MBPR 40psi, column temp 40 C) of a mixture which prepared in an analogous manner of that in scheme 3 from 4-methoxy-6-((5-methylpyrrolidin-3-yl)methyl)pyrimidine and *N*-(4-fluoro-5-formylthiazol-2-yl)acetamide. LCMS (ESI): [M+H] 380. ¹H NMR (400 MHz, Methanol-d₄) δ 8.61 (d, *J*=1.00 Hz, 1H), 6.73 (d, *J*=1.00 Hz, 1H), 3.97 (s, 3H), 3.88-3.94 (m, 1H), 3.55 (d, *J*=14.56 Hz, 1H), 3.07 (dd, *J*=6.90, 9.16 Hz, 1H), 2.56-2.72 (m, 4H), 2.18 (s, 3H), 2.12 (t, *J*=9.16 Hz, 1H), 1.69-1.79 (m, 1H), 1.56-1.67 (m, 1H), 1.14 (d, *J*=6.02 Hz, 3H).

### Example 3-47

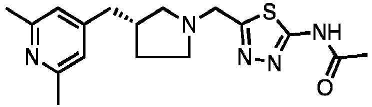

**(*S*)-*N*-(5-((3-((2,6-dimethylpyridin-4-yl)methyl)pyrrolidin-1-yl)methyl)-1,3,4-thiadiazol-2-yl)acetamide:** To a mixture of 2,6-dimethyl-4-[[(3*S*)-pyrrolidin-3-yl]methyl]pyridine (200 mg, 0.31 mmol, trifluoroacetic acid) and 5-(chloromethyl)-1,3,4-thiadiazol-2-amine (91 mg, 0.37 mmol, methanesulfonic acid) in acetonitrile (2.00 mL) and DMF (1.0 mL) was added diisopropylethylamine (320 mg, 2.48 mmol). The reaction was stirred at room temperature for 2h. The mixture was concentrated *in vacuo,* diluted with EtOAc, washed with brine, and the crude residue was purified by chromatography on silica gel (20-100% EtOAc-EtOH 3:1 with 2%NH₄OH in heptane) to give 5-[[(3*S*)-3-[(2,6-dimethyl-4-pyridyl)methyl]pyrrolidin-1-yl]methyl]-1,3,4-thiadiazol-2-amine (26 mg, LCMS (ESI): [M+H] 304) which was dissolved in dichloromethane (1.0 mL) stirred at room temperature, diisopropylethylamine (21 mg, 0.17 mmol) was then added, followed by acetic anhydride (10 mg, 0.99 mmol), and the mixture was stirred at room temperature overnight. The mixture was concentrated *in* vacuo and residue was purified by preparative TLC to afford the title compound (6 mg, 26% yield). LCMS (ESI): [M+H] 346. ¹H NMR (400 MHz, Methanol-d₄) δ 7.21 (br s, 2H), 4.09-4.25 (m, 2H), 3.72 (br d, *J*=4.77 Hz, 1H), 2.93 (br d, *J*=6.78 Hz, 2H), 2.80 (d, *J*=7.28 Hz, 2H), 2.57-2.71 (m, 2H), 2.55 (s, 6H), 2.25 (s, 2H), 2.24-2.26 (m, 1H), 2.03-2.11 (m, 1H), 1.60-1.68 (m, 1H).

### Example 3-48

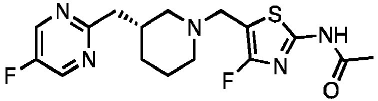

**(*R*)-*N*-(4-fluoro-5-((3-((5-fluoropyrimidin-2-yl)methyl)piperidin-1-yl)methyl)thiazol-2-yl)acetamide:** The title compound was prepared according to the general procedure described in scheme 3 and using (*R*)-5-fluoro-2-(piperidin-3-ylmethyl)pyrimidine and *N*-(4-fluoro-5-formylthiazol-2-yl)acetamide. LCMS (ESI): [M+H] 368. ¹HNMR: (400 MHz, Methanol-d₄) δ 8.63 (d, *J*=0.75 Hz, 2H), 3.56 (s, 2H), 2.70-2.95 (m, 4H), 2.14-2.29 (m, 1H), 2.18 (s, 3H), 2.02-2.12 (m, 1H), 1.82-1.94 (m, 1H), 1.64-1.78 (m, 2H), 1.46-1.62 (m, 1H), 0.92-1.15 (m, 1H).

### Example 3-49

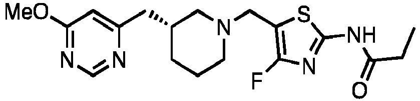

**(*R*)-*N*-(4-fluoro-5-((3-((6-methoxypyrimidin-4-yl)methyl)piperidin-1-yl)methyl)thiazol-2-yl)propionamide:** The title compound was prepared according to the general procedure described in scheme 3 and using (R)-4-methoxy-6-(piperidin-3-ylmethyl)pyrimidine and *N-*(4-fluoro-5-formylthiazol-2-yl)propionamide. LCMS (ESI): [M+H] 394. ¹HNMR: (400 MHz, Methanol-d₄) δ 8.61 (d, *J*=1.00 Hz, 1H), 6.72 (d, *J*=1.00 Hz, 1H), 3.97 (s, 3H), 3.60 (s, 2H), 2.70-2.91 (m, 2H), 2.51-2.67 (m, 2H), 2.39-2.52 (m, 2H), 2.00-2.23 (m, 2H), 1.82-1.97 (m, 1H), 1.44-1.77 (m, 3H), 1.19 (t, *J*=7.53 Hz, 3H), 0.89-1.09 (m, 1H). 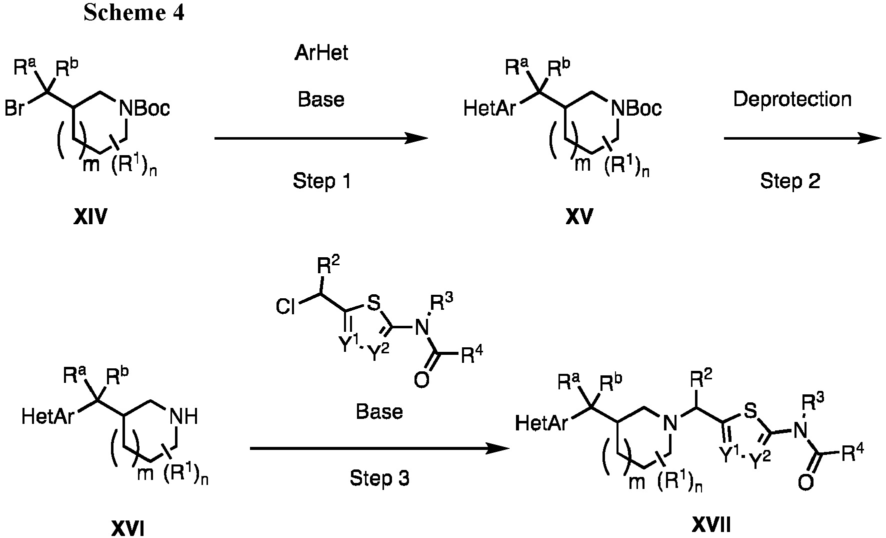 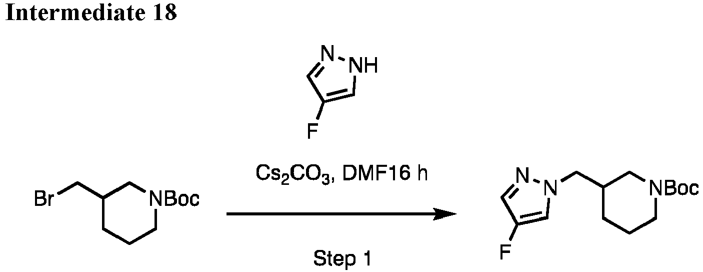 ***tert-butyl* 3-((4-fluoro-1*H*-pyrazol-1-yl)methyl)piperidine-1-carboxylate:** 4-Fluoro-1*H-*pyrazole (0.10 g, 1.16 mmol), cesium carbonate (1.14 g, 3.50 mmol), and tert-butyl 3-(bromomethyl)piperidine-1-carboxylate (0.323 g, 1.16 mmol) were suspended in DMF (5.00 mL) and the mixture was heated to 100°C for 16 h. The reaction mixture cooled to room temperature, filtered, and concentrated *in vacuo* to afford the title compound as a crude mixture. LCMS (ESI): [M+H] 284. 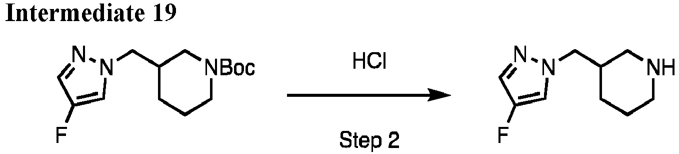 **3-((4-fluoro-1*H*-pyrazol-1-yl)methyl)piperidine hydrochloride:** A solution of HCl in dioxane (4.0 M, 4.76 mL, 27.0 mmol) was added to the crude mixture of *tert*-butyl 3-((4-fluoro-1*H*-pyrazol-1-yl)methyl)piperidine-1-carboxylate from the previous step and the mixture was stirred for 4 h at room temperature. The mixture was concentrated *in vacuo* to afford the title compound as a crude mixture. LCMS (ESI): [M+H] 184.

### Example 4-1 (Reference)

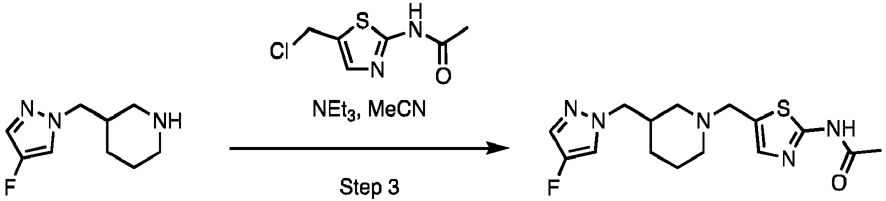

***N*-(5-((3-((4-fluoro-1*H*-pyrazol-1-yl)methyl)piperidin-1-yl)methyl)thiazol-2-yl)acetamide** A crude mixture of 3-((4-fluoro-1*H*-pyrazol-1-yl)methyl)piperidine hydrochloride from the previous step was dissolved in MeCN (4.00 mL). To the mixture was added triethylamine (0.46 g, 4.56 mmol, 0.63 mL) and *N*-[5-(chloromethyl)thiazol-2-yl]acetamide (0.145 mg, 0.760 mmol). The mixture was stirred for 16 h at room temperature. The reaction mixture was filtered and concentrated *in vacuo.* The residue was purified by Prep-HPLC {(Column: Waters Sunfire OBD 50×100mm, 5um; conditions: 95% water/5% ACN 20 minutes in 0.1% TFA (flow rate: 80mL/min))} to afford the title compound. LCMS (ESI): [M+H] 338. ¹HNMR: (500 MHz, Methanol-*d*4) δ 7.58 (d, *J*=4.6 Hz, 1H), 7.32-7.34 (m, 1H), 7.21 (s, 1H), 3.96 (d, *J*=7.3 Hz, 2H), 3.68 (s, 2H), 2.78 (br d, *J*=11.0 Hz, 1H), 2.64 (br d, *J*=9.8 Hz, 1H), 2.12-2.21 (m, 5H), 1.90 (br t, *J*=10.4 Hz, 1H), 1.69-1.77 (m, 1H), 1.52-1.64 (m, 2H), 1.01-1.10 (m, 1H).

### Example 4-2 (Reference)

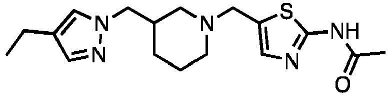

***N*-(5-((3-((4-ethyl-1*H*-pyrazol-1-yl)methyl)piperidin-1-yl)methyl)thiazol-2-yl)acetamide:** The title compound was prepared in an analogous manner of that in scheme 4 from tert-butyl 3-(bromomethyl)piperidine-1-carboxylate, 4-ethyl-1*H*-pyrazole, and *N*-[5-(chloromethyl)thiazol-2-yl]acetamide. LCMS (ESI): [M+H] 348. ¹HNMR: (500 MHz, Methanol-*d*4) δ 7.55 (s, 1H), 7.41 (s, 1H), 7.32-7.36 (m, 1H), 4.52 (q, *J*=14.4 Hz, 2H), 4.13 (br dd, *J*=14.0, 5.5 Hz, 1H), 4.02 (br dd, *J*=14.0, 7.9 Hz, 1H), 3.53 (br d, *J*=12.2 Hz, 1H), 3.22 (br d, *J*=11.6 Hz, 1H), 2.82-2.95 (m, 1H), 2.73 (br t, *J*=12.2 Hz, 1H), 2.44-2.50 (m, 2H), 2.30 (br s, 1H), 2.22-2.24 (m, 3H), 1.92-2.10 (m, 1H), 1.68-1.87 (m, 2H), 1.14-1.19 (t, 3H).

### Example 4-3 (Reference)

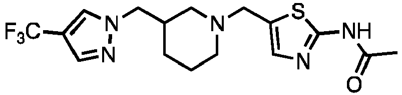

***N*-(5-((3-((4-(trifluoromethyl)-1*H*-pyrazol-1-yl)methyl)piperidin-1-yl)methyl)thiazol-2-yl)acetamide:** The title compound was prepared in an analogous manner of that in scheme 4 from *tert*-butyl 3-(bromomethyl)piperidine-1-carboxylate, 4-(trifluoromethyl)-1*H*-pyrazole, and *N*-[5-(chloromethyl)thiazol-2-yl]acetamide. LCMS (ESI): [M+H] 388.

### Example 4-4 (Reference)

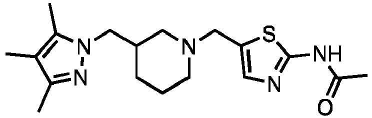

***N*-(5-((3-((3,4,5-trimethyl-1*H*-pyrazol-1-yl)methyl)piperidin-1-yl)methyl)thiazol-2-yl)acetamide:** The title compound was prepared in an analogous manner of that in scheme 4 from *tert*-butyl 3-(bromomethyl)piperidine-1-carboxylate, 3,4,5-trimethyl-1*H*-pyrazole, and *N*-[5-(chloromethyl)thiazol-2-yl]acetamide. LCMS (ESI): [M+H] 363.

### Example 4-5 (Reference)

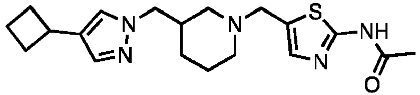

***N*-(5-((3-((4-cyclobutyl-1*H*-pyrazol-1-yl)methyl)piperidin-1-yl)methyl)thiazol-2-yl)acetamide:** The title compound was prepared in an analogous manner of that in scheme 4 from *tert*-butyl 3-(bromomethyl)piperidine-1-carboxylate, 4-cyclobutyl-1*H*-pyrazole, and *N-*[5-(chloromethyl)thiazol-2-yl]acetamide. LCMS (ESI): [M+H] 375. ¹HNMR: (500 MHz, Methanol-*d*4) δ 7.38 (s, 1H), 7.29 (s, 1H), 7.19 (s, 1H), 3.94-4.02 (m, 2H), 3.60-3.68 (m, 2H), 3.32-3.39 (m, 1H), 2.76 (br d, *J*=10.4 Hz, 1H), 2.57 (br d, *J*=10.4 Hz, 1H), 2.23-2.34 (m, 2H), 2.19 (s, 3H), 2.12-2.16 (m, 2H), 1.91-2.02 (m, 3H), 1.81-1.90 (m, 2H), 1.73 (dt, *J*=12.8, 4.0 Hz, 1H), 1.52-1.65 (m, 2H), 1.06 (br d, *J*=11.0 Hz, 1H).

### Example 4-6 (Reference)

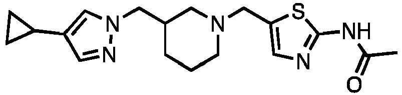

***N*-(5-((3-((4-cyclopropyl-1*H*-pyrazol-1-yl)methyl)piperidin-1-yl)methyl)thiazol-2-yl)acetamide:** The title compound was prepared in an analogous manner of that in scheme 4 from *tert*-butyl 3-(bromomethyl)piperidine-1-carboxylate, 4-cyclopropyl-1*H*-pyrazole, and *N*-[5-(chloromethyl)thiazol-2-yl]acetamide. LCMS (ESI): [M+H] 360.

### Example 4-7 (Reference)

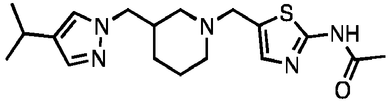

***N*-(5-((3-((4-isopropyl-1*H*-pyrazol-1-yl)methyl)piperidin-1-yl)methyl)thiazol-2-yl)acetamide:** The title compound was prepared in an analogous manner of that in scheme 4 from *tert*-butyl 3-(bromomethyl)piperidine-1-carboxylate, 4-isopropyl-1*H*-pyrazole, and *N-*[5-(chloromethyl)thiazol-2-yl]acetamide. LCMS (ESI): [M+H] 363. ¹HNMR: (500 MHz, Methanol-*d*4) δ 7.30 (s, 1H), 7.22 (s, 1H), 7.19 (s, 1H), 3.91-4.00 (m, 2H), 3.61-3.67 (m, 2H), 2.75 (br d, *J*=10.4 Hz, 1H), 2.56 (br d, *J*=9.8 Hz, 1H),2.20 (s, 3H), 2.10-2.12 (m, 2H), 1.84 (br t, *J*=10.1 Hz, 1H), 1.52-1.74 (m, 4H), 1.05 (br d, *J*=11.0 Hz, 1H), 0.78-0.83 (m, 2H), 0.41-0.46 (m, 2H).

### Example 4-8 (Reference)

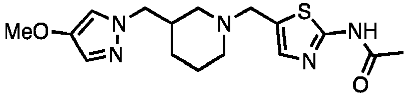

***N*-(5-((3-((4-methoxy-1*H*-pyrazol-1-yl)methyl)piperidin-1-yl)methyl)thiazol-2-yl)acetamide:** The title compound was prepared in an analogous manner of that in scheme 4 from *tert*-butyl 3-(bromomethyl)piperidine-1-carboxylate, 4-methoxy-1*H*-pyrazole, and *N*-[5-(chloromethyl)thiazol-2-yl]acetamide. LCMS (ESI): [M+H] 350.

### Example 4-9 (Reference)

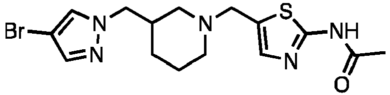

***N*-(5-((3-((4-bromo-1*H*-pyrazol-1-yl)methyl)piperidin-1-yl)methyl)thiazol-2-yl)acetamide:** The title compound was prepared in an analogous manner of that in scheme 4 from *tert*-butyl 3-(bromomethyl)piperidine-1-carboxylate, 4-bromo-1*H*-pyrazole, and *N*-[5-(chloromethyl)thiazol-2-yl]acetamide. LCMS (ESI): [M+H] 399.

### Example 4-10 (Reference)

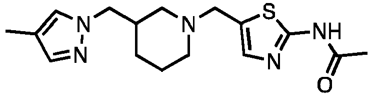

***N*-(5-((3-((4-methyl-1*H*-pyrazol-1-yl)methyl)piperidin-1-yl)methyl)thiazol-2-yl)acetamide:** The title compound was prepared in an analogous manner of that in scheme 4 from *tert*-butyl 3-(bromomethyl)piperidine-1-carboxylate, 4-methyl-1*H*-pyrazole, and *N*-[5-(chloromethyl)thiazol-2-yl]acetamide. LCMS (ESI): [M+H] 334.

### Example 4-11 (Reference)

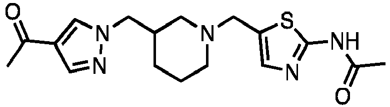

***N*-(5-((3-((4-acetyl-1*H*-pyrazol-1-yl)methyl)piperidin-1-yl)methyl)thiazol-2-yl)acetamide:** The title compound was prepared in an analogous manner of that in scheme 4 from *tert*-butyl 3-(bromomethyl)piperidine-1-carboxylate, 1-(1*H*-pyrazol-4-yl)ethan-1-one, and *N*-[5-(chloromethyl)thiazol-2-yl]acetamide. LCMS (ESI): [M+H] 362. 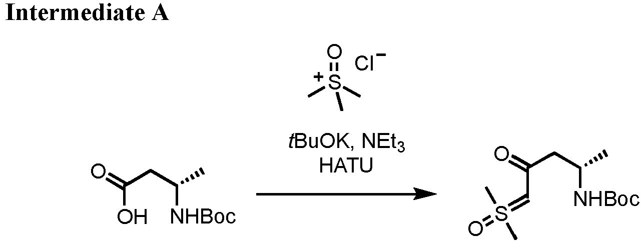 ***t*-butyl (*S*)-(5-(dimethyl(oxo)-l6-sulfaneylidene)-4-oxopentan-2-yl)carbamate:** A suspension (*S*)-3-((*tert*-butoxycarbonyl)amino)butanoic acid (19 g, 93.5 mmol) and HATU (38.4 g, 101 mmol) in THF (370 mL) was treated with TEA (55 mL, 390 mmol) and the resulting solution was stirred at rt for 16 h. In another flask a suspension of potassium *tert-*butoxide (37.8 g, 337 mmol) and trimethylsulfoxonium chloride (43.3 g, 337 mmol) in THF (370 mL) was heated at 60°C for 2 h, and then cooled in an ice-water bath during 15 min. The solution of activated ester was then added drop-wise at 0 °C over a period of 45 min. The reaction mixture was further stirred for 1 h, after which the reaction was concentrated under reduced pressure. The residue was partitioned between dichloromethane (1000 mL) and water (1000 mL). After separating the layers, the organic phase was washed with saturated aqueous NaCl (1000 mL), dried over Na₂SO₄, filtered, and concentrated under reduced pressure. The crude material was purified on silica gel column chromatography using a gradient of 0-5% MeOH in dichloromethane to afford the title compound (14 g, 56% yield). 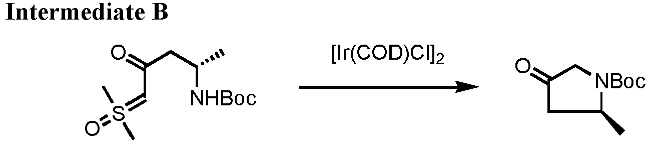 ***t*-butyl (*S*)-2-methyl-4-oxopyrrolidine-1-carboxylate:** *tert*-Butyl (*S*)-(5-(dimethyl(oxo)-l6-sulfaneylidene)-4-oxopentan-2-yl)carbamate (14 g, 50.5 mmol) was dissolved in 1,2-dichloroethane (500 mL). After deaeration, di-µ-chlorobis-[(η-cycloocta-1,5-diene)]diiridium (I) (1 g) was added under an argon atmosphere followed by raising the temperature and allowing to react at 70 °C for 2 h. The solvent of the reaction mixture was distilled off under reduced pressure, and the resulting residue was subjected to silica gel column chromatography (hexane:ethyl acetate =2:1) to afford the title compound (6.12 g, 62% yield). 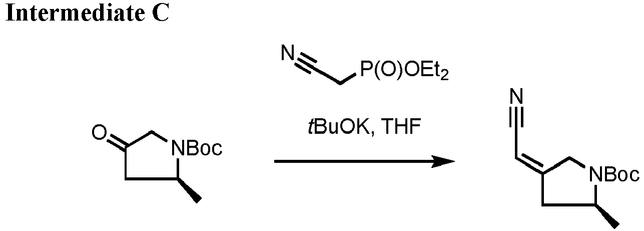 ***t*-butyl (*S*)-4-(cyanomethylene)-2-methylpyrrolidine-1-carboxylate:** A solution of diethyl cyanomethylphosphonate (5.5 g, 31 mmol) in anhydrous tetrahydrofuran (100 mL) was degassed with nitrogen. Subsequently, potassium *tert*-butoxide (3.5 g, 31 mmol) was added at room temperature, and the reaction mixture was degassed with nitrogen and stirred for 20 minutes. *tert*-butyl (*S*)-2-methyl-4-oxopyrrolidine-1-carboxylate (6.12 g, 31 mmol) in anhydrous tetrahydrofuran (20 mL) was added. After stirring for 20 hours, the reaction mixture was concentrated affording brown oil. The residue was suspended in chloroform (500 mL), washed with saturated NaHCO₃ (3×250 mL), dried over Na₂SO₄, and concentrated *in vacuo* to afford the title compound (5.16 g, 75% yield) as a brown oil. 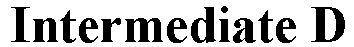 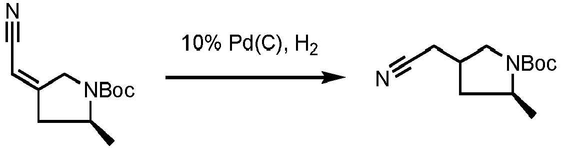 ***t*-butyl (2*S*)-4-(cyanomethyl)-2-methylpyrrolidine-1-carboxylate:** A solution of the ***t*-butyl** (*S*,)-4-(cyanomethylene)-2-methylpyrrolidine-1-carboxylate (5.16 g, 23 mmol) in methanol (500 mL) containing 10% Pd on carbon (2 g) was stirred under hydrogen at 60 psi for 24 h. The suspension was filtered through celite, concentrated *in vacuo,* and the residue was purified by silica gel chromatography, eluting with 15% EtOAc-hexane to provide the title compound (4.43g, 86% yield). 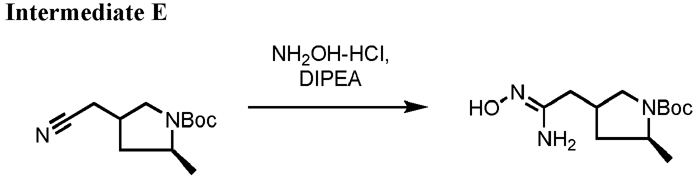 ***t*-butyl (2*S*)-4-((*Z*)-2-amino-2-(hydroxyimino)ethyl)-2-methylpyrrolidine-1-carboxylate:** A mixture of ***t*-butyl** (2*S*)-4-(cyanomethyl)-2-methylpyrrolidine-1-carboxylate (4.43 g, 20 mmol) and hydroxylamine hydrochloride (2.76 g, 40 mmol) was dissolved in EtOH (100 mL) to give a colorless suspension. Then DIPEA (5 g, 47 mmol) was added and the resulting mixture was stirred at 100°C for 6 h. The crude reaction mixture was concentrated *in vacuo* and washed with hexane (50 mL) to afford the title compound (3.96 g, 77% yield). 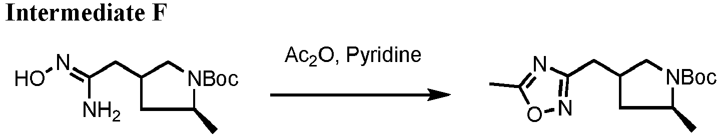 ***t*-butyl (2*S*)-2-methyl-4-((5-methyl-1,2,4-oxadiazol-3-yl)methyl)pyrrolidine-1-carboxylate:** *t*-Butyl (2*S*)-4-((*Z*)-2-amino-2-(hydroxyimino)ethyl)-2-methylpyrrolidine-1-carboxylate (3.96 g, 15.4 mmol) was dissolved in pyridine (50 mL) was added acetic anhydride (1.5 g, 15 mmol) and then it was heated at 90 °C for 24 h. The residue was partitioned between dichloromethane (1000 mL) and water (1000 mL). After separating the layers, the organic phase was washed with saturated aqueous NaCl (1000 mL), dried over Na₂SO₄, filtered, concentrated under reduced pressure. The obtained residue was subjected to column chromatography on silica gel as eluent DCM:MeOH to give the title compound (0.65 g, 17% yield).

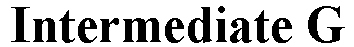

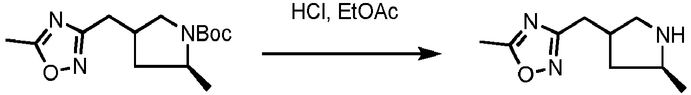

### 5-methyl-3-(((5S)-5-methylpyrrolidin-3-yl)methyl)-1,2,4-oxadiazole

To a solution of *tert*-butyl (2*S*)-2-methyl-4-((5-methyl-1,2,4-oxadiazol-3-yl)methyl)pyrrolidine-1-carboxylate (0.65 g, 2.3 mmol) in EtOAc (20 mL), 18M HCl in EtOAc (10 mL) was added dropwise. After stirring for 4 h at room temperature, the formed precipitate was collected by filtration, washed with EtOAc (20 mL), and dried in high vacuum to afford the title compound (0.305 g, 61% yield). LC-MS (ESI) m/z [M+H]⁺ 182. ¹H NMR (400 MHz, d₂o) δ 1.25 (dd, *J=* 6.6, 3.2 Hz, 1.5H), 1.29^{∗} (dd, *J=* 6.4, 3.1 Hz, 1.5H), 1.35 (m, 0.5H), 1.85 (m, 1H), 2.28^{∗} (m, 0.5H), 2.47 (s, 3H), 2.80 (m, 3H), 2.94 (m, 1H), 3.45 (m, 1H), 3.61 (m, 0.5H), 3.76^{∗} (m, 0.5H)

### Example 4-12

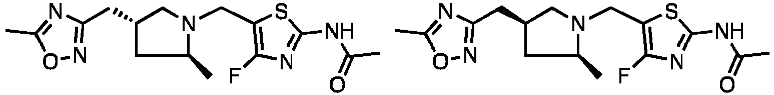

***N*-(4-fluoro-5-(((2*S*,4*R*)-2-methyl-4-((5-methyl-1,2,4-oxadiazol-3-yl)methyl)pyrrolidin-1-yl)methyl)thiazol-2-yl)acetamide and *N*-(4-fluoro-5-(((2*S*,4*S*)-2-methyl-4-((5-methyl-1,2,4-oxadiazol-3-yl)methyl)pyrrolidin-1-yl)methyl)thiazol-2-yl)acetamide:** The title compound was prepared according to the general procedure described in example 1-1 and using 5-methyl-3-(((5*S*)-5-methylpyrrolidin-3-yl)methyl)-1,2,4-oxadiazole and *N*-(4-fluoro-5-formylthiazol-2-yl)acetamide. The resulting isomers were purified over SiO₂ (ethyl acetate 100%) to afford two isomers (*trans* or *cis*), which were assigned arbitrarily as:
Peak 1: LCMS (ESI): [M+H] 354. ¹HNMR: (400 MHz, Methanol-d₄) δ 3.91 (dd, *J*=1.13, 14.43 Hz, 1H), 3.52 (d, *J*=14.31 Hz, 1H), 2.83 (dd, *J*=3.26, 9.54 Hz, 1H), 2.70-2.78 (m, 2H), 2.54 (s, 3H), 2.43-2.59 (m, 3H), 2.18 (s, 3H), 2.09-2.24 (m, 1H), 1.19-1.27 (m, 1H), 1.17 (d, *J*=6.27 Hz, 3H)
Peak 2: LCMS (ESI): [M+H] 354. ¹HNMR: (400 MHz, Methanol-d₄) δ 3.86-3.96 (m, 1H), 3.54 (d, *J*=14.56 Hz, 1H), 3.11-3.21 (m, 1H), 2.67-2.73 (m, 2H), 2.54-2.66 (m, 2H), 2.53 (s, 3H), 2.18 (s, 3H), 2.12 (t, *J*=9.16 Hz, 1H), 1.59-1.82 (m, 2H), 1.15 (d, *J*=6.27 Hz, 3H).

### Examples 4-13 and 4-14 (Reference)

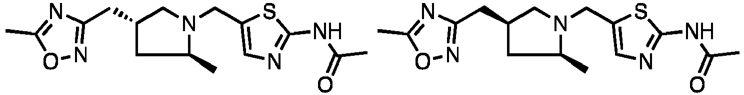

***N*-(5-(((2*S*,4*R*)-2-methyl-4-((5-methyl-1,2,4-oxadiazol-3-yl)methyl)pyrrolidin-1-yl)methyl)thiazol-2-yl)acetamide and *N*-(5-(((2*S*,4*S*)-2-methyl-4-((5-methyl-1,2,4-oxadiazol-3-yl)methyl)pyrrolidin-1-yl)methyl)thiazol-2-yl)acetamide:** The title compound was prepared according to the general procedure described in example 1-2 and using 5-Methyl-3-(((5S)-5-methylpyrrolidin-3-yl)methyl)-1,2,4-oxadiazole. The resulting isomers were purified over SiO₂ (EtOAc/EtOH 3/1) to afford two isomers (*trans* or *cis*):
***N*-(5-(((2*S*,4*R*)-2-methyl-4-((5-methyl-1,2,4-oxadiazol-3-yl)methyl)pyrrolidin-1-yl)methyl)thiazol-2-yl)acetamide** (early fractions): LCMS (ESI): [M+H] 336. ¹HNMR: (400 MHz, Methanol-d₄) δ 7.23 (s, 1H), 4.07 (dd, *J*=0.88, 14.18 Hz, 1H), 3.47-3.57 (m, 1H), 2.72-2.84 (m, 3H), 2.41-2.61 (m, 3H), 2.53 (s, 3H), 2.10-2.24 (m, 1H), 2.19 (s, 3H), 1.20-1.32 (m, 1H), 1.17 (d, *J*=6.02 Hz, 3H).
***N*-(5-(((2*S*,4*S*)-2-methyl-4-((5-methyl-1,2,4-oxadiazol-3-yl)methyl)pyrrolidin-1-yl)methyl)thiazol-2-yl)acetamide** (later fractions): LCMS (ESI): [M+H] 336. ¹HNMR: (400 MHz, Methanol-d₄) δ 7.24 (s, 1H), 4.07 (dd, *J*=0.88, 14.18 Hz, 1H), 3.56 (d, *J*=14.31 Hz, 1H), 3.13 (dd, *J*=7.03, 9.29 Hz, 1H), 2.53-2.72 (m, 4H), 2.53 (s, 3H), 2.19 (s, 3H), 2.08 (t, *J*=9.29 Hz, 1H), 1.63-1.83 (m, 2H), 1.15 (d, *J*=6.02 Hz, 3H).

### Examples 4-15 and 4-16

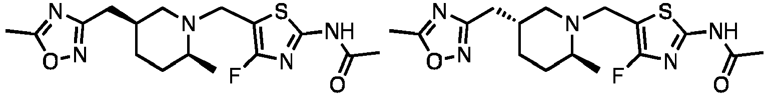

***N*-(4-fluoro-5-(((2*S*,5*S*)-2-methyl-5-((5-methyl-1,2,4-oxadiazol-3-yl)methyl)piperidin-1-yl)methyl)thiazol-2-yl)acetamide and *N*-(4-fluoro-5-(((2*S*,5*R*)-2-methyl-5-((5-methyl-1,2,4-oxadiazol-3-yl)methyl)piperidin-1-yl)methyl)thiazol-2-yl)acetamide:** The title compound was prepared according to the general procedure described in example 1-1 and using 5-methyl-3-(((6*S*)-6-methylpiperidin-3-yl)methyl)-1,2,4-oxadiazole and *N*-(4-fluoro-5-formylthiazol-2-yl)acetamide. The resulting isomers were purified over SiO₂ (EtOAc 100%) to afford two isomers (*cis* and *trans*):
***N*-(4-fluoro-5-(((2*S*,5*S*)-2-methyl-5-((5-methyl-1,2,4-oxadiazol-3-yl)methyl)piperidin-1-yl)methyl)thiazol-2-yl)acetamide** (early fractions): LCMS (ESI): [M+H] 368. ¹HNMR: (400 MHz, Methanol-d₄) δ 3.59-3.81 (m, 2H), 2.68-2.76 (m, 3H), 2.43-2.60 (m, 2H), 2.53 (s, 3H), 2.18 (s, 3H), 2.04-2.13 (m, 1H), 1.60-1.75 (m, 1H), 1.49-1.58 (m, 2H), 1.35-1.47 (m, 1H), 1.11 (d, *J*=6.27 Hz, 3H).
***N*-(4-fluoro-5-(((2*S*,5*R*)-2-methyl-5-((5-methyl-1,2,4-oxadiazol-3-yl)methyl)piperidin-1-yl)methyl)thiazol-2-yl)acetamide** (later fractions): LCMS (ESI): [M+H] 368. ¹HNMR: (400 MHz, Methanol-d₄) δ 3.73-3.96 (m, 2H), 2.90-2.95 (m, 1H), 2.51-2.60 (m, 2H), 2.54 (s, 3H), 2.23-2.34 (m, 1H), 2.19 (s, 3H), 1.96-2.07 (m, 2H), 1.64-1.80 (m, 2H), 1.26-1.42 (m, 1H), 1.20 (d, *J*=6.27 Hz, 3H), 0.95-1.14 (m, 1H).

### Example 4-17 (Reference)

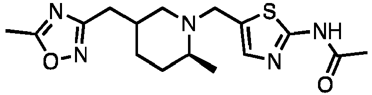

***N*-(5-(((2S)-2-methyl-5-((5-methyl-1,2,4-oxadiazol-3-yl)methyl)piperidin-1-yl)methyl)thiazol-2-yl)acetamide:** The title compound was prepared according to the general procedure described in example 1-2 and using 5-methyl-3-(((6*S*)-6-methylpiperidin-3-yl)methyl)-1,2,4-oxadiazole. LCMS (ESI): [M+H] 350. ¹H NMR (400 MHz, Methanol-d₄) δ 7.24 (s, 1H), 3.96-4.07 (m, 1H), 3.77-3.90 (m, 1H), 2.87-2.96 (m, 1H), 2.51-2.58 (m, 2H), 2.53 (s, 3H), 2.23-2.32 (m, 1H), 2.20 (s, 3H), 1.98-2.11 (m, 1H), 1.89-1.96 (m, 1H), 1.64-1.79 (m, 2H), 1.29-1.43 (m, 1H), 1.22 (d, *J*=6.02 Hz, 3H), 0.93-1.10 (m, 1H).

### Example 4-18

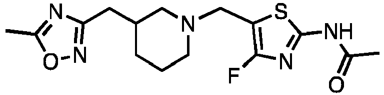

***N*-(4-fluoro-5-((3-((5-methyl-1,2,4-oxadiazol-3-yl)methyl)piperidin-1-yl)methyl)thiazol-2-yl)acetamide:** The title compound was prepared according to the general procedure described in example 1-1 and using 5-methyl-3-(piperidin-3-ylmethyl)-1,2,4-oxadiazole and *N*-(4-fluoro-5-formylthiazol-2-yl)acetamide. LCMS (ESI): [M+H] 354. ¹HNMR: (400 MHz, METHANOL-d₄) δ 3.64 (s, 2H), 2.78-2.95 (m, 2H), 2.62 (d, *J*=7.28 Hz, 2H), 2.54 (s, 3H), 2.18 (s, 3H), 2.03-2.16 (m, 2H), 1.89-2.00 (m, 1H), 1.67-1.79 (m, 2H), 1.50-1.65 (m, 1H), 0.95-1.14 (m, 1H).

### Example 4-19 (Reference)

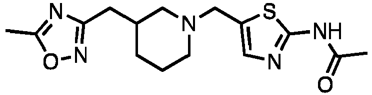

***N*-(5-((3-((5-methyl-1,2,4-oxadiazol-3-yl)methyl)piperidin-1-yl)methyl)thiazol-2-yl)acetamide:** The title compound was prepared according to the general procedure described in example 1-1 and using 5-methyl-3-(piperidin-3-ylmethyl)-1,2,4-oxadiazoleoxadiazole. LCMS (ESI): [M+H] 336. ¹HNMR: (400 MHz, DMSO-*d*₆) δ 11.80 (s, 1H), 7.09 (s, 1H), 3.58 (q, *J* = 13.9 Hz, 2H), 2.81 - 2.73 (m, 1H), 2.74 - 2.66 (m, 1H), 2.57 - 2.52 (m, 5H), 2.11 (s, 3H), 1.99 (s, 2H), 1.85 (t, *J* = 10.2 Hz, 1H), 1.66 (t, *J* = 14.0 Hz, 2H), 1.50 (d, *J* = 12.1 Hz, 1H), 1.02 (t, *J* = 11.7 Hz, 1H).

### Example 4-20 (Reference)

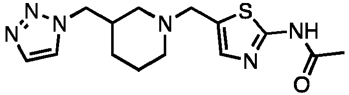

***N*-(5-((3-((1H-1,2,3-triazol-1-yl)methyl)piperidin-1-yl)methyl)thiazol-2-yl)acetamide:** The title compound was prepared according to the general procedure described in example 1-1 and using 3-((1H-1,2,3-triazol-1-yl)methyl)piperidine. LCMS (ESI): [M+H] 321.

### Example 4-21 (Reference)

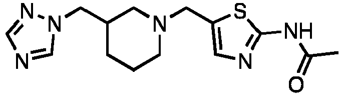

***N*-(5-((3-((1*H*-1,2,4-triazol-1-yl)methyl)piperidin-1-yl)methyl)thiazol-2-yl)acetamide:** The title compound was prepared according to the general procedure described in example 1-1 and using 3-((1H-1,2,4-triazol-1-yl)methyl)piperidine. LCMS (ESI): [M+H] 321. ¹HNMR (400 MHz, Chloroform-d) δ 12.25 (s, 1H), 8.01 (s, 1H), 7.91 (s, 1H), 7.15 (s, 1H), 4.18 (dd, J = 13.7, 7.5 Hz, 1H), 4.09 (dd, J = 13.7, 6.8 Hz, 1H), 3.69 - 3.53 (m, 2H), 2.66 - 2.48 (m, 2H), 2.35 - 2.16 (m, 4H), 2.03 (t, J = 9.6 Hz, 1H), 1.73 - 1.64 (m, 1H), 1.64 - 1.49 (m, 2H), 1.22 - 1.07 (m, 2H).

### Example 4-22 (Reference)

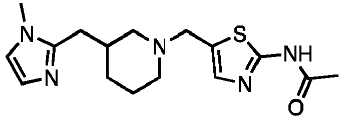

***N*-(5-((3-((1-methyl-1*H*-imidazol-2-yl)methyl)piperidin-1-yl)methyl)thiazol-2-yl)acetamide:** The title compound was prepared according to the general procedure described in example 1-1 and using 3-((1-methyl-1H-imidazol-2-yl)methyl)piperidine. LCMS (ESI): [M+H] 334. ¹HNMR: (400 MHz, Chloroform-*d*) δ 12.48 (s, 1H), 7.15 (s, 1H), 6.94 - 6.85 (m, 1H), 6.81 - 6.72 (m, 1H), 3.71 - 3.58 (m, 2H), 3.57 (s, 3H), 2.83 - 2.50 (m, 4H), 2.29 (s, 3H), 2.14 - 2.00 (m, 2H), 1.94 (t, *J=* 10.0 Hz, 1H), 1.76 - 1.58 (m, 2H), 1.59 - 1.43 (m, 1H), 1.12 - 1.00 (m, 1H).

### Example 4-23 (Reference)

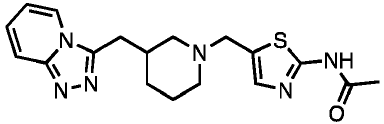

***N*-(5-((3-([1,2,4]triazolo[4,3-a]pyridin-3-ylmethyl)piperidin-1-yl)methyl)thiazol-2-yl)acetamide:** The title compound was prepared according to the general procedure described in example 1-1 and using 3-(piperidin-3-ylmethyl)-[1,2,4]triazolo[4,3-a]pyridine. LCMS (ESI): [M+H] 371. ¹HNMR: (400 MHz, DMSO-*d*₆) δ 11.82 (s, 1H), 8.35 (d, *J* = 7.0 Hz, 1H), 7.62 (d, *J* = 9.3 Hz, 1H), 7.25 (dd, *J* = 9.2, 6.5 Hz, 1H), 7.09 (s, 1H), 6.87 (t, *J* = 6.7 Hz, 1H), 3.65 - 3.51 (m, 2H), 3.14 - 3.01 (m, 2H), 2.79 (d, *J* = 10.7 Hz, 1H), 2.66 (d, *J* = 10.6 Hz, 1H), 2.26 - 2.15 (m, 1H), 2.11 (s, 3H), 2.10 - 2.03 (m, 1H), 1.98 (t, *J* = 10.0 Hz, 1H), 1.76 - 1.56 (m, 2H), 1.59 - 1.42 (m, 1H), 1.13 (d, *J* = 10.5 Hz, 1H).

### Example 4-24 (Reference)

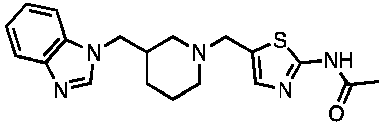

***N*-(5-((3-((1H-benzo[d]imidazol-1-yl)methyl)piperidin-1-yl)methyl)thiazol-2-yl)acetamide:** The title compound was prepared according to the general procedure described in example 1-1 and using 1-(piperidin-3-ylmethyl)-1H-benzo[d]imidazole. LCMS (ESI): [M+H] 370. ¹HNMR: (400 MHz, Chloroform-*d*) δ 12.28 (s, 1H), 7.84 (s, 1H), 7.78 (d, *J* = 7.1 Hz, 1H), 7.39 (d, *J* = 7.8 Hz, 1H), 7.33 - 7.19 (m, 2H), 7.14 (s, 1H), 4.19 (dd, *J* = 14.3, 7.7 Hz, 1H), 4.05 (dd, *J=* 14.3, 7.0 Hz, 1H), 3.61 (s, 2H), 3.47 (s, 3H), 2.69 - 2.42 (m, 1H), 2.29 (s, 2H), 2.27 - 2.14 (m, 1H), 2.10 - 1.93 (m, 1H), 1.75 - 1.62 (m, 1H), 1.59 (s, 1H), 1.56 - 1.40 (m, 1H), 1.22 - 1.07 (m, 1H).

### Example 4-25 (Reference)

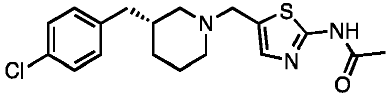

***N*-(5-((3-(4-chlorobenzyl)piperidin-1-yl)methyl)thiazol-2-yl)acetamide:** The title compound was prepared according to the general procedure described in example 1-1 and using 3-(4-chlorobenzyl)piperidine. LCMS (ESI): [M+H] 364. ¹HNMR: (400 MHz, DMSO-*d*₆) δ 11.80 (s, 1H), 7.20 (d, *J=* 8.0 Hz, 2H), 7.10 (d, *J=* 8.2 Hz, 2H), 7.07 (s, 1H), 3.54 (q, *J* = 13.8 Hz, 2H), 2.76 - 2.61 (m, 2H), 2.59 - 2.46 (m, 1H), 2.42 (dd, *J* = 13.6, 6.4 Hz, 1H), 2.12 (s, 3H), 2.03 - 1.88 (m, 1H), 1.87 - 1.69 (m, 2H), 1.69 - 1.53 (m, 2H), 1.44 (dd, *J =* 23.5, 11.1 Hz, 1H), 0.93 (dd, *J* = 20.1, 8.6 Hz, 1H).

### Example 4-26 (Reference)

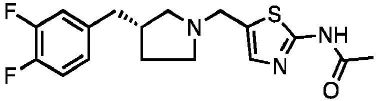

***N*-(5-((3-(3,4-difluorobenzyl)pyrrolidin-1-yl)methyl)thiazol-2-yl)acetamide:** The title compound was prepared according to the general procedure described in example 1-1 and using 3-(3,4-difluorobenzyl)pyrrolidine. LCMS (ESI): [M+H] 352.

### Biological Data

### OGA enzyme inhibition biochemical assay

Recombinant full length human OGA enzyme was purchased from Origene. 4-MUGlCNAc substrate was purchased from Sigma. All other reagents were purchased from Sigma or Fisher. Assay buffer consists of the McIlvaine buffer system, pH 6.4 (0.2M Na₂HPO₄ mixed with 0.1M citric acid) and 0.01% BSA. Reactions consist of InM OGA, 100µM 4-MUGlcNAc (Kₘ), and compound in a final volume of 10µl. Reactions were incubated for 90 minutes at room temperature and quenched with 40µl of 3M glycine, pH 10 and read on a Perkin Elmer Envision plate reader (Ex: 355nm/Em: 460nm). Compounds were tested with a 10-point dose-response starting from 20µM with a 4-fold dilution. Data was fit using GraphPad Prism using a 4-paramter fit with variable slope.

The following Table 1 shows the activity data for some of the compounds of the present invention.

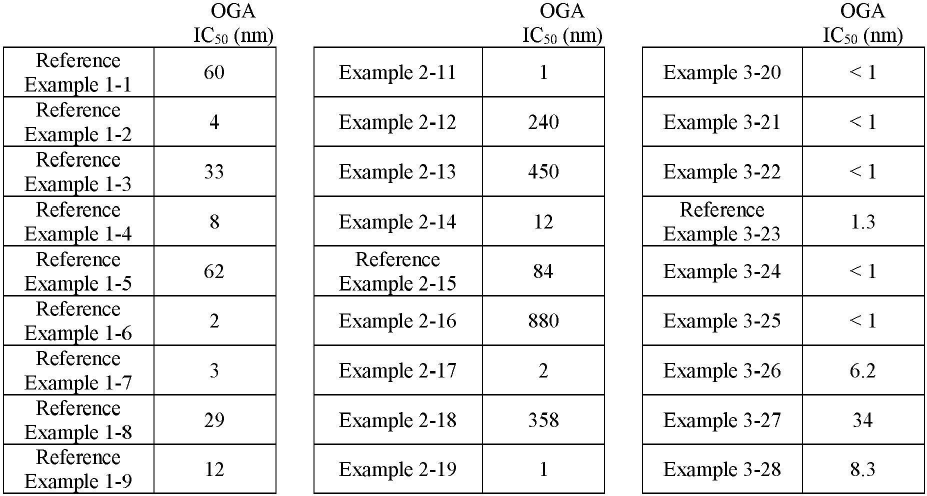

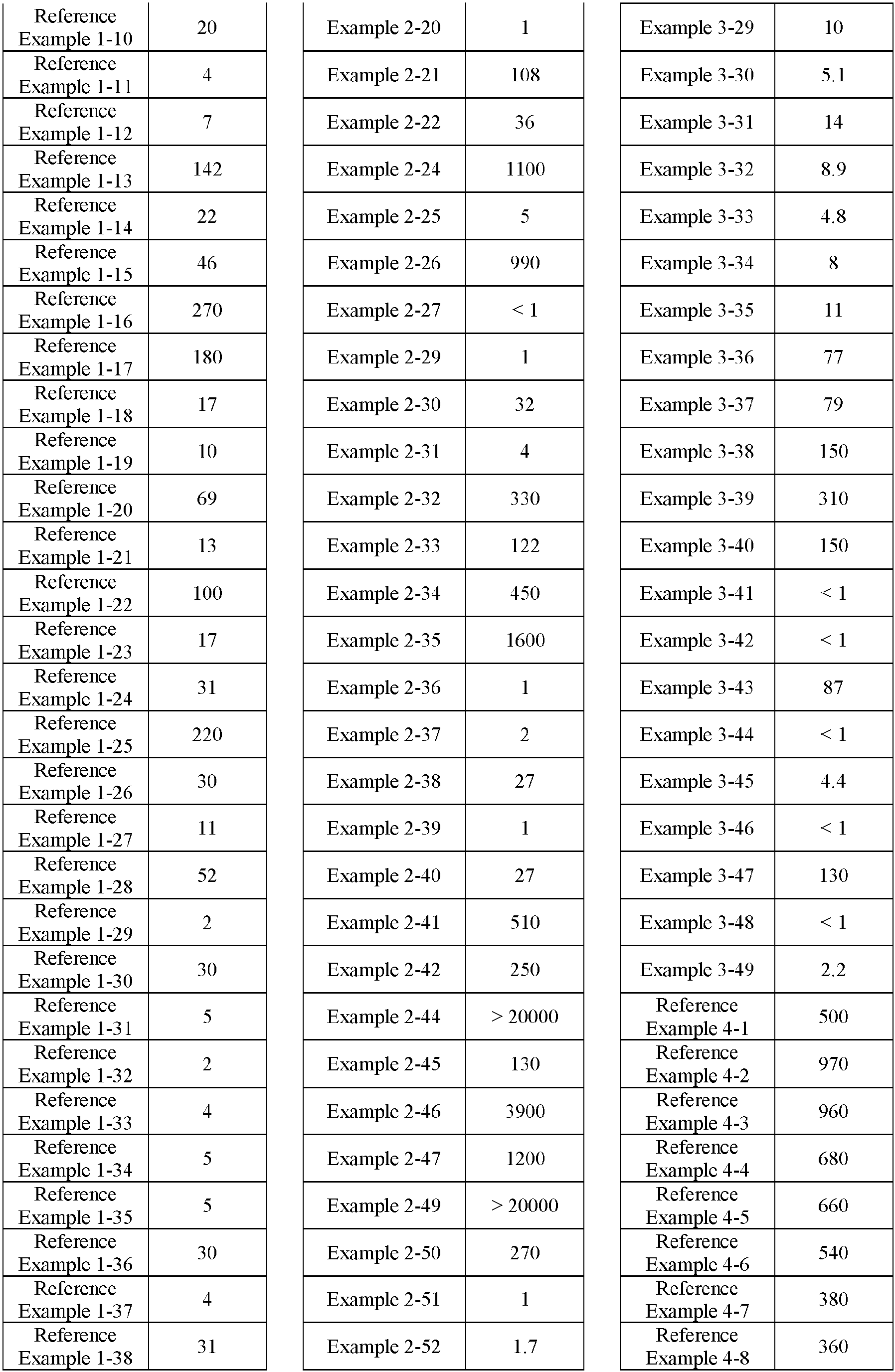

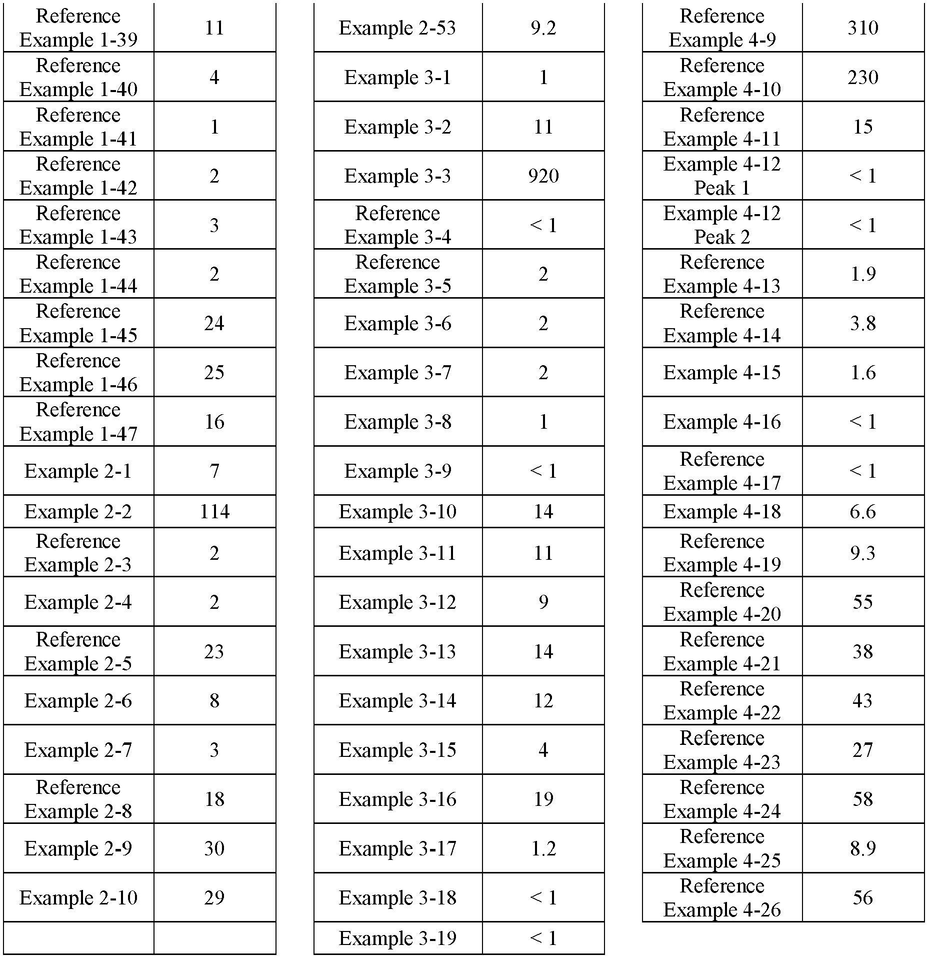

While we have described a number of embodiments of this, it is apparent that our basic examples may be altered to provide other embodiments that utilize the compounds and methods of this disclosure. Therefore, it will be appreciated that the scope of this disclosure is to be defined by the appended claims rather than by the specific embodiments that have been represented by way of example.

Unless otherwise defined, all technical and scientific terms used herein are accorded the meaning commonly known to one with ordinary skill in the art.

## Claims

1. A compound represented by the following structural formulae:
(i) 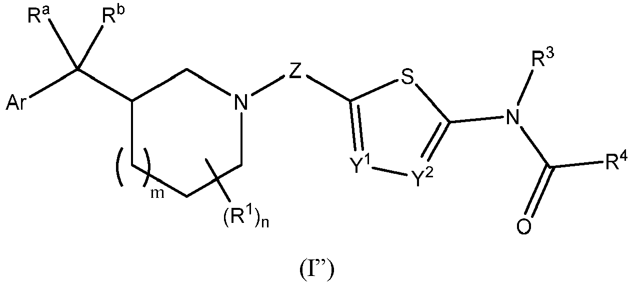 or a pharmaceutically acceptable salt thereof, wherein:
Ar is an optionally substituted 5- to 10-membered heteroaryl, an optionally substituted phenyl or an optionally substituted phenyl fused to an optionally substituted non-aromatic 5-to 6-membered heterocycle;
Y¹ and Y² are each CR^{c} or N, wherein at least one of Y¹ or Y² is N;
Z is CR²R², C(=O), (CR²R²)₂, CH₂C(=O), or C(=O)CH₂;
R^{a} and R^{b} are each independently -H, halo, C₁-C₄ alkyl, C₁-C₄ haloalkyl, or C₁-C₄ alkoxy, or R^{a} and R^{b} taken together with their intervening carbon atom form a C₃-C₆ cycloalkyl;
R^{c} is halo, C₁-C₄ alkyl, C₁-C₄ haloalkyl, or C₁-C₄ alkoxy;
m is 0 or 1;
n is 0 or an integer from 1 to 7;
when n is other than 0, R¹, for each occurrence, is independently halo, C₁-C₄ alkyl, C₁-C₄ haloalkyl, C₁-C₄ alkoxy or C₁-C₄ haloalkoxy;
R², for each occurrence, is independently -H, halo, C₁-C₄ alkyl, C₁-C₄ haloalkyl, C₃-C₁₀ cycloalkyl, or C₃-C₁₀ halocycloalkyl;
or alternatively two R² together with the carbon atom to which they are attached form a C₃-C₁₀ cycloalkyl;
R³ is -H or C₁-C₄ alkyl; and
R⁴ is -H, C₁-C₄ alkyl, C₁-C₄ haloalkyl, or C₃-C₆ cycloalkyl;
or alternatively R³ and R⁴ taken together with their intervening atoms form an optionally substituted 5- to 7-membered heterocyclyl; or
(ii) 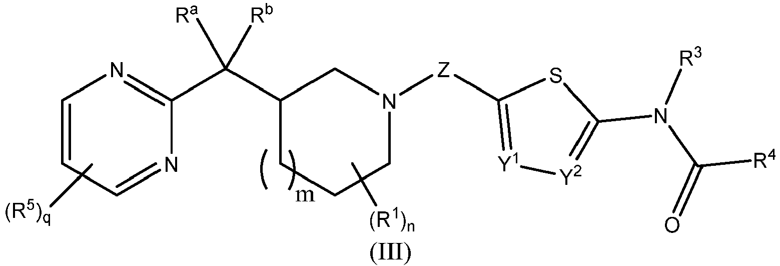 or a pharmaceutically acceptable salt thereof; wherein:
Y¹ and Y² are each CR^{c} or N, wherein at least one of Y¹ or Y² is N;
Z is CR²R², C(=O), (CR²R²)₂, CH₂C(=O), or C(=O)CH₂;
R^{a}, R^{b} and R^{c} are each independently -H, halo, C₁-C₄ alkyl, C₁-C₄ haloalkyl, or C₁-C₄ alkoxy, or R^{a} and R^{b} taken together with their intervening carbon atom form a C₃-C₆ cycloalkyl;
m is 0 or 1;
n is 0 or an integer from 1 to 7;
when n is other than 0, R¹, for each occurrence, is independently halo, C₁-C₄ alkyl, C₁-C₄ haloalkyl, C₁-C₄ alkoxy or C₁-C₄ haloalkoxy;
R², for each occurrence, is independently -H, halo, C₁-C₄ alkyl, C₁-C₄ haloalkyl, C₃-C₁₀ cycloalkyl, or C₃-C₁₀ halocycloalkyl;
or alternatively two R² together with the carbon atom to which they are attached form a C₃-C₁₀ cycloalkyl;
R³ is -H or C₁-C₄ alkyl; and
R⁴ is -H, C₁-C₄ alkyl, C₁-C₄ haloalkyl, or C₃-C₆ cycloalkyl;
or alternatively R³ and R⁴ taken together with their intervening atoms form an optionally substituted 5- to 7-membered heterocyclyl;
R⁵, for each occurrence, is selected from C₁-C₄ alkyl, C₁-C₄ haloalkyl, C₃-C₆ cycloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, -C(=O)R^{x}, phenyl and monocyclic heteroaryl; wherein
when R⁵ is a C₁-C₄ alkyl group, the C₁-C₄ alkyl group is optionally and independently substituted with -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{x}, -C(=O)NR^{x}R^{y}, -NR^{x}C(=O)R^{y}, -C(=S)NR^{x}R^{y} -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{y}, C₃-C₆ cycloalkyl (optionally substituted with one or more groups selected from -CH₃, halomethyl, halo, methoxy and halomethoxy), monocyclic heteroaryl (optionally substituted with one or more groups selected from -CH₃, halomethyl, halo, methoxy or halomethoxy) and phenyl (optionally substituted with one or more groups selected from - CH₃, halomethyl, halo, methoxy and halomethoxy);
when R⁵ is a C₃-C₆ cycloalkyl, phenyl or a monocyclic heteroaryl, the cycloalkyl, phenyl or a monocyclic heteroaryl is optionally and independently substituted with C₁-C₄ alkyl, C₁-C₄ haloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{y}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{x};
each R^{x} and each R^{y} is independently -H, C₁-C₄ alkyl, or C₃-C₈ cycloalkyl; wherein the C₁-C₄ alkyl or C₃-C₈ cycloalkyl represented by R^{x} or R^{y} is optionally substituted with one or more substituents selected from halo, hydroxyl, C₃-C₆ cycloalkyl and phenyl (optionally substituted with one or more groups selected from -CH₃, halomethyl, halo, methoxy or halomethoxy);
R^{z} is -H, C₁-C₄ alkyl, or C₃-C₈ cycloalkyl; wherein the C₁-C₄ alkyl or C₃-C₈ cycloalkyl group represented by R^{z} is optionally substituted with one or more substituents selected from halo, hydroxyl, C₃-C₆ cycloalkyl and phenyl (optionally substituted with one or more groups selected from -CH₃, halomethyl, halo, methoxy and halomethoxy); and
i is 0, 1, or 2; and
q 0, 1, 2, or 3.

2. The compound according to claim 1, wherein the compound is represented by the following structural formulae:
(i) 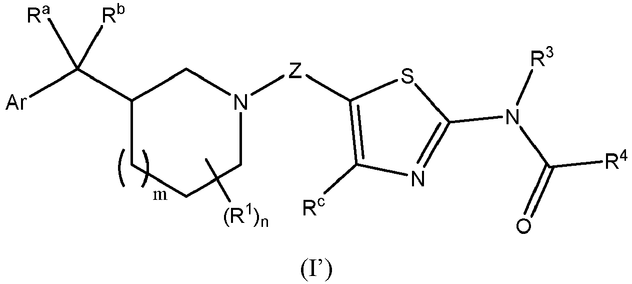 or a pharmaceutically acceptable salt thereof;
(ii) 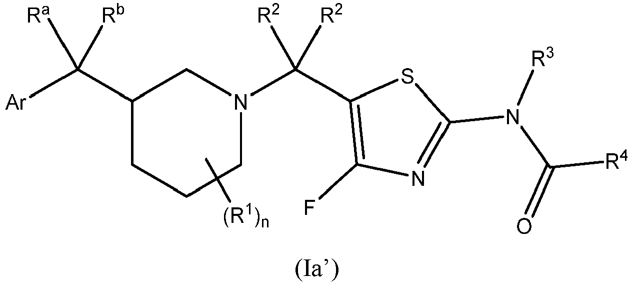 or a pharmaceutically acceptable salt thereof; wherein
R^{a} and R^{b} are each independently -H or C₁-C₄ alkyl; and
R², for each occurrence, is independently -H, halo, C₁-C₄ alkyl;
(iii) 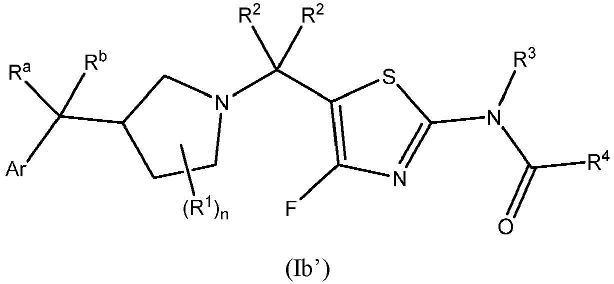 or a pharmaceutically acceptable salt thereof; wherein R², for each occurrence, is independently -H, halo, C₁-C₄ alkyl;
(iv) 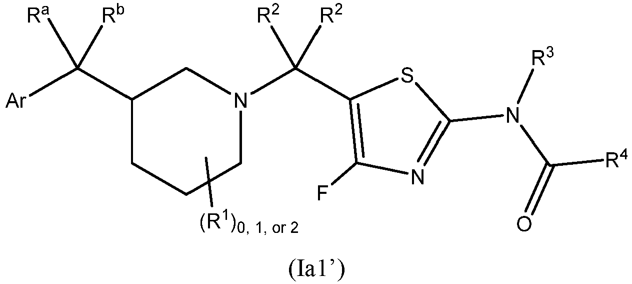 or a pharmaceutically acceptable salt thereof; wherein
R^{a} and R^{b} are each independently -H or methyl;
R^{z} is halo, C₁-C₄ alkyl or C₁-C₄ haloalkyl; and
R², for each occurrence, is independently -H or C₁-C₄ alkyl;
(v) 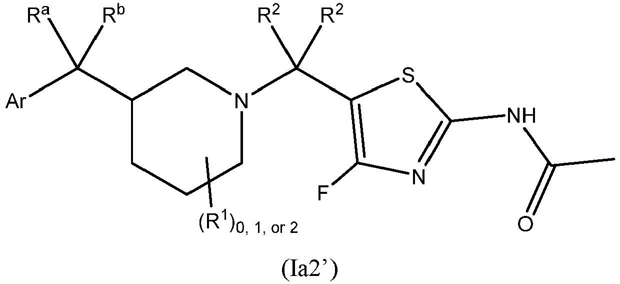 or a pharmaceutically acceptable salt thereof; wherein
R^{a} and R^{b} are each independently -H or methyl;
R², for each occurrence, is independently -H or methyl; and
R^{z} is -F or methyl;
(vi) 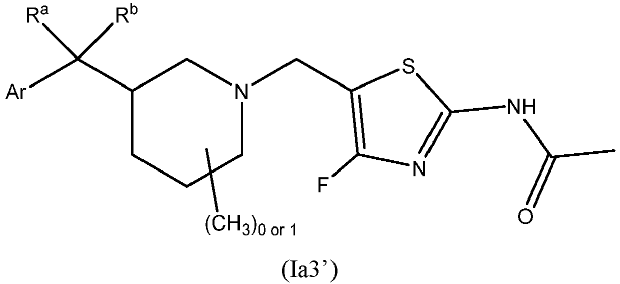 or a pharmaceutically acceptable salt thereof; or
(vii) 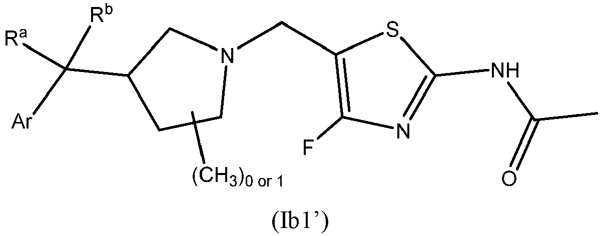 or a pharmaceutically acceptable salt thereof.

3. The compound according to claim 1 or claim 2, wherein the compound is represented by the following structural formulae:
(i) 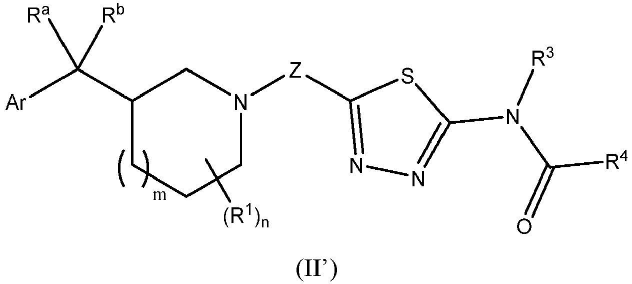 or a pharmaceutically acceptable salt thereof;
(ii) 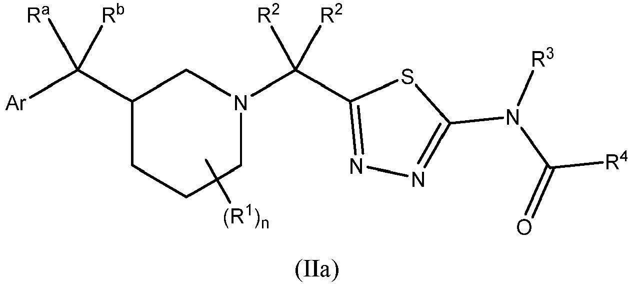 or a pharmaceutically acceptable salt thereof; wherein
R^{a} and R^{b} are each independently -H or C₁-C₄ alkyl; and
R², for each occurrence, is independently -H, halo, C₁-C₄ alkyl;
(iii) 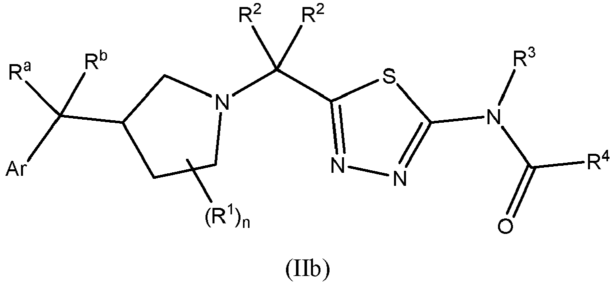 or a pharmaceutically acceptable salt thereof; wherein R², for each occurrence, is independently -H, halo, C₁-C₄ alkyl;
(iv) 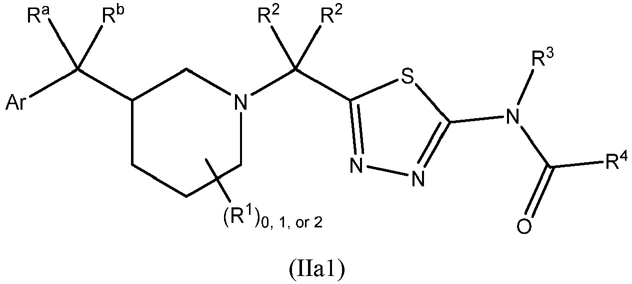 or a pharmaceutically acceptable salt thereof; wherein
R^{a} and R^{b} are each independently -H or methyl;
R^{z} is halo, C₁-C₄ alkyl or C₁-C₄ haloalkyl; and
R², for each occurrence, is independently -H or C₁-C₄ alkyl;
(v) 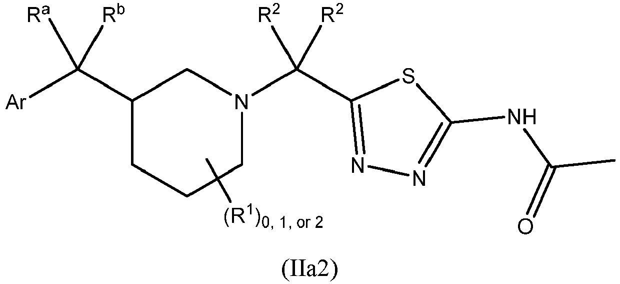 or a pharmaceutically acceptable salt thereof; wherein
R^{a} and R^{b} are each independently -H or methyl;
R², for each occurrence, is independently -H or methyl; and
R¹ is -F or methyl;
(vi) 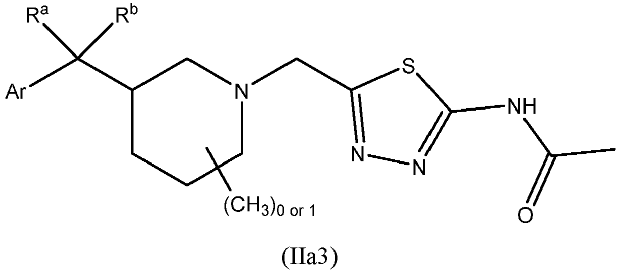 or a pharmaceutically acceptable salt thereof; or
(vii) 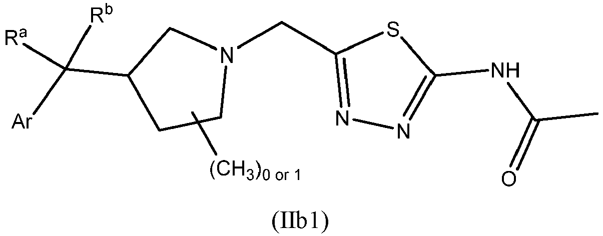 or a pharmaceutically acceptable salt thereof.

4. The compound according to any one of claims 1-3, or a pharmaceutically acceptable salt thereof, wherein
(i) Ar is optionally substituted pyrazolyl, optionally substituted imidazolyl, optionally substituted thiazolyl, optionally substituted phenyl, optionally substituted pyridinyl, optionally substituted pyrimidinyl, optionally substituted pyrazinyl, optionally substituted imidazo[1,2-a]pyiidinyl, optionally substituted thieno[2,3-d]pyrimidinyl, or optionally substituted thieno[3,2-d]pyrimidinyl;
(ii) Ar is optionally substituted 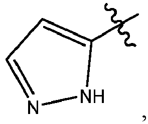 optionally substituted 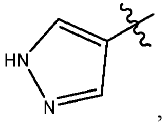 optionally substituted 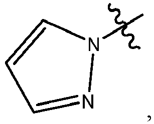 optionally substituted 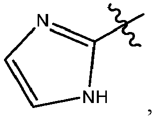 optionally substituted 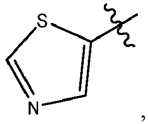 optionally substituted 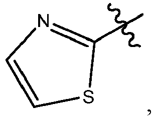 optionally substituted 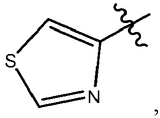 optionally substituted 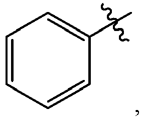 optionally substituted 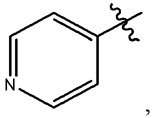 optionally substituted 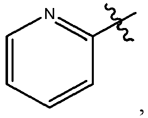 optionally substituted 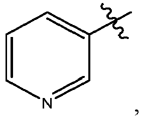 optionally substituted 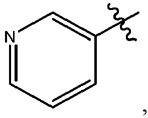 optionally substituted 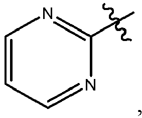 optionally substituted 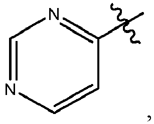 optionally substituted 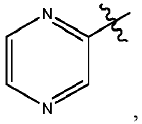 optionally substituted 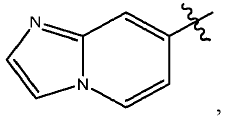 or optionally substituted 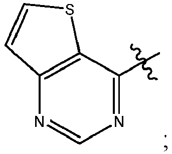
(iii) Ar is optionally substituted 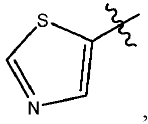 optionally substituted 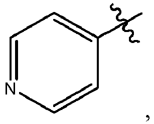 optionally substituted 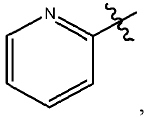 optionally substituted 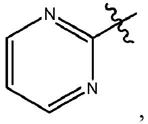 optionally substituted 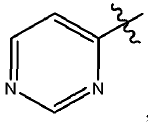 , optionally substituted 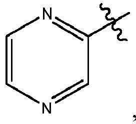 optionally substituted 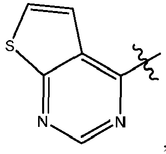 , or optionally substituted 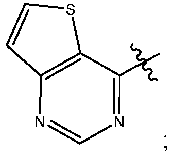 or
(iv) Ar is optionally substituted 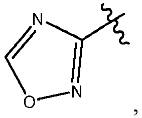 optionally substituted 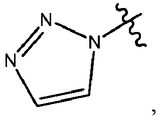 optionally substituted 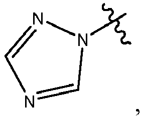 optionally substituted 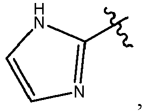 optionally substituted 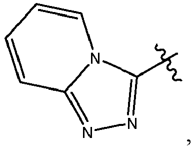 or optionally substituted 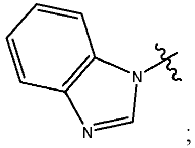 or
(v) Ar is optionally substituted

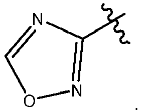

5. The compound according to any one of claims 1-4 or a pharmaceutically acceptable salt thereof, wherein:
Ar is optionally substituted with one or more groups selected from C₁-C₄ alkyl, C₁-C₄ haloalkyl, C₃-C₆ cycloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NRW, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, -C(=O)R^{x}, phenyl and monocyclic heteroaryl; wherein
the C₁-C₄ alkyl group substituent on Ar is optionally substituted with-CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{x}, -C(=O)NR^{x}R^{y}, -NR^{x}C(=O)R^{y}, -C(=S)NR^{x}R^{y} -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{y}, C₃-C₆ cycloalkyl (optionally substituted with one or more groups selected from -CH₃, halomethyl, halo, methoxy and halomethoxy), monocyclic heteroaryl (optionally substituted with one or more groups selected from -CH₃, halomethyl, halo, methoxy or halomethoxy) and phenyl (optionally substituted with one or more groups selected from - CH₃, halomethyl, halo, methoxy and halomethoxy);
the C₃-C₆ cycloalkyl, phenyl and monocyclic heteroaryl group substituent on Ar are optionally and independently substituted with C₁-C₄ alkyl, C₁-C₄ haloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{y}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NRW, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{x};
each R^{x} and each R^{y} is independently -H, C₁-C₄ alkyl, or C₃-C₈ cycloalkyl; wherein the C₁-C₄ alkyl or C₃-C₈ cycloalkyl represented by R^{x} or R^{y} is optionally substituted with one or more substituents selected from halo, hydroxyl, C₃-C₆ cycloalkyl and phenyl (optionally substituted with one or more groups selected from -CH₃, halomethyl, halo, methoxy or halomethoxy);
R^{z} is -H, C₁-C₄ alkyl, or C₃-C₈ cycloalkyl; wherein the C₁-C₄ alkyl or C₃-C₈ cycloalkyl group represented by R^{z} is optionally substituted with one or more substituents selected from halo, hydroxyl, C₃-C₆ cycloalkyl and phenyl (optionally substituted with one or more groups selected from -CH₃, halomethyl, halo, methoxy and
halomethoxy); and
i is 0, 1, or 2.

6. The compound according to any one of claims 1-5 or a pharmaceutically acceptable salt thereof, wherein
(i) Ar is optionally substituted with one or more groups selected from C₁-C₄ alkyl, C₁-C₄ haloalkyl, C₃-C₆ cycloalkyl, halo, -CN, -OR^{z}, -NR^{x}R^{y}, -C(=O)NR^{x}R^{y}, -C(=S)NR^{x}R^{y}, -O(C=O)NR^{x}R^{y}, -O(C=S)NR^{x}R^{y}, -C(=O)OR^{x}, -NR^{x}C(=O)R^{y} phenyl, -C(=O)R^{x}, and optionally substituted monocyclic heteroaryl;
(ii) Ar is optionally substituted with one or more groups selected from C₁-C₄ alkyl, C₁-C₄ haloalkyl, C₃-C₆ cycloalkyl, halo, -OR^{z}, -C(=O)R^{x}, and monocyclic heteroaryl optionally substituted with C₁-C₄ alkyl, C₁-C₄ haloalkyl, halo;
(iii) Ar is optionally substituted with one or more groups selected from -CH₃, - CH₂CH₃, halomethyl, cyclopentyl, cyclobutyl, halo, -OR^{z}, -C(=O)R^{x}, and a 5- or 6-membered monocyclic heteroaryl containing one or two heteroatoms selected from S and N and optionally substituted with C₁-C₄ alkyl; wherein R^{x} is -H or C₁-C₄ alkyl; and wherein R^{z} is optionally substituted C₁-C₄ alkyl; or
(iv) Ar is optionally substituted with one or more groups selected from -CH₃, - CH₂CH₃, -CHF₂, -CF₃, cyclopentyl, cyclobutyl, -F, -Cl, -Br, -OCH₃, -C(=O)CH₃, and a thiazolyl.

7. The compound according to claim 1, wherein the compound is represented by the following structural formula: 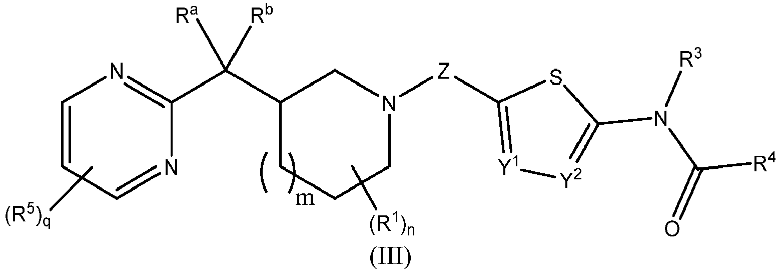 or a pharmaceutically acceptable salt thereof; wherein:
Y¹ and Y² are each CR^{c} or N, wherein at least one of Y¹ or Y² is N;
Z is CR²R², C(=O), (CR²R²)₂, CH₂C(=O), or C(=O)CH₂;
R^{a}, R^{b} and R^{c} are each independently -H, halo, C₁-C₄ alkyl, C₁-C₄ haloalkyl, or C₁-C₄ alkoxy, or R^{a} and R^{b} taken together with their intervening carbon atom form a C₃-C₆ cycloalkyl;
m is 0 or 1;
n is 0 or an integer from 1 to 7;
when n is other than 0, R¹, for each occurrence, is independently halo, C₁-C₄ alkyl, C₁-C₄ haloalkyl, C₁-C₄ alkoxy or C₁-C₄ haloalkoxy;
R², for each occurrence, is independently -H, halo, C₁-C₄ alkyl, C₁-C₄ haloalkyl, C₃-C₁₀ cycloalkyl, or C₃-C₁₀ halocycloalkyl;
or alternatively two R² together with the carbon atom to which they are attached form a C₃-C₁₀ cycloalkyl;
R³ is -H or C₁-C₄ alkyl; and
R⁴ is -H, C₁-C₄ alkyl, C₁-C₄ haloalkyl, or C₃-C₆ cycloalkyl;
or alternatively R³ and R⁴ taken together with their intervening atoms form an optionally substituted 5- to 7-membered heterocyclyl;
R⁵, for each occurrence, is selected from C₁-C₄ alkyl, C₁-C₄ haloalkyl, C₃-C₆ cycloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, -C(=O)R^{x}, phenyl and monocyclic heteroaryl; wherein
when R⁵ is a C₁-C₄ alkyl group, the C₁-C₄ alkyl group is optionally and independently substituted with -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{x}, -C(=O)NR^{x}R^{y}, -NR^{x}C(=O)R^{y}, -C(=S)NR^{x}R^{y} -NR^{x}C(=S)R^{y}, -NR^{x}(C=O)OR^{y}, -O(C=O)NRW, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{y}, C₃-C₆ cycloalkyl (optionally substituted with one or more groups selected from -CH₃, halomethyl, halo, methoxy and halomethoxy), monocyclic heteroaryl (optionally substituted with one or more groups selected from -CH₃, halomethyl, halo, methoxy or halomethoxy) and phenyl (optionally substituted with one or more groups selected from - CH₃, halomethyl, halo, methoxy and halomethoxy);
when R⁵ is a C₃-C₆ cycloalkyl, phenyl or a monocyclic heteroaryl, the cycloalkyl, phenyl or a monocyclic heteroaryl is optionally and independently substituted with C₁-C₄ alkyl, C₁-C₄ haloalkyl, halo, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{y}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, and -C(=O)R^{x};
each R^{x} and each R^{y} is independently -H, C₁-C₄ alkyl, or C₃-C₈ cycloalkyl; wherein the C₁-C₄ alkyl or C₃-C₈ cycloalkyl represented by R^{x} or R^{y} is optionally substituted with one or more substituents selected from halo, hydroxyl, C₃-C₆ cycloalkyl and phenyl (optionally substituted with one or more groups selected from -CH₃, halomethyl, halo, methoxy or halomethoxy);
R^{z} is -H, C₁-C₄ alkyl, or C₃-C₈ cycloalkyl; wherein the C₁-C₄ alkyl or C₃-C₈ cycloalkyl group represented by R^{z} is optionally substituted with one or more substituents selected from halo, hydroxyl, C₃-C₆ cycloalkyl and phenyl (optionally substituted with one or more groups selected from -CH₃, halomethyl, halo, methoxy and halomethoxy); and
i is 0, 1, or 2; and
q 0, 1, 2, or 3.

8. The compound according to claim 1, wherein the compound is represented by the following structural formulae:
(i) 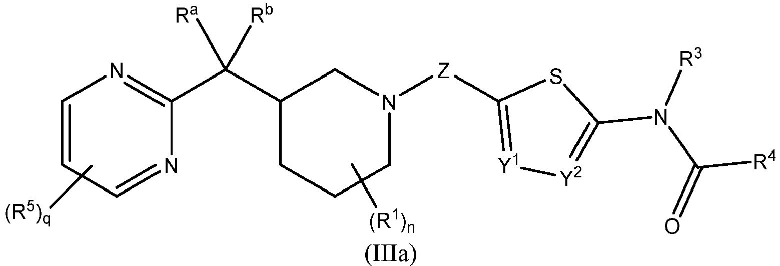 or a pharmaceutically acceptable salt thereof;
(ii) 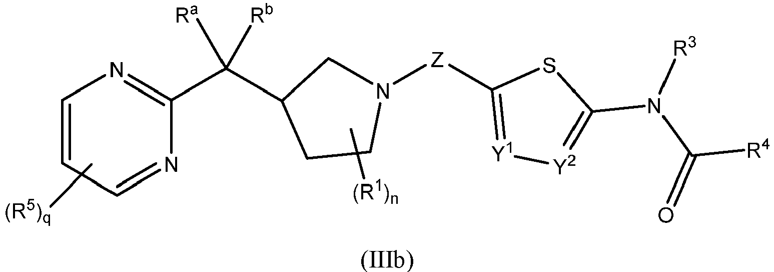 or a pharmaceutically acceptable salt thereof; wherein q is 0, 1, 2, or 3;
(iii) 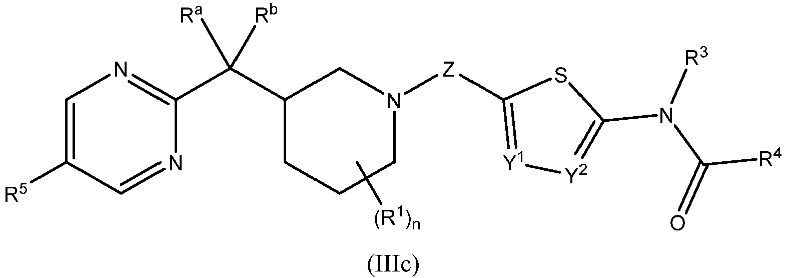 or a pharmaceutically acceptable salt thereof;
(iv) 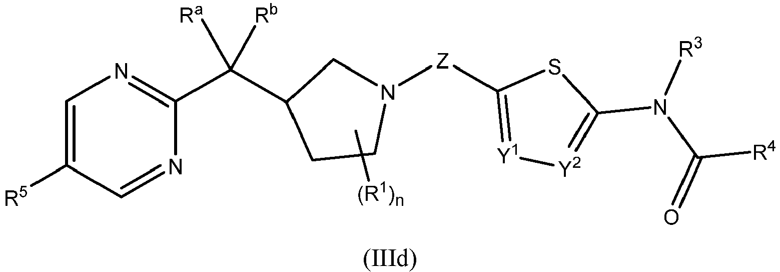 or a pharmaceutically acceptable salt thereof;
(v) 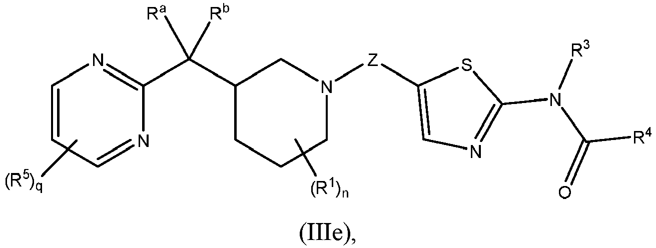 or a pharmaceutically acceptable salt thereof;
(vi) 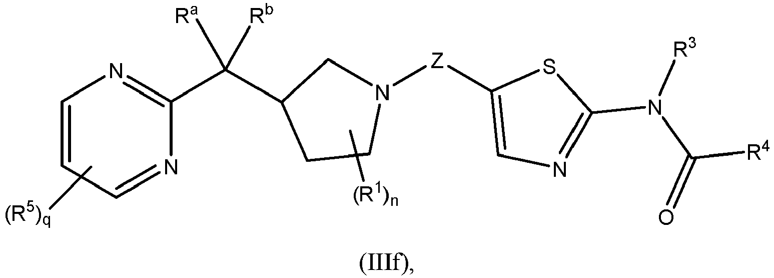 or a pharmaceutically acceptable salt thereof; wherein q is 0, 1, 2, or 3;
(vii) 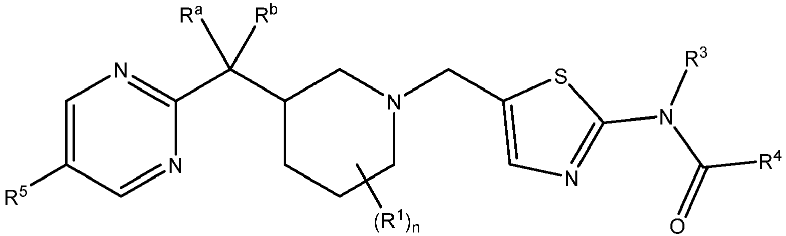 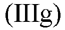 or a pharmaceutically acceptable salt thereof;
(viii) 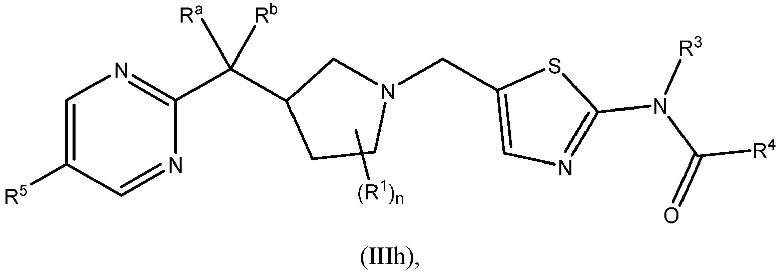 or a pharmaceutically acceptable salt thereof;
(ix) 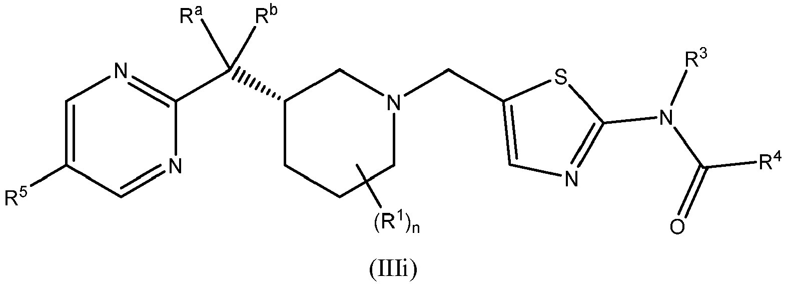 or a pharmaceutically acceptable salt thereof;
(x) 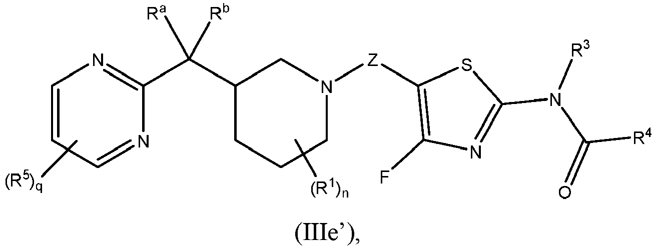 or a pharmaceutically acceptable salt thereof, wherein q is 0, 1, 2, or 3;
(xi) 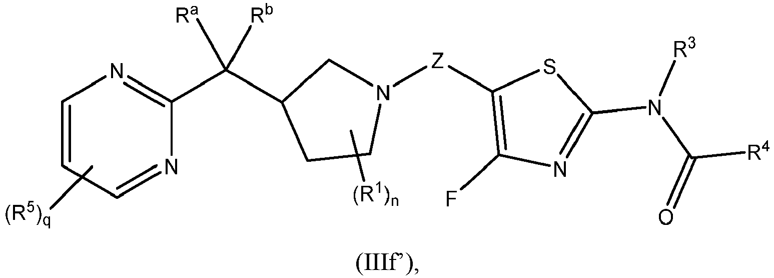 or a pharmaceutically acceptable salt thereof;
(xii) 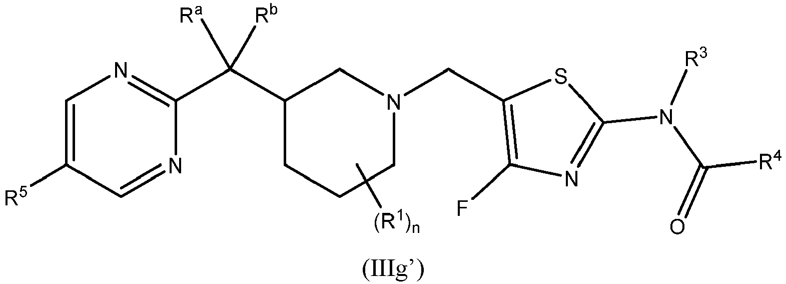 or a pharmaceutically acceptable salt thereof;
(xiii) 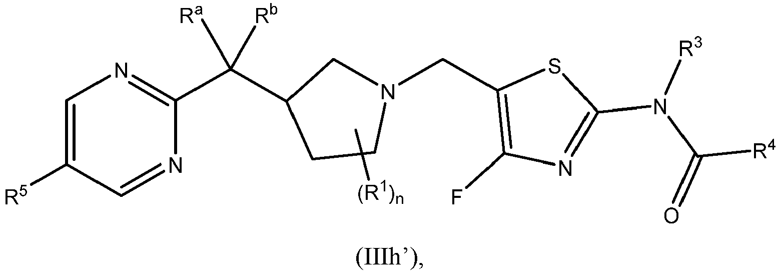 or a pharmaceutically acceptable salt thereof;
(xiv) 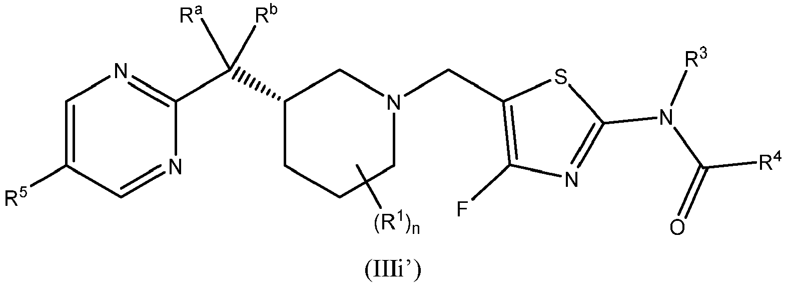 or a pharmaceutically acceptable salt thereof;
(xv) 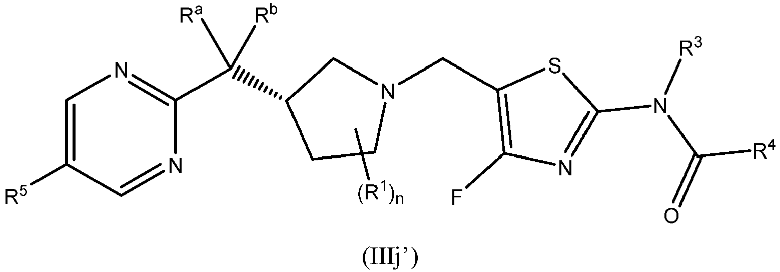 or a pharmaceutically acceptable salt thereof; or
(xvi) 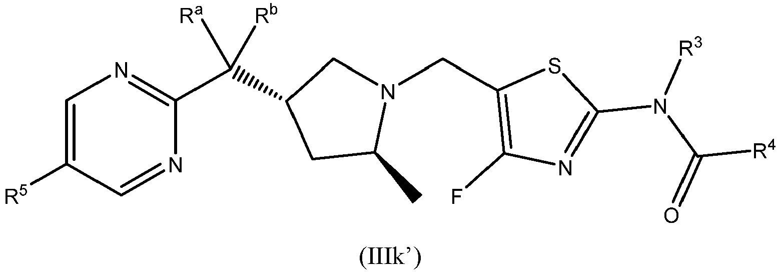 or a pharmaceutically acceptable salt thereof.

9. The compound according to any one of claims 1-8 or a pharmaceutically acceptable salt thereof, wherein R³ is -H.

10. The compound according to any one of claims 1-9 or a pharmaceutically acceptable salt thereof, wherein R⁴ is -CH₃.

11. The compound according to any one of claims 1 or 9-10 or a pharmaceutically acceptable salt thereof, wherein R⁵ is selected from C₁-C₄ alkyl, C₁-C₄ haloalkyl, C₃-C₆ cycloalkyl, halo, -CN, -OR^{z}, -NR^{x}R^{y}, -C(=O)NR^{x}R^{y}, -C(=S)NR^{x}R^{y}, -O(C=O)NR^{x}R^{y}, -O(C=S)NR^{x}R^{y}, -C(=O)OR^{x}, -NR^{x}C(=O)R^{y} phenyl, -C(=O)R^{x}, and optionally substituted monocyclic heteroaryl; optionally, wherein:
(i) R⁵ is selected from C₁-C₄ alkyl, C₁-C₄ haloalkyl, C₃-C₆ cycloalkyl, halo, -OR^{z}, -C(=O)R^{x}, and monocyclic heteroaryl optionally substituted with C₁-C₄ alkyl, C₁-C₄ haloalkyl, halo;
(ii) R⁵ is selected from -CH₃, -CH₂CH₃, halomethyl, cyclopentyl, cyclobutyl, halo, -OR^{z}, -C(=O)R^{x}, and a 5- or 6-membered monocyclic heteroaryl containing one or two heteroatoms selected from S and N and optionally substituted with C₁-C₄ alkyl; wherein R^{z} is -H or C₁-C₄ alkyl; and wherein R^{z} is optionally substituted C₁-C₄ alkyl;
(iii) R⁵ is selected from -CH₃, -CH₂CH₃, -CHF₂, -CF₃, cyclopentyl, cyclobutyl, -F, -Br, Cl, -OCH₃, -C(=O)CH₃, and a thiazolyl; or
(iv) R⁵ is selected from -F, -Br, and Cl.

12. The compound according to any one of 1-11 or a pharmaceutically acceptable salt thereof, wherein one of R^{a} and R^{b} is -H and the other is selected from -CH₃, -CF₃, and - OCH₃.

13. The compound according to any one of 1-12 or a pharmaceutically acceptable salt thereof, wherein n is 0 or 1.

14. A pharmaceutical composition comprising the compound according to any one of claims 1-13 or a pharmaceutically acceptable salt thereof and a pharmaceutically acceptable carrier or diluent.

15. A compound according to any one of claims 1-13 or a pharmaceutical composition according to claim 14 for use in the treatment of a disease or condition selected from a neurodegenerative disease, a tauopathy, diabetes, cancer, and stress, optionally, wherein:
(i) the disease or condition is selected from Acute ischemic stroke (AIS), Alzheimer's disease, Dementia, Amyotrophic lateral sclerosis (ALS), Amyotrophic lateral sclerosis with cognitive impairment (ALSci), Argyrophilic grain dementia, Bluit disease, Corticobasal degeneration (CBP), Dementia pugilistica, Diffuse neurofibrillary tangles with calcification, Down's syndrome, epilepsy, Familial British dementia, Familial Danish dementia, Frontotemporal dementia with parkinsonism linked to chromosome 17 (FTDP-17), Gerstmann-Straussler-Scheinker disease, Guadeloupean parkinsonism, Hallevorden-Spatz disease (neurodegeneration with brain iron accumulation type 1), ischemic stroke, mild cognitive impairment (MCI), Multiple system atrophy, Myotonic dystrophy, Niemann-Pick disease (type C), Pallido-ponto-nigral degeneration, Parkinsonism-dementia complex of Guam, Pick's disease (PiD), Postencephalitic parkinsonism (PEP), Prion diseases (including Creutzfeldt- Jakob Disease (GJD), Variant Creutzfeldt-Jakob Disease (vCJD), Fatal Familial Insomnia, Kuru, Progressive supercortical gliosis, Progressive supranuclear palsy (PSP), Steele- Richardson-Olszewski syndrome, Subacute sclerosing panencephalitis, Tangle-only dementia, Huntington's disease, and Parkinson's disease;
(ii) the disease or condition is selected from Acute ischemic stroke (AIS), Alzheimer's disease, Dementia, Amyotrophic lateral sclerosis (ALS), Amyotrophic lateral sclerosis with cognitive impairment (ALSci), Argyrophilic grain dementia, epilepsy, ischemic stroke, mild cognitive impairment (MCI), Huntington's disease, and Parkinson's disease; or
(iii) the disease or condition is Alzheimer's disease.

## Patentansprüche

1. Verbindung, dargestellt durch die folgenden Strukturformeln:
(i) 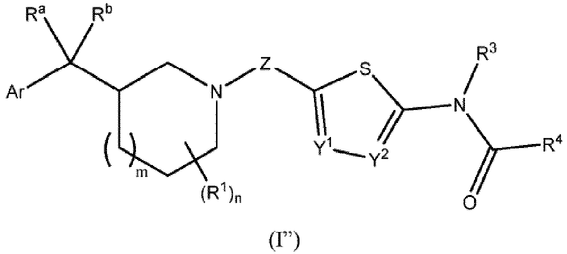 oder ein pharmazeutisch verträgliches Salz davon, wobei:
Ar ein optional substituiertes 5- bis 10-gliedriges Heteroaryl, ein optional substituiertes Phenyl oder ein optional substituiertes Phenyl ist, das an einen optional substituierten nichtaromatischen 5- bis 6-gliedrigen Heterocyclus anelliert ist;
Y¹ und Y² jeweils CR^{c} oder N sind, wobei mindestens eines von Y¹ oder Y² N ist;
Z CR²R², C(=O), (CR²R²)₂, CH₂C(=O) oder C(=O)CH₂ ist;
R^{a} und R^{b} sind jeweils unabhängig -H, Halogen, C₁-C₄-Alkyl, C₁-C₄-Halogenalkyl oder C₁-C₄-Alkoxy sind, oder R^{a} und R^{b} zusammen mit ihrem dazwischenliegenden Kohlenstoffatom ein C₃-C₆-Cycloalkyl bilden;
R^{c} Halogen, C₁-C₄-Alkyl, C₁-C₄-Halogenalkyl oder C₁-C₄-Alkoxy ist;
m 0 oder 1 ist;
n 0 oder eine ganze Zahl von 1 bis 7 ist;
wenn n nicht 0 ist, R¹ bei jedem Auftreten unabhängig Halogen, C₁-C₄-Alkyl, C₁-C₄-Halogenalkyl, C₁-C₄-Alkoxy oder C₁-C₄-Halogenalkoxy ist;
R² bei jedem Auftreten unabhängig -H, Halogen, C₁-C₄-Alkyl, C₁-C₄-Halogenalkyl, C₃-C₁₀-Cycloalkyl oder C₃-C₁₀-Halogencycloalkyl ist;
oder alternativ zwei R² zusammen mit dem Kohlenstoffatom, an das sie gebunden sind, ein C₃-C₁₀-Cycloalkyl bilden;
R³ -H oder C₁-C₄-Alkyl ist; und
R⁴ -H, C₁-C₄-Alkyl, C₁-C₄-Halogenalkyl oder C₃-C₆-Cycloalkyl ist;
oder alternativ R³ und R⁴ zusammen mit ihren dazwischenliegenden Atomen ein optional substituiertes 5- bis 7-gliedriges Heterocyclyl bilden; oder
(ii) 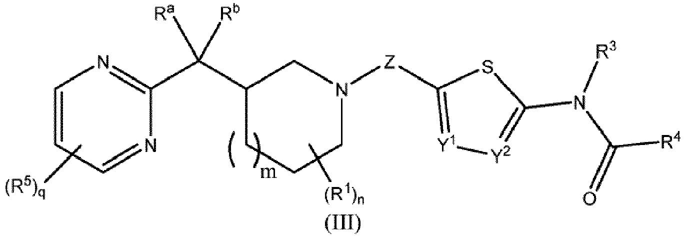 oder ein pharmazeutisch verträgliches Salz davon; wobei:
Y¹ und Y² jeweils CR^{c} oder N sind, wobei mindestens eines von Y¹ oder Y² N ist;
Z CR²R², C(=O), (CR²R²)₂, CH₂C(=O) oder C(=O)CH₂ ist;
R^{a}, R^{b} und R^{c} jeweils unabhängig -H, Halogen, C₁-C₄-Alkyl, C₁-C₄-Halogenalkyl oder C₁-C₄-Alkoxy sind, oder R^{a} und R^{b} zusammen mit ihrem dazwischenliegenden Kohlenstoffatom ein C₃-C₆-Cycloalkyl bilden;
m 0 oder 1 ist;
n 0 oder eine ganze Zahl von 1 bis 7 ist;
wenn n nicht 0 ist, R¹ bei jedem Auftreten unabhängig Halogen, C₁-C₄-Alkyl, C₁-C₄-Halogenalkyl, C₁-C₄-Alkoxy oder C₁-C₄-Halogenalkoxy ist;
R² bei jedem Auftreten unabhängig -H, Halogen, C₁-C₄-Alkyl, C₁-C₄-Halogenalkyl, C₃-C₁₀-Cycloalkyl oder C₃-C₁₀-Halogencycloalkyl ist;
oder alternativ zwei R² zusammen mit dem Kohlenstoffatom, an das sie gebunden sind, ein C₃-C₁₀-Cycloalkyl bilden;
R³ -H oder C₁-C₄-Alkyl ist; und
R⁴ -H, C₁-C₄-Alkyl, C₁-C₄-Halogenalkyl oder C₃-C₆-Cycloalkyl ist;
oder alternativ R³ und R⁴ zusammen mit ihren dazwischenliegenden Atomen ein optional substituiertes 5- bis 7-gliedriges Heterocyclyl bilden;
R⁵ bei jedem Auftreten aus C₁-C₄-Alkyl, C₁-C₄-Halogenalkyl, C₃-C₆-Cycloalkyl, Halogen, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, -NR^{x}C(=O)R^{y}, -C(=S)NR^{x}R^{y}, -NR^{x}C(=S)R^{y}, - NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, - NR^{x}(C=S)NR^{x}R^{y}, -C(=S)R^{x}, -C(=O)R^{x}, Phenyl und monocyclisches Heteroaryl ausgewählt ist;
wobei
wenn R⁵ eine C₁-C₄-Alkylgruppe ist, die C₁-C₄-Alkylgruppe optional und unabhängig mit -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{x}, -C(=O)NR^{x}R^{y}, -NR^{x}C(=O)R^{y}, -C(=S)NR^{x}R^{y}, -NR^{x}C(=S)R^{y}, - NR^{x}(C=O)OR^{Y}, -O(C=O)NR^{x}R^{y}, -NR^{x}(C=S)OR^{y}, -O(C=S)NR^{x}R^{y}, -NR^{x}(C=O)NR^{x}R^{y}, - NR^{x}(C=S)NR^{x}R^{y}, -C(=S)R^{x} und -C(=O)R^{y}, C₃-C₆-Cycloalkyl (optional substituiert mit einer oder mehreren Gruppen ausgewählt aus -CH₃, Halogenmethyl, Halogen, Methoxy und Halogenmethoxy), monocyclischem Heteroaryl (optional substituiert mit einer oder mehreren Gruppen ausgewählt aus -CH₃, Halogenmethyl, Halogen, Methoxy oder Halogenmethoxy) und Phenyl (optional substituiert mit einer oder mehreren Gruppen ausgewählt aus -CH₃, Halogenmethyl, Halogen, Methoxy und Halogenmethoxy) substituiert ist;
wenn R⁵ ein C₃-C₆-Cycloalkyl, Phenyl oder ein monocyclisches Heteroaryl ist, das Cycloalkyl, Phenyl oder ein monocyclisches Heteroaryl optional und unabhängig mit C₁-C₄-Alkyl, C₁-C₄-Halogenalkyl, Halogen, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{y}, -C(=O)NR^{x}R^{y}, -NR^{x}C(=O)R^{y}, - C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, - O(C=S)NR^{x}R^{y}, -NR^{x}(C=O)NR^{x}R^{y}, -NR^{x}(C=S)NR^{x}R^{y}, -C(=S)R^{x} und -C(=O)R^{x} substituiert ist;
jedes R^{x} und jedes R^{y} unabhängig -H, C₁-C₄-Alkyl oder C₃-C₈-Cycloalkyl ist; wobei das durch R^{x} oder R^{y} dargestellte C₁-C₄-Alkyl oder C₃-C₈-Cycloalkyl optional mit einem oder mehreren Substituenten ausgewählt aus Halogen, Hydroxyl, C₃-C₆-Cycloalkyl und Phenyl (optional substituiert mit einer oder mehreren Gruppen ausgewählt aus -CH₃, Halogenmethyl, Halogen, Methoxy oder Halogenmethoxy) substituiert ist;
R^{z} -H, C₁-C₄-Alkyl oder C₃-C₈-Cycloalkyl ist; wobei die durch R^{z} dargestellte C₁-C₄-Alkyl- oder C₃-C₈-Cycloalkylgruppe optional mit einem oder mehreren Substituenten ausgewählt aus Halogen, Hydroxyl, C₃-C₆-Cycloalkyl und Phenyl (optional substituiert mit einer oder mehreren Gruppen ausgewählt aus -CH₃, Halogenmethyl, Halogen, Methoxy und Halogenmethoxy) substituiert ist; und
i 0, 1 oder 2 ist; und
q 0, 1, 2 oder 3 ist.

2. Verbindung nach Anspruch 1, wobei die Verbindung durch die folgenden Strukturformeln dargestellt ist:
(i) 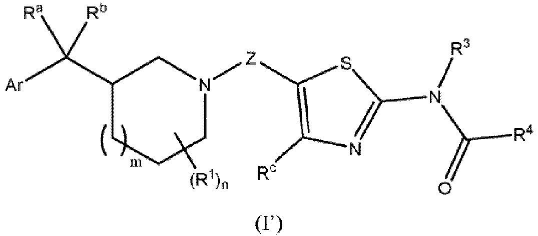 oder ein pharmazeutisch verträgliches Salz davon;
(ii) 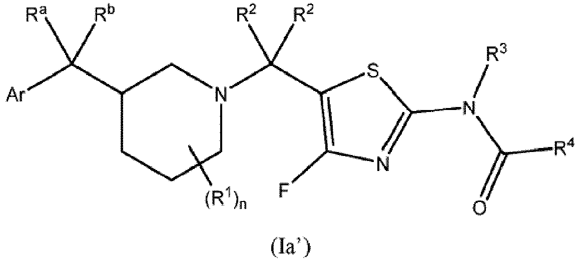 oder ein pharmazeutisch verträgliches Salz davon; wobei
R^{a} und R^{b} jeweils unabhängig -H oder C₁-C₄-Alkyl sind; und
R² bei jedem Auftreten unabhängig -H, Halogen, C₁-C₄-Alkyl ist;
(iii) 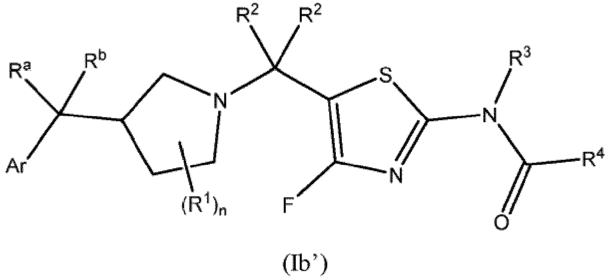 oder ein pharmazeutisch verträgliches Salz davon; wobei R² bei jedem Auftreten unabhängig -H, Halogen, C₁-C₄-Alkyl ist;
(iv) 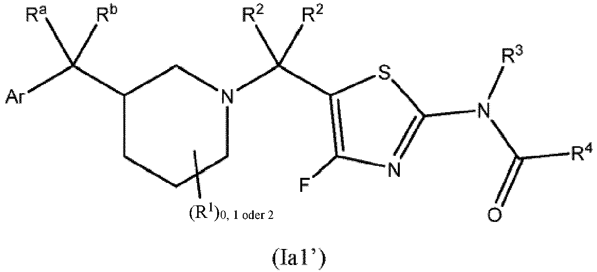 oder ein pharmazeutisch verträgliches Salz davon; wobei
R^{a} und R^{b} jeweils unabhängig -H oder Methyl sind;
R¹ Halogen, C₁-C₄-Alkyl oder C₁-C₄-Halogenalkyl ist; und
R² bei jedem Auftreten unabhängig -H oder C₁-C₄-Alkyl ist;
(v) 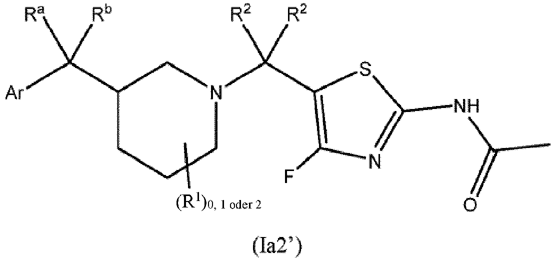 oder ein pharmazeutisch verträgliches Salz davon; wobei
R^{a} und R^{b} jeweils unabhängig -H oder Methyl sind;
R² bei jedem Auftreten unabhängig -H oder Methyl ist; und
R¹ -F oder Methyl ist;
(vi) 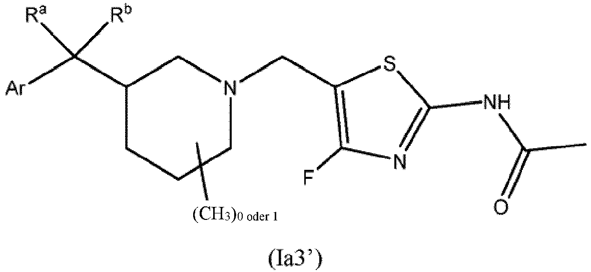 oder ein pharmazeutisch verträgliches Salz davon; oder
(vii) 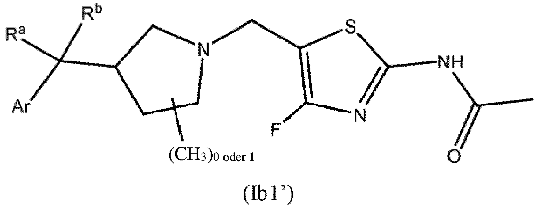 oder ein pharmazeutisch verträgliches Salz davon.

3. Verbindung nach Anspruch 1 oder Anspruch 2, wobei die Verbindung durch die folgenden Strukturformeln dargestellt ist:
(i) 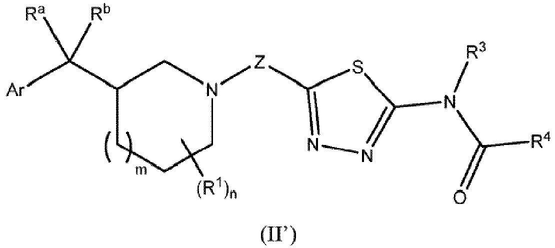 oder ein pharmazeutisch verträgliches Salz davon;
(ii) 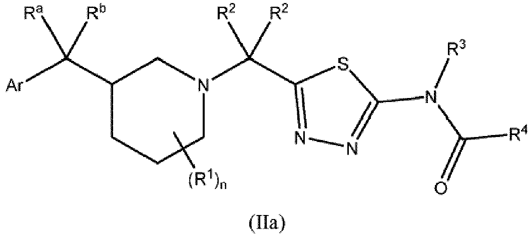 oder ein pharmazeutisch verträgliches Salz davon; wobei
R^{a} und R^{b} jeweils unabhängig -H oder C₁-C₄-Alkyl sind; und
R² bei jedem Auftreten unabhängig -H, Halogen, C₁-C₄-Alkyl ist;
(iii) 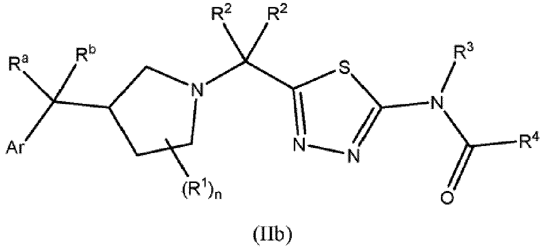 oder ein pharmazeutisch verträgliches Salz davon; wobei R² bei jedem Auftreten unabhängig -H, Halogen, C₁-C₄-Alkyl ist;
(iv) 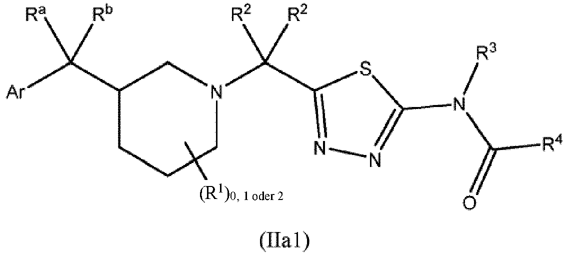 oder ein pharmazeutisch verträgliches Salz davon; wobei
R^{a} und R^{b} jeweils unabhängig -H oder Methyl sind;
R¹ Halogen, C₁-C₄-Alkyl oder C₁-C₄-Halogenalkyl ist; und
R² bei jedem Auftreten unabhängig -H oder C₁-C₄-Alkyl ist;
(v) 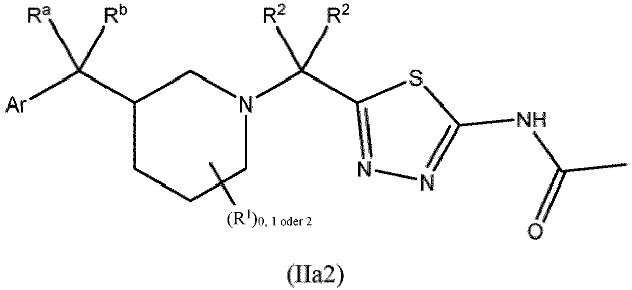 oder ein pharmazeutisch verträgliches Salz davon; wobei
R^{a} und R^{b} jeweils unabhängig -H oder Methyl sind;
R² bei jedem Auftreten unabhängig -H oder Methyl ist; und
R¹ -F oder Methyl ist;
(vi) 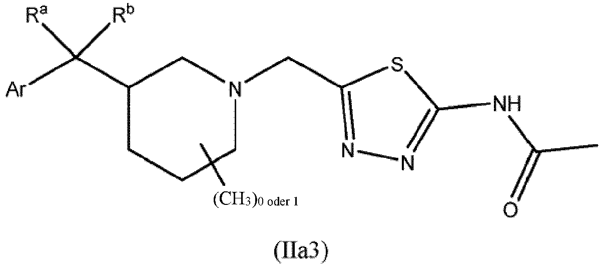 oder ein pharmazeutisch verträgliches Salz davon; oder
(vii) 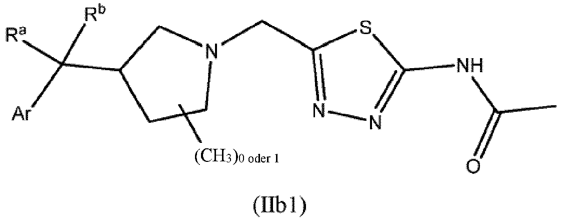 oder ein pharmazeutisch verträgliches Salz davon.

4. Verbindung nach einem der Ansprüche 1-3 oder ein pharmazeutisch verträgliches Salz davon, wobei
(i) Ar optional substituiertes Pyrazolyl, optional substituiertes Imidazolyl, optional substituiertes Thiazolyl, optional substituiertes Phenyl, optional substituiertes Pyridinyl, optional substituiertes Pyrimidinyl, optional substituiertes Pyrazinyl, optional substituiertes Imidazo[1,2-a]pyridinyl, optional substituiertes Thieno[2,3-d]pyrimidinyl oder optional substituiertes Thieno[3,2-d]pyrimidinyl ist;
(ii) Ar optional substituiertes 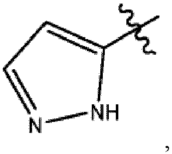 optional substituiertes 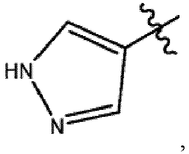 optional substituiertes 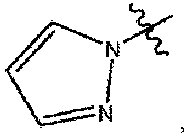 optional substituiertes 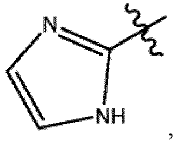 optional substituiertes 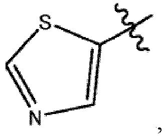 optional substituiertes 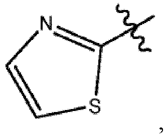 optional substituiertes 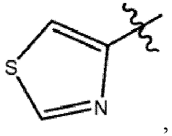 optional substituiertes 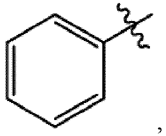 optional substituiertes 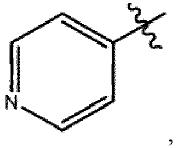 optional substituiertes 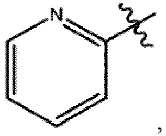 optional substituiertes 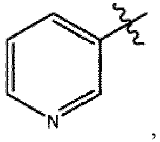 optional substituiertes 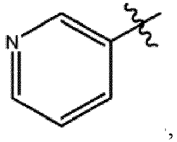 optional substituiertes 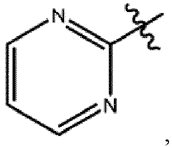 optional substituiertes 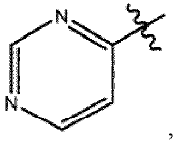 optional substituiertes 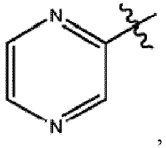 optional substituiertes 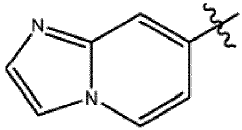 oder optional substituiertes 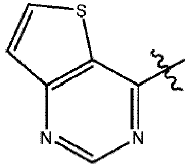 ist;
(iii) Ar optional substituiertes 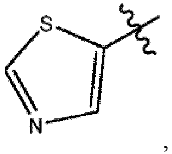 optional substituiertes 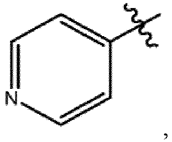 optional substituiertes 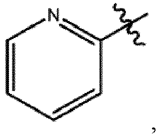 optional substituiertes 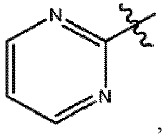 optional substituiertes 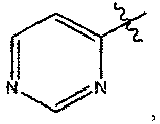 optional substituiertes 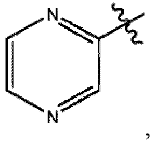 optional substituiertes 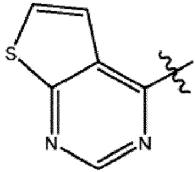 oder optional substituiertes 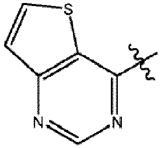 ist; oder
(iv) Ar optional substituiertes 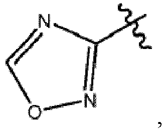 optional substituiertes 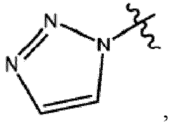 optional substituiertes 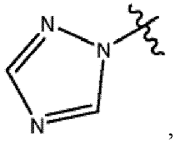 optional substituiertes 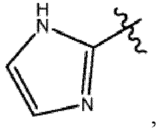 optional substituiertes 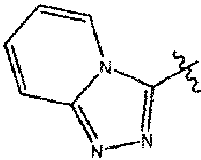 oder optional substituiertes 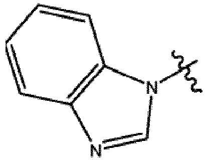 ist; oder
(v) Ar optional substituiertes 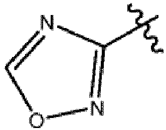 ist.

5. Verbindung nach einem der Ansprüche 1-4 oder ein pharmazeutisch verträgliches Salz davon, wobei:
Ar optional mit einer oder mehreren Gruppen ausgewählt aus C₁-C₄-Alkyl, C₁-C₄-Halogenalkyl, C₃-C₆-Cycloalkyl, Halogen, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, - NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, -C(=O)R^{x}, Phenyl und monocyclisches Heteroaryl substituiert ist;
wobei
der C₁-C₄-Alkylgruppensubstituent an Ar optional mit -CN, -NO₂, -OR^{z}, -NR^{x}R^{y}, - S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{x}, - C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, - NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x} und - C(=O)R^{y}, C₃-C₆-Cycloalkyl (optional substituiert mit einer oder mehreren Gruppen ausgewählt aus -CH₃, Halogenmethyl, Halogen, Methoxy und Halogenmethoxy), monocyclischem Heteroaryl (optional substituiert mit einer oder mehreren Gruppen ausgewählt aus -CH₃, Halogenmethyl, Halogen, Methoxy oder Halogenmethoxy) und Phenyl (optional substituiert mit einer oder mehreren Gruppen ausgewählt aus -CH₃, Halogenmethyl, Halogen, Methoxy und Halogenmethoxy) substituiert ist;
die C₃-C₆-Cycloalkyl-, Phenyl- und monocyclischen Heteroarylgruppen-Substituenten an Ar optional und unabhängig mit C₁-C₄-Alkyl, C₁-C₄-Halogenalkyl, Halogen, -CN, -NO₂, -OR^{z}, - NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, - O(C=S)R^{y}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, - O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, - C(=S)R^{x} und -C(= O)R^{x} substituiert sind;
jedes R^{x} und jedes R^{y} unabhängig -H, C₁-C₄-Alkyl oder C₃-C₈-Cycloalkyl ist; wobei das durch R^{x} oder R^{y} dargestellte C₁-C₄-Alkyl oder C₃-C₈-Cycloalkyl optional mit einem oder mehreren Substituenten ausgewählt aus Halogen, Hydroxyl, C₃-C₆-Cycloalkyl und Phenyl (optional substituiert mit einem oder mehrere Gruppen ausgewählt aus -CH₃, Halogenmethyl, Halogen, Methoxy oder Halogenmethoxy) substituiert ist;
R^{z} -H, C₁-C₄-Alkyl oder C₃-C₈-Cycloalkyl ist; wobei die durch R^{z} dargestellte C₁-C₄-Alkyl- oder C₃-C₈-Cycloalkylgruppe optional mit einem oder mehreren Substituenten ausgewählt aus Halogen, Hydroxyl, C₃-C₆-Cycloalkyl und Phenyl (optional substituiert mit einer oder mehreren Gruppen ausgewählt aus -CH₃, Halogenmethyl, Halogen, Methoxy und Halogenmethoxy) substituiert ist; und
i 0, 1 oder 2 ist.

6. Verbindung nach einem der Ansprüche 1-5 oder ein pharmazeutisch verträgliches Salz davon, wobei
(i) Ar optional mit einer oder mehreren Gruppen ausgewählt aus C₁-C₄-Alkyl, C₁-C₄-Halogenalkyl, C₃-C₆-Cycloalkyl, Halogen, -CN, -OR^{z}, -NR^{x}R^{y}, -C(=O)NR^{x}R^{y}, -C(=S)NR^{x}R^{y}, - O(C=O)NR^{x}R^{y}, -O(C=S)NR^{x}R^{y}, -C(=O)OR^{x}, -NR^{x}C(=O)R^{y} Phenyl, -C(=O)R^{x} und optional substituiertem monocyclischem Heteroaryl substituiert ist;
(ii) Ar optional mit einer oder mehreren Gruppen ausgewählt aus C₁-C₄-Alkyl, C₁-C₄-Halogenalkyl, C₃-C₆-Cycloalkyl, Halogen, -OR^{z}, -C(=O)R^{x} substituiert ist, und monocyclisches Heteroaryl optional mit C₁-C₄-Alkyl, C₁-C₄-Halogenalkyl, Halogen substituiert ist;
(iii) Ar optional mit einer oder mehreren Gruppen ausgewählt aus -CH₃, -CH₂CH₃, Halogenmethyl, Cyclopentyl, Cyclobutyl, Halogen, -OR^{z}, -C(=O)R^{x} und einem 5- oder 6-gliedrigen monocyclischen Heteroaryl enthaltend ein oder zwei Heteroatome ausgewählt aus S und N und optional substituiert mit C₁-C₄-Alkyl substituiert ist; wobei R^{x} -H oder C₁-C₄-Alkyl ist; und wobei R^{z} optional substituiertes C₁-C₄-Alkyl ist; oder
(iv) Ar optional mit einer oder mehreren Gruppen ausgewählt aus -CH₃, -CH₂CH₃, -CHF₂, -CF₃, Cyclopentyl, Cyclobutyl, -F, -Cl, -Br, -OCH₃, -C(=O)CH₃ und einem Thiazolyl substituiert ist.

7. Verbindung nach Anspruch 1, wobei die Verbindung durch die folgende Strukturformel dargestellt ist: 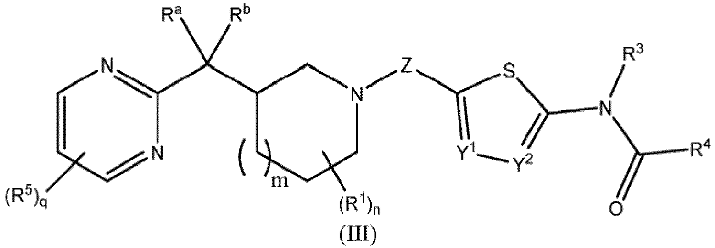 oder ein pharmazeutisch verträgliches Salz davon; wobei:
Y¹ und Y² jeweils CR^{c} oder N sind, wobei mindestens eines von Y¹ oder Y² N ist;
Z CR²R², C(=O), (CR²R²)₂, CH₂C(=O) oder C(=O)CH₂ ist;
R^{a}, R^{b} und R^{c} jeweils unabhängig -H, Halogen, C₁-C₄-Alkyl, C₁-C₄-Halogenalkyl oder C₁-C₄-Alkoxy sind oder R^{a} und R^{b} zusammen mit ihrem dazwischenliegenden Kohlenstoffatom ein C₃-C₆-Cycloalkyl bilden;
m 0 oder 1 ist;
n 0 oder eine ganze Zahl von 1 bis 7 ist;
wenn n nicht 0 ist, R¹ bei jedem Auftreten unabhängig Halogen, C₁-C₄-Alkyl, C₁-C₄-Halogenalkyl, C₁-C₄-Alkoxy oder C₁-C₄-Halogenalkoxy ist;
R² bei jedem Auftreten unabhängig -H, Halogen, C₁-C₄-Alkyl, C₁-C₄-Halogenalkyl, C₃-C₁₀-Cycloalkyl oder C₃-C₁₀-Halogencycloalkyl ist;
oder alternativ zwei R² zusammen mit dem Kohlenstoffatom, an das sie gebunden sind, ein C₃-C₁₀-Cycloalkyl bilden;
R³ -H oder C₁-C₄-Alkyl ist; und
R⁴ -H, C₁-C₄-Alkyl, C₁-C₄-Haloalkyl oder C₃-C₆-Cycloalkyl ist;
oder alternativ R³ und R⁴ zusammen mit ihren dazwischenliegenden Atomen ein optional substituiertes 5- bis 7-gliedriges Heterocyclyl bilden;
R⁵ bei jedem Auftreten aus C₁-C₄-Alkyl, C₁-C₄-Halogenalkyl, C₃-C₆-Cycloalkyl, Halogen, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, -NR^{x}C(=O)R^{y}, -C(=S)NR^{x}R^{y}, -NR^{x}C(=S)R^{y}, - NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, - NR^{x}(C=S)NR^{x}R^{y}, -C(=S)R^{x}, -C(=O)R^{x}, Phenyl und monocyclischem Heteroaryl ausgewählt ist; wobei
wenn R⁵ eine C₁-C₄-Alkylgruppe ist, die C₁-C₄-Alkylgruppe optional und unabhängig mit -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{x}, -O(C=S)R^{x}, -C(=O)NR^{x}R^{y}, -NR^{x}C(=O)R^{y}, -C(=S)NR^{x}R^{y}, -NR^{x}C(=S)R^{y}, - NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, - NR^{x}(C=S)NR^{x}R^{y}, -C(=S)R^{x} und -C(=O)R^{y}, C₃-C₆-Cycloalkyl (optional substituiert mit einer oder mehreren Gruppen ausgewählt aus -CH₃, Halogenmethyl, Halogen, Methoxy und Halogenmethoxy), monocyclischem Heteroaryl (optional substituiert mit einer oder mehreren Gruppen ausgewählt aus -CH₃, Halogenmethyl, Halogen, Methoxy oder Halogenmethoxy) und Phenyl (optional substituiert mit einer oder mehreren Gruppen ausgewählt aus -CH₃, Halogenmethyl, Halogen, Methoxy und Halogenmethoxy) substituiert ist;
wenn R⁵ ein C₃-C₆-Cycloalkyl, Phenyl oder ein monocyclisches Heteroaryl ist, das Cycloalkyl, Phenyl oder ein monocyclisches Heteroaryl optional und unabhängig mit C₁-C₄-Alkyl, C₁-C₄-Halogenalkyl, Halogen, -CN, -NO₂, -OR^{z}, -NR^{x}R^{y}, -S(O)ᵢR^{x}, -NR^{x}S(O)ᵢR^{y}, - S(O)ᵢNR^{x}R^{y}, -C(=O)OR^{x}, -OC(=O)OR^{x}, -C(=S)OR^{x}, -O(C=S)R^{y}, -C(=O)NR^{x}R^{y}, - NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x} und -C(=O)R^{x} substituiert ist;
jedes R^{x} und jedes R^{y} unabhängig -H, C₁-C₄-Alkyl oder C₃-C₈-Cycloalkyl ist; wobei das durch R^{x} oder R^{y} dargestellte C₁-C₄-Alkyl oder C₃-C₈-Cycloalkyl optional mit einem oder mehreren Substituenten ausgewählt aus Halogen, Hydroxyl, C₃-C₆-Cycloalkyl und Phenyl (optional substituiert mit einer oder mehreren Gruppen ausgewählt aus -CH₃, Halogenmethyl, Halogen, Methoxy oder Halogenmethoxy) substituiert ist;
R^{z} -H, C₁-C₄-Alkyl oder C₃-C₈-Cycloalkyl ist; wobei die durch R^{z} dargestellte C₁-C₄-Alkyl- oder C₃-C₈-Cycloalkylgruppe optional mit einem oder mehreren Substituenten ausgewählt aus Halogen, Hydroxyl, C₃-C₆-Cycloalkyl und Phenyl (optional substituiert mit einer oder mehreren Gruppen ausgewählt aus -CH₃, Halogenmethyl, Halogen, Methoxy und Halogenmethoxy) substituiert ist; und
i 0, 1 oder 2 ist; und
q 0, 1, 2 oder 3 ist.

8. Verbindung nach Anspruch 1, wobei die Verbindung durch die folgenden Strukturformeln dargestellt ist:
(i) 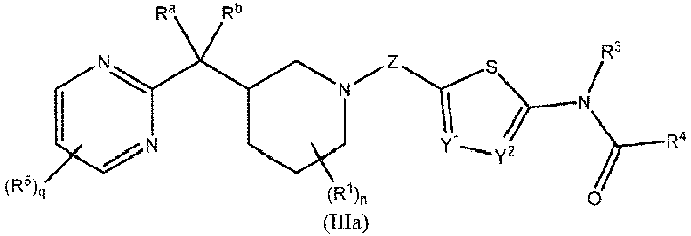 oder ein pharmazeutisch verträgliches Salz davon;
(ii) 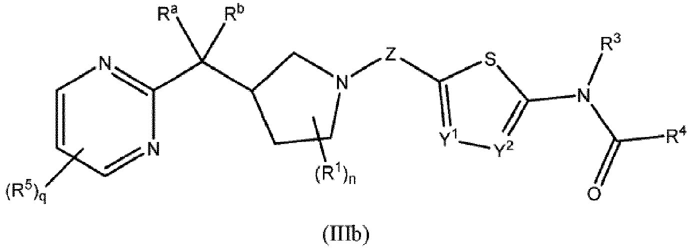 oder ein pharmazeutisch verträgliches Salz davon; wobei q 0, 1, 2 oder 3 ist;
(iii) 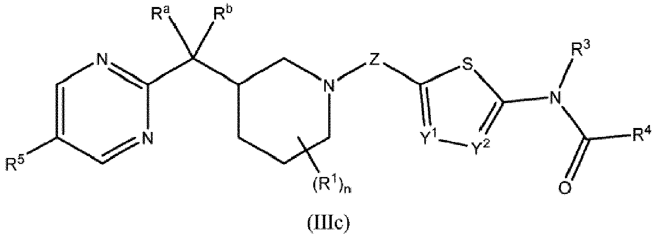 oder ein pharmazeutisch verträgliches Salz davon;
(iv) 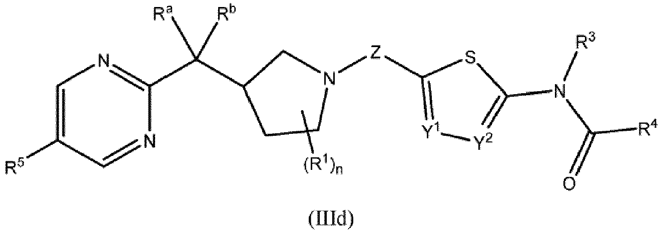 oder ein pharmazeutisch verträgliches Salz davon;
(v) 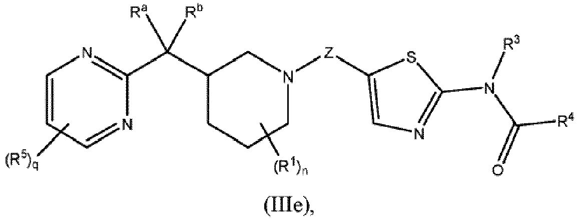 oder ein pharmazeutisch verträgliches Salz davon;
(vi) 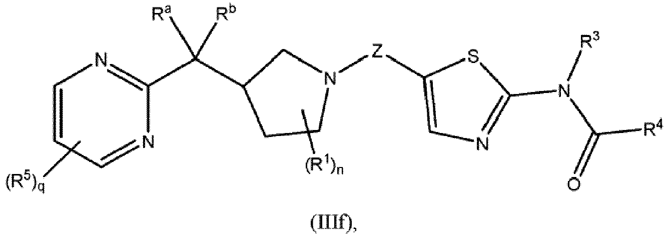 oder ein pharmazeutisch verträgliches Salz davon; wobei q 0, 1, 2 oder 3 ist;
(vii) 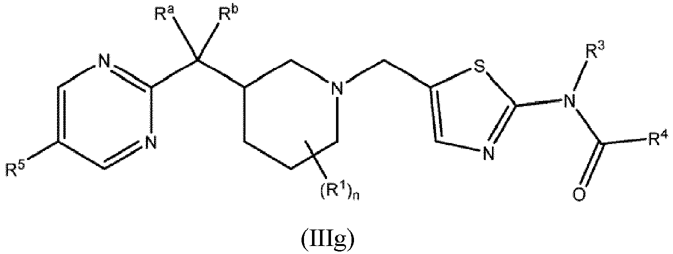 oder ein pharmazeutisch verträgliches Salz davon;
(viii) 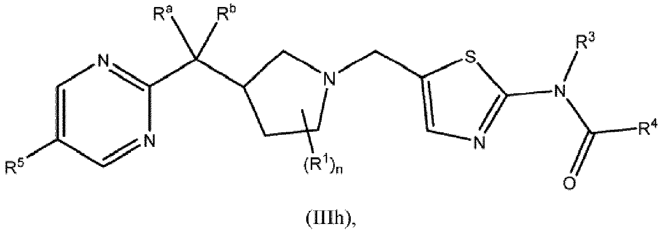 oder ein pharmazeutisch verträgliches Salz davon;
(ix) 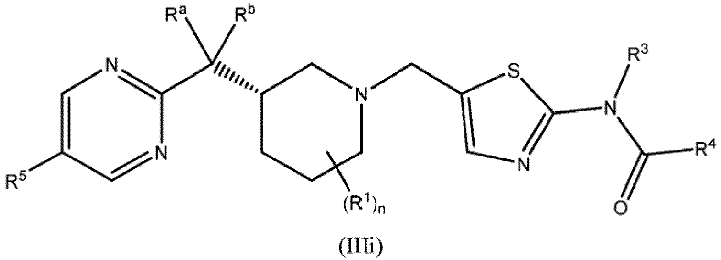 oder ein pharmazeutisch verträgliches Salz davon;
(x) 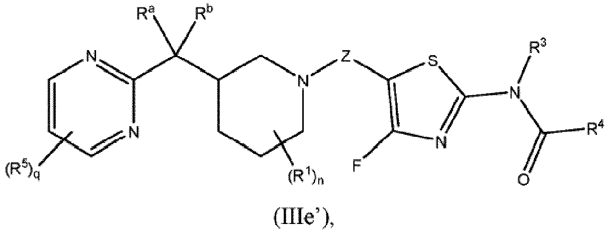 oder ein pharmazeutisch verträgliches Salz davon, wobei q 0, 1, 2 oder 3 ist;
(xi) 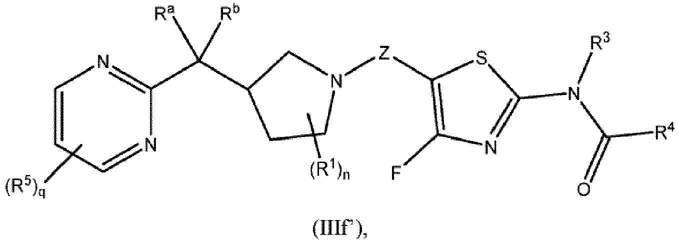 oder ein pharmazeutisch verträgliches Salz davon;
(xii) 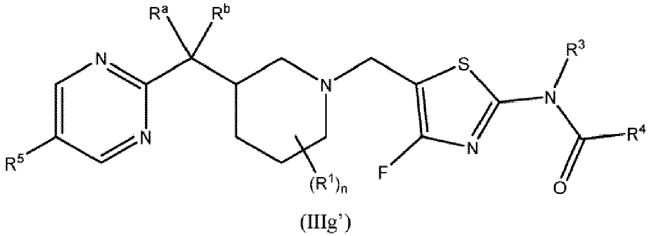 oder ein pharmazeutisch verträgliches Salz davon;
(xiii) 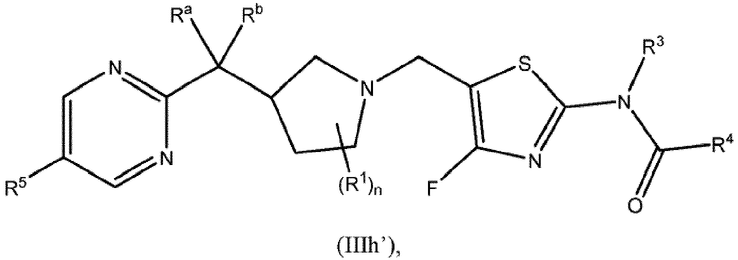 oder ein pharmazeutisch verträgliches Salz davon;
(xiv) 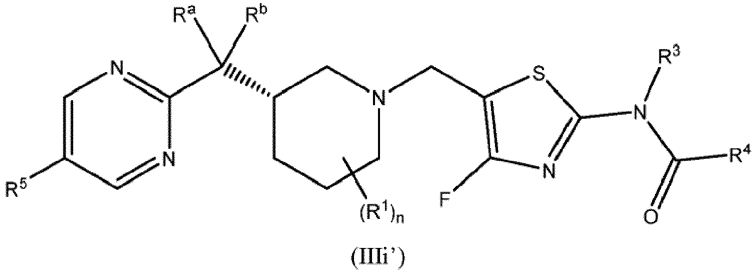 oder ein pharmazeutisch verträgliches Salz davon;
(xv) 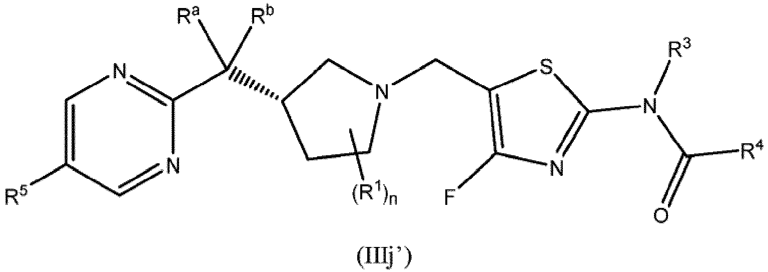 oder ein pharmazeutisch verträgliches Salz davon; oder
(xvi) 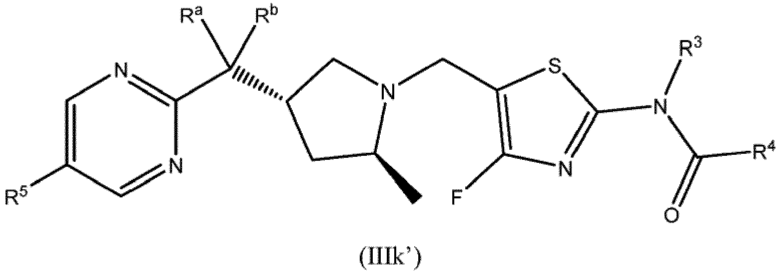 oder ein pharmazeutisch verträgliches Salz davon.

9. Verbindung nach einem der Ansprüche 1-8 oder ein pharmazeutisch verträgliches Salz davon, wobei R³ -H ist.

10. Verbindung nach einem der Ansprüche 1-9 oder ein pharmazeutisch verträgliches Salz davon, wobei R⁴ -CH₃ ist.

11. Verbindung nach einem der Ansprüche 1 oder 9-10 oder ein pharmazeutisch verträgliches Salz davon, wobei R⁵ aus C₁-C₄-Alkyl, C₁-C₄-Halogenalkyl, C₃-C₆-Cycloalkyl, Halogen, -CN, - OR^{z}, -NR^{x}R^{y}, -C(=O)NR^{x}R^{y}, -C(=S)NR^{x}R^{y}, -O(C=O)NR^{x}R^{y}, -O(C=S)NR^{x}R^{y}, -C(=O)OR^{x}, - NR^{x}C(=O)R^{y} Phenyl, -C(=O)R^{x} und optional substituiertem monocyclischem Heteroaryl ausgewählt ist; optional, wobei:
(i) R⁵ aus C₁-C₄-Alkyl, C₁-C₄-Halogenalkyl, C₃-C₆-Cycloalkyl, Halogen, -OR^{z}, -C(=O)R^{x} und monocyclischem Heteroaryl optional substituiert mit C₁-C₄-Alkyl, C₁-C₄-Halogenalkyl, Halogen ausgewählt ist;
(ii) R⁵ aus -CH₃, -CH₂CH₃, Halogenmethyl, Cyclopentyl, Cyclobutyl, Halogen, -OR^{z}, - C(=O)R^{x} und einem 5- oder 6-gliedrigen monocyclischen Heteroaryl enthaltend ein oder zwei Heteroatome ausgewählt aus S und N und optional substituiert mit C₁-C₄-Alkyl ausgewählt ist; wobei R^{X} -H oder C₁-C₄-Alkyl ist; und wobei R^{z} optional substituiertes C₁-C₄-Alkyl ist;
(iii) R⁵ aus -CH₃, -CH₂CH₃, -CHF₂, -CF₃, Cyclopentyl, Cyclobutyl, -F, -Br, Cl, -OCH₃, -C(=O)CH₃ und ein Thiazolyl ausgewählt ist; oder
(iv) R⁵ aus -F, -Br und Cl ausgewählt ist.

12. Verbindung nach einem von 1-11 oder ein pharmazeutisch verträgliches Salz davon, wobei eines von R^{a} und R^{b} -H ist und das andere aus -CH₃, -CF₃ und -OCH₃ ausgewählt ist.

13. Verbindung nach einem von 1-12 oder ein pharmazeutisch verträgliches Salz davon, wobei n 0 oder 1 ist.

14. Pharmazeutische Zusammensetzung, umfassend die Verbindung nach einem der Ansprüche 1-13 oder ein pharmazeutisch verträgliches Salz davon und einen pharmazeutisch verträglichen Träger oder ein pharmazeutisch verträgliches Verdünnungsmittel.

15. Verbindung nach einem der Ansprüche 1-13 oder eine pharmazeutische Zusammensetzung nach Anspruch 14 zur Verwendung bei der Behandlung einer Krankheit oder eines Zustands ausgewählt aus einer neurodegenerativen Krankheit, einer Tauopathie, Diabetes, Krebs und Stress, optional wobei:
(i) die Krankheit oder der Zustand aus akutem ischämischem Schlaganfall (AIS), Alzheimer-Krankheit, Demenz, amyotropher Lateralsklerose (ALS), amyotropher Lateralsklerose mit kognitiver Beeinträchtigung (ALSci), Demenz mit argyrophilen Körperchen, Bluit-Krankheit, kortikobasaler Degeneration (CBP), Demenz pugilistica, diffusen Neurofibrillenverschlingungen mit Verkalkung, Down-Syndrom, Epilepsie, familiärer britischer Demenz, familiärer dänischer Demenz, frontotemporaler Demenz mit Parkinsonismus in Verbindung mit Chromosom 17 (FTDP-17), Gerstmann-Straussler-Scheinker-Krankheit, Guadeloupe-Parkinsonismus, Hallevorden-Spatz-Krankheit (Neurodegeneration mit Eisenakkumulation im Gehirn Typ 1), ischämischem Schlaganfall, leichter kognitiver Beeinträchtigung (MCI), multipler Systematrophie, myotoner Dystrophie, Niemann-Pick-Krankheit (Typ C), Pallido-ponto-nigraler Degeneration, Guam-Parkinson-Demenz Komplex, Morbus Pick (PiD), postenzephalitischem Parkinsonismus (PEP), Prionenerkrankungen (einschließlich Creutzfeldt-Jakob-Krankheit (GJD), varianter Creutzfeldt-Jakob-Krankheit (vCJD), tödlicher familiärer Schlaflosigkeit, Kuru, progressiver superkortikaler Gliose, progressiver supranukleärer Lähmung (PSP), Steele-Richardson-Olszewski-Syndrom, subakuter sklerosierender Panenzephalitis, Tangle-only-Demenz, Huntington-Krankheit und Parkinson-Krankheit ausgewählt ist;
(ii) die Krankheit oder der Zustand aus akutem ischämischem Schlaganfall (AIS), Alzheimer-Krankheit, Demenz, amyotropher Lateralsklerose (ALS), amyotropher Lateralsklerose mit kognitiver Beeinträchtigung (ALSci), Demenz mit argyrophilen Körperchen, Epilepsie, ischämischem Schlaganfall, leichter kognitiver Beeinträchtigung (MCI), Huntington-Krankheit und Parkinson-Krankheit ausgewählt ist; oder
(iii) die Krankheit oder der Zustand Alzheimer-Krankheit ist.

## Revendications

1. Composé représenté par la formule développée suivante :
(i) 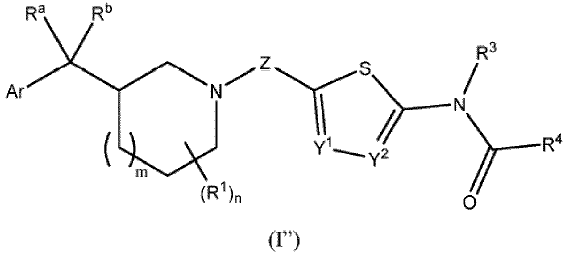 ou sel pharmaceutiquement acceptable de celui-ci, dans laquelle :
Ar représente un groupe hétéroaryle de 5 à 10 chaînons éventuellement substitué, un groupe phényle éventuellement substitué ou un groupe phényle éventuellement substitué fusionné à un groupe hétérocycle de 5 à 6 chaînons non aromatique éventuellement substitué ;
Y¹ et Y² représentent chacun un groupe CR^{c} ou un atome N, au moins un groupe parmi Y¹ ou Y² représentant un atome N ;
Z représente un groupe CR²R², C(=O), (CR²R²)₂, CH₂C(=O) ou C(=O)CH₂ ;
R^{a} et R^{b} représentent chacun indépendamment un groupe -H, halogéno, C₁-C₄ alkyle, C₁-C₄ halogénoalkyle ou C₁-C₄ alcoxy, ou R^{a} et R^{b} pris conjointement avec leur atome de carbone intermédiaire forment un groupe C₃-C₆ cycloalkyle ;
R^{c} représente un groupe halogéno, C₁-C₄ alkyle, C₁-C₄ halogénoalkyle ou C₁-C₄ alcoxy ;
m vaut 0 ou 1 ;
n vaut 0 ou représente un entier allant de 1 à 7 ;
lorsque n est différent de 0, R¹, pour chaque occurrence, représente indépendamment un groupe halogéno, C₁-C₄ alkyle, C₁-C₄ halogénoalkyle, C₁-C₄ alcoxy ou C₁-C₄ halogénoalcoxy ;
R², pour chaque occurrence, représente indépendamment un groupe -H, halogéno, C₁-C₄ alkyle, C₁-C₄ halogénoalkyle, C₃-C₁₀ cycloalkyle ou C₃-C₁₀ halogénocycloalkyle ;
ou sinon deux groupes R² conjointement avec l'atome de carbone auquel ils sont liés forment un groupe C₃-C₁₀ cycloalkyle ;
R³ représente un groupe -H ou un groupe C₁-C₄ alkyle ; et
R⁴ représente un groupe -H, C₁-C₄ alkyle, C₁-C₄ halogénoalkyle ou C₃-C₆ cycloalkyle ;
ou sinon R³ et R⁴ pris conjointement avec leurs atomes intermédiaires forment un groupe hétérocyclyle de 5 à 7 chaînons éventuellement substitué ; ou
(ii) 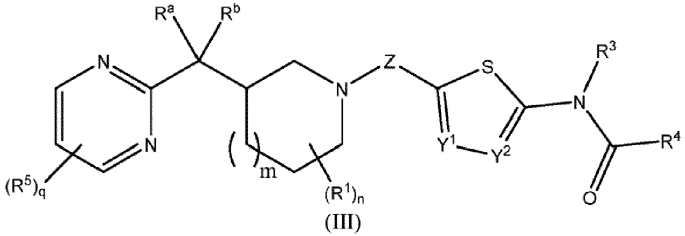 ou sel pharmaceutiquement acceptable de celui-ci ; dans laquelle :
Y¹ et Y² représentent chacun un groupe CR^{c} ou un atome N, au moins un groupe parmi Y¹ ou Y² représentant un atome N ;
Z représente un groupe CR²R², C(=O), (CR²R²)₂, CH₂C(=O) ou C(=O)CH₂ ;
R^{a}, R^{b} et R^{c} représentent chacun indépendamment un groupe -H, halogéno, C₁-C₄ alkyle, C₁-C₄ halogénoalkyle ou C₁-C₄ alcoxy, ou R^{a} et R^{b} pris conjointement avec leur atome de carbone intermédiaire forment un groupe C₃-C₆ cycloalkyle ;
m vaut 0 ou 1 ;
n vaut 0 ou représente un entier allant de 1 à 7 ;
lorsque n est différent de 0, R¹, pour chaque occurrence, représente indépendamment un groupe halogéno, C₁-C₄ alkyle, C₁-C₄ halogénoalkyle, C₁-C₄ alcoxy ou C₁-C₄ halogénoalcoxy ;
R², pour chaque occurrence, représente indépendamment un groupe -H, halogéno, C₁-C₄ alkyle, C₁-C₄ halogénoalkyle, C₃-C₁₀ cycloalkyle or C₃-C₁₀ halogénocycloalkyle ;
ou sinon deux groupes R² conjointement avec l'atome de carbone auquel ils sont liés forment un groupe C₃-C₁₀ cycloalkyle ;
R³ représente un groupe -H ou un groupe C₁-C₄ alkyle ; et
R⁴ représente un groupe -H, C₁-C₄ alkyle, C₁-C₄ halogénoalkyle ou C₃-C₆ cycloalkyle ;
ou sinon R³ et R⁴ pris conjointement avec leurs atomes intermédiaires forment un groupe hétérocyclyle de 5 à 7 chaînons éventuellement substitué ;
R⁵, pour chaque occurrence, est choisi parmi les groupes C₁-C₄ alkyle, C₁-C₄ halogénoalkyle, C₃-C₆ cycloalkyle, halogéno, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, -C(=O)R^{x}, phényle et hétéroaryle monocyclique ;
dans laquelle
lorsque R⁵ représente un groupe C₁-C₄ alkyle, le groupe C₁-C₄ alkyle est éventuellement et indépendamment substitué par un groupe -CN, -NO₂, -OR^{z}, -NR^{x}R^{y}, -S(O)ᵢR^{x}, -NR^{x}S(O)ᵢR^{y}, - S(O)ᵢNR^{x}R^{y}, -C(=O)OR^{x}, -OC(=O)OR^{x}, -C(=S)OR^{x}, -O(C=S)R^{x}, -C(=O)NR^{x}R^{y}, -NR^{x}C(=O)R^{y},-C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x} et -C(=O)R^{y}, C₃-C₆ cycloalkyle (éventuellement substitué par un ou plusieurs groupes choisis parmi les groupes -CH₃, halogénométhyle, halogéno, méthoxy et halogénométhoxy), hétéroaryle monocyclique (éventuellement substitué par un ou plusieurs groupes choisis parmi un groupe -CH₃, halogénométhyle, halogéno, méthoxy ou halogénométhoxy) et phényle (éventuellement substitué par un ou plusieurs groupes choisis parmi les groupes -CH₃, halogénométhyle, halogéno, méthoxy et halogénométhoxy) ;
lorsque R⁵ représente un groupe C₃-C₆ cycloalkyle, phényle ou un groupe hétéroaryle monocyclique, le groupe cycloalkyle, phényle ou un groupe hétéroaryle monocyclique est éventuellement et indépendamment substitué par les groupes C₁-C₄ alkyle, C₁-C₄ halogénoalkyle, halogéno, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, - OC(=O)OR^{x}, -C(=S)OR^{x}, -O(C=S)R^{y}, -C(=O)NR^{x}R^{y}, -NR^{x}C(=O)R^{y}, -C(=S)NR^{x}R^{y}, - NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, - NR^{x}(C=O)NR^{x}R^{y}, -NR^{x}(C=S)NR^{x}R^{y}, -C(=S)R^{x} et -C(=O)R^{x} ;
chaque groupe R^{x} et chaque groupe R^{y} représentent indépendamment un groupe -H, C₁-C₄ alkyle ou C₃-C₈ cycloalkyle ; ledit groupe C₁-C₄ alkyle ou C₃-C₈ cycloalkyle représenté par R^{x} ou R^{y} étant éventuellement substitué par un ou plusieurs substituants choisis parmi les groupes halogéno, hydroxy, C₃-C₆ cycloalkyle et phényle (éventuellement substitué par un ou plusieurs groupes choisis parmi un groupe -CH₃, halogénométhyle, halogéno, méthoxy ou halogénométhoxy) ;
R^{z} représente un groupe -H, C₁-C₄ alkyle ou C₃-C₈ cycloalkyle ; ledit groupe C₁-C₄ alkyle ou C₃-C₈ cycloalkyle représenté par R^{z} étant éventuellement substitué par un ou plusieurs substituants choisis parmi les groupes halogéno, hydroxy, C₃-C₆ cycloalkyle et phényle (éventuellement substitué par un ou plusieurs groupes choisis parmi les groupes -CH₃, halogénométhyle, halogéno, méthoxy et halogénométhoxy) ; et
i vaut 0, 1 ou 2 ; et
q vaut 0, 1, 2 ou 3.

2. Composé selon la revendication 1, ledit composé étant représenté par les formules développées suivantes :
(i) 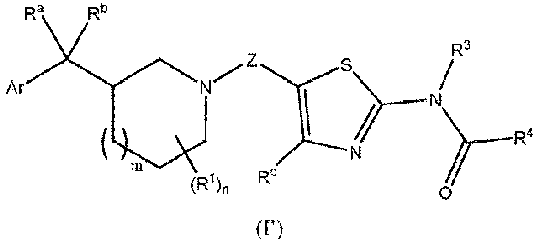 ou un sel pharmaceutiquement acceptable de celui-ci ;
(ii) 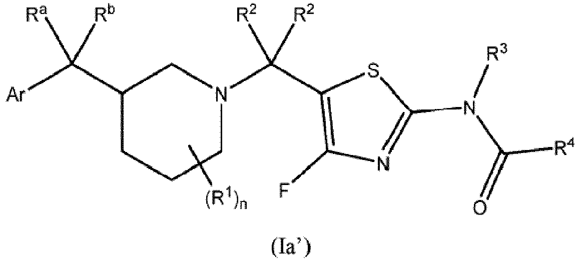 ou un sel pharmaceutiquement acceptable de celui-ci ; dans laquelle
R^{a} et R^{b} représentent chacun indépendamment un groupe -H ou C₁-C₄ alkyle ; et
R², pour chaque occurrence, représente indépendamment un groupe -H, halogéno, C₁-C₄ alkyle ;
(iii) 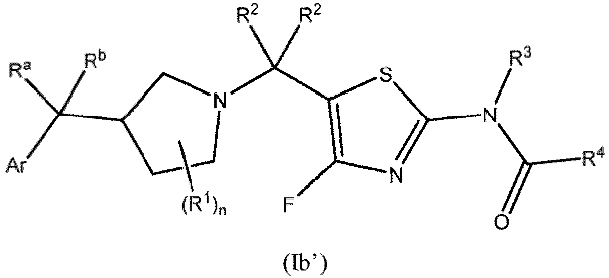 ou un sel pharmaceutiquement acceptable de celui-ci ; dans laquelle R², pour chaque occurrence, représente indépendamment un groupe -H, halogéno, C₁-C₄ alkyle ;
(iv) 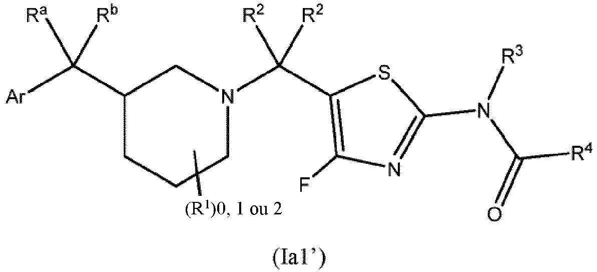 ou un sel pharmaceutiquement acceptable de celui-ci ; dans laquelle
R^{a} et R^{b} représentent chacun indépendamment un groupe -H ou méthyle ;
R¹ représente un groupe halogéno, C₁-C₄ alkyle ou C₁-C₄ halogénoalkyle ; et
R², pour chaque occurrence, représente indépendamment un groupe -H ou C₁-C₄ alkyle ;
(v) 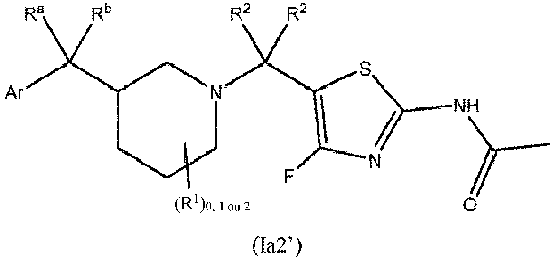 ou un sel pharmaceutiquement acceptable de celui-ci ; dans laquelle
R^{a} et R^{b} représentent chacun indépendamment un groupe -H ou méthyle ;
R², pour chaque occurrence, représente indépendamment un groupe -H ou méthyle ; et
R¹ représente un groupe -F ou méthyle ;
(vi) 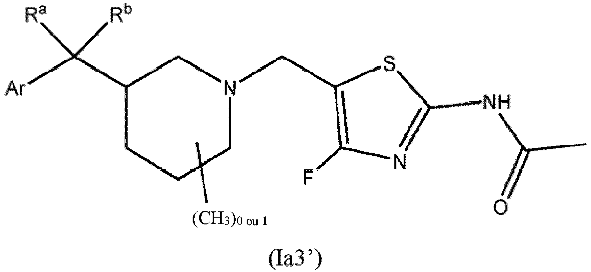 ou un sel pharmaceutiquement acceptable de celui-ci ; ou
(vii) 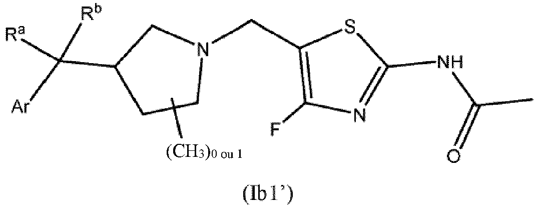 ou un sel pharmaceutiquement acceptable de celui-ci.

3. Composé selon la revendication 1 ou la revendication 2, ledit composé étant représenté par les formules développées suivantes :
(i) 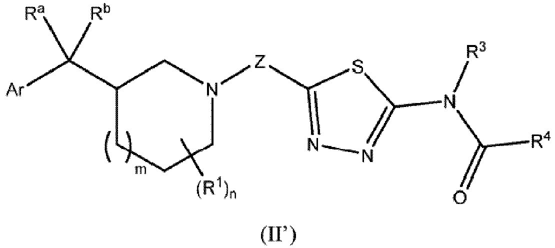 ou un sel pharmaceutiquement acceptable de celui-ci ;
(ii) 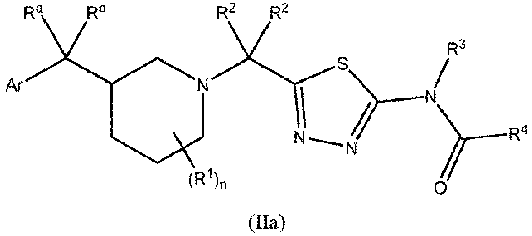 ou un sel pharmaceutiquement acceptable de celui-ci ; dans laquelle
R^{a} et R^{b} représentent chacun indépendamment un groupe -H ou C₁-C₄ alkyle ; et
R², pour chaque occurrence, représente indépendamment un groupe -H, halogéno, C₁-C₄ alkyle ;
(iii) 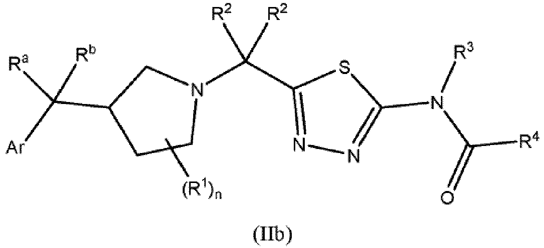 ou un sel pharmaceutiquement acceptable de celui-ci ; dans laquelle R², pour chaque occurrence, représente indépendamment un groupe -H, halogéno, C₁-C₄ alkyle ;
(iv) 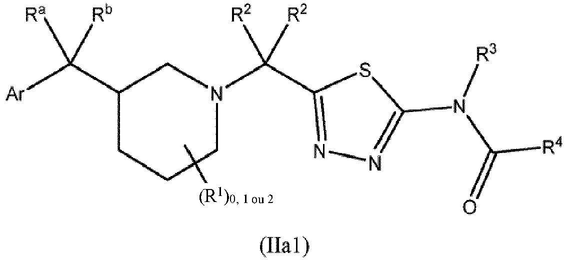 ou un sel pharmaceutiquement acceptable de celui-ci ; dans laquelle
R^{a} et R^{b} représentent chacun indépendamment un groupe -H ou méthyle ;
R¹ représente un groupe halogéno, C₁-C₄ alkyle ou C₁-C₄ halogénoalkyle ; et
R², pour chaque occurrence, représente indépendamment un groupe -H ou C₁-C₄ alkyle ;
(v) 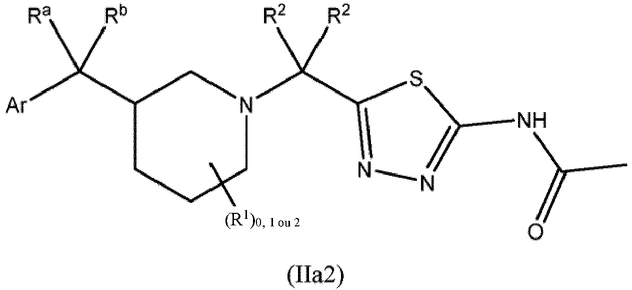 ou un sel pharmaceutiquement acceptable de celui-ci ; dans laquelle
R^{a} et R^{b} représentent chacun indépendamment un groupe -H ou méthyle ;
R², pour chaque occurrence, représente indépendamment un groupe -H ou méthyle ; et
R¹ représente un groupe -F ou méthyle ;
(vi) 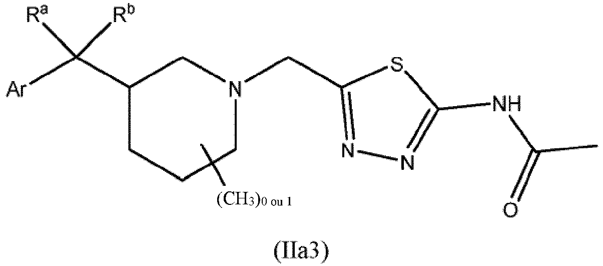 ou un sel pharmaceutiquement acceptable de celui-ci ; ou
(vii) 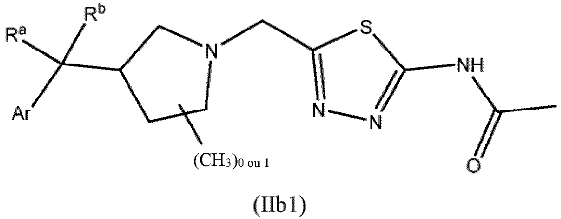 ou un sel pharmaceutiquement acceptable de celui-ci.

4. Composé selon l'une quelconque des revendications 1 à 3, ou sel pharmaceutiquement acceptable de celui-ci,
(i) Ar représentant un groupe pyrazolyle éventuellement substitué, un groupe imidazolyle éventuellement substitué, un groupe thiazolyle éventuellement substitué, un groupe phényle éventuellement substitué, un groupe pyridinyle éventuellement substitué, un groupe pyrimidinyle éventuellement substitué, un groupe pyrazinyle éventuellement substitué, un groupe imidazo[1,2-*a*]pyridinyle éventuellement substitué, un groupe thiéno[2,3-d]pyrimidinyle éventuellement substitué ou un groupe thiéno[3,2-d]pyrimidinyle éventuellement substitué ;
(ii) Ar représentant un groupe 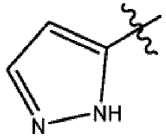 éventuellement substitué, un groupe 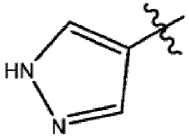 éventuellement substitué, un groupe 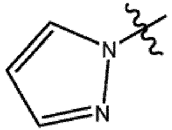 éventuellement substitué, un groupe 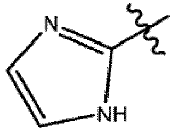 éventuellement substitué, un groupe 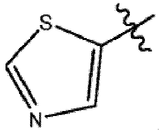 éventuellement substitué, un groupe 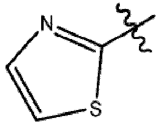 éventuellement substitué, un groupe 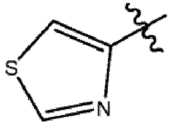 éventuellement substitué, un groupe 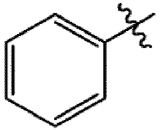 éventuellement substitué, un groupe 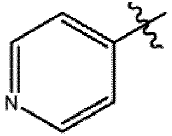 éventuellement substitué, un groupe 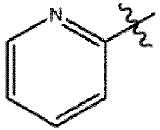 éventuellement substitué, un groupe 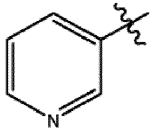 éventuellement substitué, un groupe 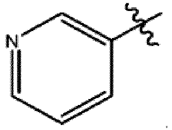 éventuellement substitué, un groupe 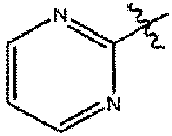 éventuellement substitué, un groupe 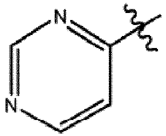 éventuellement substitué, un groupe 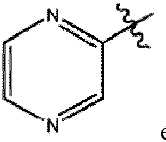 éventuellement substitué, un groupe 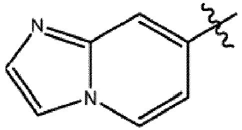 éventuellement substitué, ou un groupe 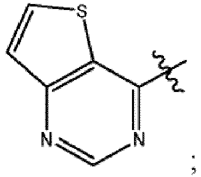 éventuellement substitué ;
(iii) Ar représentant un groupe 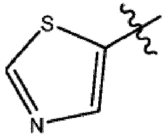 éventuellement substitué, un groupe 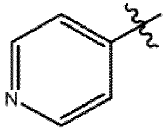 éventuellement substitué, un groupe 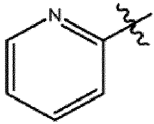 éventuellement substitué, un groupe 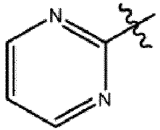 éventuellement substitué, un groupe 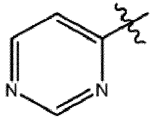 éventuellement substitué, un groupe 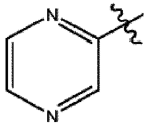 éventuellement substitué, un groupe 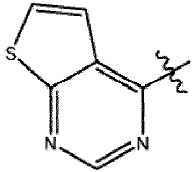 éventuellement substitué ou un groupe 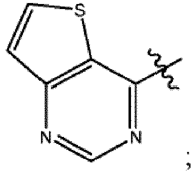 éventuellement substitué ; ou
(iv) Ar représentant un groupe 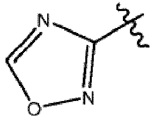 éventuellement substitué, un groupe 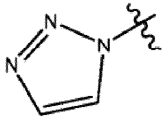 éventuellement substitué, un groupe 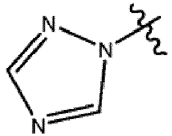 éventuellement substitué, un groupe 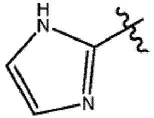 éventuellement substitué, un groupe 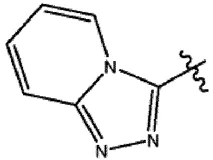 éventuellement substitué ou un groupe 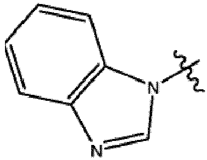 éventuellement substitué ; ou
(v) Ar représentant un groupe 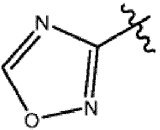 éventuellement substitué.

5. Composé selon l'une quelconque des revendications 1 à 4 ou sel pharmaceutiquement acceptable de celui-ci :
Ar étant éventuellement substitué par un ou plusieurs groupes choisis parmi les groupes C₁-C₄ alkyle, C₁-C₄ halogénoalkyle, C₃-C₆ cycloalkyle, halogéno, -CN, -NO₂, -OR^{z}, -NR^{x}R^{y}, - S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, - C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, - NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, -C(=O)R^{x}, phényle et hétéroaryle monocyclique ;
ledit substituent groupe C₁-C₄ alkyle sur Ar étant éventuellement substitué par un groupe -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, - C(=S)OR^{x}, -O(C=S)R^{x}, -C(=O)NR^{x}R^{y}, -NR^{x}C(=O)R^{y}, -C(=S)NR^{x}R^{y}, -NR^{x}C(=S)R^{y}, - NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, - NR^{x}(C=S)NR^{x}R^{y}, -C(=S)R^{x} et -C(=O)R^{y}, C₃-C₆ cycloalkyle (éventuellement substitué par un ou plusieurs groupes choisis parmi les groupes -CH₃, halogénométhyle, halogéno, méthoxy et halogénométhoxy), hétéroaryle monocyclique (éventuellement substitué par un ou plusieurs groupes choisis parmi un groupe -CH₃, halogénométhyle, halogéno, méthoxy ou halogénométhoxy) et phényle (éventuellement substitué par un ou plusieurs groupes choisis parmi les groupes -CH₃, halogénométhyle, halogéno, méthoxy et halogénométhoxy) ;
lesdits substituants groupes C₃-C₆ cycloalkyle, phényle et hétéroaryle monocyclique sur Ar étant éventuellement et indépendamment substitués par les groupes C₁-C₄ alkyle, C₁-C₄ halogénoalkyle, halogéno, -CN, -NO₂, -OR^{z}, -NR^{x}R^{y}, -S(O)ᵢR^{x}, -NR^{x}S(O)ᵢR^{y}, -S(O)ᵢNR^{x}R^{y}, - C(=O)OR^{x}, -OC(=O)OR^{x}, -C(=S)OR^{x}, -O(C=S)R^{y}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, - NR^{x}(C=O)NR^{x}R^{y}, -NR^{x}(C=S)NR^{x}R^{y}, -C(=S)R^{x} et -C(=O)R^{x} ;
chaque groupe R^{x} et chaque groupe R^{y} représentant indépendamment un groupe -H, C₁-C₄ alkyle ou C₃-C₈ cycloalkyle ; ledit groupe C₁-C₄ alkyle ou C₃-C₈ cycloalkyle représenté par R^{x} ou R^{y} étant éventuellement substitué par un ou plusieurs substituants choisis parmi les groupes halogéno, hydroxy, C₃-C₆ cycloalkyle et phényle (éventuellement substitué par un ou plusieurs groupes choisis parmi un groupe -CH₃, halogénométhyle, halogéno, méthoxy ou halogénométhoxy) ;
R^{z} représentant un groupe -H, C₁-C₄ alkyle ou C₃-C₈ cycloalkyle ; ledit groupe C₁-C₄ alkyle ou C₃-C₈ cycloalkyle représenté par R^{z} étant éventuellement substitué par un ou plusieurs substituants choisis parmi les groupes halogéno, hydroxy, C₃-C₆ cycloalkyle et phényle (éventuellement substitué par un ou plusieurs groupes choisis parmi les groupes -CH₃, halogénométhyle, halogéno, méthoxy et halogénométhoxy) ; et
i vaut 0, 1 ou 2.

6. Composé selon l'une quelconque des revendications 1 à 5 ou sel pharmaceutiquement acceptable de celui-ci,
(i) Ar étant éventuellement substitué par un ou plusieurs groupes choisis parmi les groupes
C₁-C₄ alkyle, C₁-C₄ halogénoalkyle, C₃-C₆ cycloalkyle, halogéno, -CN, -OR^{z}, -NR^{x}R^{y}, - C(=O)NR^{x}R^{y}, -C(=S)NR^{x}R^{y}, -O(C=O)NR^{x}R^{y}, -O(C=S)NR^{x}R^{y}, -C(=O)OR^{x}, -NR^{x}C(=O)R^{y} phényle, -C(=O)R^{x}, et hétéroaryle monocyclique éventuellement substitué ;
(ii) Ar étant éventuellement substitué par un ou plusieurs groupes choisis parmi les groupes C₁-C₄ alkyle, C₁-C₄ halogénoalkyle, C₃-C₆ cycloalkyle, halogéno, -OR^{z}, -C(=O)R^{x}, et hétéroaryle monocyclique éventuellement substitué par un groupe C₁-C₄ alkyle, C₁-C₄ halogénoalkyle, halogéno ;
(iii) Ar étant éventuellement substitué par un ou plusieurs groupes choisis parmi les groupes -CH₃, -CH₂CH₃, halogénométhyle, cyclopentyle, cyclobutyle, halogéno, -OR^{z}, -C(=O)R^{x}, et un groupe hétéroaryle monocyclique de 5 ou 6 chaînons contenant un ou deux hétéroatomes choisis parmi les atomes S et N et éventuellement substitué par un groupe C₁-C₄ alkyle ; R^{x} représentant un groupe -H ou C₁-C₄ alkyle ; et R^{z} représentant un groupe C₁-C₄ alkyle éventuellement substitué ; ou
(iv) Ar étant éventuellement substitué par un ou plusieurs groupes choisis parmi les groupes -CH₃, - CH₂CH₃, -CHF₂,-CF₃, cyclopentyle, cyclobutyle, -F, -Cl, -Br, -OCH₃, - C(=O)CH₃, et un groupe thiazolyle.

7. Composé selon la revendication 1, ledit composé étant représenté par la formule développée suivante : 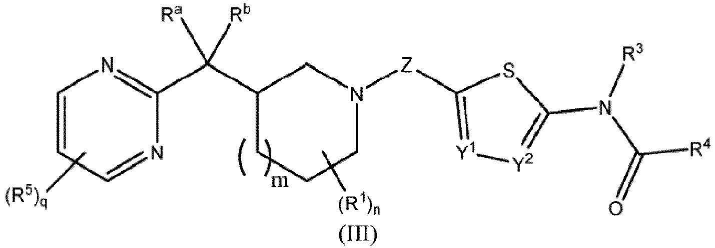 ou un sel pharmaceutiquement acceptable de celui-ci ; dans laquelle :
Y¹ et Y² représentent chacun un groupe CR^{c} ou un atome N, au moins un groupe parmi Y¹ ou Y² représentant un atome N ;
Z représente un groupe CR²R², C(=O), (CR²R²)₂, CH₂C(=O) ou C(=O)CH₂ ;
R^{a}, R^{b} et R^{c} représentent chacun indépendamment un groupe -H, halogéno, C₁-C₄ alkyle, C₁-C₄ halogénoalkyle ou C₁-C₄ alcoxy, ou R^{a} et R^{b} pris conjointement avec leur atome de carbone intermédiaire forment un groupe C₃-C₆ cycloalkyle ;
m vaut 0 ou 1 ;
n vaut 0 ou représente un entier allant de 1 à 7 ;
lorsque n est différent de 0, R¹, pour chaque occurrence, représente indépendamment un groupe halogéno, C₁-C₄ alkyle, C₁-C₄ halogénoalkyle, C₁-C₄ alcoxy ou C₁-C₄ halogénoalcoxy ;
R², pour chaque occurrence, représente indépendamment un groupe -H, halogéno, C₁-C₄ alkyle, C₁-C₄ halogénoalkyle, C₃- C₁₀ cycloalkyle ou C₃-C₁₀ halogénocycloalkyle ;
ou sinon deux groupes R² conjointement avec l'atome de carbone auquel ils sont liés forment un groupe C₃-C₁₀ cycloalkyle ;
R³ représente un groupe -H ou C₁-C₄ alkyle ; et
R⁴ représente un groupe -H, C₁-C₄ alkyle, C₁-C₄ halogénoalkyle ou C₃-C₆ cycloalkyle ;
ou sinon R³ et R⁴ pris conjointement avec leurs atomes intermédiaires forment un groupe hétérocyclyle de 5 à 7 chaînons éventuellement substitué ;
R⁵, pour chaque occurrence, est choisi parmi les groupes C₁-C₄ alkyle, C₁-C₄ halogénoalkyle, C₃-C₆ cycloalkyle, halogéno, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, -OC(=O)OR^{x}, -C(=S)OR^{y}, -O(C=S)R^{x}, -C(=O)NR^{x}R^{y}, -NR^{x}C(=O)R^{y}, -C(=S)NR^{x}R^{y}, -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, -NR^{x}(C=O)NR^{x}R^{y}, -NR^{x}(C=S)NR^{x}R^{y}, -C(=S)R^{x}, -C(=O)R^{x}, phényle et hétéroaryle monocyclique ;
lorsque R⁵ représente un groupe C₁-C₄ alkyle, le groupe C₁-C₄ alkyle étant éventuellement et indépendamment substitué par les groupes -CN, -NO₂, -OR^{z}, -NR^{x}R^{y}, -S(O)ᵢR^{x}, -NR^{x}S(O)ᵢR^{y}, - S(O)ᵢNR^{x}R^{y}, -C(=O)OR^{x}, -OC(=O)OR^{x}, -C(=S)OR^{x}, -O(C=S)R^{x}, -C(=O)NR^{x}R^{y}, -NR^{x}C(=O)R^{y}, - C(=S)NR^{x}R^{y} -NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, - NR^{x}(C=O)NR^{x}R^{y}, -NR^{x}(C=S)NR^{x}R^{y}, -C(=S)R^{x} et -C(=O)R^{y}, un groupe C₃-C₆ cycloalkyle (éventuellement substitué par un ou plusieurs groupes choisis parmi les groupes -CH₃, halogénométhyle, halogéno, méthoxy et halogénométhoxy), hétéroaryle monocyclique (éventuellement substitué par un ou plusieurs groupes choisis parmi un groupe -CH₃, halogénométhyle, halogéno, méthoxy ou halogénométhoxy) et phényle (éventuellement substitué par un ou plusieurs groupes choisis parmi les groupes -CH₃, halogénométhyle, halogéno, méthoxy et halogénométhoxy) ;
lorsque R⁵ représente un groupe C₃-C₆ cycloalkyle, phényle ou un groupe hétéroaryle monocyclique, le groupe cycloalkyle, phényle ou un groupe hétéroaryle monocyclique est éventuellement et indépendamment substitué par les groupes C₁-C₄ alkyle, C₁-C₄ halogénoalkyle, halogéno, -CN, -NO₂, -OR^{z}, -NR^{x}R^{y}, -S(O)ᵢR^{x}, -NR^{x}S(O)ᵢR^{y}, -S(O)ᵢNR^{x}R^{y}, -C(=O)OR^{x}, - OC(=O)OR^{x}, -C(=S)OR^{x}, -O(C=S)R^{y}, -C(=O)NR^{x}R^{y}, -NR^{x}C(=O)R^{y}, -C(=S)NR^{x}R^{y}, - NR^{x}C(=S)R^{y}, -NR^{x}(C=O)OR^{y}, -O(C=O)NR^{x}R^{y}, -NR^{x}(C=S)OR^{y}, -O(C=S)NR^{x}R^{y}, - NR^{x}(C=O)NR^{x}R^{y}, -NR^{x}(C=S)NR^{x}R^{y}, -C(=S)R^{x} et -C(=O)R^{x} ;
chaque groupe R^{x} et chaque groupe R^{y} représentent indépendamment un groupe -H, C₁-C₄ alkyle ou C₃-C₈ cycloalkyle ; ledit groupe C₁-C₄ alkyle ou C₃-C₈ cycloalkyle représenté par R^{x} ou R^{y} étant éventuellement substitué par un ou plusieurs substituants choisis parmi les groupes halogéno, hydroxy, C₃-C₆ cycloalkyle et phényle (éventuellement substitué par un ou plusieurs groupes choisis parmi un groupe -CH₃, halogénométhyle, halogéno, méthoxy ou halogénométhoxy) ;
R^{z} représente un groupe -H, C₁-C₄ alkyle ou C₃-C₈ cycloalkyle ; ledit groupe C₁-C₄ alkyle ou C₃-C₈ cycloalkyle représenté par R^{z} étant éventuellement substitué par un ou plusieurs substituants choisis parmi les groupes halogéno, hydroxy, C₃-C₆ cycloalkyle et phényle (éventuellement substitué par un ou plusieurs groupes choisis parmi les groupes -CH₃, halogénométhyle, halogéno, méthoxy et halogénométhoxy) ; et
i vaut 0, 1 ou 2 ; et
q vaut 0, 1, 2 ou 3.

8. Composé selon la revendication 1, ledit composé étant représenté par les formules développées suivantes :
(i) 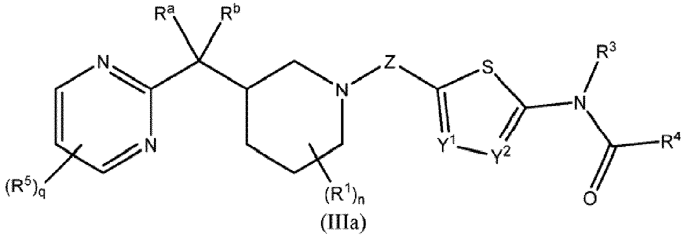 ou un sel pharmaceutiquement acceptable de celui-ci ;
(ii) 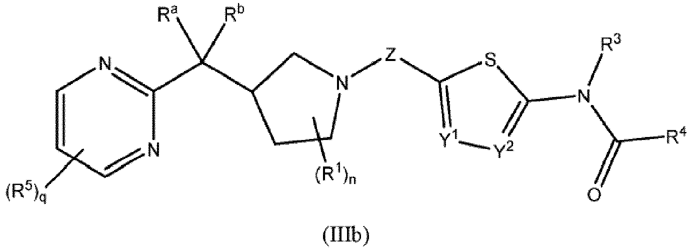 ou un sel pharmaceutiquement acceptable de celui-ci ; dans laquelle q vaut 0, 1, 2 ou 3 ;
(iii) 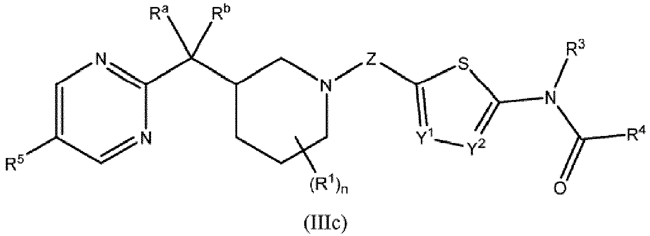 ou un sel pharmaceutiquement acceptable de celui-ci ;
(iv) 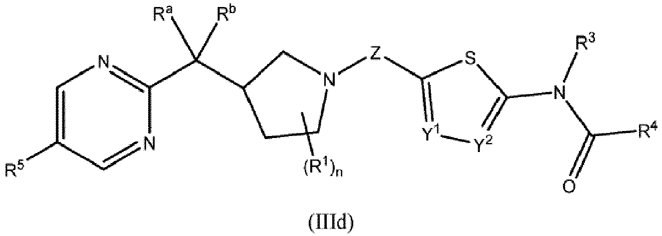 ou un sel pharmaceutiquement acceptable de celui-ci ;
(v) 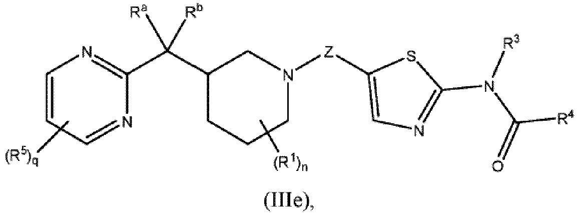 ou un sel pharmaceutiquement acceptable de celui-ci ;
(vi) 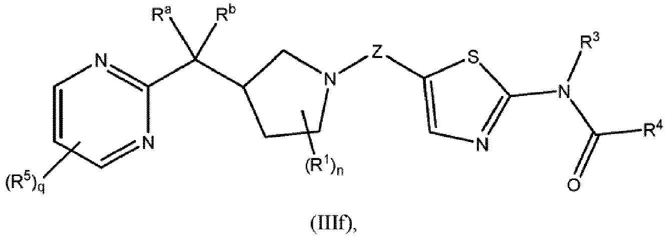 ou un sel pharmaceutiquement acceptable de celui-ci ; dans laquelle q vaut 0, 1, 2 ou 3 ;
(vii) 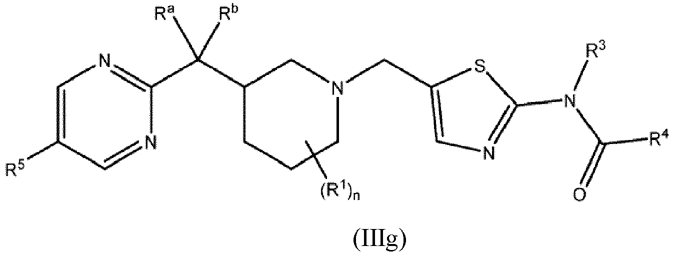 ou un sel pharmaceutiquement acceptable de celui-ci ;
(viii) 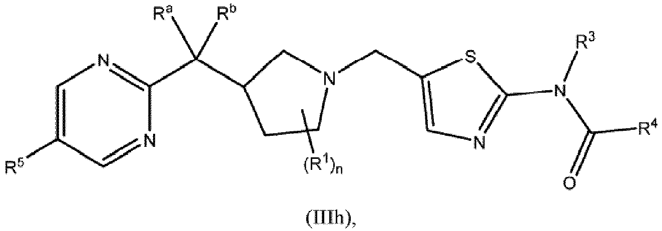 ou un sel pharmaceutiquement acceptable de celui-ci ;
(ix) 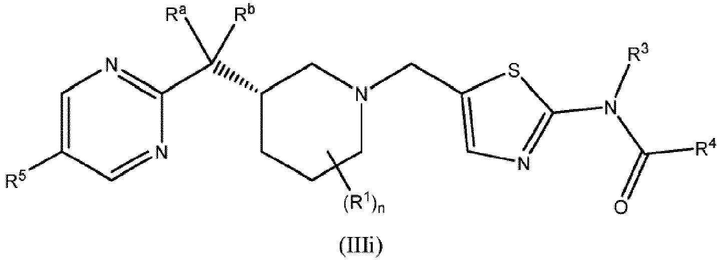 ou un sel pharmaceutiquement acceptable de celui-ci ;
(x) 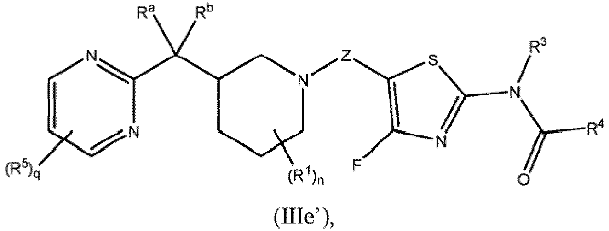 ou un sel pharmaceutiquement acceptable de celui-ci ; dans laquelle q vaut 0, 1, 2 ou 3 ;
(xi) 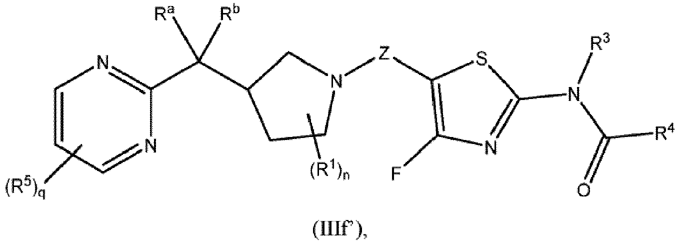 ou un sel pharmaceutiquement acceptable de celui-ci ;
(xii) 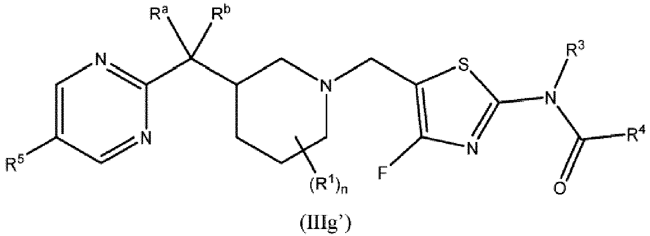 ou un sel pharmaceutiquement acceptable de celui-ci ;
(xiii) 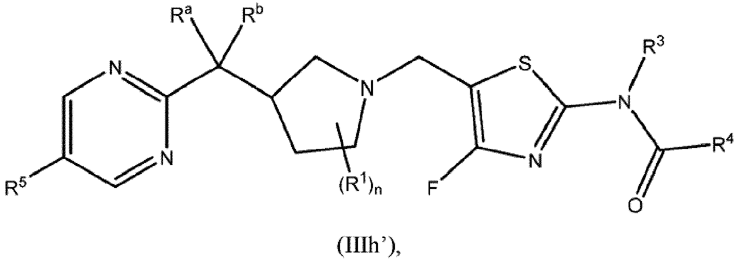 ou un sel pharmaceutiquement acceptable de celui-ci ;
(xiv) 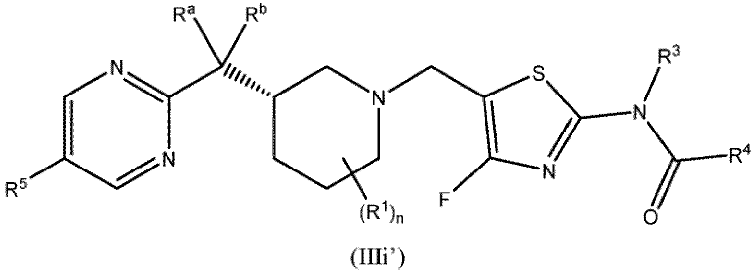 ou un sel pharmaceutiquement acceptable de celui-ci ;
(xv) 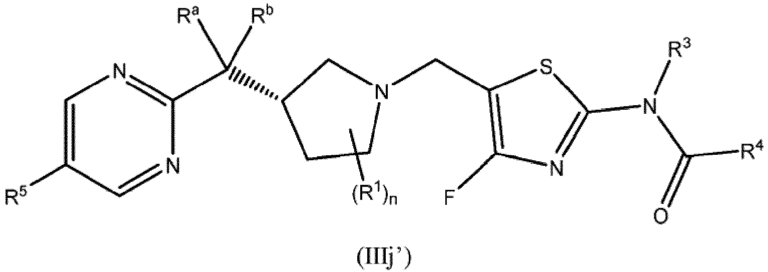 ou un sel pharmaceutiquement acceptable de celui-ci ; ou
(xvi) 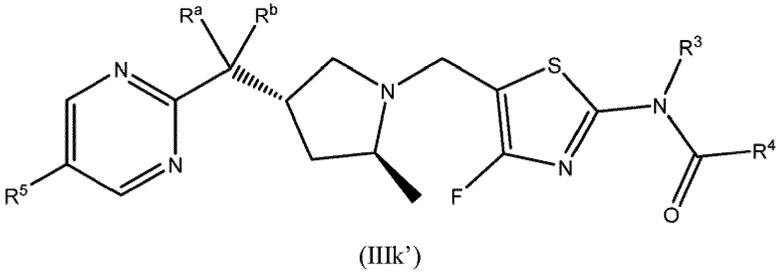 ou un sel pharmaceutiquement acceptable de celui-ci.

9. Composé selon l'une quelconque des revendications 1 à 8 ou sel pharmaceutiquement acceptable de celui-ci, R³ représentant un groupe -H.

10. Composé selon l'une quelconque des revendications 1 à 9 ou sel pharmaceutiquement acceptable de celui-ci, R⁴ représentant un groupe -CH₃.

11. Composé selon l'une quelconque des revendications 1 ou 9 à 10 ou sel pharmaceutiquement acceptable de celui-ci, R⁵ étant choisi parmi les groupes C₁-C₄ alkyle, C₁-C₄ halogénoalkyle, C₃-C₆ cycloalkyle, halogéno, -CN, -OR^{z}, -NR^{x}R^{y}, -C(=O)NR^{x}R^{y}, -C(=S)NR^{x}R^{y}, -O(C=O)NR^{x}R^{y}, -O(C=S)NR^{x}R^{y}, -C(=O)OR^{x}, -NR^{x}C(=O)R^{y} phényle, -C(=O)R^{x}, et hétéroaryle monocyclique éventuellement substitué ; éventuellement :
(i) R⁵ étant choisi parmi les groupes C₁-C₄ alkyle, C₁-C₄ halogénoalkyle, C₃-C₆ cycloalkyle, halogéno, -OR^{z}, -C(=O)R^{x}, et un groupe hétéroaryle monocyclique éventuellement substitué par un groupe C₁-C₄ alkyle, C₁-C₄ halogénoalkyle, halogéno ;
(ii) R⁵ étant choisi parmi les groupes -CH₃, -CH₂CH₃, halogénométhyle, cyclopentyle, cyclobutyle, halogéno, -OR^{z}, -C(=O)R^{x}, et un groupe hétéroaryle monocyclique de 5 ou 6 chaînons contenant un ou deux hétéroatomes choisis parmi les atomes S et N et éventuellement substitué par un groupe C₁-C₄ alkyle ; R^{x} représentant un groupe -H ou C₁-C₄ alkyle ; et R^{z} représentant un groupe C₁-C₄ alkyle éventuellement substitué ;
(iii) R⁵ étant choisi parmi les groupes -CH₃, -CH₂CH₃, -CHF₂,-CF₃, cyclopentyle, cyclobutyle, -F, -Br, Cl, -OCH₃, -C(=O)CH₃, et un groupe thiazolyle ; ou
(iv) R⁵ étant choisi parmi les groupes -F, -Br et Cl.

12. Composé selon l'une quelconque des 1 à 11 ou sel pharmaceutiquement acceptable de celui-ci, un groupe parmi R^{a} et R^{b} représentant un groupe -H et l'autre groupe étant choisi parmi les groupes -CH₃, -CF₃ et - OCH3.

13. Composé selon l'une quelconque des 1 à 12 ou sel pharmaceutiquement acceptable de celui-ci, n valant 0 ou 1.

14. Composition pharmaceutique comprenant le composé selon l'une quelconque des revendications 1 à 13 ou un sel pharmaceutiquement acceptable de celui-ci et un vecteur ou diluant pharmaceutiquement acceptable.

15. Composé selon l'une quelconque des revendications 1 à 13 ou composition pharmaceutique selon la revendication 14 pour utilisation dans le traitement d'une maladie ou d'un état choisi parmi une maladie neurodégénérative, une tauopathie, le diabète, le cancer et le stress, éventuellement :
(i) ladite maladie ou ledit état étant choisi parmi un accident ischémique cérébral aigu (AIS), la maladie d'Alzheimer, la démence, la sclérose latérale amyotrophique (SLA), la sclérose latérale amyotrophique avec déficit cognitif (ALSci), la démence à grains argyrophiles, la maladie de Bluit, la dégénérescence corticobasale (CBP), la démence pugilistique, les enchevêtrements neurofibrillaires diffus avec calcification, le syndrome de Down, l'épilepsie, la démence familiale britannique, la démence familiale danoise, la démence frontotemporale avec parkinsonisme liée au chromosome 17 (FTDP-17), la maladie de Gerstmann-Straussler-Scheinker, le parkinsonisme guadeloupéen, la maladie de Hallevorden-Spatz (neurodégénérescence avec accumulation de fer dans le cerveau de type 1), un accident vasculaire cérébral ischémique, une déficience cognitive légère (MCI), une atrophie multi-systématisée, une dystrophie myotonique, la maladie de Niemann-Pick (type C), une dégénérescence pallido-ponto-nigrale, le complexe parkinsonisme-démence de Guam, la maladie de Pick (PiD), le parkinsonisme post-encéphalitique (PEP), les maladies à prions (dont la maladie de Creutzfeldt-Jakob (MCJ), la variante de la maladie de Creutzfeldt-Jakob (vMCJ), l'insomnie familiale fatale, le kuru, une gliose supercorticale progressive, la paralysie supranucléaire progressive (PSP), le syndrome de Steele-Richardson-Olszewski, la panencéphalite sclérosante subaiguë, la démence à enchevêtrement seul, la maladie de Huntington et la maladie de Parkinson ;
(ii) ladite maladie ou ledit état étant choisi parmi un accident vasculaire cérébral ischémique aigu (AIS), la maladie d'Alzheimer, la démence, la sclérose latérale amyotrophique (SLA), la sclérose latérale amyotrophique avec déficience cognitive (ALSci), la démence à grains argyrophiles, l'épilepsie, un accident vasculaire cérébral ischémique, une déficience cognitive légère (MCI), la maladie de Huntington et la maladie de Parkinson ; ou
(iii) ladite maladie ou ledit état étant la maladie d'Alzheimer.
